# EUROPEAN PATENT APPLICATION

(11) **EP 4 206 325 A1**
(43) Date of publication of application: **05.07.2023**
(21) Application number: 21861646.4
(22) Date of filing: 25.08.2021
(51) Int. Cl.: C12N 15/113, A61K 31/712, A61K 31/7125, A61K 48/00, A61P 11/00, A61P 35/00

(54) **NOVEL NUCLEIC ACID MOLECULE INHIBITING EXPRESSION OF TARGET GENE**

(30) Priority: 25.08.2020 JP 2020142170
(71) Applicant: BONAC CORPORATION, Kurume-shi, Fukuoka 839-0861 (JP)
(72) Inventor: SHIBATA, Atsushi, Kurume-shi, Fukuoka 839-0861 (JP); OHGI, Tadaaki, Kurume-shi, Fukuoka 839-0861 (JP); ABE, Tomoaki, Kurume-shi, Fukuoka 839-0861 (JP); EMURA, Chisato, Kurume-shi, Fukuoka 839-0861 (JP); AOKI, Eriko, Kurume-shi, Fukuoka 839-0861 (JP); SHIROHZU, Hisao, Kurume-shi, Fukuoka 839-0861 (JP); IWAKAMI, Yusuke, Kurume-shi, Fukuoka 839-0861 (JP); ACOSTA RAMIREZ, Melisa, Kurume-shi, Fukuoka 839-0861 (JP); NAITO, Kota, Kurume-shi, Fukuoka 839-0861 (JP)
(74) Representative: J A Kemp LLP
(86) International application number: PCT/JP2021/031252
(87) International publication number: WO 2022/045224

(57) **Abstract**

The present invention aims to provide a new nucleic acid molecule for suppressing expression of the target gene, which (1) has a gene expression suppressing activity equivalent to or higher than that of siRNA, (2) shows no off-target effect of the sense strand, and (3) makes it possible to design a wider range of antisense strand sequences (extends the range of targetable sequences). Since the nucleic acid molecule of the following formula: 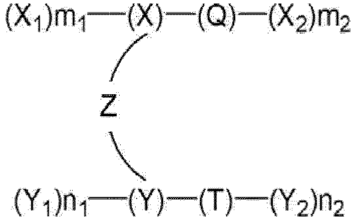 wherein each symbol is as defined in the DESCRIPTION, has the superior properties of the above-mentioned (1) to (3), it is extremely useful as a novel gene expression inhibitor that replaces conventional siRNA.

## Description

### [Technical Field]

The present invention relates to a novel nucleic acid molecule that suppresses expression of the target gene, a composition containing the nucleic acid molecule, and a method for suppressing expression of the target gene by using the nucleic acid molecule.

### [Background Art]

As a technique for inhibiting gene expression, for example, RNA interference (RNAi) is known. RNAi is a method for suppressing gene expression in order to analyze the functions of genes and proteins in a wide range of cells, and is a very powerful tool for studying molecular biology and cellular biology. Inhibition of gene expression by RNA interference is generally carried out, for example, by administering a short double-stranded RNA molecule consisting of a sense strand and an antisense strand to a cell or the like. The aforementioned double-stranded RNA molecule is generally called small interfering RNA (siRNA), and is generally a 21-25nt double-stranded RNA (dsRNA) with dinucleotide 3' overhang.

In RNAi, siRNA is incorporated into the RNA-induced silencing complex (RISC) and functions as a guide molecule. siRNA does not work alone, and exhibits its function only when it is incorporated into Argonaute family protein (AGO), which is the central protein of RISC. siRNA in RISC functions as a guide for recognizing target mRNA, and RISC constituent factors such as AGO act, whereby translational suppression and degradation of target mRNA occur.

RNAi is a method widely used in this technical field, but strand bias is a problem in carrying out RNAi. Strand bias is a phenomenon in which one of the two strands constituting the siRNA is selectively used due to the thermodynamic stability bias at both ends of the siRNA. If siRNA is a symmetrical molecule free of bias in thermodynamic stability, both the sense strand and the antisense strand should be equally incorporated into RISC. In practice, however, due to the bias described above, one of the strands is incorporated more than the other strand and utilized in the RNAi pathway.

In siRNA, an AU-rich region has loose base-pairing, and a GC-rich region has strong base-pairing. When base pairing near the 5'-end of siRNA is loose, it binds to the MID domain of AGO protein and functions as a guide strand. When the base pairing is strong, it cannot bind to the MID domain and cannot function as siRNA. Thus, the base-pairing strength near the 5'-end affects the frequency (probability) of binding to the MID domain. When the pairing strength is different between the 5'-ends of the sense strand and the antisense strand, the frequency of incorporation into RISC also becomes different and is observed as strand bias. A strand bias towards the antisense strand affords a strong gene expression suppressing effect, and a bias towards the sense strand affords a weak gene expression suppressing effect. Therefore, all existing siRNA design algorithms are programmed, taking strand bias into account, to select sequences that are loosely paired on the 5' side of the antisense strand and tightly paired on the 3' side.

When gene expression is suppressed by RNAi using siRNA, etc., a phenomenon is known in which a non-specific gene expression suppressing effect appears. This is called an off-target effect. Such off-target effect causes unexpected suppression of gene expression, thus posing a big problem from the aspect of the safety of RNAi. In siRNA, strand bias towards the sense strand also increases the off-target effect by the sense strand, which is not preferred.

SomaGenics has developed a synthetic short hairpin RNA (sshRNA) with reduced off-target effect by sense strand, by ligating the 3'-end of an antisense strand and the 5'-end of a sense strand via a loop consisting of short nucleotides (e.g., dTdT, dUdU) containing nucleotides with a modified 2'-position (Patent Literatures 1, 2, and 3).

However, there is still a need for the development of a new nucleic acid molecule for suppressing expression of a target gene, wherein the molecule (1) has a gene expression suppressing activity equivalent to or higher than that of siRNA, (2) has no off-target effect due to the sense strand, and (3) is not bound by the above-mentioned strand bias rule and allows for the design of a wider range of antisense strand sequences (extends the range of targetable sequences).

### [Citation List]

### [Patent Literature]

[PTL 1]
   US Patent No. 9,816,091
[PTL 2]
   US Patent No. 8,871,730
[PTL 3]
   Japanese Translation of PCT Application Publication No. 2012-505657

### [Summary of Invention]

### [Technical Problem]

The problem of the present invention is to provide a novel nucleic acid molecule having a target gene expression suppressive activity combined with advantageous properties of the above-mentioned (1) to (3).

### [Solution to Problem]

The present inventors have developed a single-stranded nucleic acid molecule in which an antisense strand and a sense strand are linked via a non-nucleotide linker near one end of a double-stranded nucleic acid molecule having gene expression-suppressing activity. During the process, they have found that a single-stranded nucleic acid molecule obtained by linking the antisense strand and the sense strand by cross-linking between the 2' and 2' sugar moieties of the nucleosides has a target gene expression-suppressing activity equivalent to that of siRNA, and also reduces the off-target effect due to the sense strand.

The present inventors synthesized nucleic acid molecules in which various gene expression-suppressing sequences (antisense strand sequences) and their complementary strand sequences (sense strand sequences) are linked using an alkyl chain having an amide bond inside as a linker, and compared gene expression-suppressing activity with that of siRNA consisting of the same antisense strand and sense strand sequences. As a result, the nucleic acid molecule also exhibited an activity equal to or higher than that of the corresponding siRNA. Furthermore, the off-target effect by the sense strand sequence (expression suppressing activity of a nucleic acid having a sequence complementary to the sense strand sequence) was compared between the nucleic acid molecule and siRNA. As a result, siRNA showed a strong expression suppressing effect by sense strand but the gene expression-suppressing activity by the sense strand sequence was remarkably reduced in the nucleic acid molecule. Thus, it was demonstrated that the off-target effect by the sense strand could be markedly attenuated.

The present inventors have conducted further studies based on these findings and completed the present invention.

Accordingly, the present invention provides the following.
[1] A nucleic acid molecule represented by the following formula: 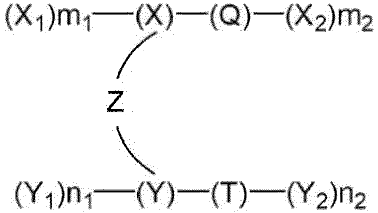
   wherein X, Y, X₁, Y₁, X₂, and Y₂ are each independently an optionally modified ribonucleotide residue or an optionally modified deoxyribonucleotide residue,
   T and Q are a sequence consisting of 14 to 30 consecutive, optionally-modified ribonucleotide residues and complementary to the target nucleic acid sequence, and a ribonucleotide sequence complementary thereto (one of which is a sequence complementary to the target nucleic acid sequence, and the other is a sequence complementary thereto),
   Z is a linker connecting the 2'-position of the sugar moiety of (X) and the 2'-position of the sugar moiety of (Y);
   m₁ and m₂ are each independently an integer of 0 to 5; and n₁ and n₂ are each independently an integer of 0 to 5.
[2] The nucleic acid molecule of [1], wherein the linker Z is a non-nucleotide structure having an alkyl chain with an amide bond therein.
[3] The nucleic acid molecule of [1] or [2], wherein the following structure 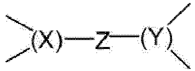
   containing the linker Z is 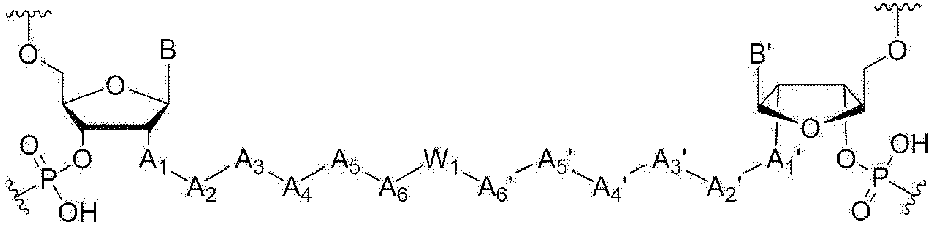
   wherein B and B' are each independently an atomic group having a nucleic acid base backbone,
   A₁ and A₁' are each independently -O-, -NR^{1a}-, -S- or -CR^{1a}R^{1b}-(wherein R^{1a} and R^{1b} are each independently a hydrogen atom or an alkyl group having 1 - 10 carbon atoms),
   A₂ and A₂' are each independently -CR^{2a}R^{2b}-, -CO-, an alkynyl group, an alkenyl group, or a single bond (wherein R^{2a} and R^{2b} are each independently a hydrogen atom or an alkyl group having 1 - 10 carbon atoms),
   A₃ and A₃' are each independently -O- or -NR^{3a}-, -S-, -CR^{3a}R^{3b}- or a single bond (wherein R^{3a} and R^{3b} are each independently a hydrogen atom or an alkyl group having 1 - 10 carbon atoms),
   A₄ and A₄' are each independently - (CR^{4a}R^{4b})n-, - (CR^{4a}R^{4b})n-ring D- (wherein ring D is an aryl group having 6 - 10 carbon atoms, a heteroaryl group having 2 - 10 carbon atoms, a cycloalkyl group having 4 - 10 carbon atoms, or a heterocycloalkyl group having 4 - 10 carbon atoms, R^{4a} and R^{4b} are each independently a hydrogen atom or an alkyl group having 1 - 10 carbon atoms) or a single bond,
   A₅ and A₅' are each independently -NR^{5a}- or a single bond (wherein R^{5a} is a hydrogen atom or an alkyl group having 1 - 10 carbon atoms),
   A₆ and A₆' are each independently -(CR^{6a}R^{6b})n- or a single bond (wherein R^{6a} and R^{6b} are each independently a hydrogen atom or an alkyl group having 1 - 10 carbon atoms), and
   W₁ is -(CR¹R²)n-, -CO-, -(CR¹R²) n-COO-(CR¹R²)n-COO-(CR¹R²)n, - (CR¹R²)n-O-(CR¹R²CR¹R²O)n-CH₂-, -(CR¹R²)n- ring D-(CR¹R²)n-, or - (CR⁴R²)n-SS-(CR⁴R²)n- (wherein ring D is an aryl group having 6 - 10 carbon atoms, a heteroaryl group having 2 - 10 carbon atoms, a cycloalkyl group having 4 - 10 carbon atoms, or a heterocycloalkyl group having 4 - 10 carbon atoms; R¹ and R², R^{1a} and R^{2a}, and R^{1b} and R^{2b} are each independently a hydrogen atom or an alkyl group having 1 - 10 carbon atoms).
[4] The nucleic acid molecule of [1] or [2], wherein the following structure 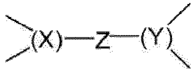
   containing the linker Z is 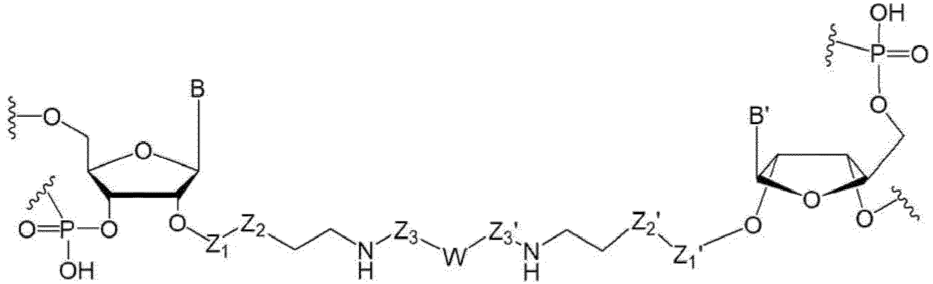
   wherein B and B' are each independently an atomic group having a nucleic acid base backbone,
   Z₁ and Z₁' are each independently -CH₂- or -CO-,
   Z₂ and Z₂' are each independently -O- or -NH-,
   Z₃ and Z₃' are each -CO-, -CH₂-, or a single bond (absent), and W is -CR¹⁰R²⁰- or -CR^{10a}R^{20a}-N(R³⁰) -CR^{10b}R^{20b}- (wherein R¹⁰ and R²⁰,
   R^{10a} and R^{20a}, and R^{10b} and R^{20b} are each independently a hydrogen atom or an alkyl group having 1 - 10 carbon atoms, R³⁰ is -COR⁴⁰-, R⁴⁰ is a hydrogen atom, an optionally substituted alkyl group having 1 - 20 carbon atoms, or an aryl group having 6 - 10 carbon atoms).
[5] The nucleic acid molecule of any of [1] to [4], comprising at least one modified nucleotide.
[6] The nucleic acid molecule of any of [1] to [5], wherein the aforementioned modified nucleotide is selected from the group consisting of 2'-O-methyl-modified nucleotide, nucleotide containing a 5'-phosphorothioate group, deoxy-nucleotide, 3'-terminal deoxy-thymine (dT) nucleotide, 2'-O-methyl-modified nucleotide, 2'-fluoro-modified nucleotide, 2'-deoxy-modified nucleotide, terminal nucleotide bound with a cholesteryl derivative or a dodecanoic acid bisdecylamide group, 2'-deoxy-2'-fluoro-modified nucleotide, fixed nucleotide, non-fixed nucleotide, conformationally restricted nucleotide, constrained ethyl nucleotide, abasic nucleotide, 2'-amino-modified nucleotide, 2'-O-allyl-modified nucleotide, 2'-C-alkyl-modified nucleotide, 2'-hydroxyl-modified nucleotide, 2'-methoxyethyl-modified nucleotide, 2'-O-alkyl-modified nucleotide, morpholino nucleotide, phosphoramidate, nucleotide containing non-natural base, tetrahydropyran-modified nucleotide, 1,5-anhydrohexitol-modified nucleotide, cyclohexenyl-modified nucleotide, nucleotide containing a phosphorothioate group, nucleotide containing a methylphosphonate group, nucleotide containing 5'-phosphate, and nucleotide containing a 5'-phosphate mimic.
[7] A medicament comprising the nucleic acid molecule of any of [1] to [6] .
[8] A target gene expression inhibitor comprising the nucleic acid molecule of any of [1] to [6], wherein the target gene comprises the aforementioned target nucleic acid sequence.
[9] A therapeutic agent for cancer or fibrosis comprising the nucleic acid molecule of [8].
[10] A method for suppressing expression of a target gene, comprising contacting an effective amount of the nucleic acid molecule of any of [1] to [6] with the target gene.
[11] A method for treating cancer or fibrosis, comprising contacting an effective amount of the nucleic acid molecule of any of [1] to [6] with a target gene.
[12] The method of [10] or [11], comprising a step of administering the aforementioned nucleic acid molecule to a cell, a tissue, or an organ.
[13] The method of any of [10] to [12], wherein the aforementioned nucleic acid molecule is administered in vivo or in vitro.
[14] The method of any of [10] to [13], wherein the aforementioned nucleic acid molecule is administered to a non-human animal.
[15] An amidite compound having the following structure 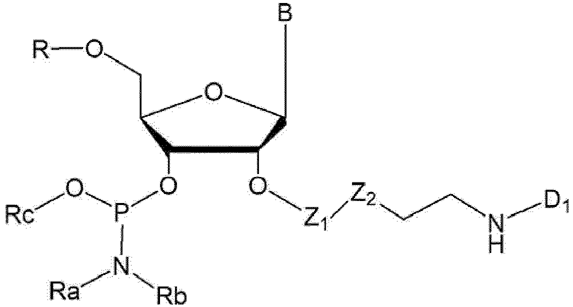
   wherein B is an atomic group having a nucleic acid base backbone,
   Z₁ is -CH₂- or -CO-,
   Z₂ is -O- or -NH-,
   R is a hydroxyl-protecting group,
   Rₐ and R_{b} are the same or different and each a hydrogen atom or a substituent; R_{c} is a hydrogen atom, an electron-withdrawing group, or a substituent optionally substituted by an electron-withdrawing group, and
   D₁ is an amino-protecting group.
[16] The amidite compound of [15], wherein Z₁ is -CH₂- and Z₂ is -O-.
[17] The amidite compound of [15], wherein Z₁ is -CO- and Z₂ is -NH-.

### [Advantageous Effects of Invention]

According to the nucleic acid molecule of the present invention, a gene expression suppressing effect by the antisense strand can be obtained with efficiency equivalent to or higher than that of siRNA molecules. The nucleic acid molecule of the present invention minimizes the off-target effect by the sense strand and has higher safety than siRNA molecules. Furthermore, the crosslinked nucleic acid molecule of the present invention is not bound by the strand bias rule.

### [Brief Description of Drawings]

[Fig. 1]

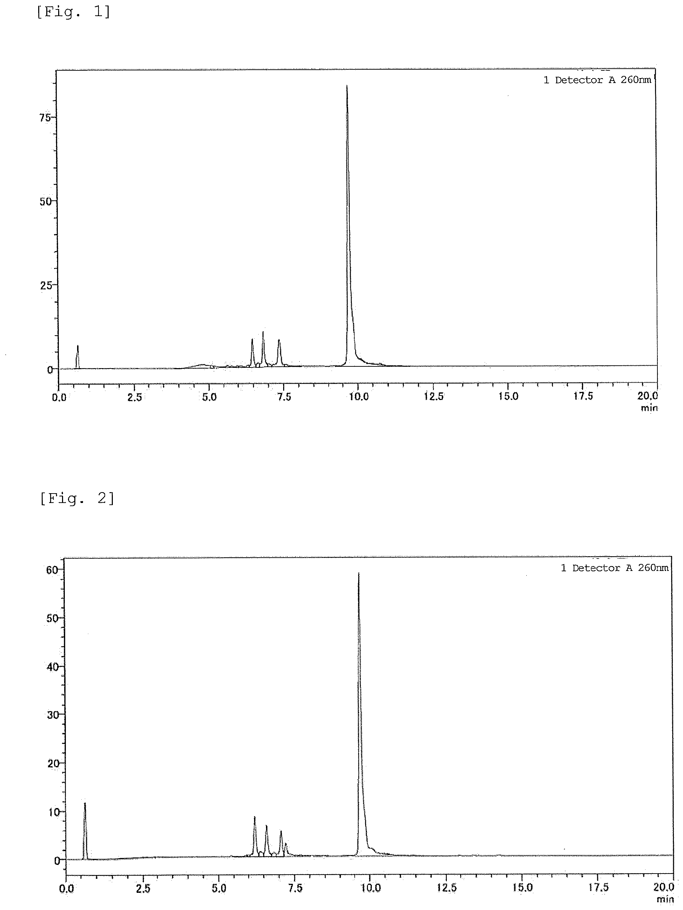

   Fig. 1 is an RP-HPLC chart of BBN-1 before purification.
[Fig. 2]
   Fig. 2 is an RP-HPLC chart of BBN-2 before purification.
[Fig. 3]

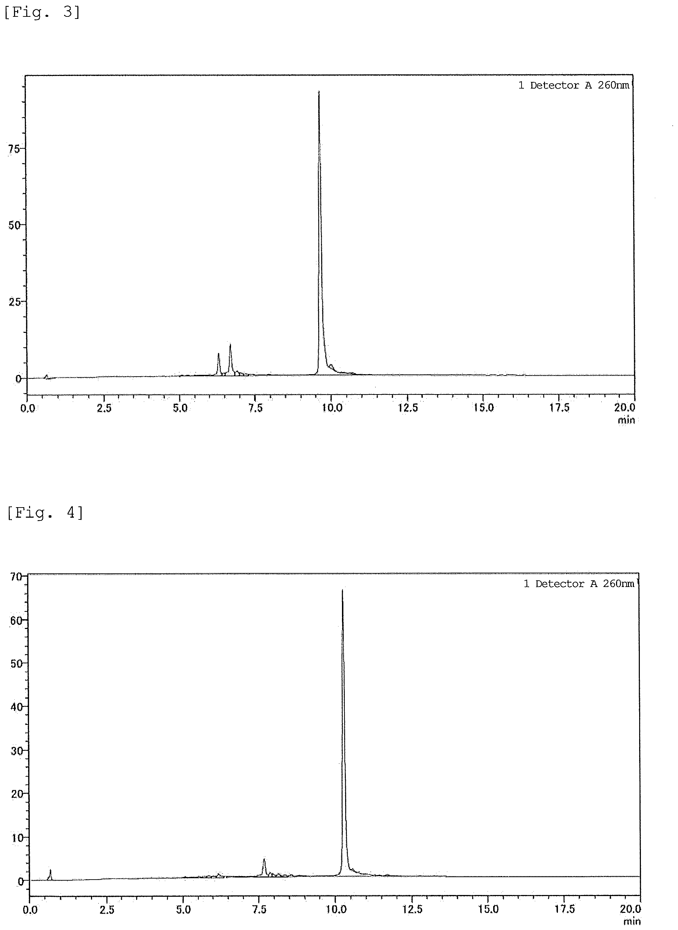

   Fig. 3 is an RP-HPLC chart of BBN-3 before purification.
[Fig. 4]
   Fig. 4 is an RP-HPLC chart of BBN-4 before purification.
[Fig. 5]

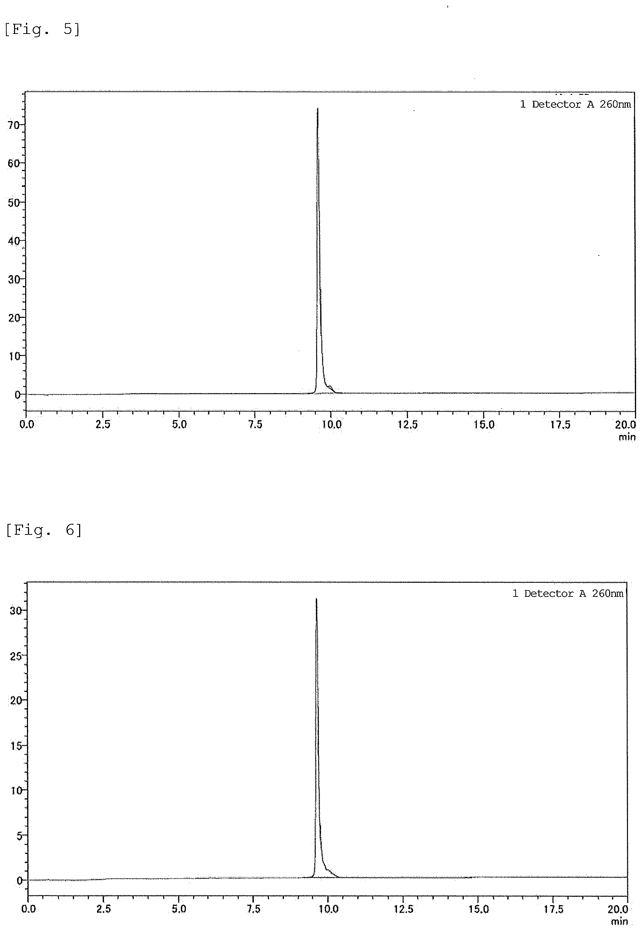

   Fig. 5 is an RP-HPLC chart of BBN-5 after purification.
[Fig. 6]
   Fig. 6 is an RP-HPLC chart of BBN-6 after purification.
[Fig. 7]

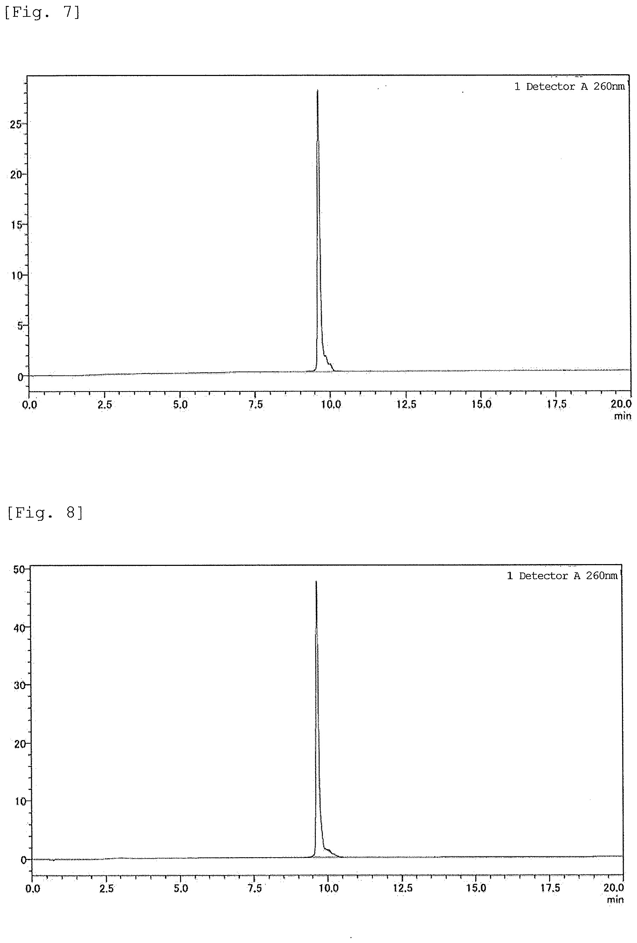

   Fig. 7 is an RP-HPLC chart of BBN-7 after purification.
[Fig. 8]
   Fig. 8 is an RP-HPLC chart of BBN-8 after purification.
[Fig. 9]

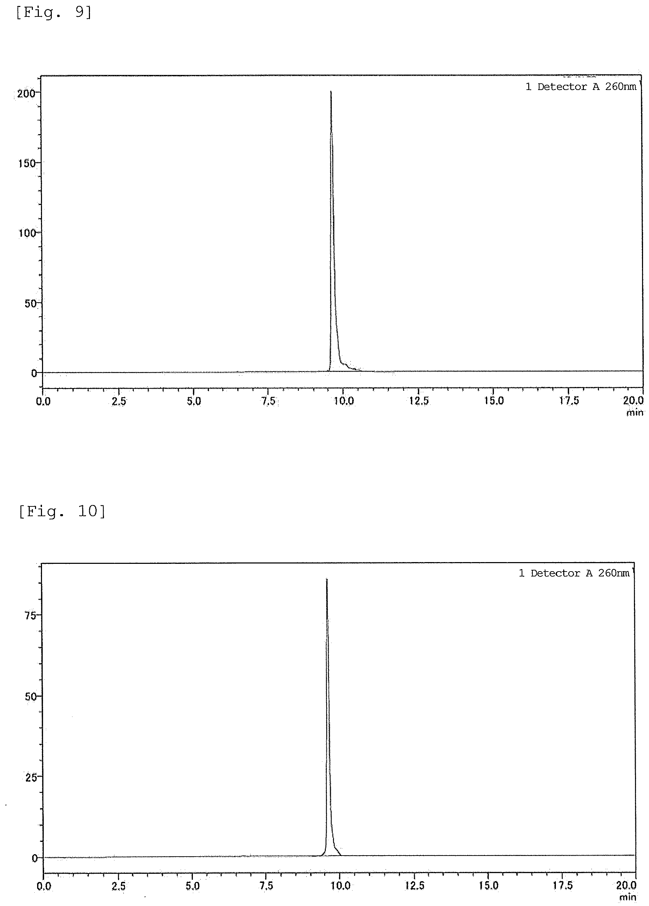

   Fig. 9 is an RP-HPLC chart of BBN-9 after purification.
[Fig. 10]
   Fig. 10 is an RP-HPLC chart of BBN-10 after purification.
[Fig. 11]

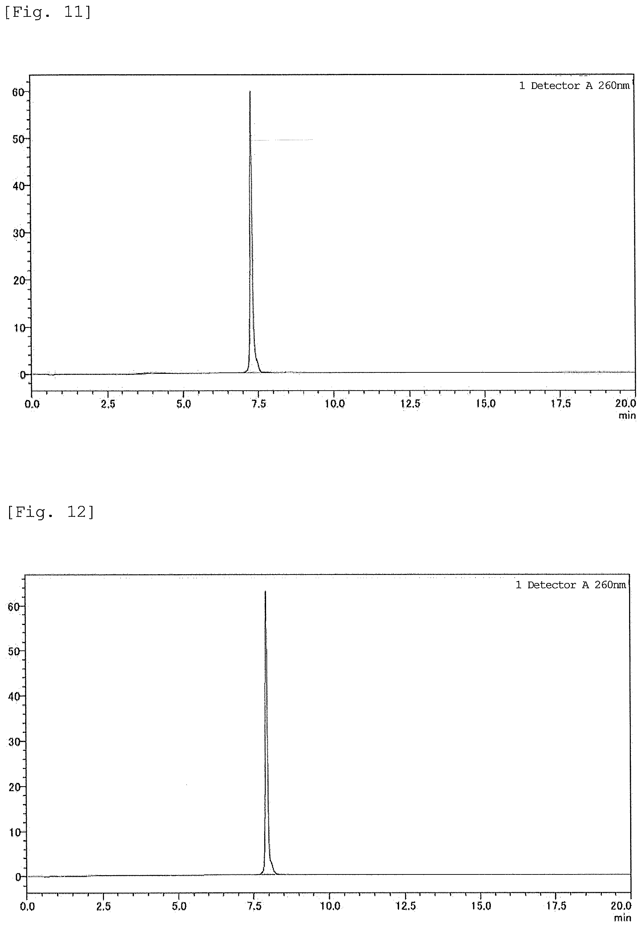

   Fig. 11 is an RP-HPLC chart of BBN-11 after purification.
[Fig. 12]
   Fig. 12 is an RP-HPLC chart of BBN-12 after purification.
[Fig. 13]

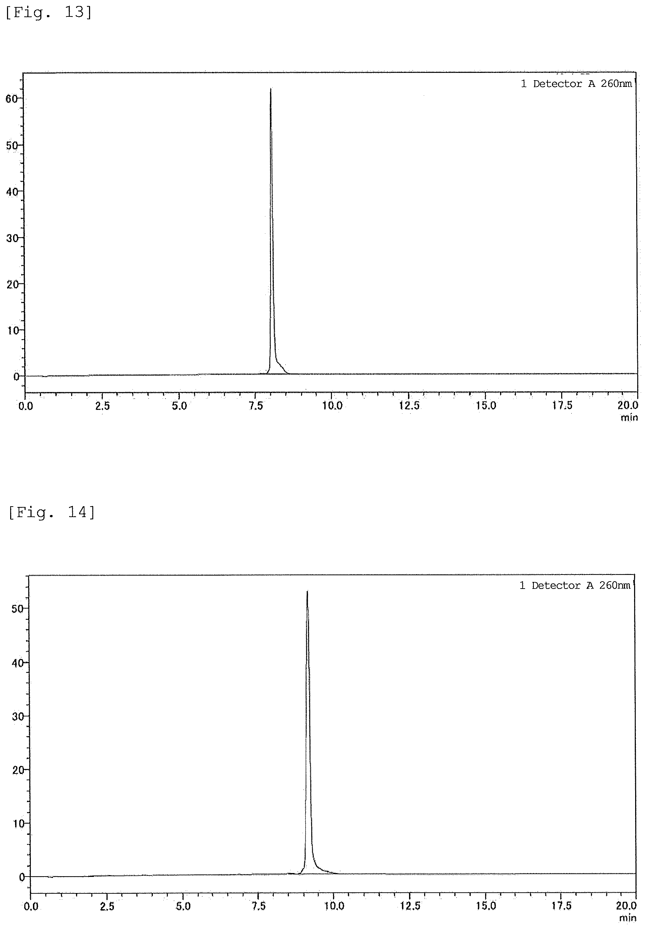

   Fig. 13 is an RP-HPLC chart of BBN-13 after purification.
[Fig. 14]
   Fig. 14 is an RP-HPLC chart of BBN-14 after purification.
[Fig. 15]

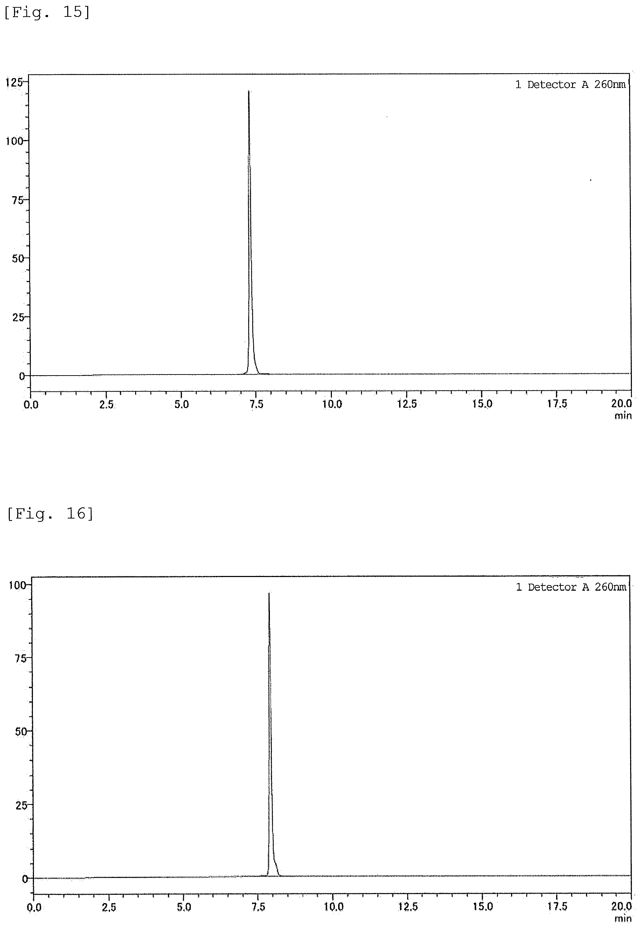

   Fig. 15 is an RP-HPLC chart of BBN-17 after purification.
[Fig. 16]
   Fig. 16 is an RP-HPLC chart of BBN-18 after purification.
[Fig. 17]

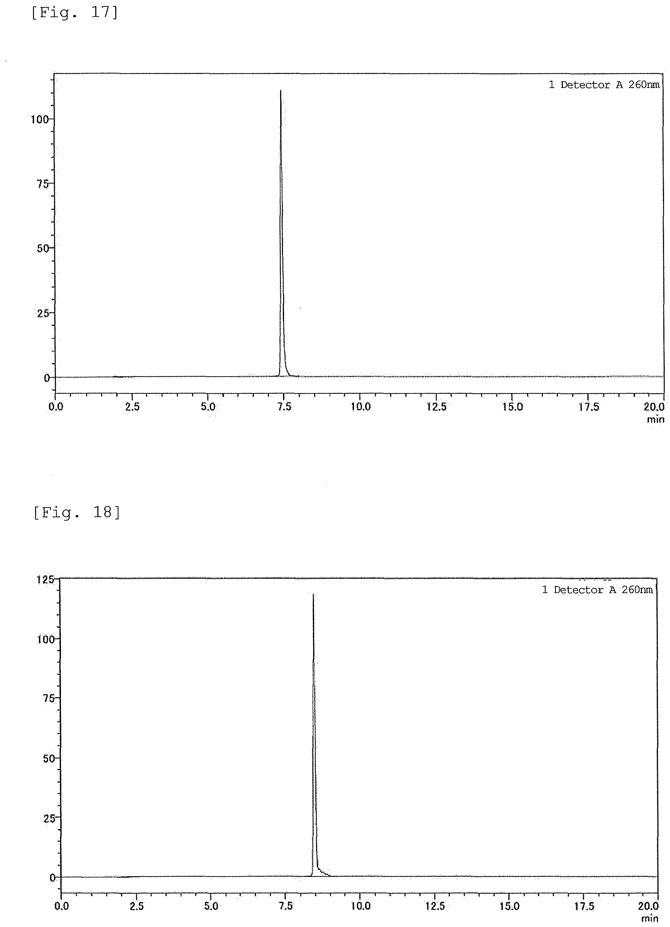

   Fig. 17 is an RP-HPLC chart of BBN-19 after purification.
[Fig. 18]
   Fig. 18 is an RP-HPLC chart of BBN-20 after purification.
[Fig. 19]

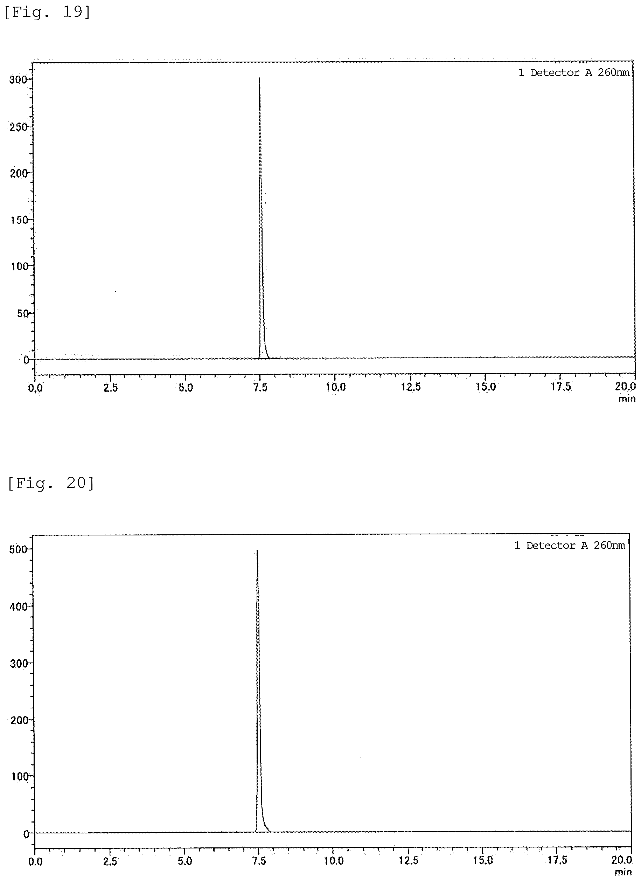

   Fig. 19 is an RP-HPLC chart of BBN-21 after purification.
[Fig. 20]
   Fig. 20 is an RP-HPLC chart of BBN-22 after purification.
[Fig. 21-1]

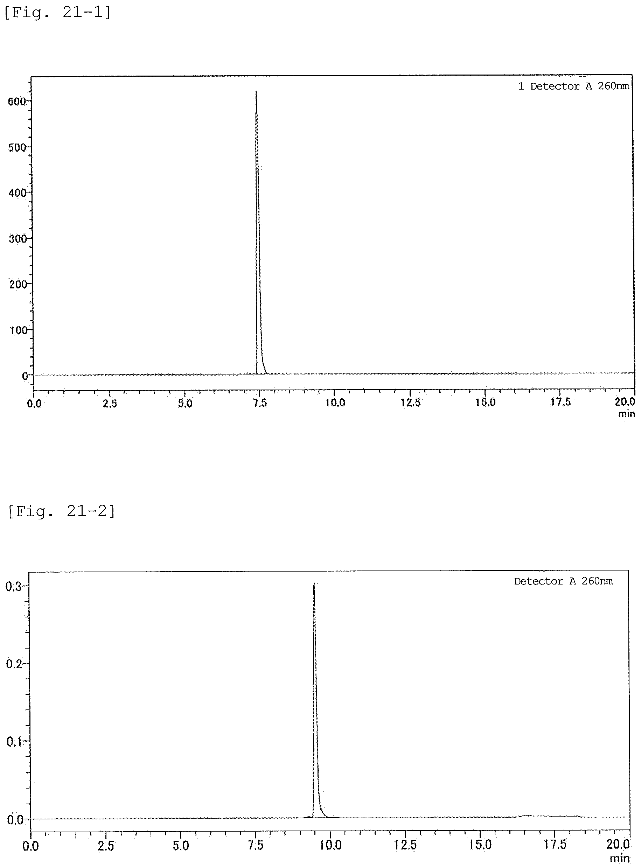

   Fig. 21-1 is an RP-HPLC chart of BBN-23 after purification.
[Fig. 21-2]
   Fig. 21-2 is an RP-HPLC chart of ESB2.2_HPRT1-3 after purification.
[Fig. 22]

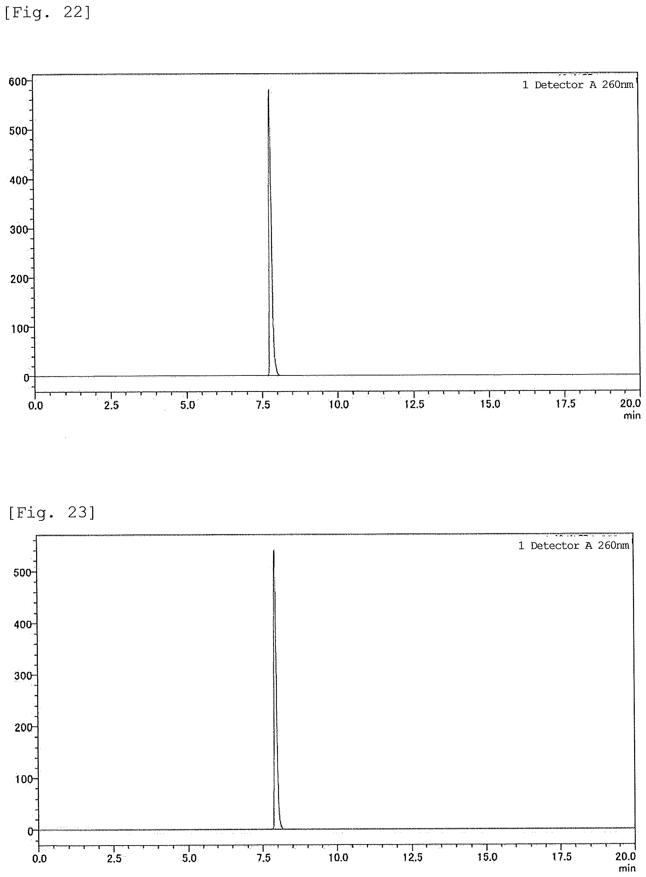

   Fig. 22 is an RP-HPLC chart of BBN-24 after purification.
[Fig. 23]
   Fig. 23 is an RP-HPLC chart of BBN-25 after purification.
[Fig. 24]

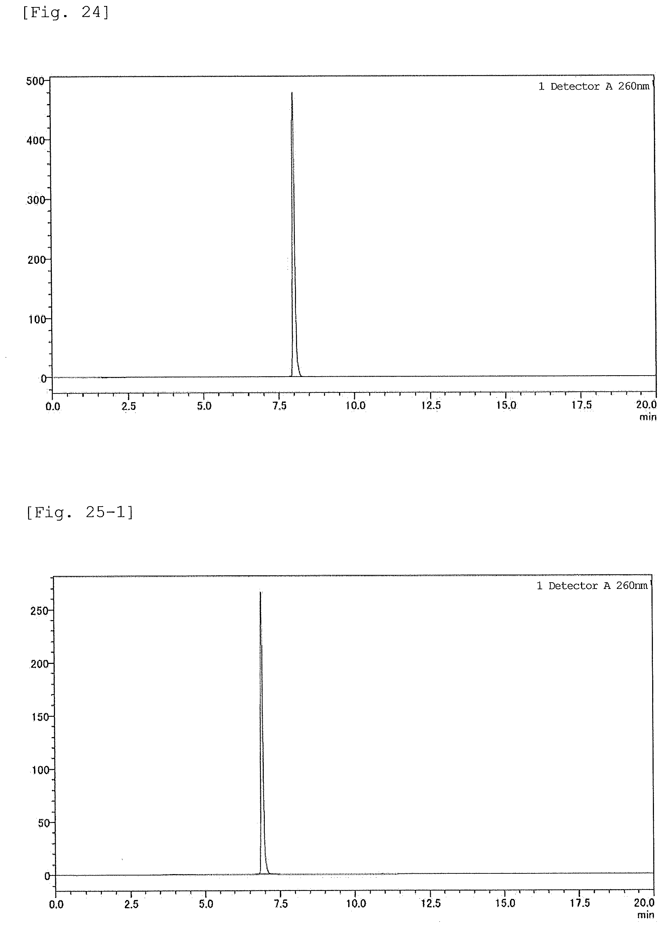

   Fig. 24 is an RP-HPLC chart of BBN-26 after purification.
[Fig. 25-1]
   Fig. 25-1 is an RP-HPLC chart of BBN-27 after purification.
[Fig. 25-2]

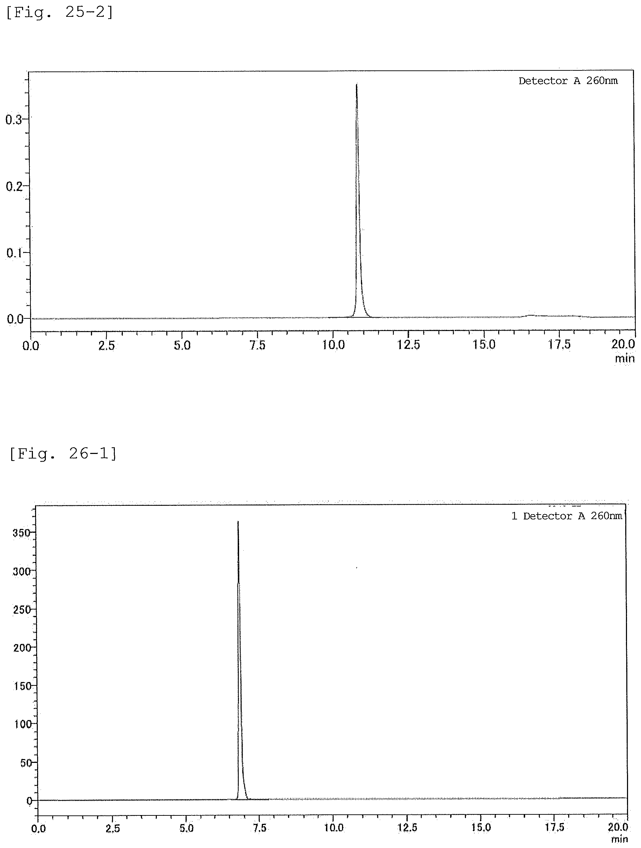

   Fig. 25-2 is an RP-HPLC chart of ESB2.2-20-2 after purification.
[Fig. 26-1]
   Fig. 26-1 is an RP-HPLC chart of BBN-28 after purification.
[Fig. 26-2]

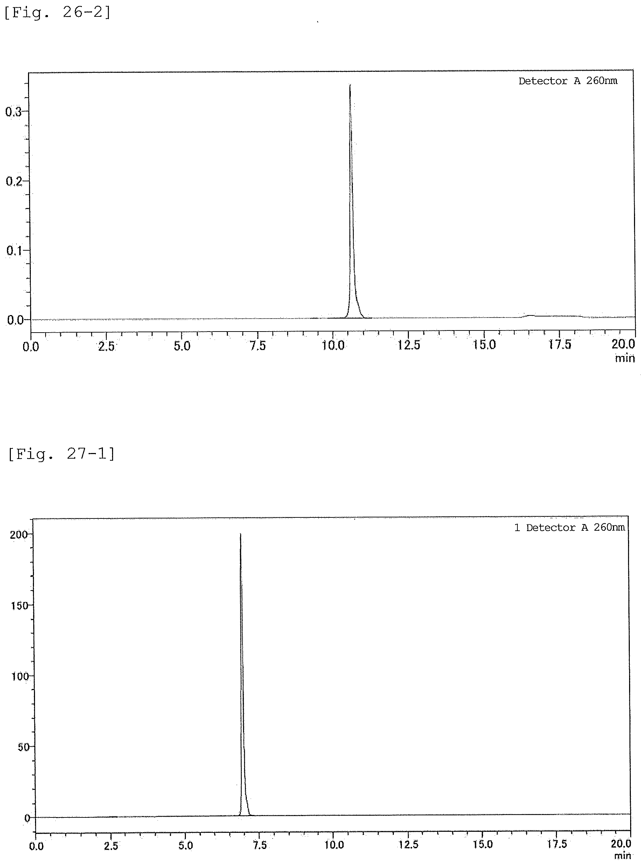

   Fig. 26-2 is an RP-HPLC chart of ESB2.2-20-3 after purification.
[Fig. 27-1]
   Fig. 27-1 is an RP-HPLC chart of BBN-29 after purification.
[Fig. 27-2]

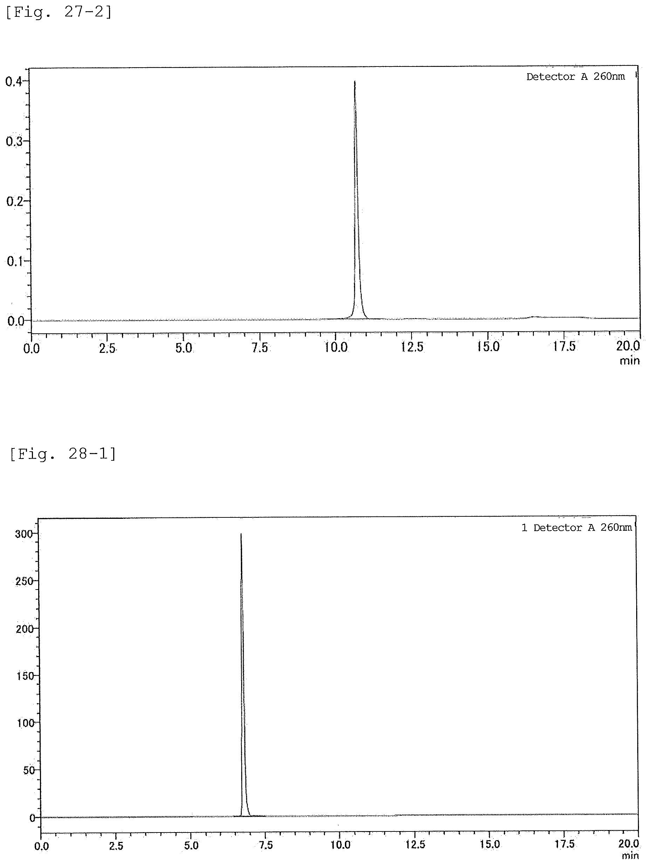

   Fig. 27-2 is an RP-HPLC chart of ESB2.2-20-4 after purification.
[Fig. 28-1]
   Fig. 28-1 is an RP-HPLC chart of BBN-30 after purification.
[Fig. 28-2]

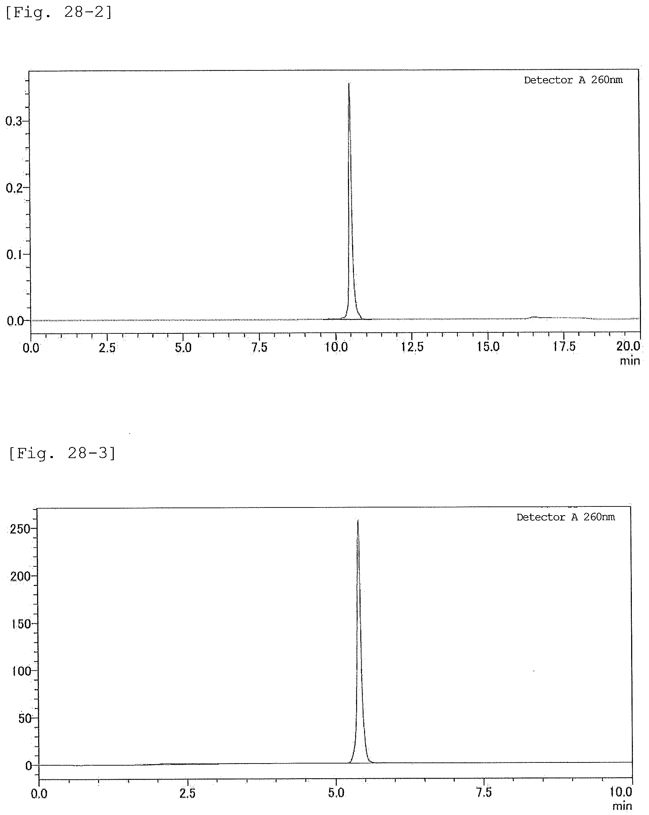

   Fig. 28-2 is an RP-HPLC chart of ESB2.2-20-5 after purification.
[Fig. 28-3]
   Fig. 28-3 is an RP-HPLC chart of ESB_HPRT1 after purification.
[Fig. 28-4]

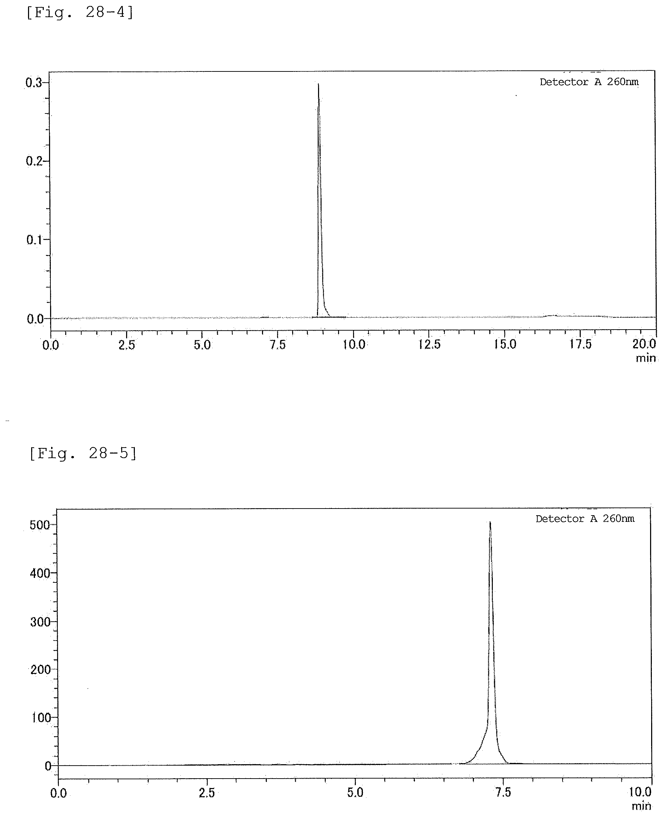

   Fig. 28-4 is an RP-HPLC chart of ESB2.2_HPRT1-4 after purification.
[Fig. 28-5]
   Fig. 28-5 is an RP-HPLC chart of 6b1 after purification.
[Fig. 29]

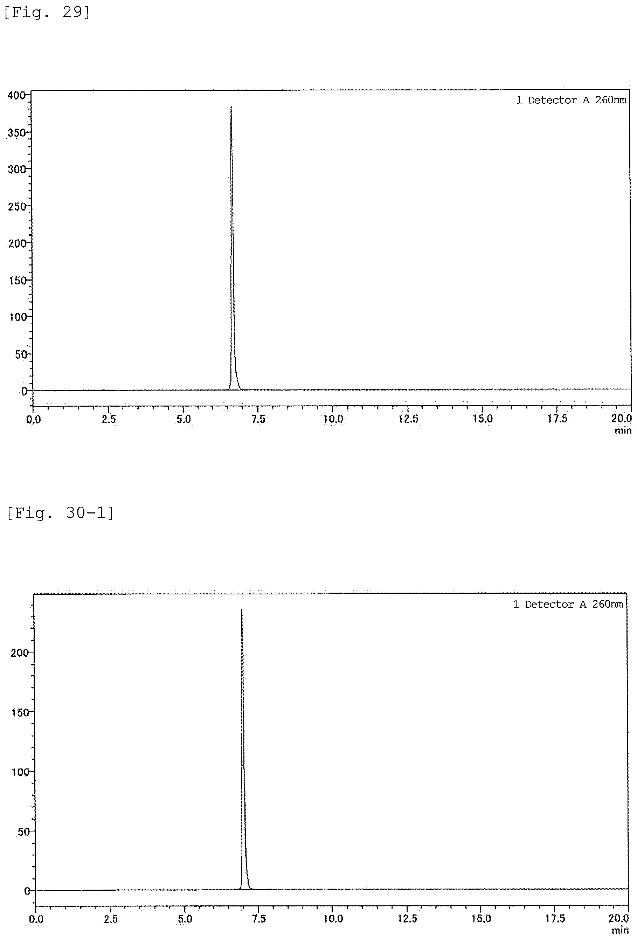

   Fig. 29 is an RP-HPLC chart of BBN-31 after purification.
[Fig. 30-1]
   Fig. 30-1 is an RP-HPLC chart of BBN-32 after purification.
[Fig. 30-2]

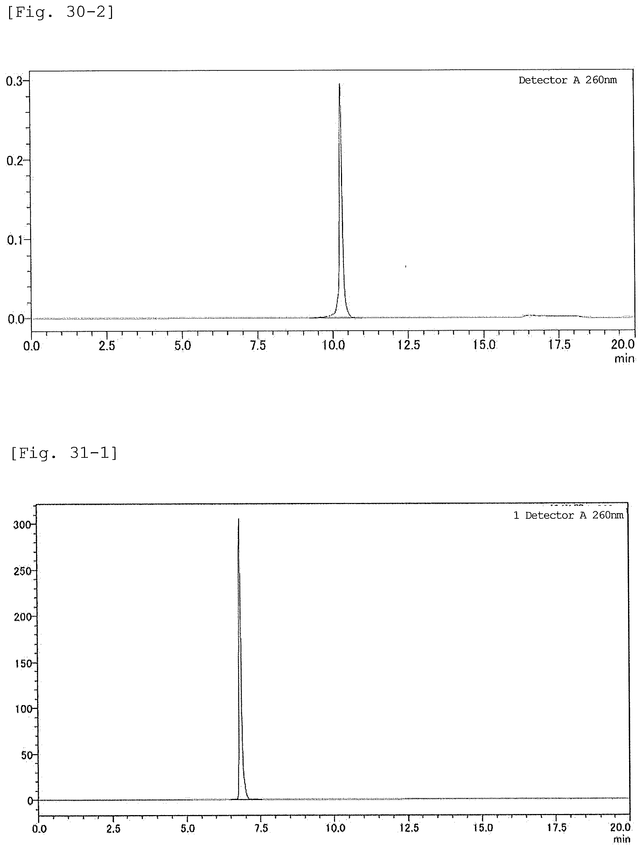

   Fig. 30-2 is an RP-HPLC chart of ESB2.2-20-6 after purification.
[Fig. 31-1]
   Fig. 31-1 is an RP-HPLC chart of BBN-33 after purification.
[Fig. 31-2]

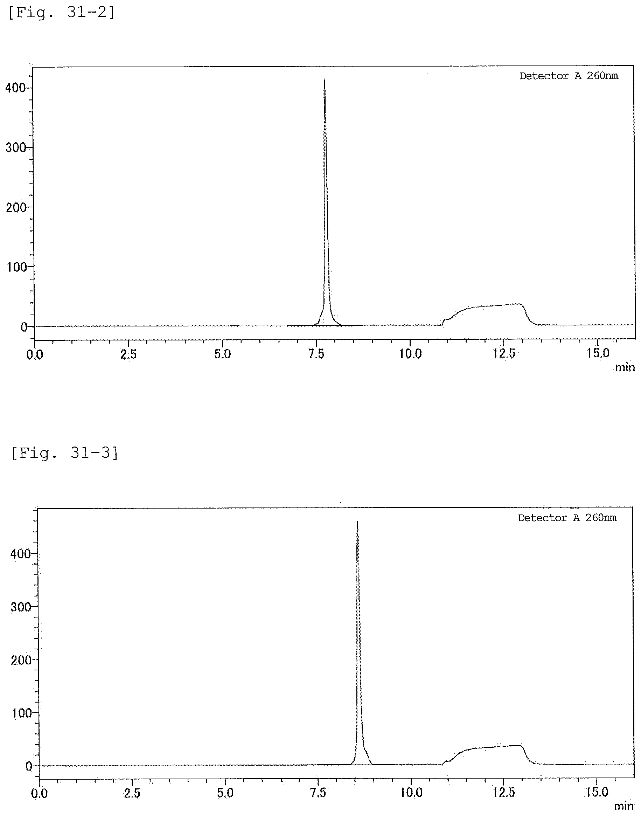

   Fig. 31-2 is an RP-HPLC chart of ESB2.2-17 after purification.
[Fig. 31-3]
   Fig. 31-3 is an RP-HPLC chart of ESB2.2-18 after purification.
[Fig. 31-4]

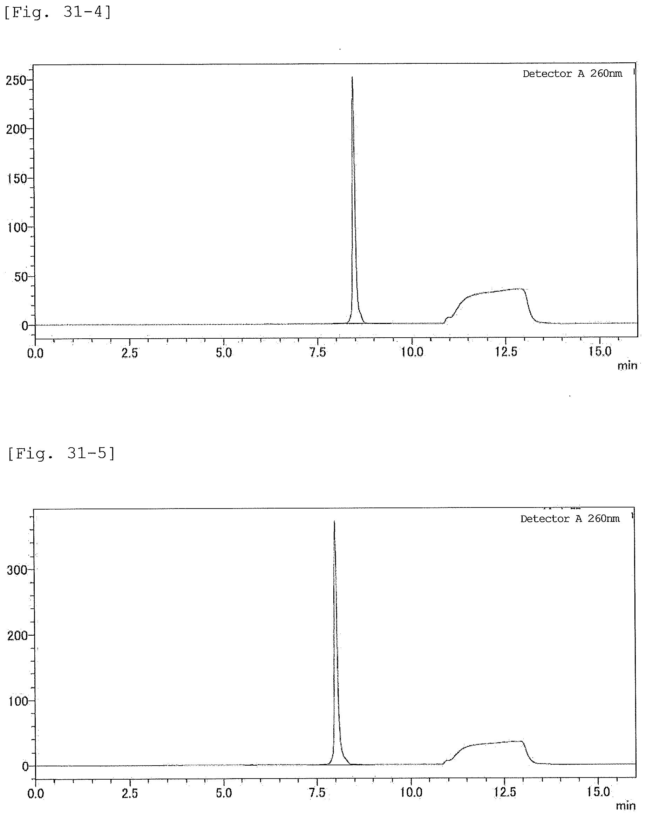

   Fig. 31-4 is an RP-HPLC chart of ESB2.2-19 after purification.
[Fig. 31-5]
   Fig. 31-5 is an RP-HPLC chart of ESB2.2-20 after purification.
[Fig. 31-6]

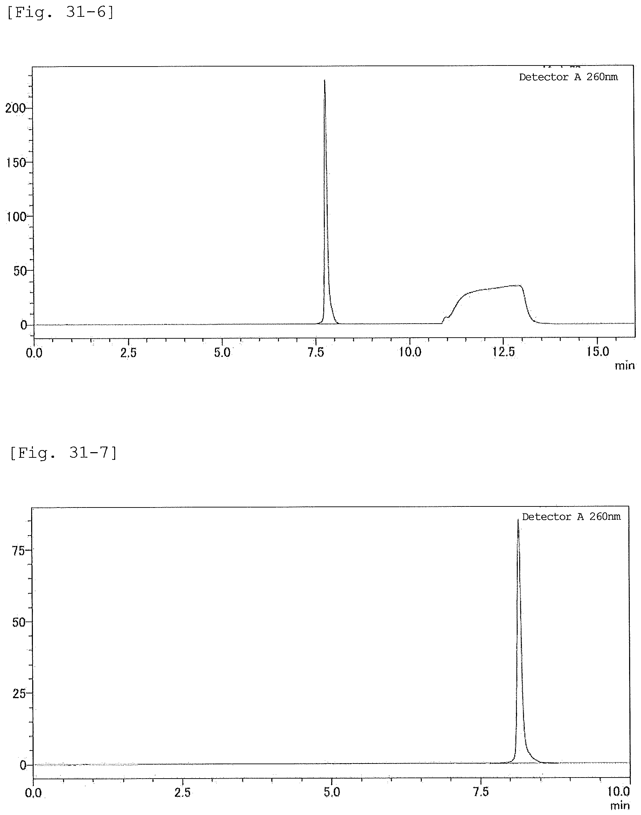

   Fig. 31-6 is an RP-HPLC chart of ESB2.2-21 after purification.
[Fig. 31-7]
   Fig. 31-7 is an RP-HPLC chart of ESB2.2-22 after purification.
[Fig. 31-8]

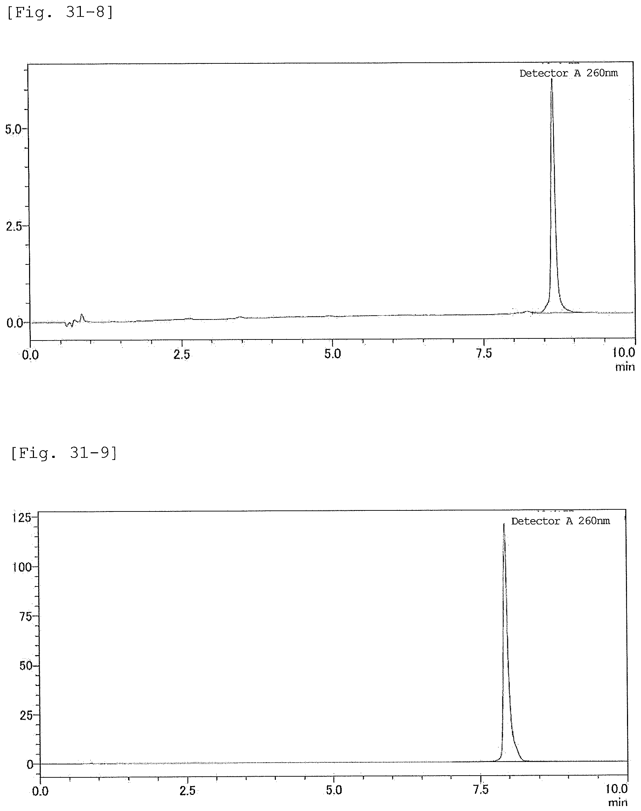

   Fig. 31-8 is an RP-HPLC chart of ESB2.2-23 after purification.
[Fig. 31-9]
   Fig. 31-9 is an RP-HPLC chart of ESB2.2-24 after purification.
[Fig. 31-10]

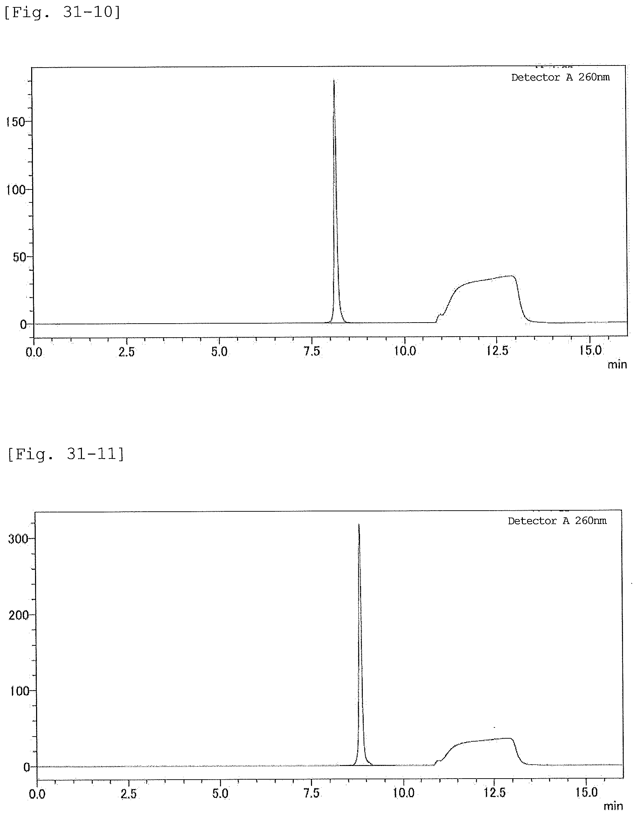

   Fig. 31-10 is an RP-HPLC chart of ESB2.2-25 after purification.
[Fig. 31-11]
   Fig. 31-11 is an RP-HPLC chart of ESB2.2-26 after purification.
[Fig. 31-12]

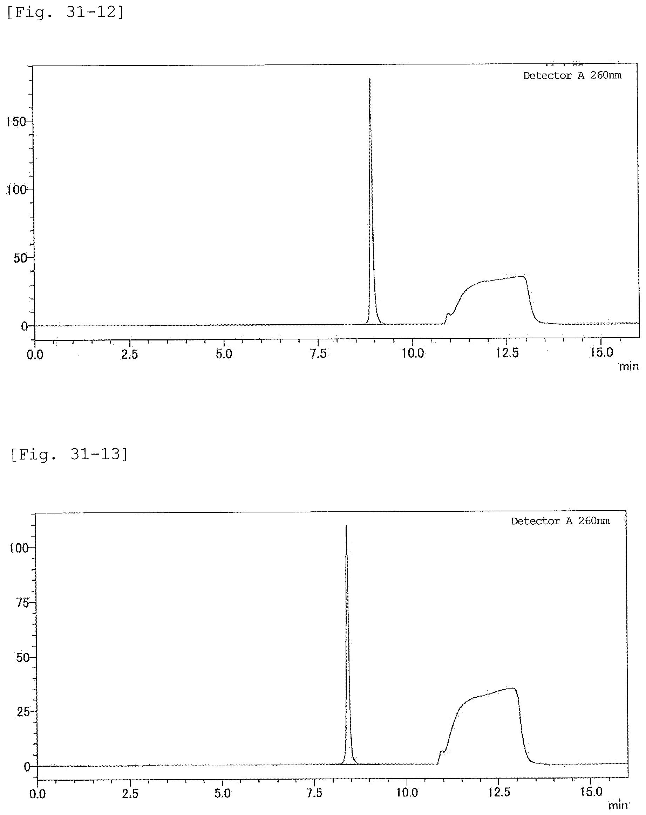

   Fig. 31-12 is an RP-HPLC chart of ESB2.2-27 after purification.
[Fig. 31-13]
   Fig. 31-13 is an RP-HPLC chart of ESB2.2-28 after purification.
[Fig. 31-14]

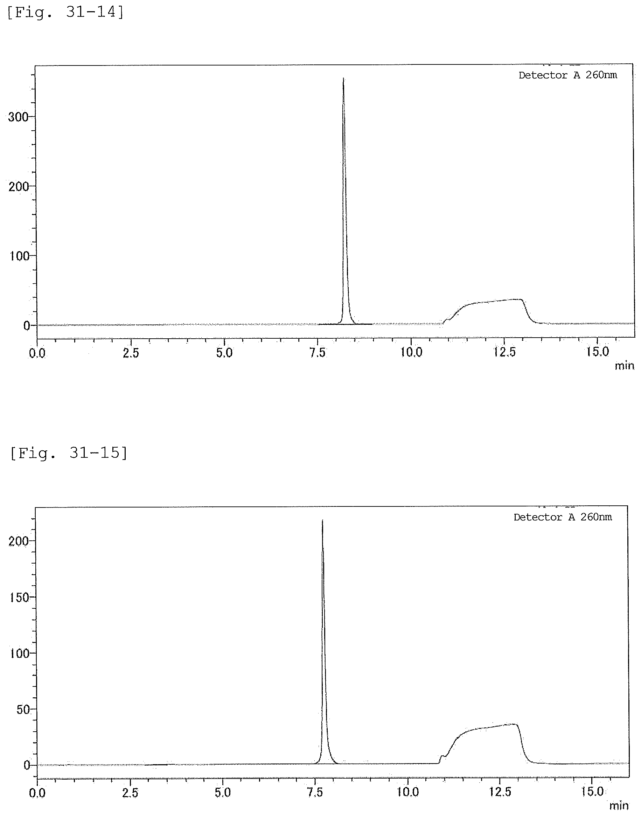

   Fig. 31-14 is an RP-HPLC chart of ESB2.2-29 after purification.
[Fig. 31-15]
   Fig. 31-15 is an RP-HPLC chart of ESB2.2-30 after purification.
[Fig. 31-16]

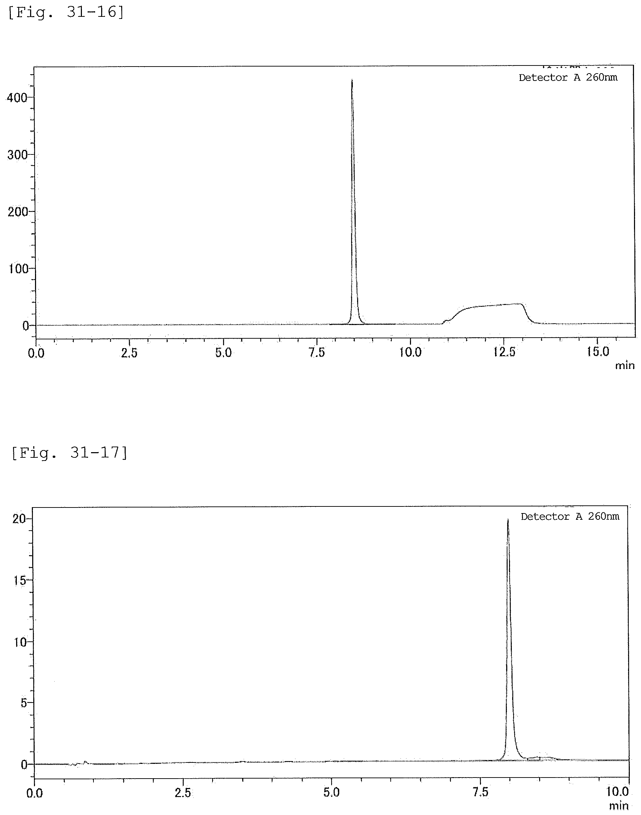

   Fig. 31-16 is an RP-HPLC chart of ESB2.2-31 after purification.
[Fig. 31-17]
   Fig. 31-17 is an RP-HPLC chart of ESB2.2-32 after purification.
[Fig. 31-18]

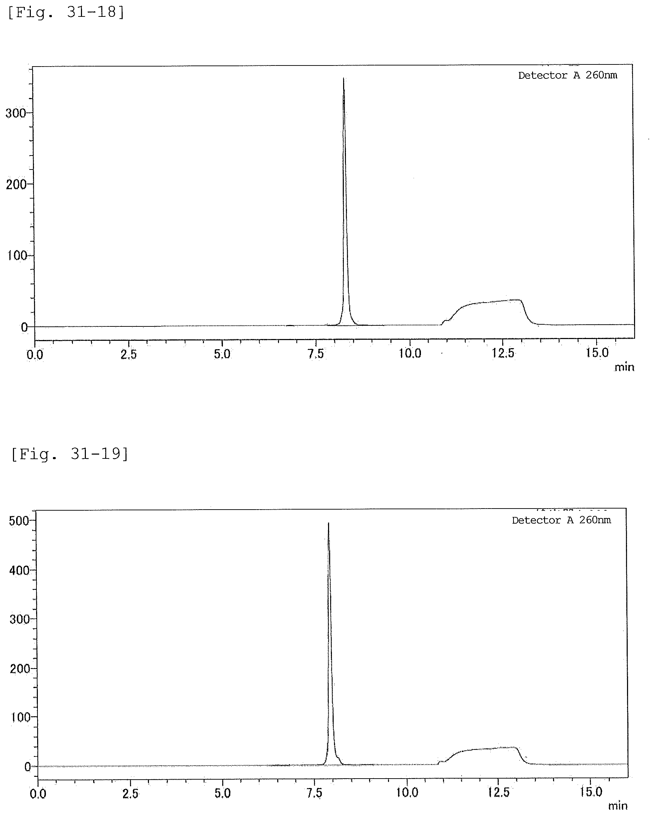

   Fig. 31-18 is an RP-HPLC chart of ESB2.2-33 after purification.
[Fig. 31-19]
   Fig. 31-19 is an RP-HPLC chart of ESB2.2-34 after purification.
[Fig. 31-20]

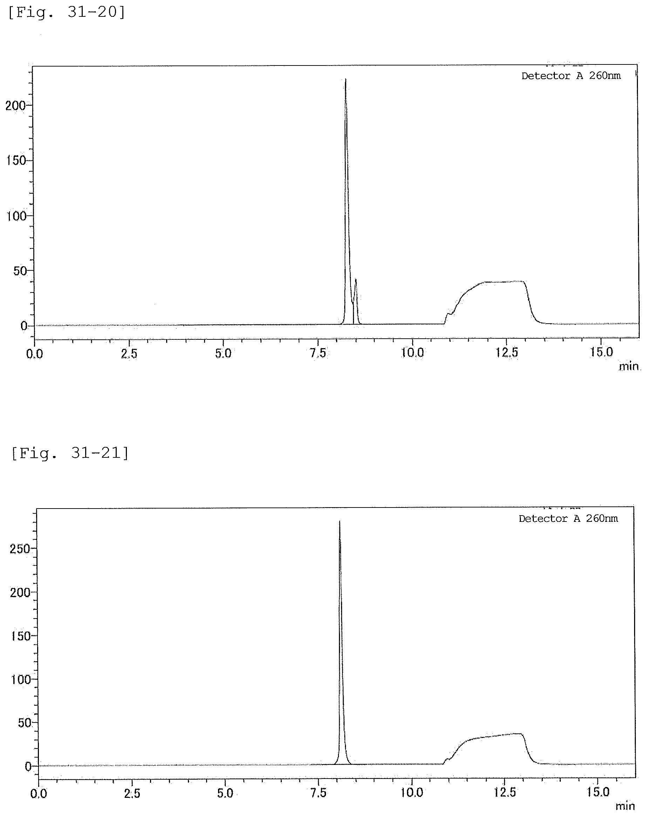

   Fig. 31-20 is an RP-HPLC chart of ESB2.2-35 after purification.
[Fig. 31-21]
   Fig. 31-21 is an RP-HPLC chart of ESB2.2-36 after purification.
[Fig. 31-22]

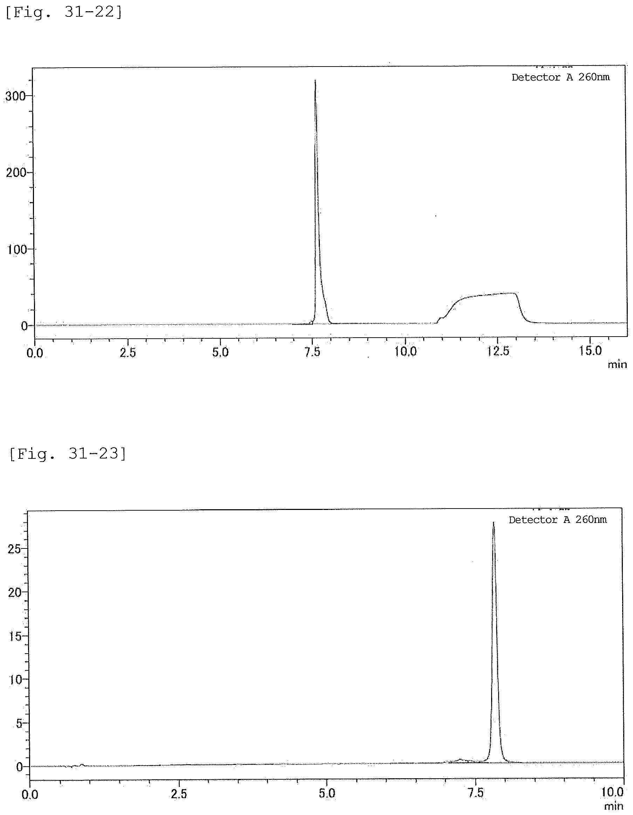

   Fig. 31-22 is an RP-HPLC chart of ESB2.2-37 after purification.
[Fig. 31-23]
   Fig. 31-23 is an RP-HPLC chart of ESB2.2-38 after purification.
[Fig. 31-24]

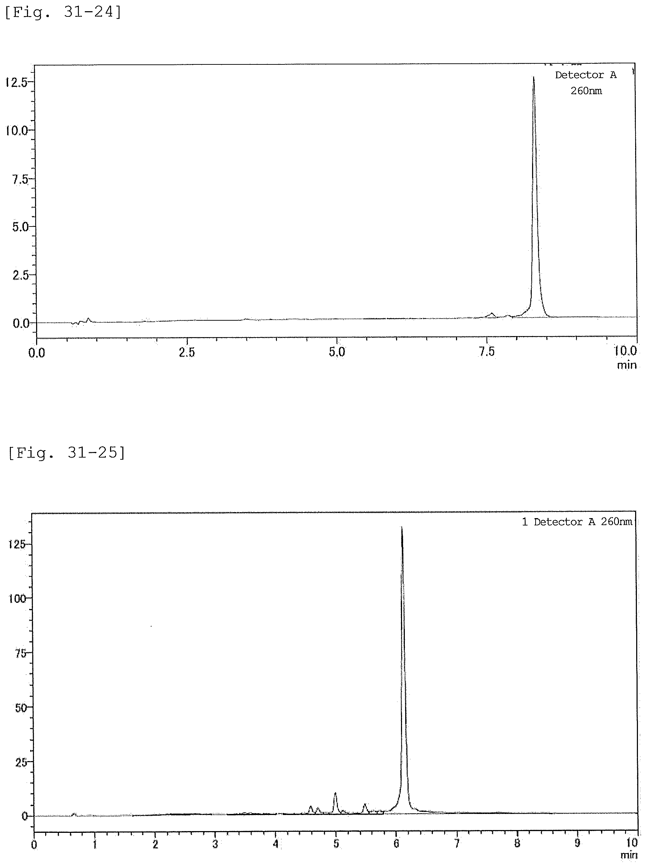

   Fig. 31-24 is an RP-HPLC chart of ESB2.2-39 after purification.
[Fig. 31-25]
   Fig. 31-25 is an RP-HPLC chart of ESB2.2-20-fd after purification.
[Fig. 31-26]

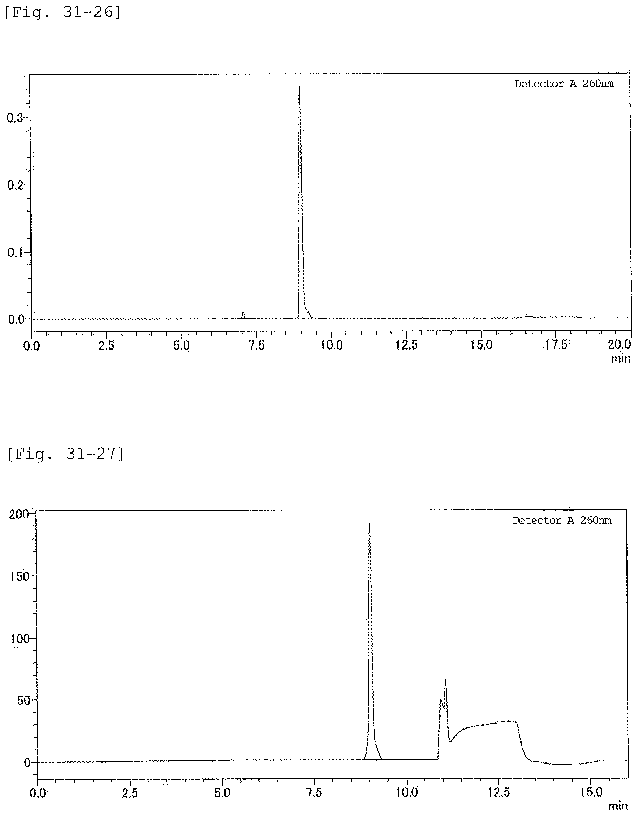

   Fig. 31-26 is an RP-HPLC chart of ESB2.2_HPRT1-1 after purification.
[Fig. 31-27]
   Fig. 31-27 is an RP-HPLC chart of 6b1-4 after purification.
[Fig. 32]

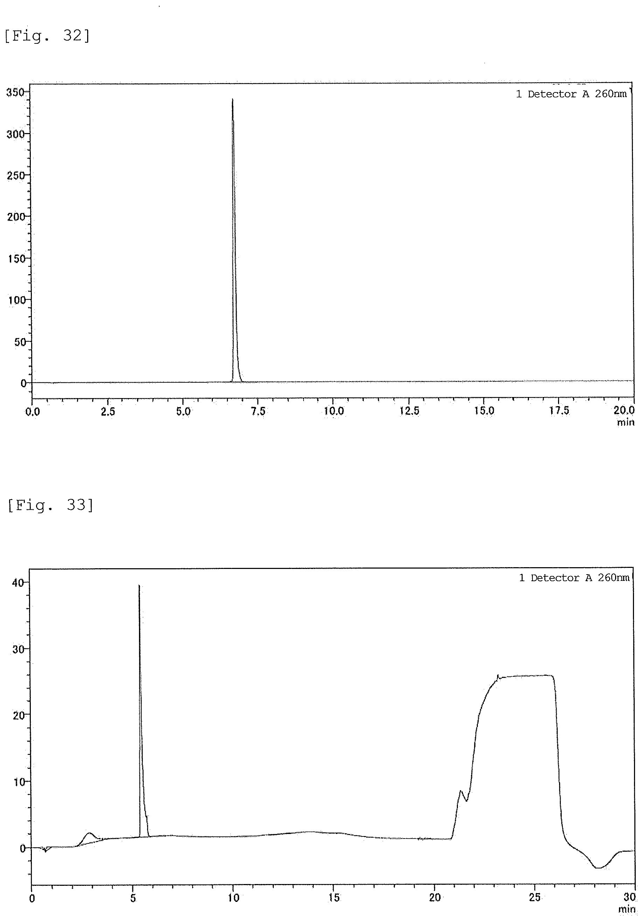

   Fig. 32 is an RP-HPLC chart of BBN-34 after purification.
[Fig. 33]
   Fig. 33 is an RP-HPLC chart of BBN-39 after purification.
[Fig. 34]

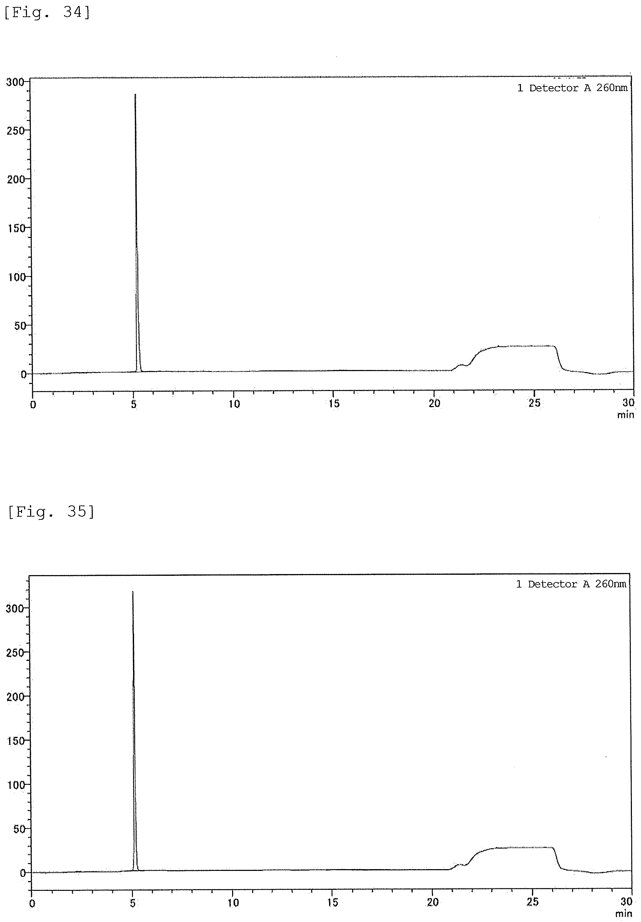

   Fig. 34 is an RP-HPLC chart of BBN-40 after purification.
[Fig. 35]

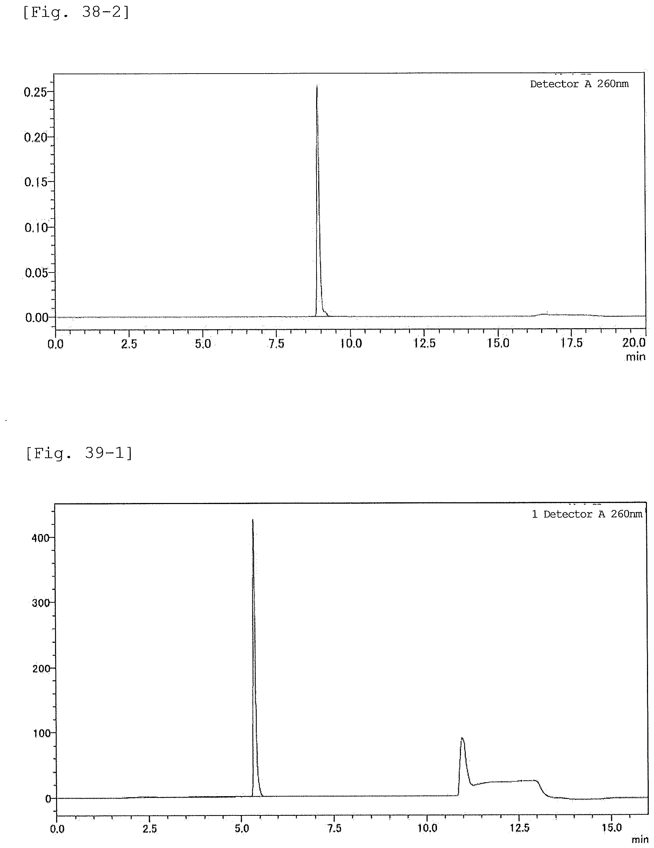

   Fig. 35 is an RP-HPLC chart of BBN-41 after purification.
[Fig. 36-1]

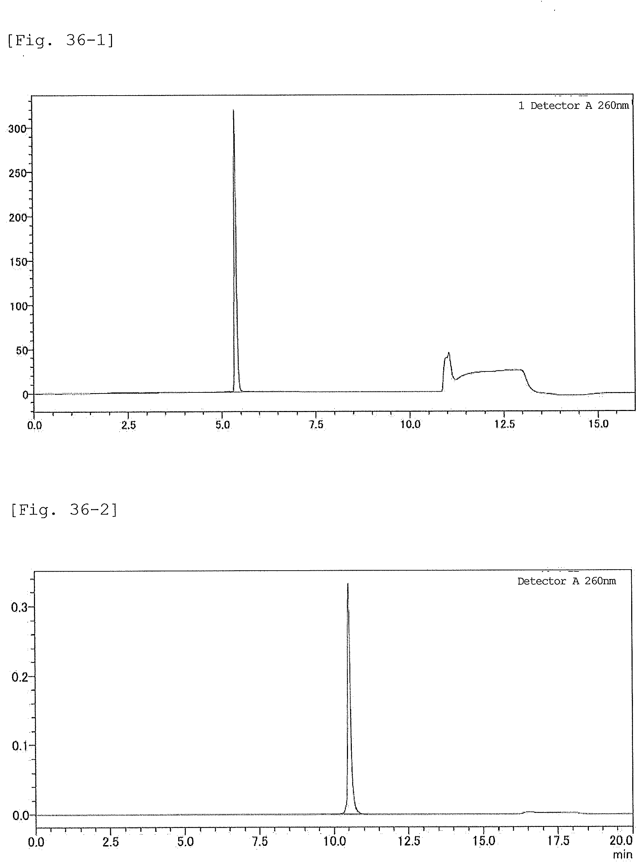

   Fig. 36-1 is an RP-HPLC chart of BBN-42 after purification.
[Fig. 36-2]
   Fig. 36-2 is an RP-HPLC chart of ESB2.2-20-1 after purification.
[Fig. 37]

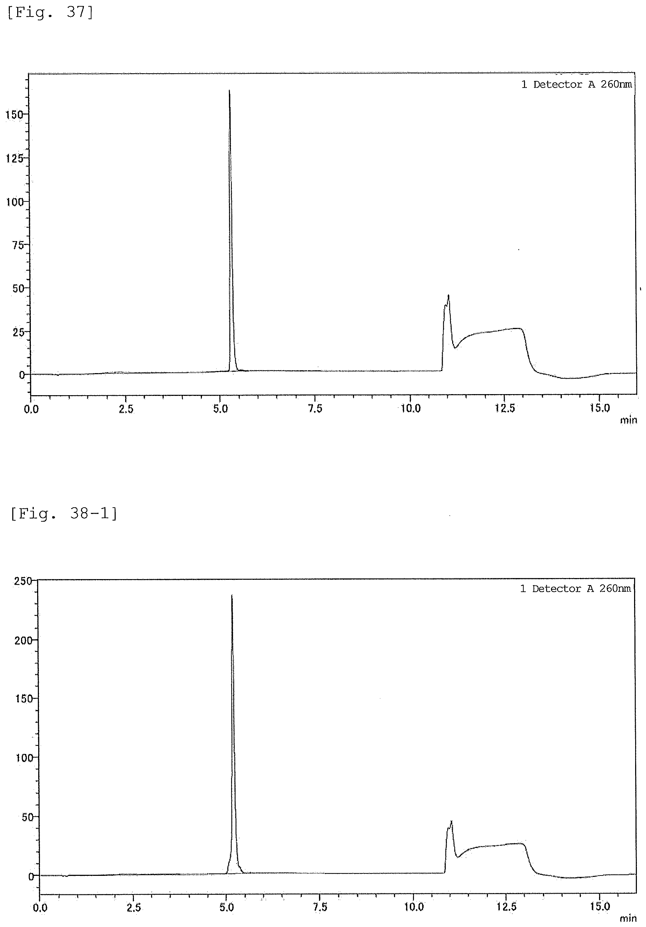

   Fig. 37 is an RP-HPLC chart of BBN-43 after purification.
[Fig. 38-1]
   Fig. 38-1 is an RP-HPLC chart of BBN-44 after purification.
[Fig. 38-2]
   Fig. 38-2 is an RP-HPLC chart of ESB2.2_HPRT1-2 after purification.
[Fig. 39-1]
   Fig. 39-1 is an RP-HPLC chart of BBN-45 after purification.
[Fig. 39-2]

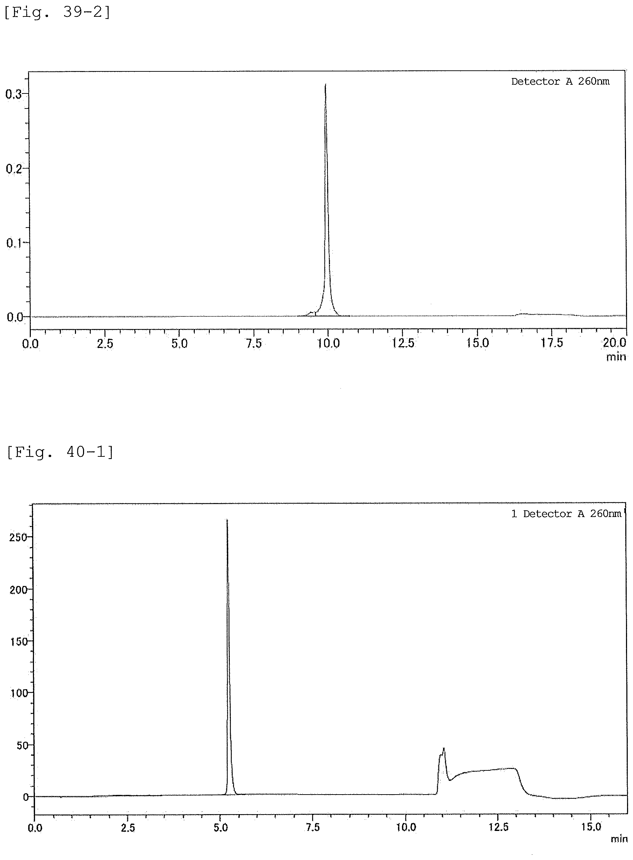

   Fig. 39-2 is an RP-HPLC chart of ESB2.2-20-8 after purification.
[Fig. 40-1]
   Fig. 40-1 is an RP-HPLC chart of BBN-46 after purification.
[Fig. 40-2]

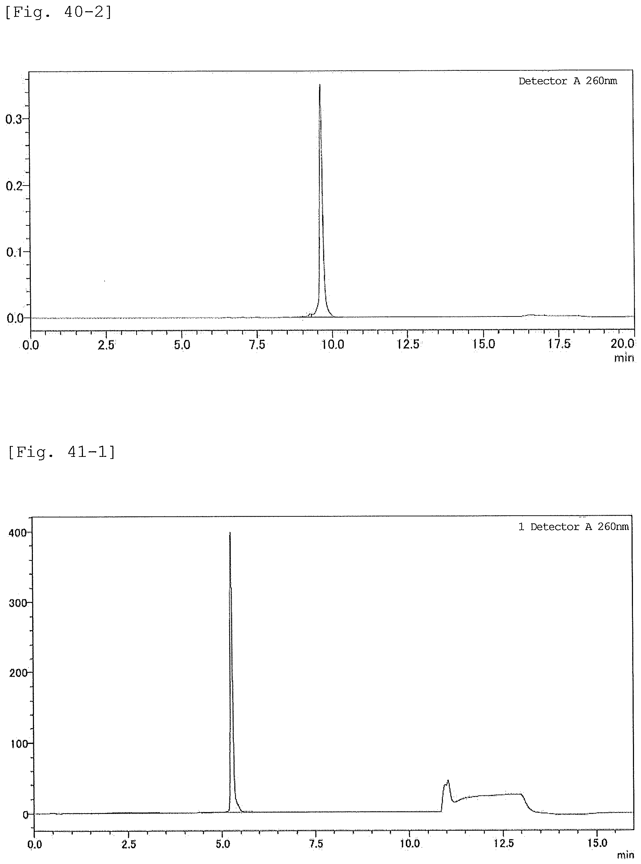

   Fig. 40-2 is an RP-HPLC chart of ESB2.2-20-9 after purification.
[Fig. 41-1]
   Fig. 41-1 is an RP-HPLC chart of BBN-47 after purification.
[Fig. 41-2]

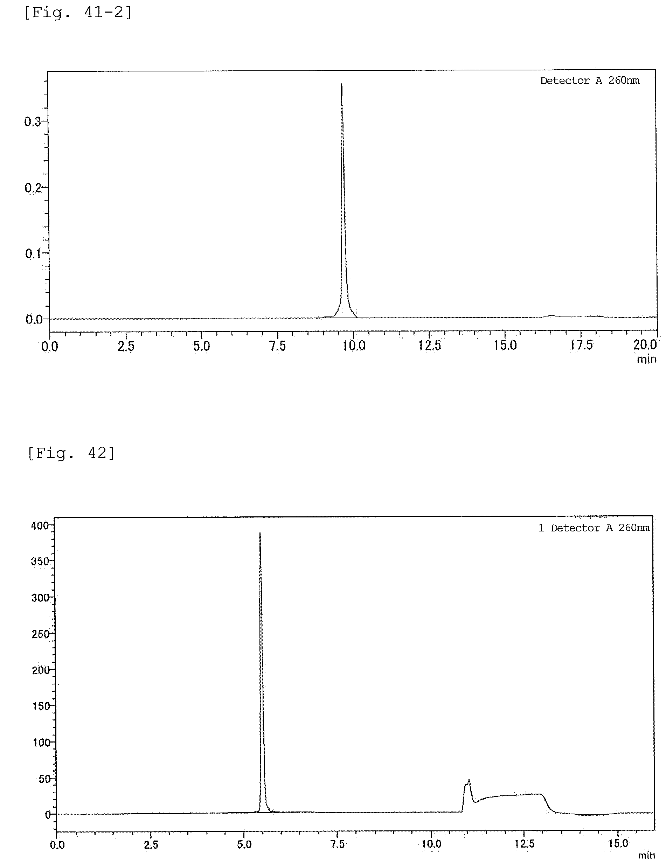

   Fig. 41-2 is an RP-HPLC chart of ESB2.2-20-10 after purification.
[Fig. 42]
   Fig. 42 is an RP-HPLC chart of BBN-48 after purification.
[Fig. 43]

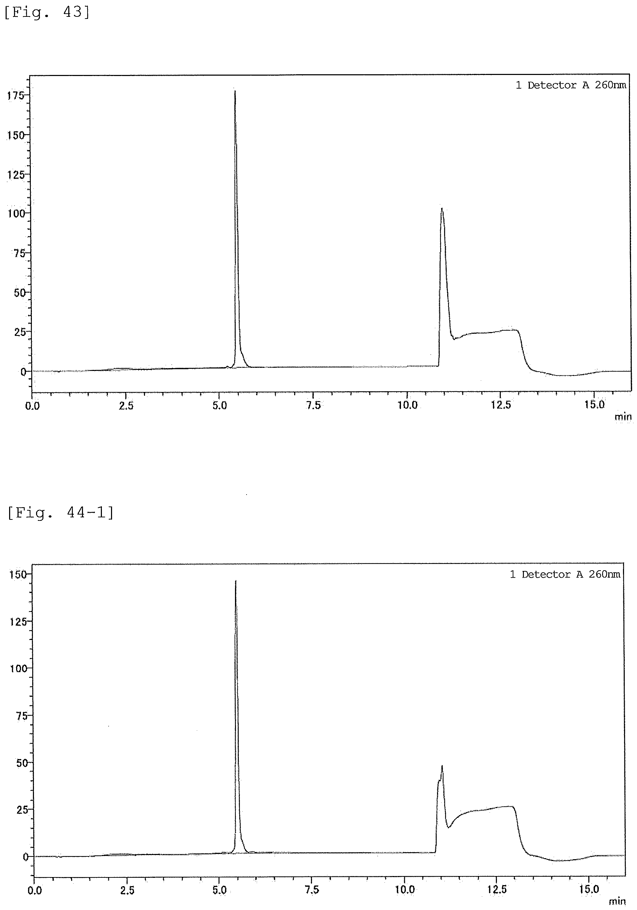

   Fig. 43 is an RP-HPLC chart of BBN-49 after purification.
[Fig. 44-1]
   Fig. 44-1 is an RP-HPLC chart of BBN-50 after purification.
[Fig. 44-2]

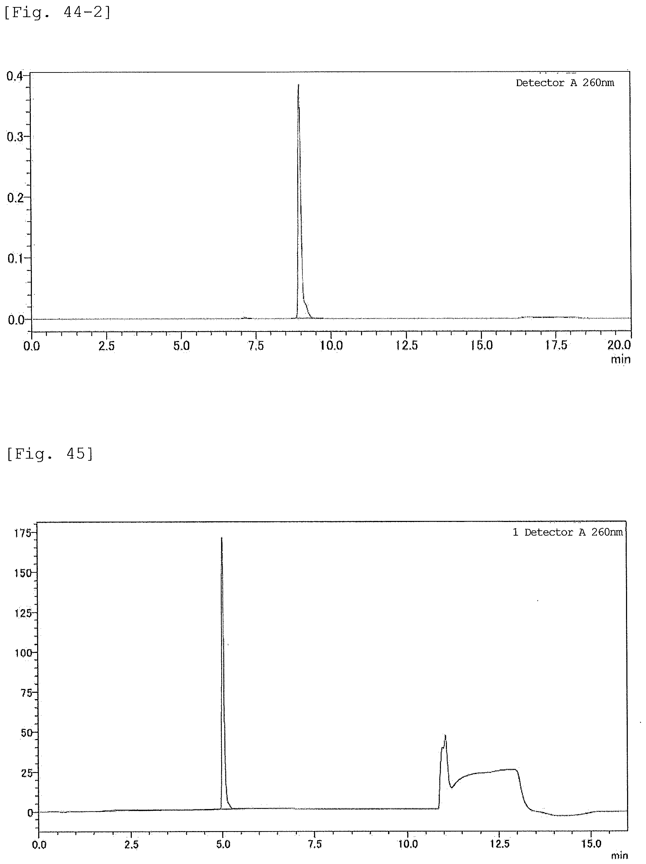

   Fig. 44-2 is an RP-HPLC chart of ESB2.2_HPRT1-5 after purification.
[Fig. 45]
   Fig. 45 is an RP-HPLC chart of BBN-51 after purification.
[Fig. 46-1]

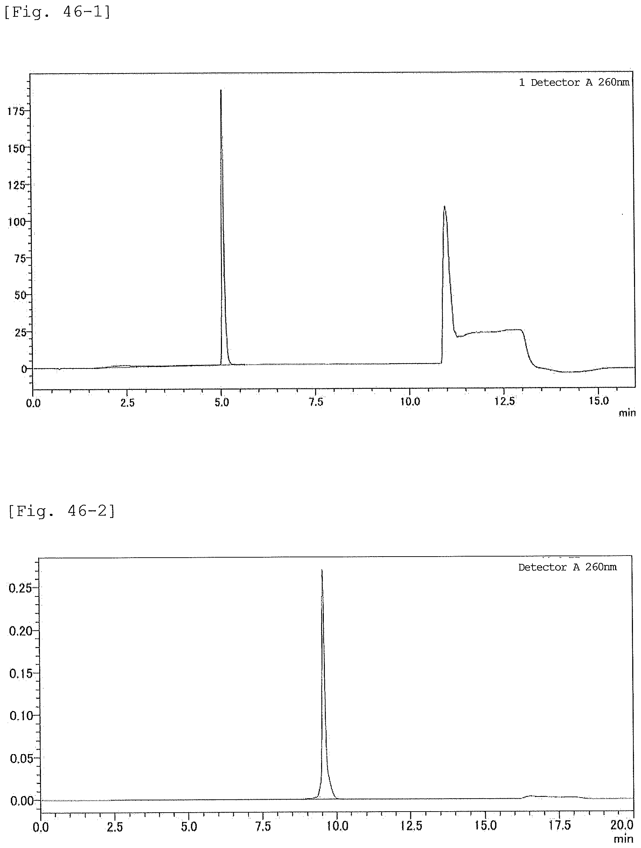

   Fig. 46-1 is an RP-HPLC chart of BBN-52 after purification.
[Fig. 46-2]
   Fig. 46-2 is an RP-HPLC chart of ESB2.2-20-7 after purification.
[Fig. 47]

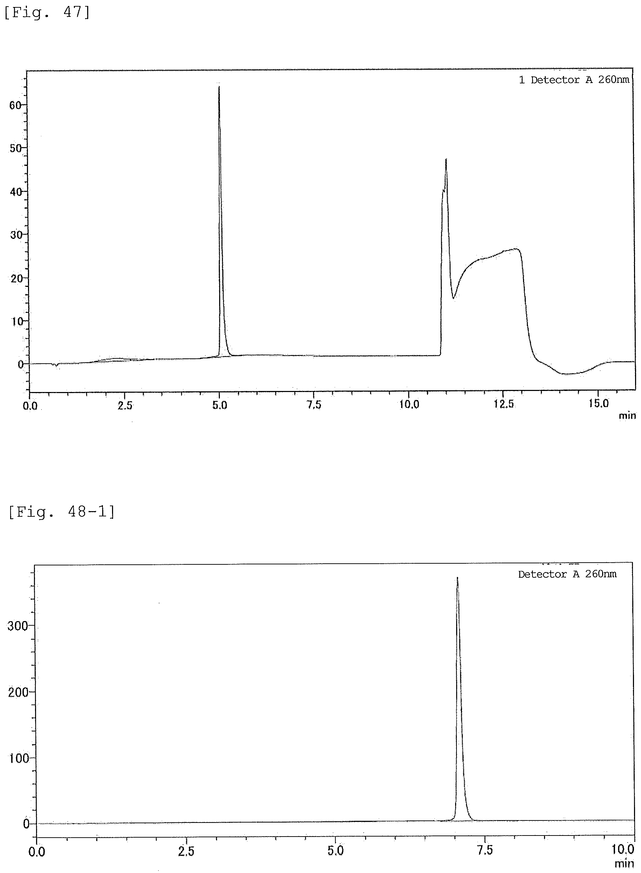

   Fig. 47 is an RP-HPLC chart of BBN-53 after purification.
[Fig. 48-1]
   Fig. 48-1 is an RP-HPLC chart of ESB2.2-1 after purification.
[Fig. 48-2]

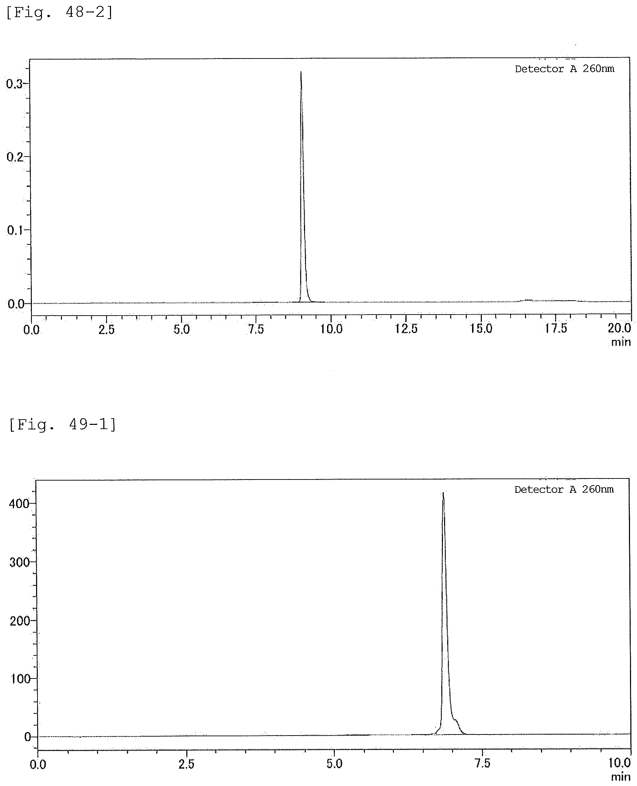

   Fig. 48-2 is an RP-HPLC chart of ESB2.2_HPRT1-6 after purification.
[Fig. 49-1]
   Fig. 49-1 is an RP-HPLC chart of ESB2.2-2 after purification.
[Fig. 49-2]

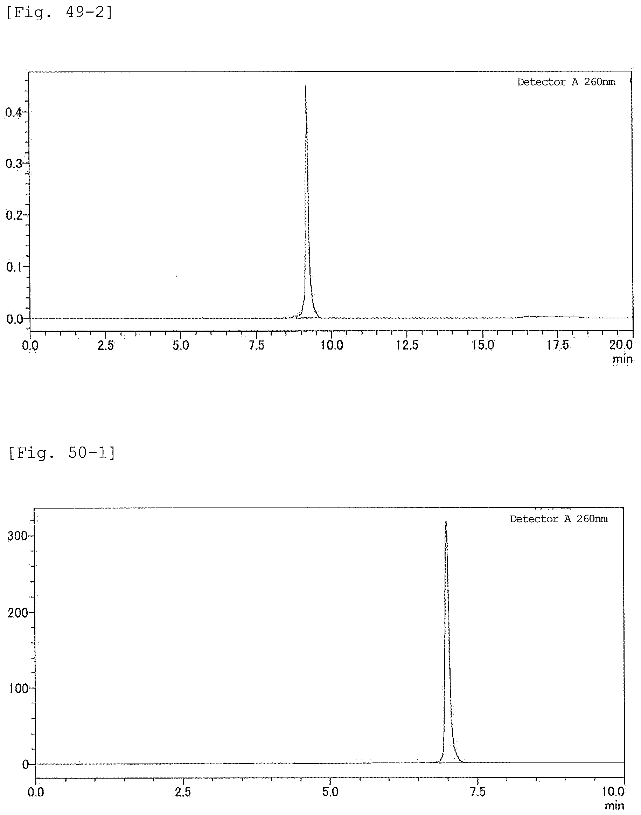

   Fig. 49-2 is an RP-HPLC chart of ESB2.2-20-15 after purification.
[Fig. 50-1]
   Fig. 50-1 is an RP-HPLC chart of ESB2.2-3 after purification.
[Fig. 50-2]

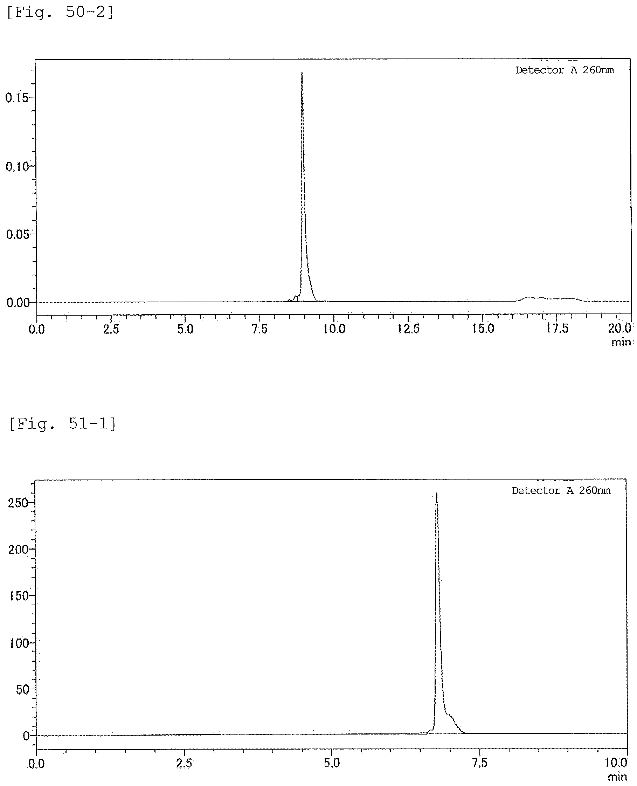

   Fig. 50-2 is an RP-HPLC chart of ESB2.2-20-16 after purification.
[Fig. 51-1]
   Fig. 51-1 is an RP-HPLC chart of ESB2.2-4 after purification.
[Fig. 51-2]

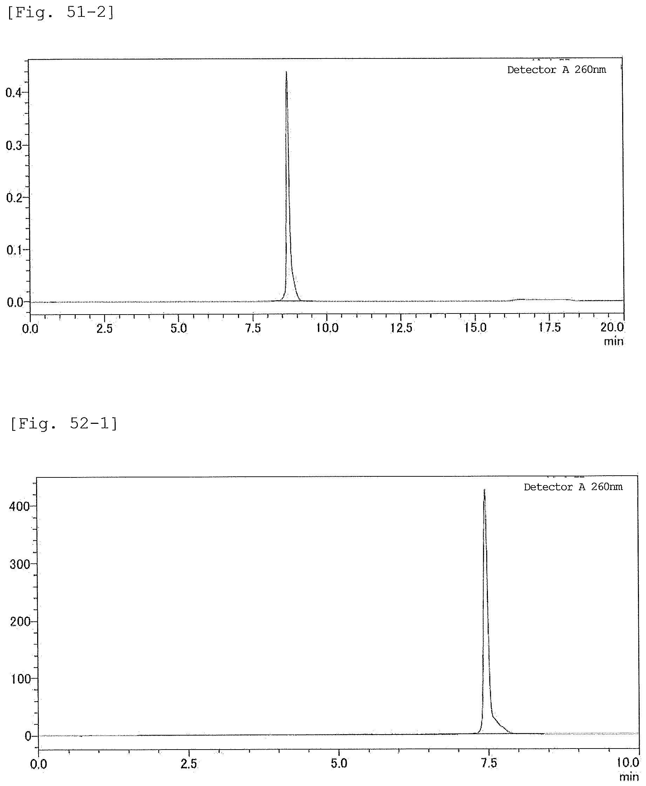

   Fig. 51-2 is an RP-HPLC chart of ESB2.2-20-17 after purification.
[Fig. 52-1]
   Fig. 52-1 is an RP-HPLC chart of ESB2.2-5 after purification.
[Fig. 52-2]

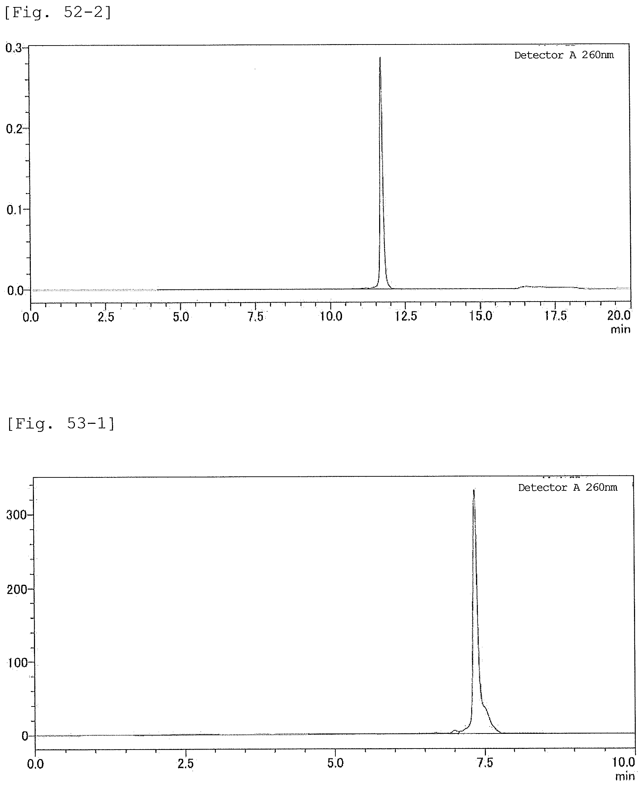

   Fig. 52-2 is an RP-HPLC chart of ESB2.2-20-22 after purification.
[Fig. 53-1]
   Fig. 53-1 is an RP-HPLC chart of ESB2.2-6 after purification.
[Fig. 53-2]

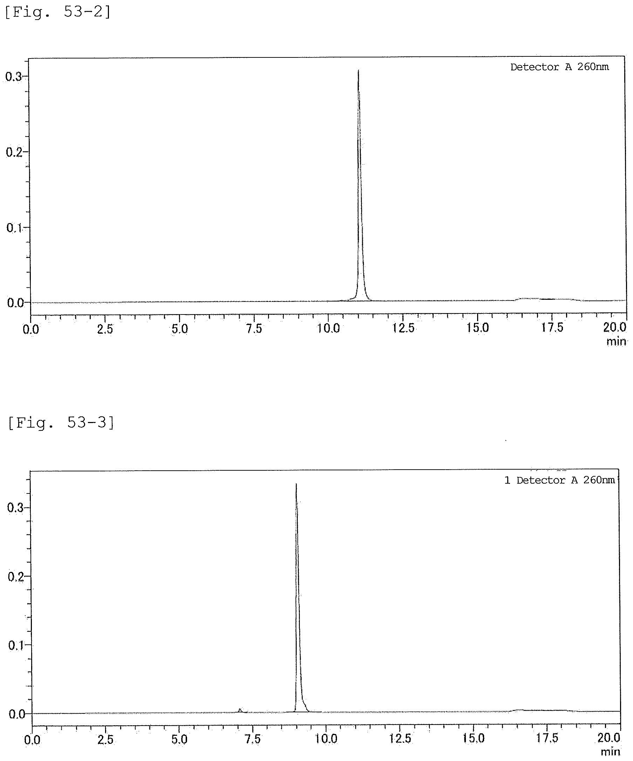

   Fig. 53-2 is an RP-HPLC chart of ESB2.2-20-21 after purification.
[Fig. 53-3]
   Fig. 53-3 is an RP-HPLC chart of ESB2.2_HPRT1-9 after purification.
[Fig. 54-1]

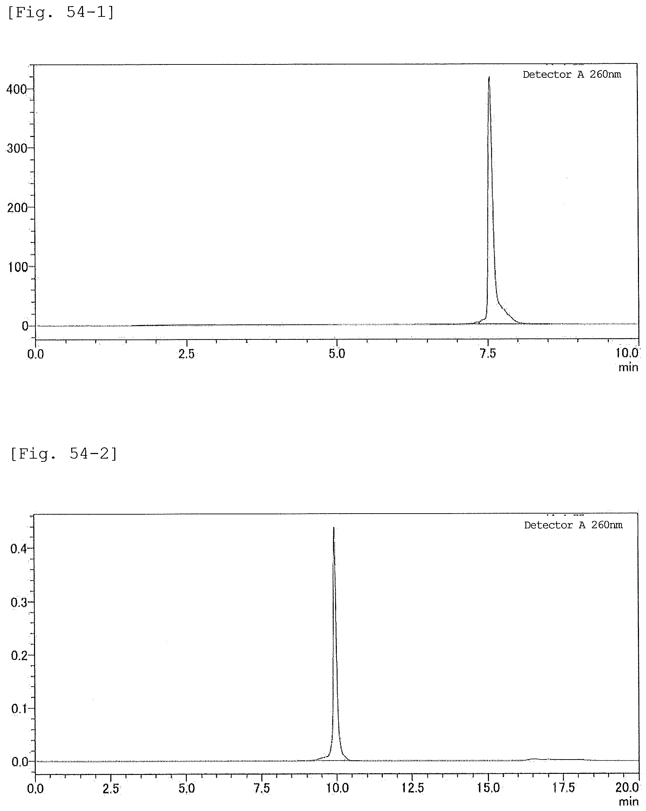

   Fig. 54-1 is an RP-HPLC chart of ESB2.2-7 after purification.
[Fig. 54-2]
   Fig. 54-2 is an RP-HPLC chart of ESB2.2-20-19 after purification.
[Fig. 55-1]

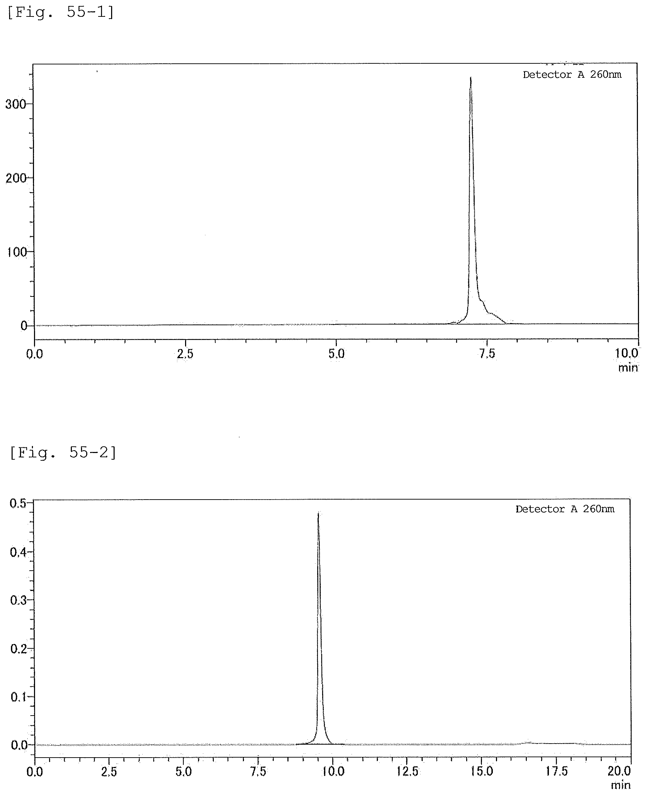

   Fig. 55-1 is an RP-HPLC chart of ESB2.2-8 after purification.
[Fig. 55-2]
   Fig. 55-2 is an RP-HPLC chart of ESB2.2-20 after purification.
[Fig. 56-1]

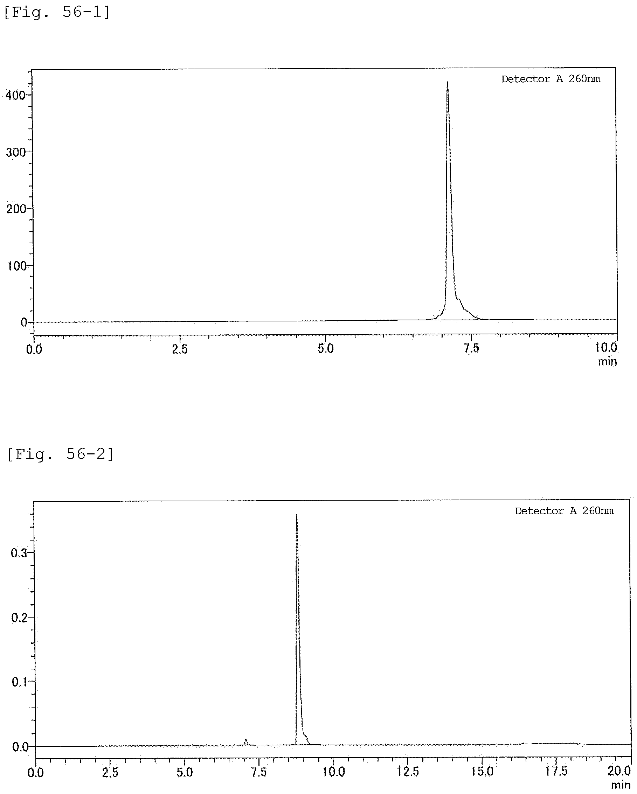

   Fig. 56-1 is an RP-HPLC chart of ESB2.2-9 after purification.
[Fig. 56-2]
   Fig. 56-2 is an RP-HPLC chart of ESB2.2_HPRT1-8 after purification.
[Fig. 57-1]

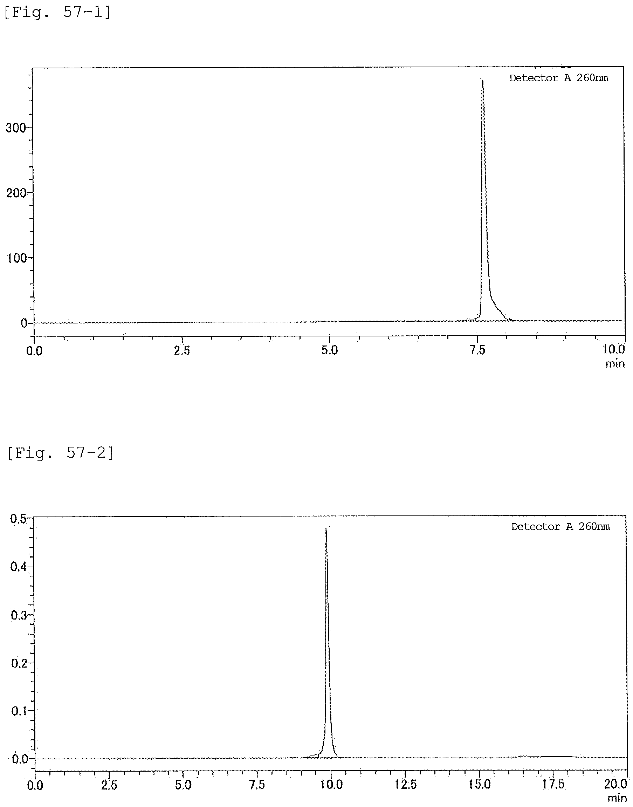

   Fig. 57-1 is an RP-HPLC chart of ESB2.2-10 after purification.
[Fig. 57-2]
   Fig. 57-2 is an RP-HPLC chart of ESB2.2-20-18 after purification.
[Fig. 58-1]

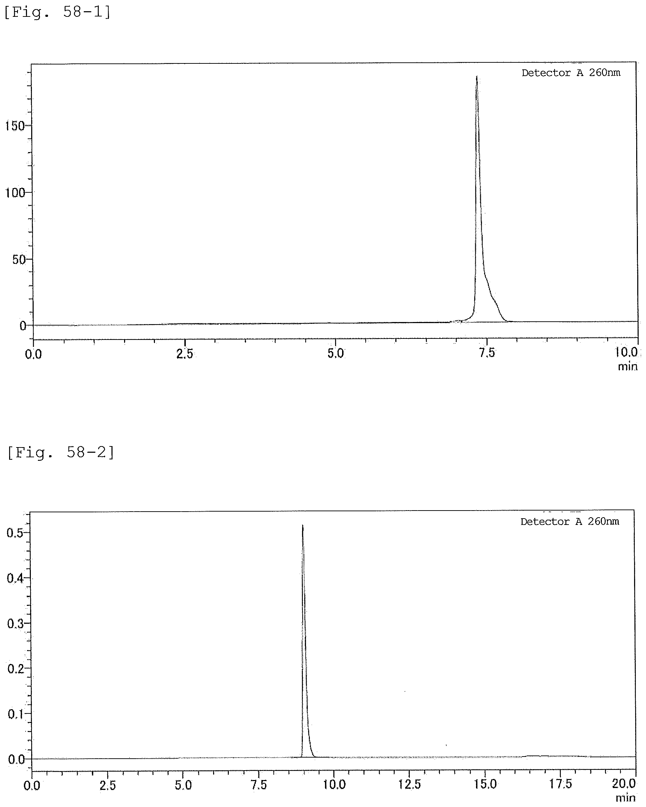

   Fig. 58-1 is an RP-HPLC chart of ESB2.2-11 after purification.
[Fig. 58-2]
   Fig. 58-2 is an RP-HPLC chart of ESB2.2_HPRT1-7 after purification.
[Fig. 59]

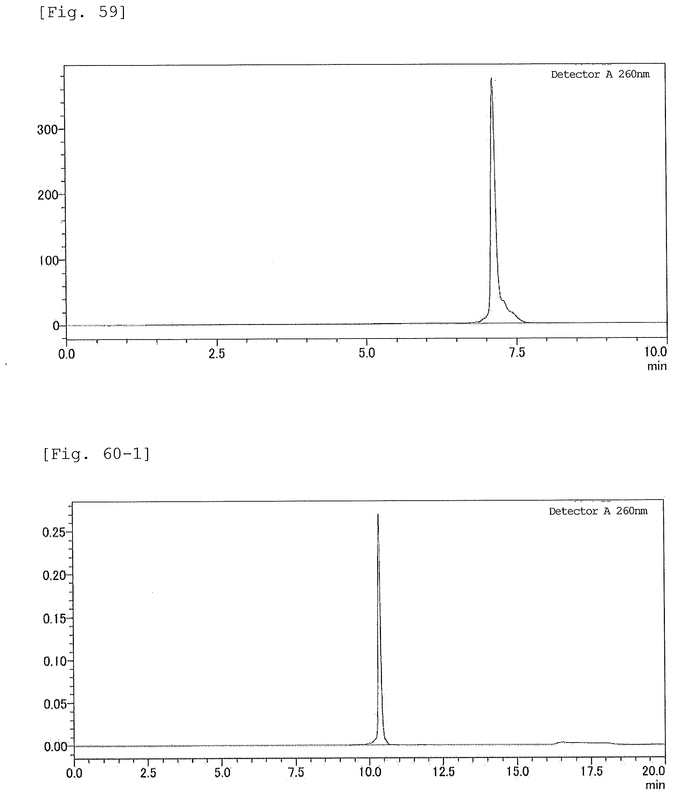

   Fig. 59 is an RP-HPLC chart of ESB2.2-12 after purification.
[Fig. 60-1]
   Fig. 60-1 is an RP-HPLC chart of ESB2.2-20-23 after purification.
[Fig. 60-2]

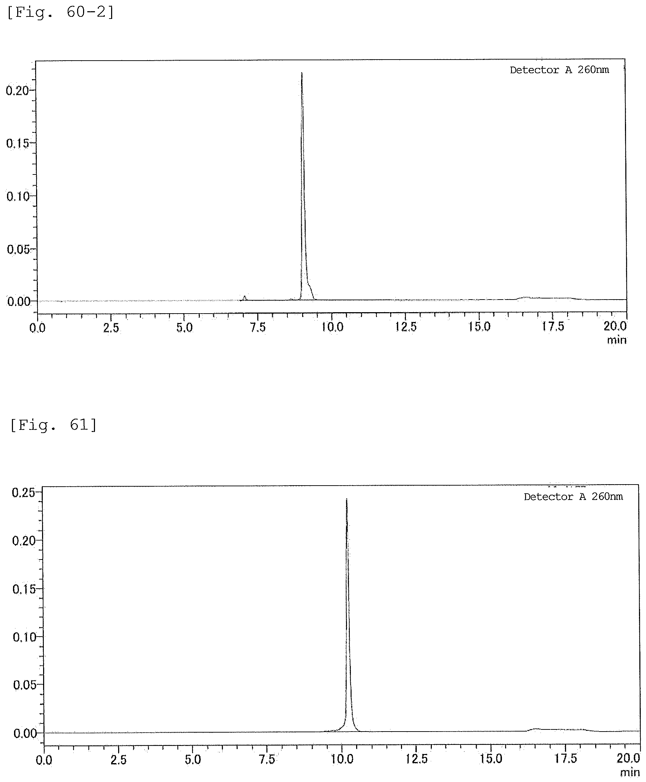

   Fig. 60-2 is an RP-HPLC chart of ESB2.2_HPRT1-10 after purification.
[Fig. 61]
   Fig. 61 is an RP-HPLC chart of ESB2.2-20-24 after purification.
[Fig. 62-1]

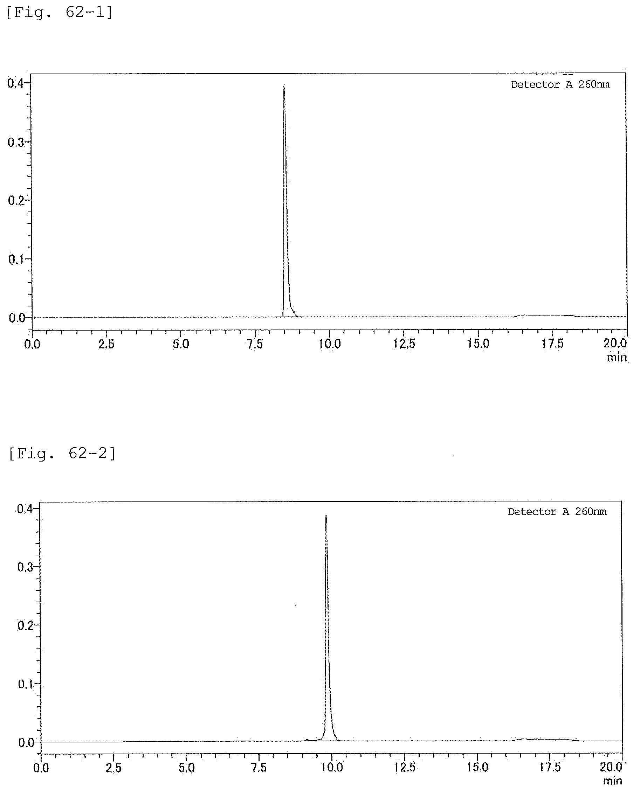

   Fig. 62-1 is an RP-HPLC chart of ESB2.2-NEK-S21 after purification.
[Fig. 62-2]
   Fig. 62-2 is an RP-HPLC chart of ESB2.2-20-S21 after purification.
[Fig. 63-1]

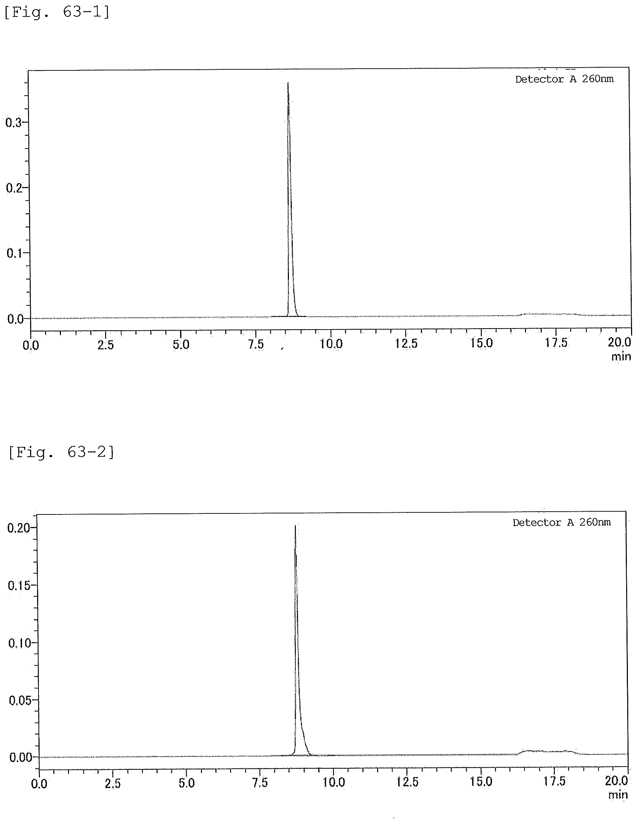

   Fig. 63-1 is an RP-HPLC chart of ESB2.2-NEK-S19 after purification.
[Fig. 63-2]
   Fig. 63-2 is an RP-HPLC chart of ESB2.2-20-S19 after purification.
[Fig. 64-1]

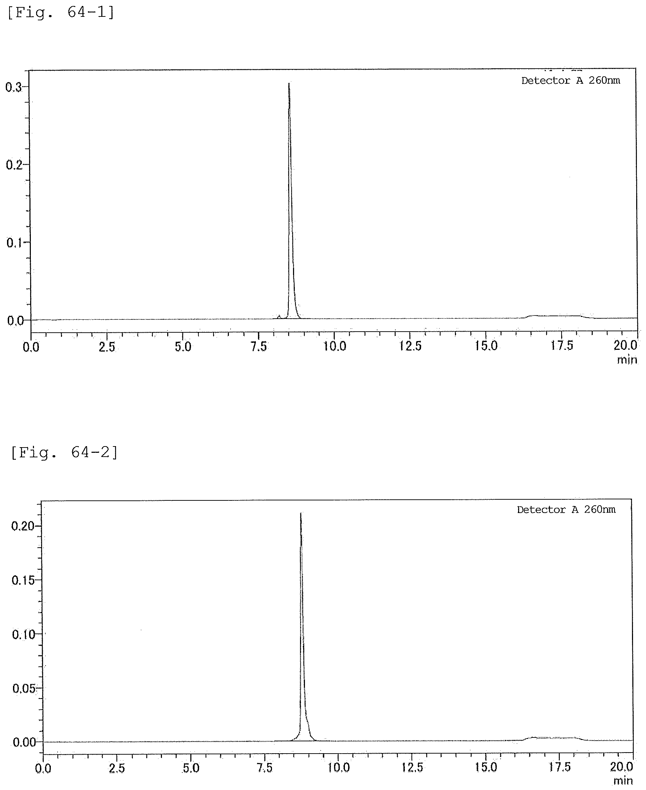

   Fig. 64-1 is an RP-HPLC chart of ESB2.2-NEK-S18 after purification.
[Fig. 64-2]
   Fig. 64-2 is an RP-HPLC chart of ESB2.2-20-S18 after purification.
[Fig. 65-1]

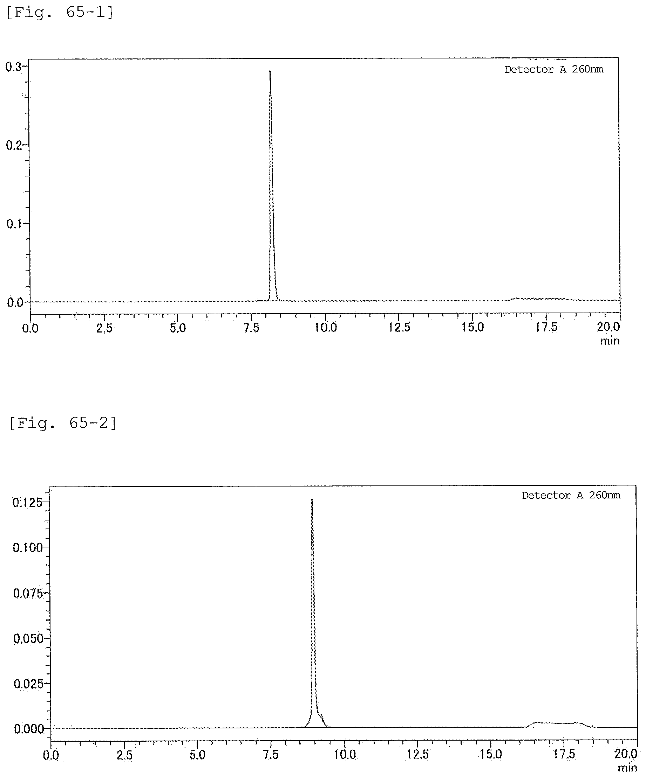

   Fig. 65-1 is an RP-HPLC chart of ESB2.2-NEK-S17 after purification.
[Fig. 65-2]
   Fig. 65-2 is an RP-HPLC chart of ESB2.2-20-S17 after purification.
[Fig. 66]

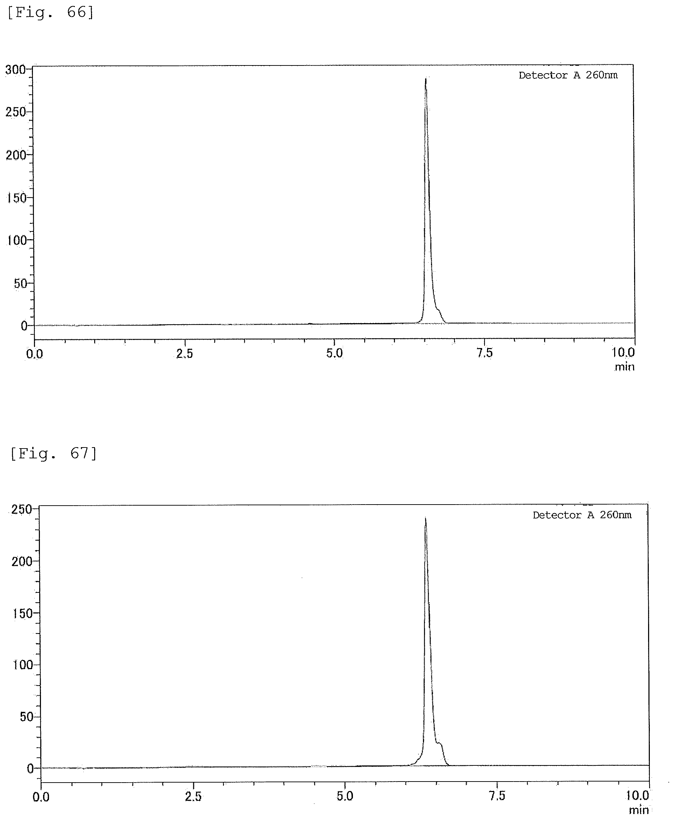

   Fig. 66 is an RP-HPLC chart of ESB2.2-15-C4 after purification.
[Fig. 67]
   Fig. 67 is an RP-HPLC chart of ESB2.2-16-C2 after purification.
[Fig. 68]

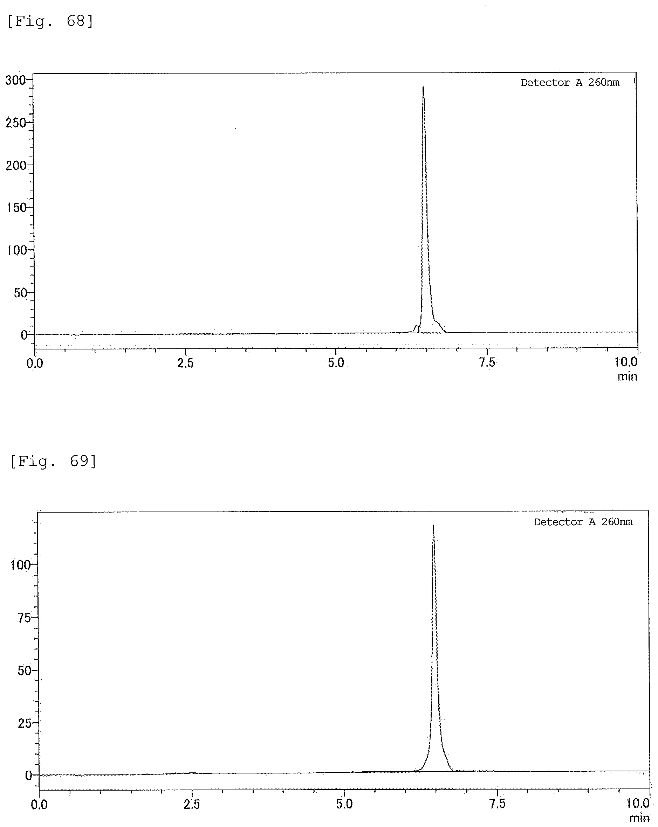

   Fig. 68 is an RP-HPLC chart of ESB2.2-16-C4 after purification.
[Fig. 69]
   Fig. 69 is an RP-HPLC chart of ESB2.2-13-C2 after purification.
[Fig. 70]

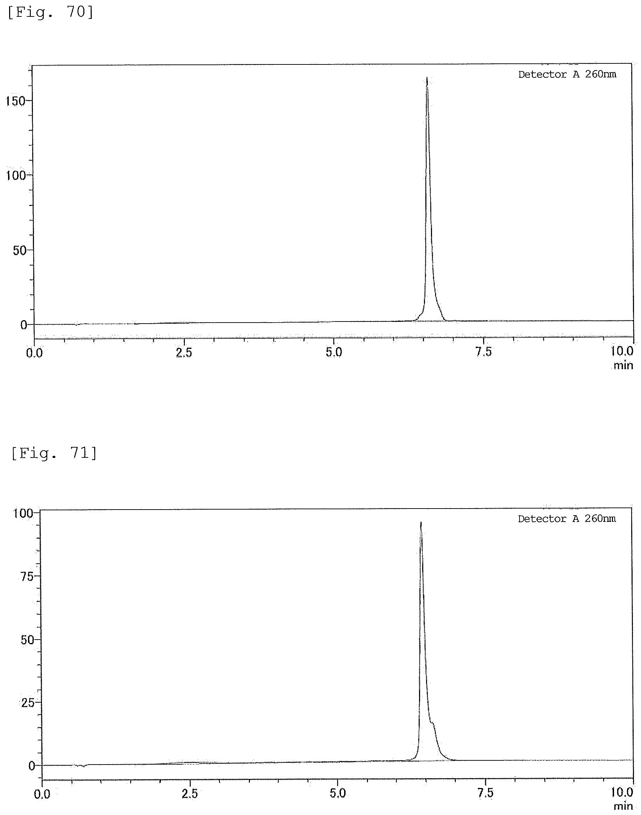

   Fig. 70 is an RP-HPLC chart of ESB2.2-13-C4 after purification.
[Fig. 71]
   Fig. 71 is an RP-HPLC chart of ESB2.2-14-C2 after purification.
[Fig. 72]

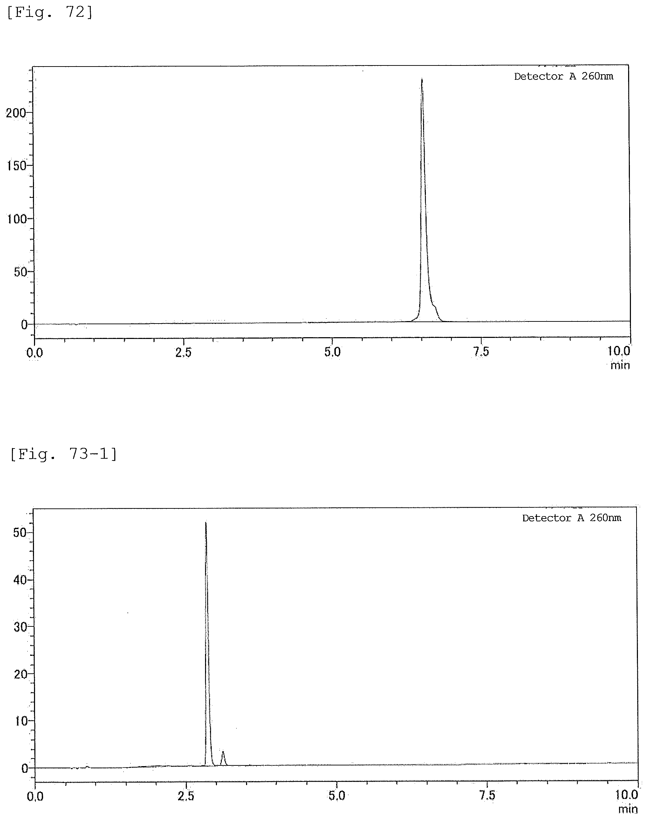

   Fig. 72 is an RP-HPLC chart of ESB2.2-14-C4 after purification.
[Fig. 73-1]
   Fig. 73-1 is an RP-HPLC chart of ESB2.2-16-C12 after purification.
[Fig. 73-2]

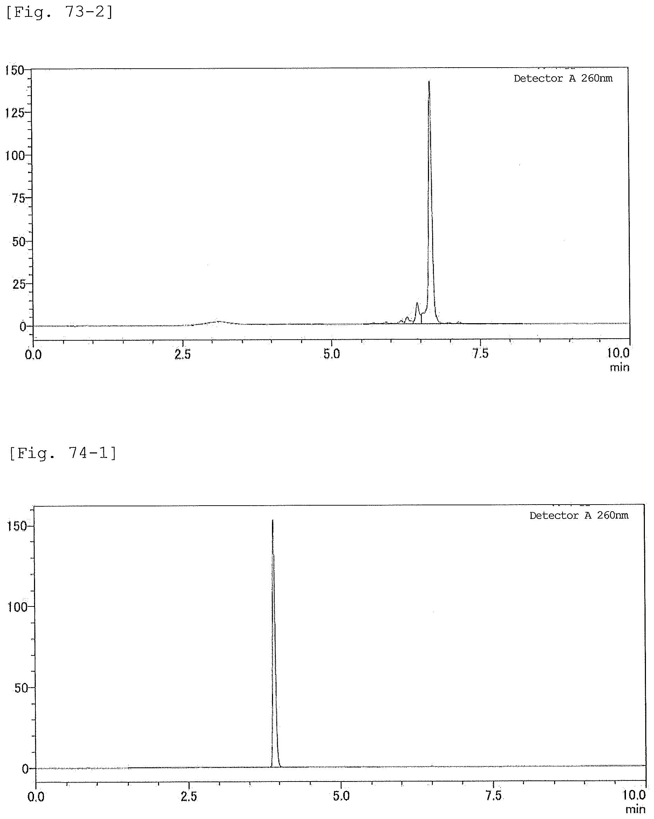

   Fig. 73-2 is an RP-HPLC chart of ESB2.2-20-C12 after purification.
[Fig. 74-1]
   Fig. 74-1 is an RP-HPLC chart of ESB2.2-16-C16 after purification.
[Fig. 74-2]

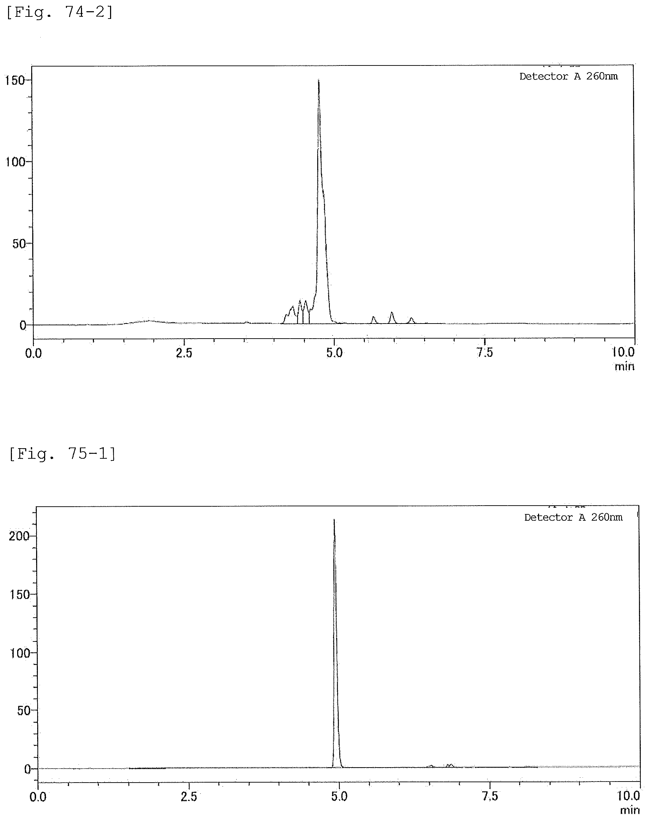

   Fig. 74-2 is an RP-HPLC chart of ESB2.2-20-C16 after purification.
[Fig. 75-1]
   Fig. 75-1 is an RP-HPLC chart of ESB2.2-16-C19 after purification.
[Fig. 75-2]

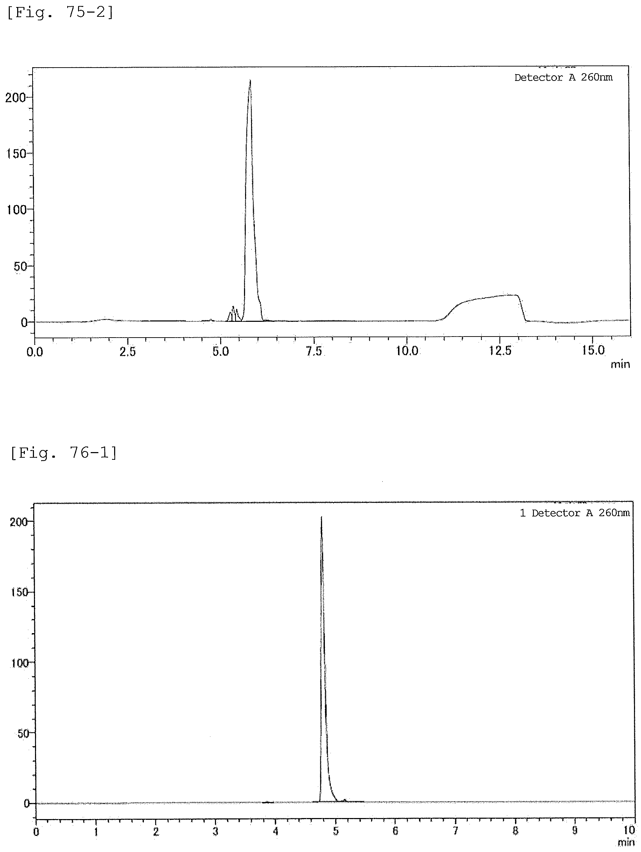

   Fig. 75-2 is an RP-HPLC chart of ESB2.2-20-C19 after purification.
[Fig. 76-1]
   Fig. 76-1 is an RP-HPLC chart of ESB2.2-16-EGS after purification.
[Fig. 76-2]

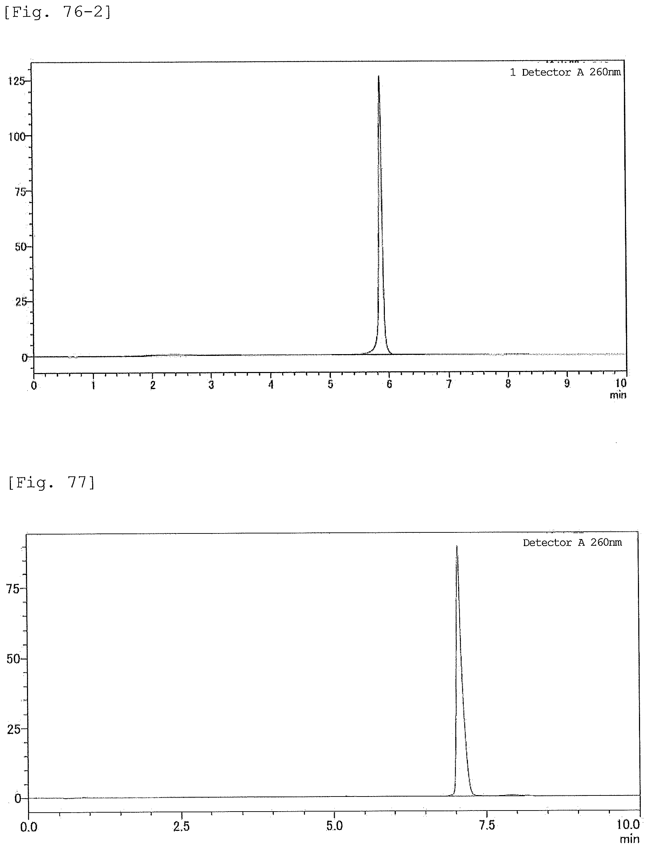

   Fig. 76-2 is an RP-HPLC chart of ESB2.2-20-EGS after purification.
[Fig. 77]
   Fig. 77 is an RP-HPLC chart of ESB2.2-16-C6O2 after purification.
[Fig. 78-1]

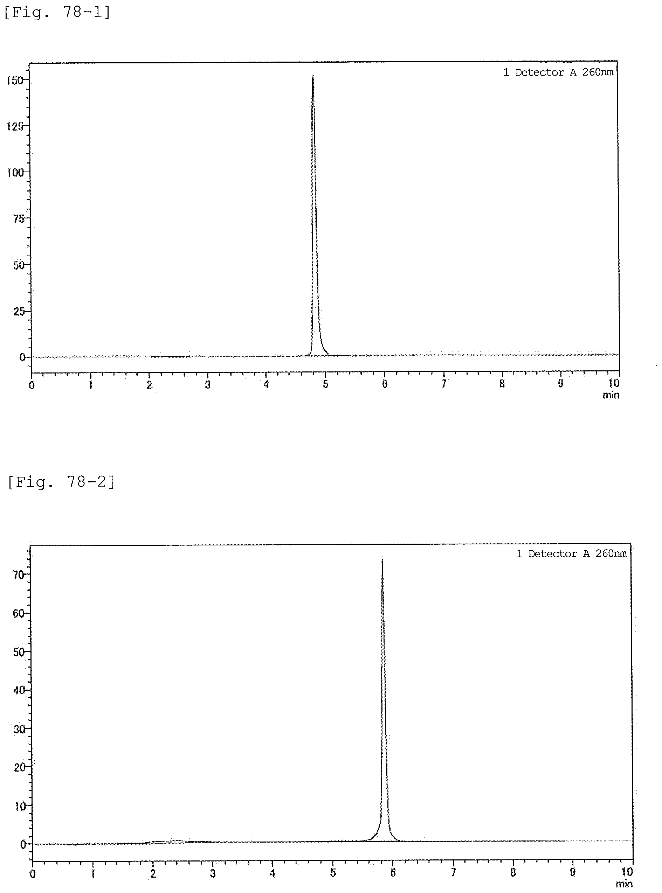

   Fig. 78-1 is an RP-HPLC chart of ESB2.2-16-PEG5 after purification.
[Fig. 78-2]
   Fig. 78-2 is an RP-HPLC chart of ESB2.2-20-PEG5 after purification.
[Fig. 79-1]

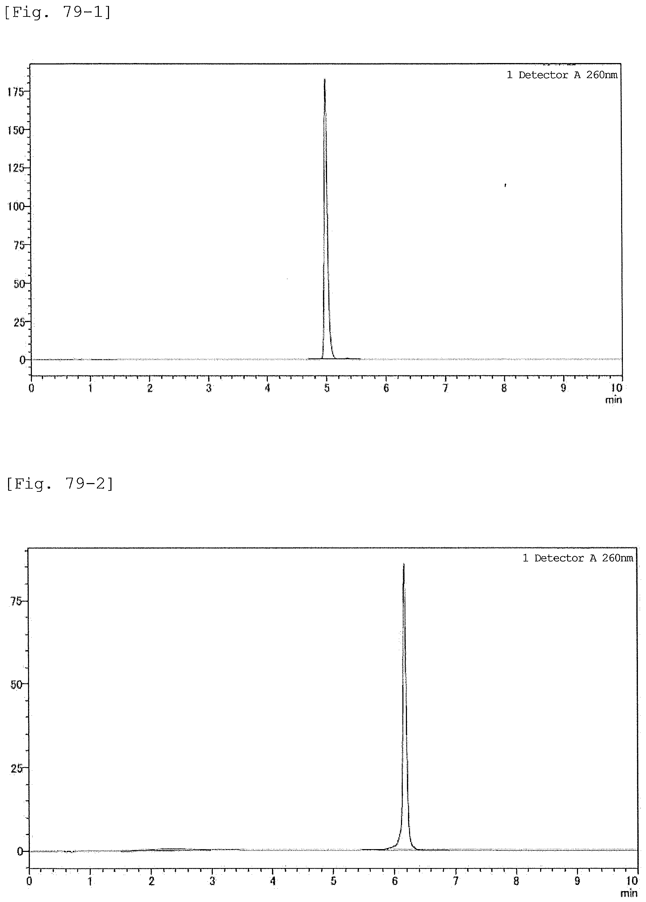

   Fig. 79-1 is an RP-HPLC chart of ESB2.2-16-PEG6 after purification.
[Fig. 79-2]
   Fig. 79-2 is an RP-HPLC chart of ESB2.2-20-PEG6 after purification.
[Fig. 80-1]

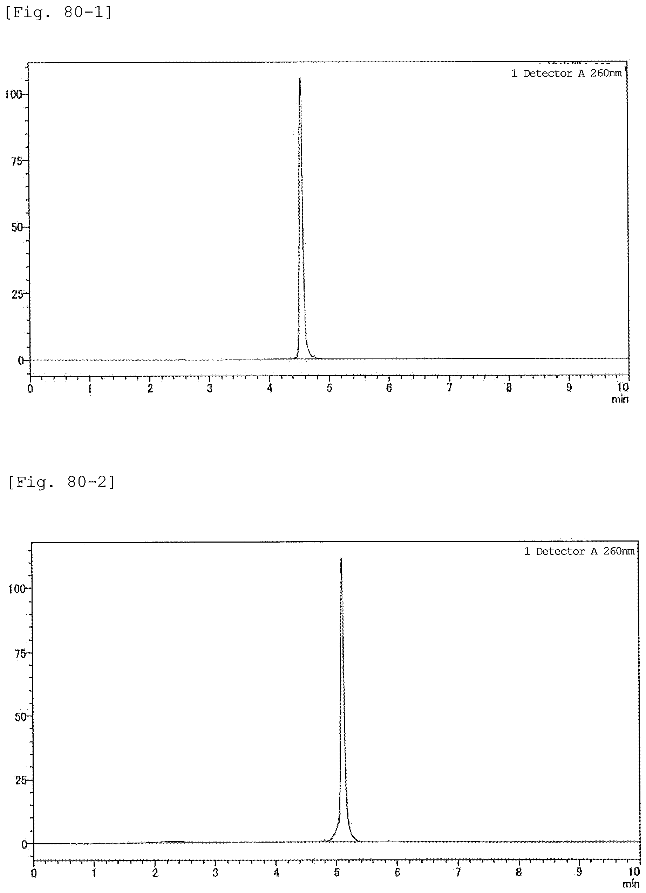

   Fig. 80-1 is an RP-HPLC chart of ESB2.2-16-DST after purification.
[Fig. 80-2]
   Fig. 80-2 is an RP-HPLC chart of ESB2.2-20-DST after purification.
[Fig. 81-1]

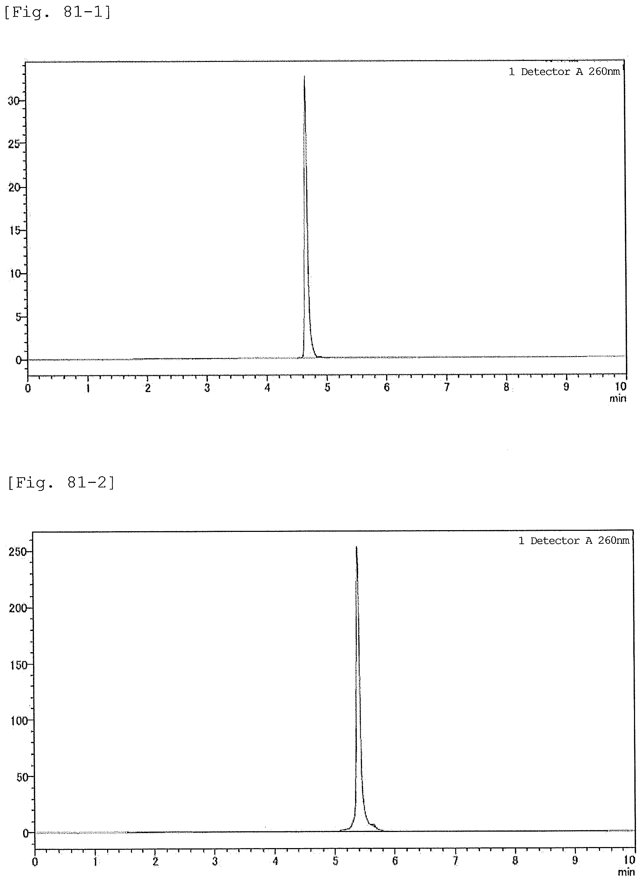

   Fig. 81-1 is an RP-HPLC chart of ESB2.2-16-trans-3-hexene after purification.
[Fig. 81-2]
   Fig. 81-2 is an RP-HPLC chart of ESB2.2-20-trans-3-hexene after purification.
[Fig. 82-1]

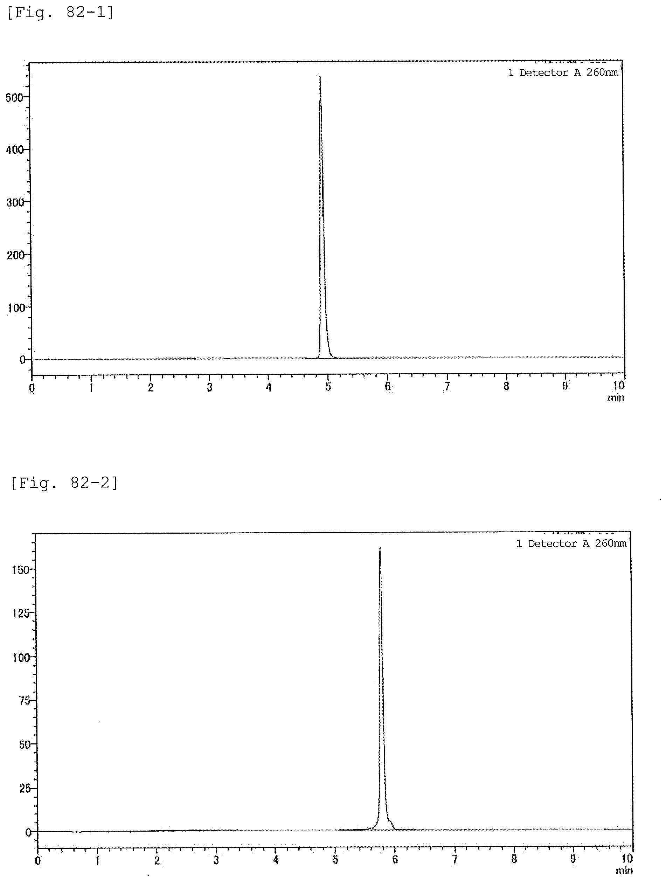

   Fig. 82-1 is an RP-HPLC chart of ESB2.2-16-PP after purification.
[Fig. 82-2]
   Fig. 82-2 is an RP-HPLC chart of ESB2.2-20-PP after purification.
[Fig. 83-1]

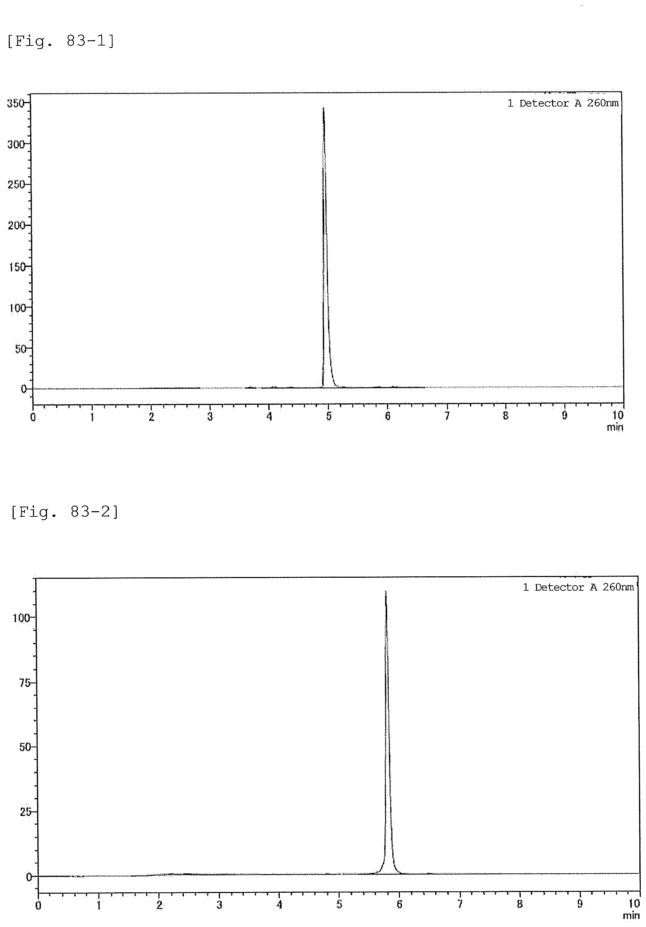

   Fig. 83-1 is an RP-HPLC chart of ESB2.2-16-MP after purification.
[Fig. 83-2]
   Fig. 83-2 is an RP-HPLC chart of ESB2.2-20-MP after purification.
[Fig. 84-1]

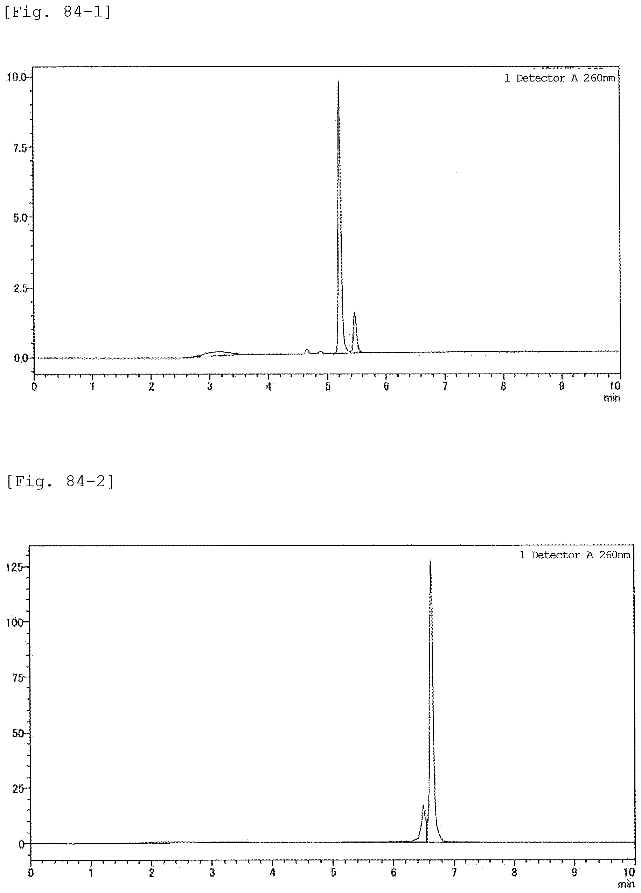

   Fig. 84-1 is an RP-HPLC chart of ESB2.2-16-adamantane after purification.
[Fig. 84-2]
   Fig. 84-2 is an RP-HPLC chart of ESB2.2-20-adamantane after purification.
[Fig. 85-1]

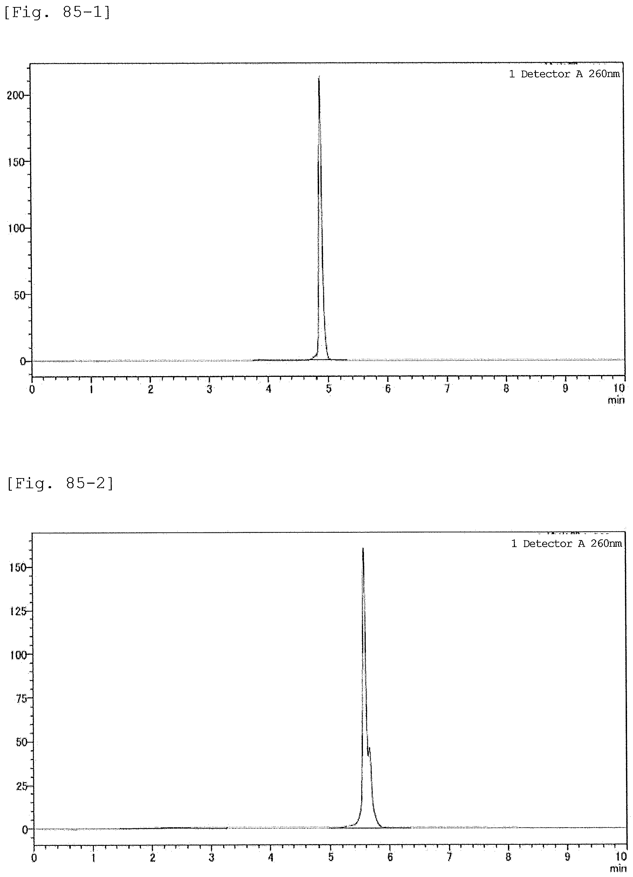

   Fig. 85-1 is an RP-HPLC chart of ESB2.2-16-pyridine after purification.
[Fig. 85-2]
   Fig. 85-2 is an RP-HPLC chart of ESB2.2-20-pyridine after purification.
[Fig. 86-1]

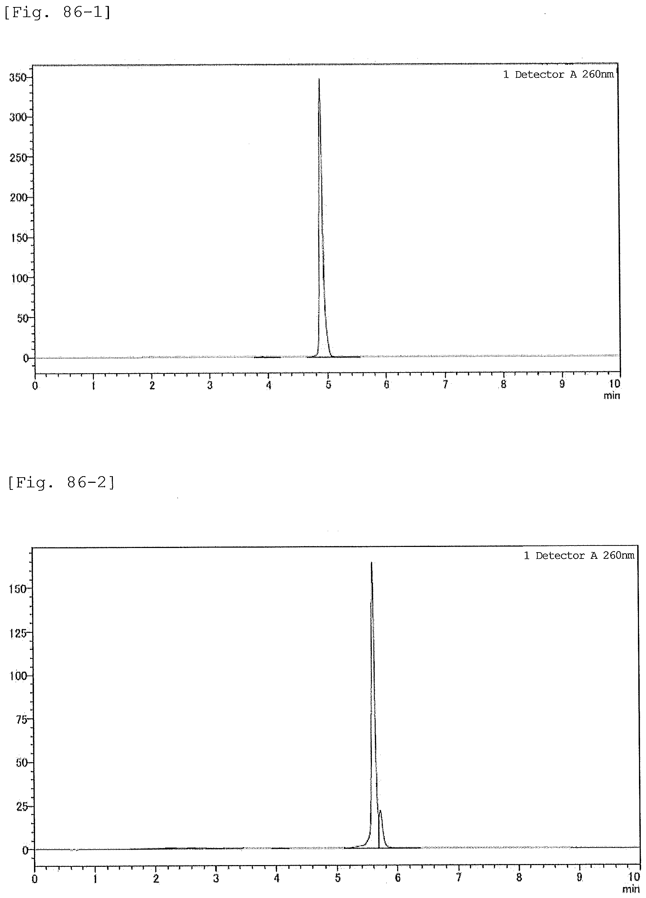

   Fig. 86-1 is an RP-HPLC chart of ESB2.2-16-furan after purification.
[Fig. 86-2]
   Fig. 86-2 is an RP-HPLC chart of ESB2.2-20-furan after purification.
[Fig. 87]

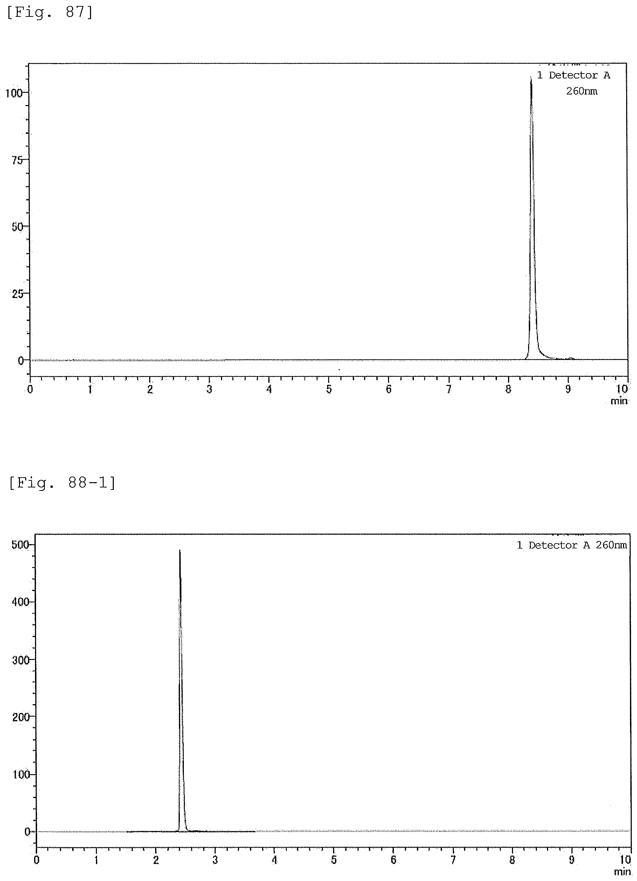

   Fig. 87 is an RP-HPLC chart of ESB2.2-16-134-72 after purification.
[Fig. 88-1]
   Fig. 88-1 is an RP-HPLC chart of ESB2.2-16-DSP after purification.
[Fig. 88-2]

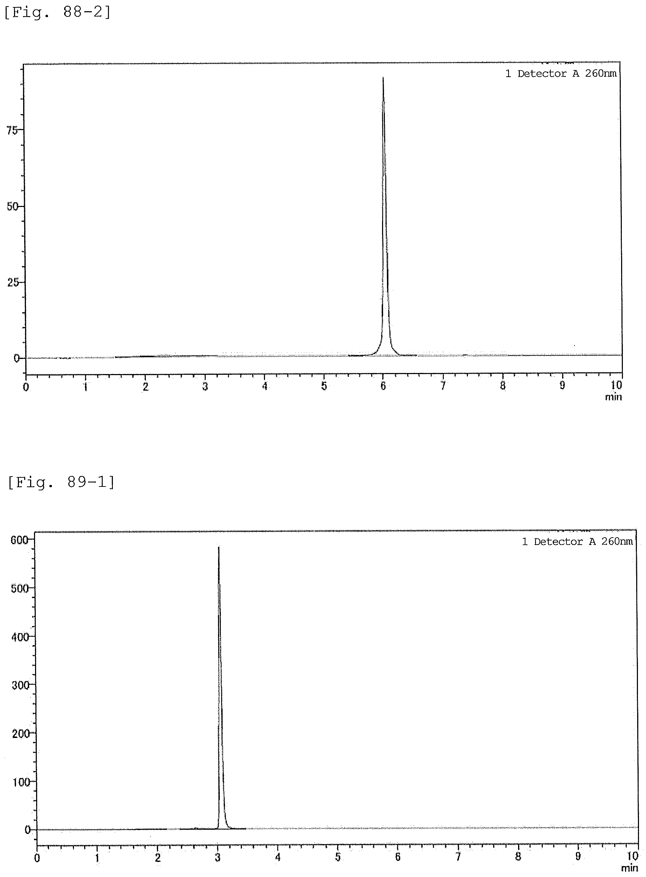

   Fig. 88-2 is an RP-HPLC chart of ESB2.2-20-DSP after purification.
[Fig. 89-1]
   Fig. 89-1 is an RP-HPLC chart of ESB2.2-16-DSH after purification.
[Fig. 89-2]

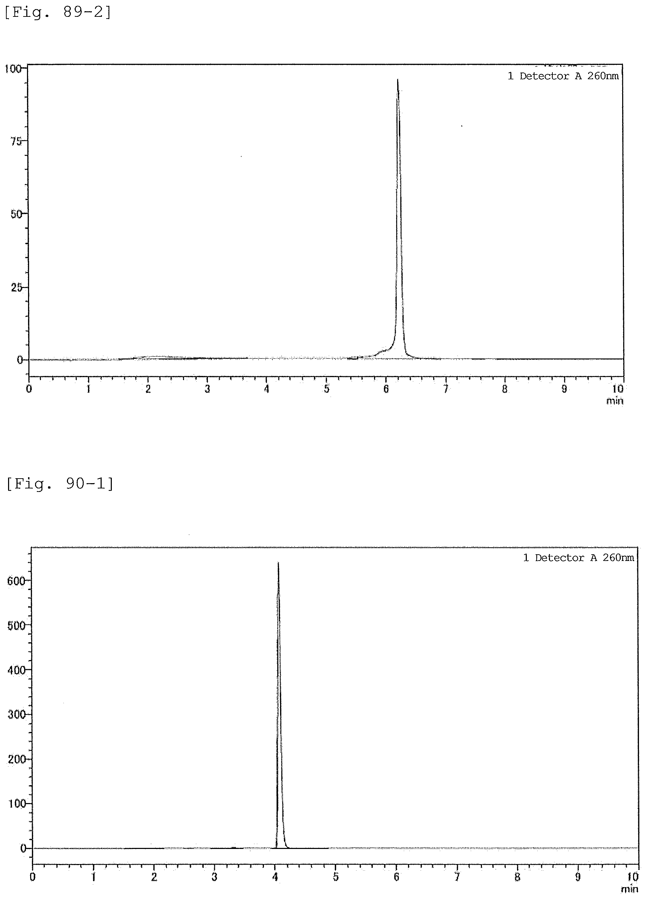

   Fig. 89-2 is an RP-HPLC chart of ESB2.2-20-DSH after purification.
[Fig. 90-1]
   Fig. 90-1 is an RP-HPLC chart of ESB2.2-16-DSO after purification.
[Fig. 90-2]

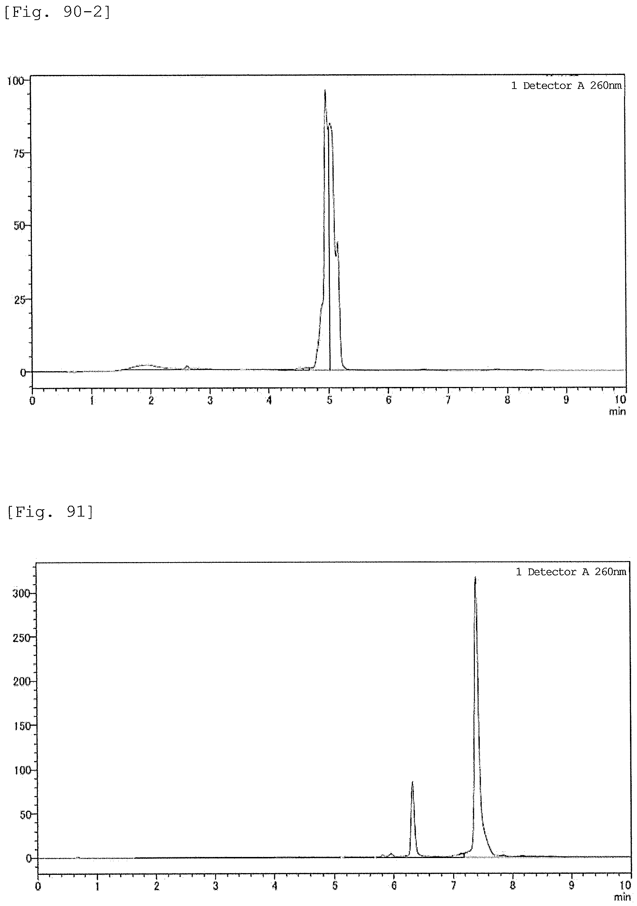

   Fig. 90-2 is an RP-HPLC chart of ESB2.2-20-DSO after purification.
[Fig. 91]
   Fig. 91 is an RP-HPLC chart of ESB2.2-20-NH2 after purification.
[Fig. 92]

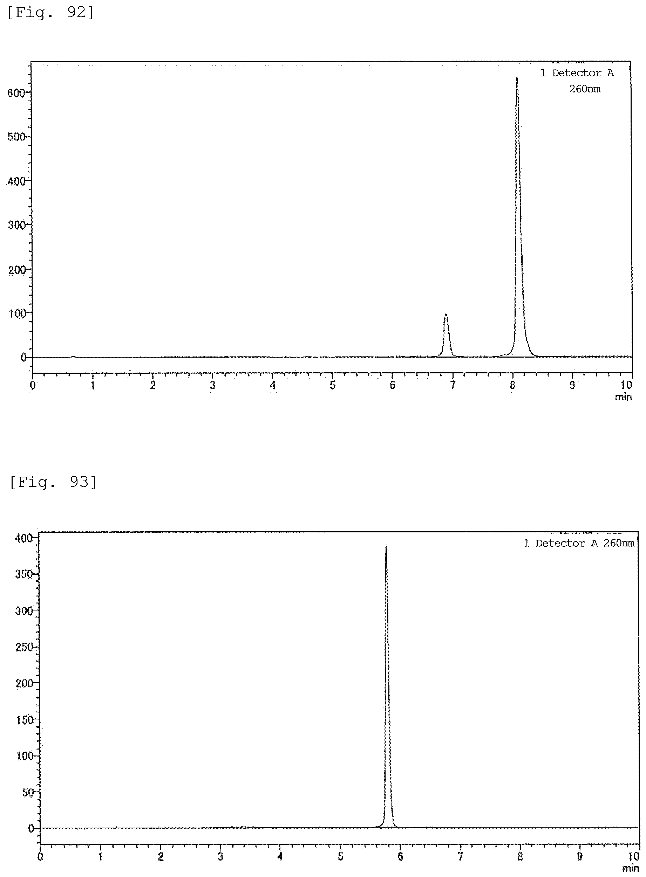

   Fig. 92 is an RP-HPLC chart of ESB2.2-20-AEC after purification.
[Fig. 93]
   Fig. 93 is an RP-HPLC chart of ESB2.2-20-asNH2-ssAEM after purification.
[Fig. 94-1]

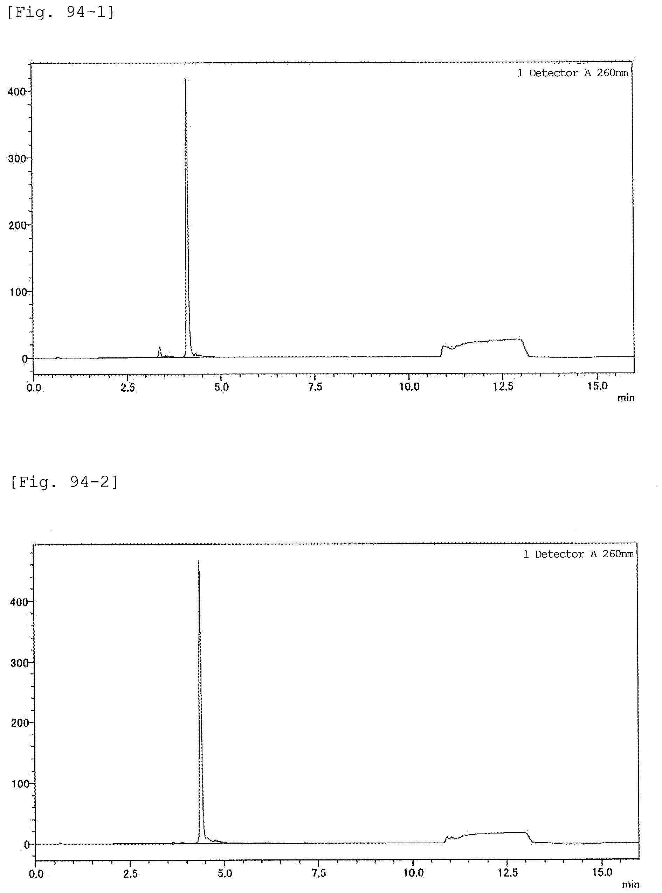

   Fig. 94-1 is an RP-HPLC chart of AEM28-azide after desalting.
[Fig. 94-2]
   Fig. 94-2 is an RP-HPLC chart of AEM8-alkyne after desalting.
[Fig. 94-3]

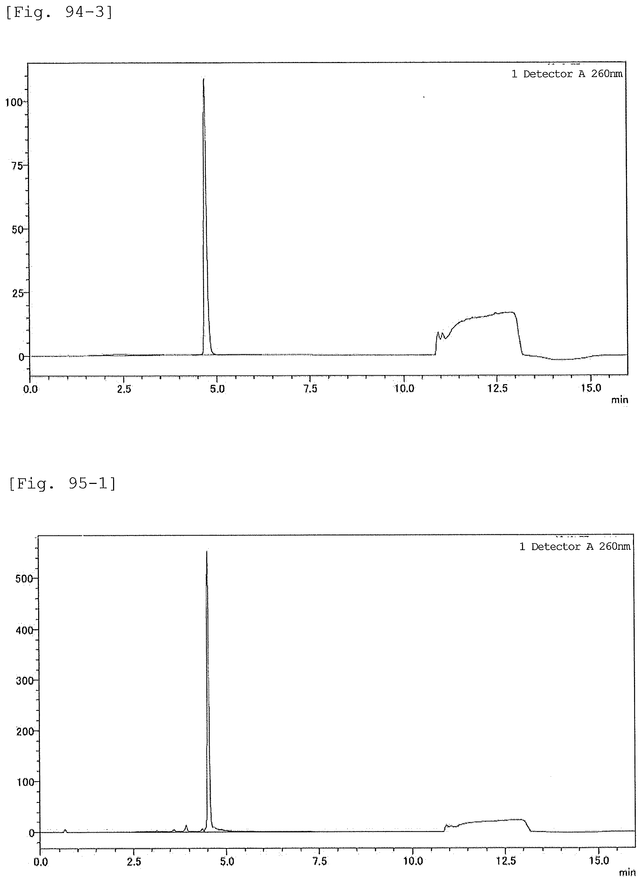

   Fig. 94-3 is an RP-HPLC chart of ESB2.2-16-141-152 after purification.
[Fig. 95-1]
   Fig. 95-1 is an RP-HPLC chart of ESB2.2-20 antisense strand azide after desalting.
[Fig. 95-2]

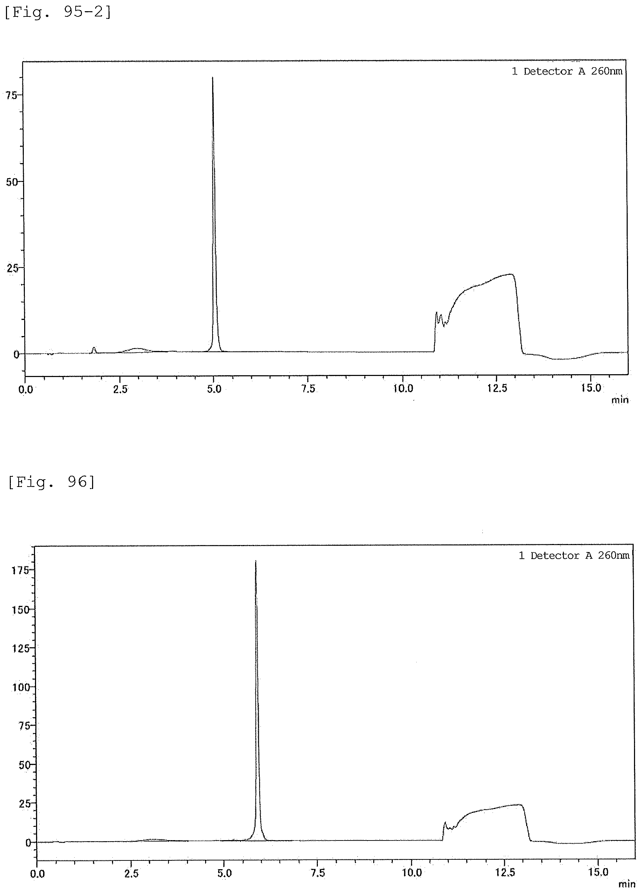

   Fig. 95-2 is an RP-HPLC chart of ESB2.2-20-141-178 after purification.
[Fig. 96]
   Fig. 96 is an RP-HPLC chart of ESB2.2-20-141-171-1 after purification.
[Fig. 97]

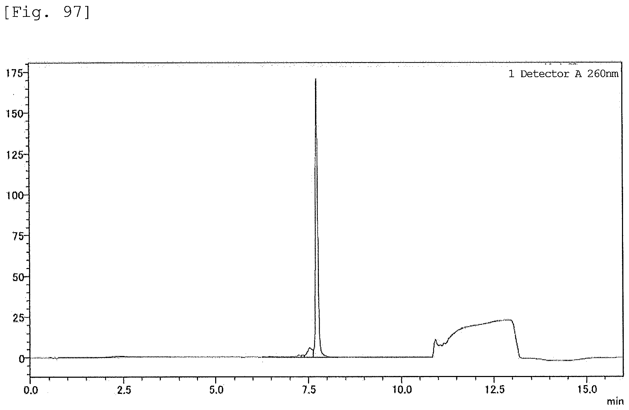

   Fig. 97 is an RP-HPLC chart of ESB2.2-20-141-171-2 after purification.
[Fig. 98]

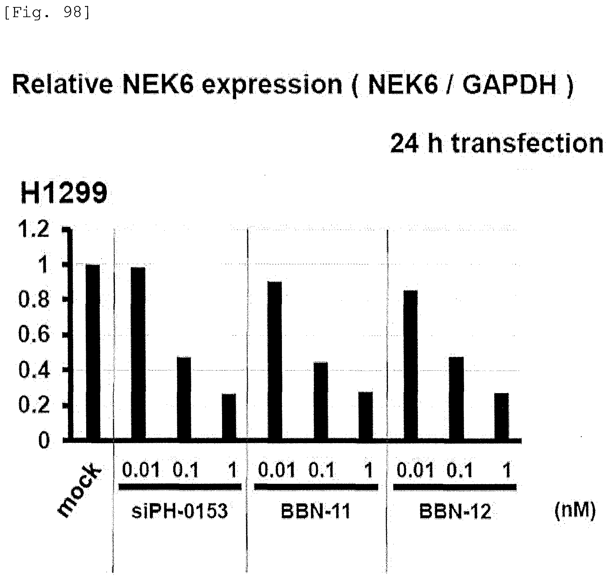

   Fig. 98 is a graph showing relative values of NEK6 gene expression levels in cells transfected with siPH-0153, BBN-11, and BBN-12, with the expression level in control (mock) cells measured by RT-PCR being 1.
[Fig. 99]

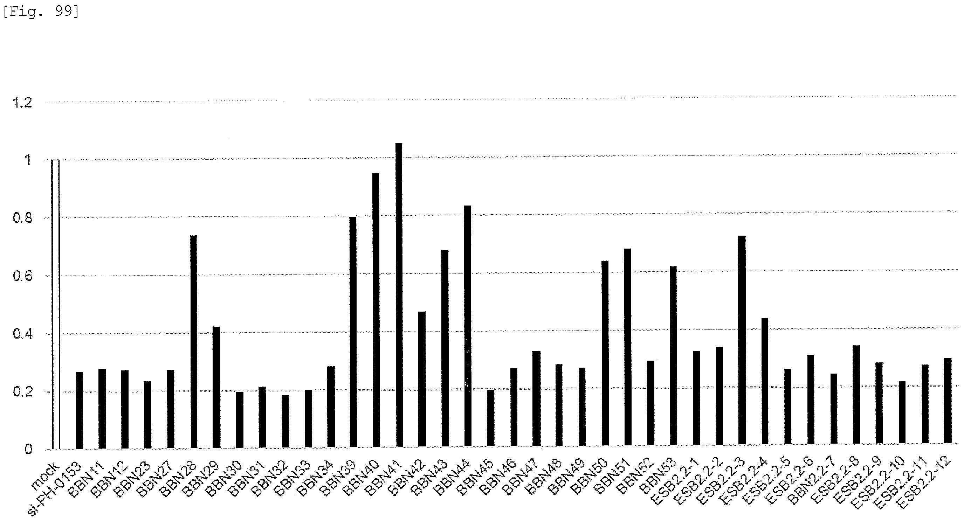

   Fig. 99 is a graph showing relative values of NEK6 gene expression levels in cells transfected with each nucleic acid molecule (final concentration 1 nmol/L), with the expression level in control (mock) cells measured by RT-PCR being 1.
[Fig. 100]

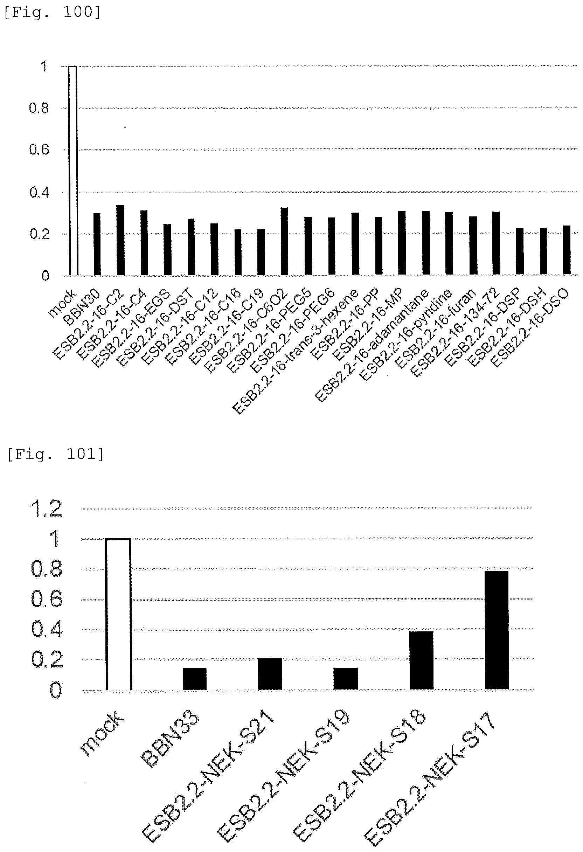

   Fig. 100 is a graph showing relative values of NEK6 gene expression levels in cells transfected with each nucleic acid molecule (final concentration 10 nmol/L), with the expression level in control (mock) cells measured by RT-PCR being 1.
[Fig. 101]
   Fig. 101 is a graph showing relative values of NEK6 gene expression levels in cells transfected with each nucleic acid molecule (final concentration 10 nmol/L), with the expression level in control (mock) cells measured by RT-PCR being 1.
[Fig. 102]

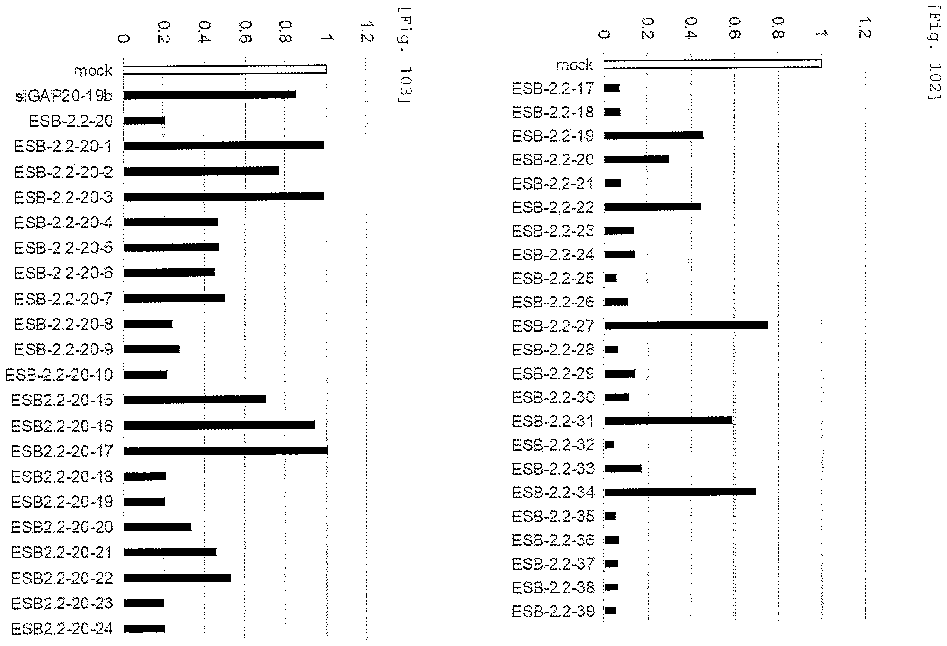

   Fig. 102 is a graph showing relative values of GAPDH gene expression levels in cells transfected with each nucleic acid molecule (final concentration 10 nmol/L), with the expression level in control (mock) cells measured by RT-PCR being 1.
[Fig. 103]
   Fig. 103 is a graph showing relative values of GAPDH gene expression levels in cells transfected with each nucleic acid molecule (final concentration 10 nmol/L), with the expression level in control (mock) cells measured by RT-PCR being 1.
[Fig. 104]

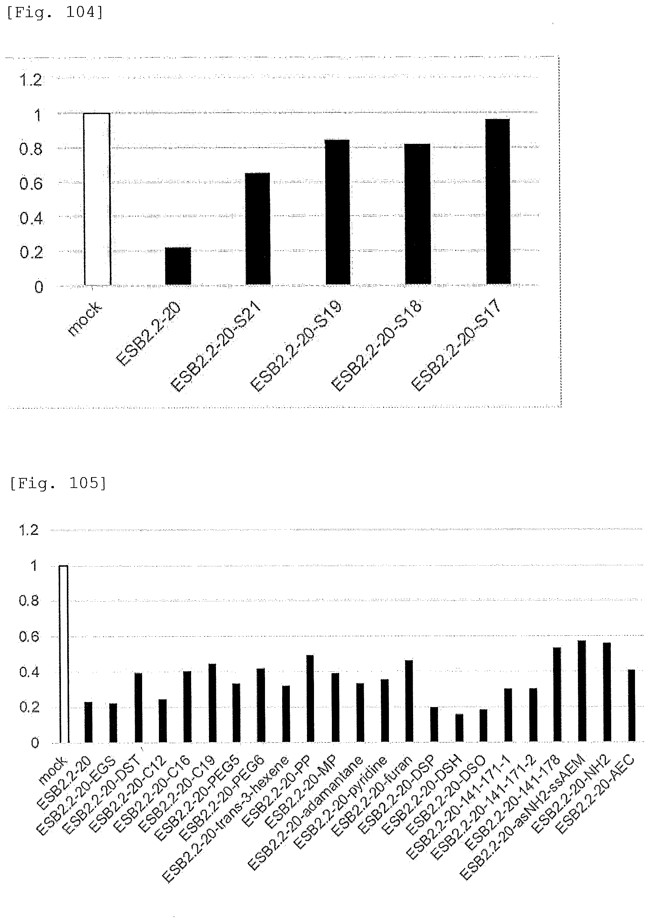

   Fig. 104 is a graph showing relative values of GAPDH gene expression levels in cells transfected with each nucleic acid molecule (final concentration 10 nmol/L), with the expression level in control (mock) cells measured by RT-PCR being 1.
[Fig. 105]
   Fig. 105 is a graph showing relative values of GAPDH gene expression levels in cells transfected with each nucleic acid molecule (final concentration 10 nmol/L), with the expression level in control (mock) cells measured by RT-PCR being 1.
[Fig. 106]

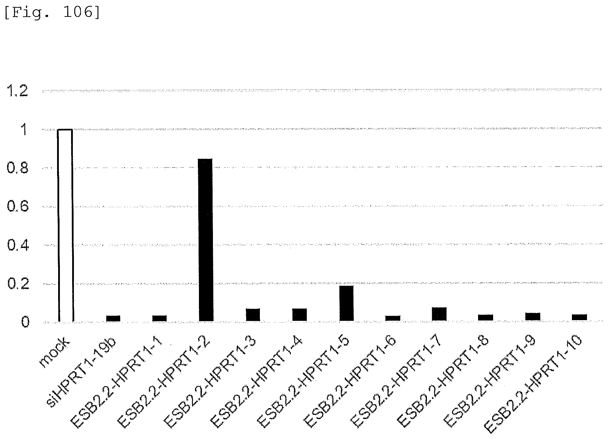

   Fig. 106 is a graph showing relative values of HPRT1 gene expression levels in cells transfected with each nucleic acid molecule (final concentration 10 nmol/L), with the expression level in control (mock) cells measured by RT-PCR being 1.
[Fig. 107]

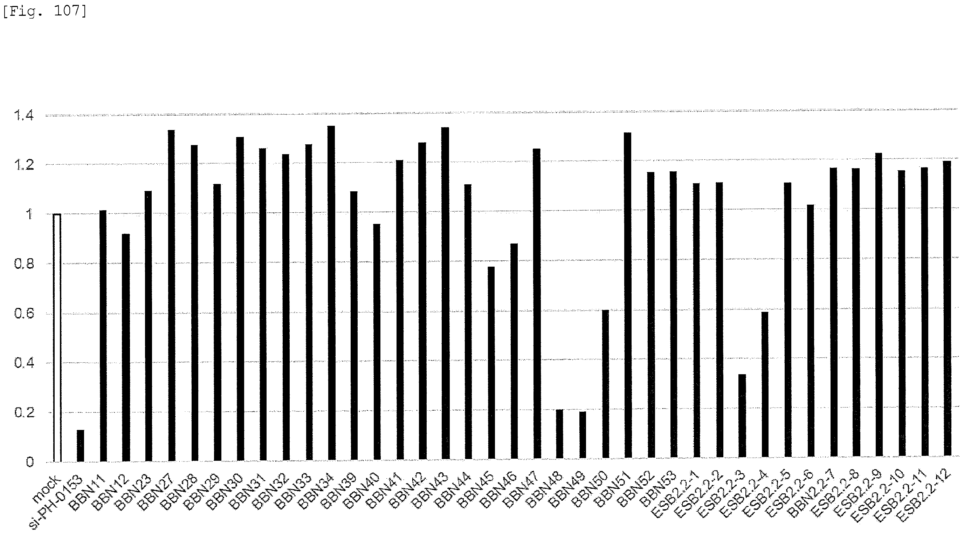

   Fig. 107 is a graph showing hRluc gene relative expression levels when cells transfected with a reporter plasmid for measuring the activity of the sense strand of a nucleic acid molecule targeting NEK6 were transfected with a nucleic acid molecule targeting the NEK6 gene (final concentration 1 nmol/L).
[Fig. 108]

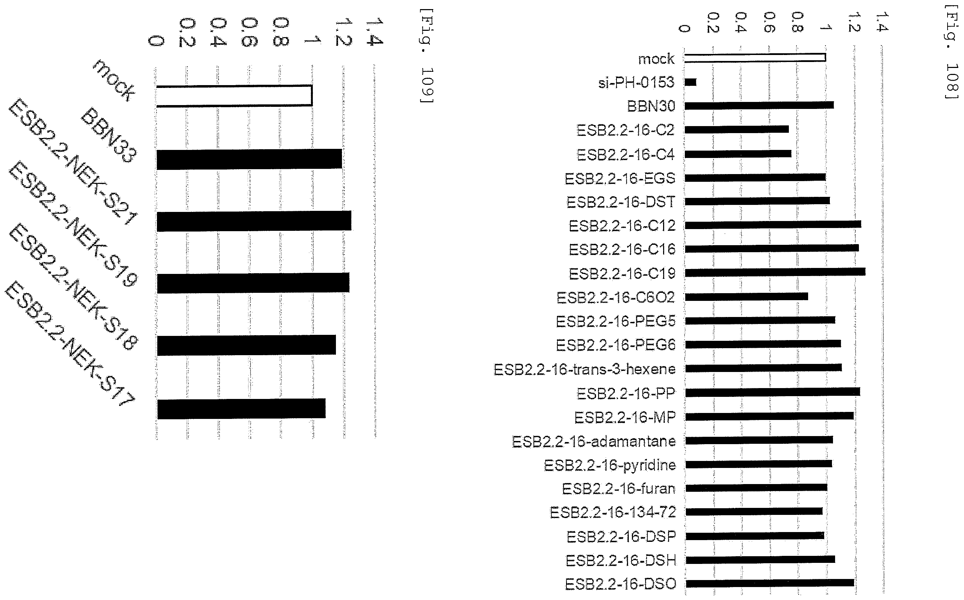

   Fig. 108 is a graph showing hRluc gene relative expression levels when cells transfected with a reporter plasmid for measuring the activity of the sense strand of a nucleic acid molecule targeting NEK6 were transfected with a nucleic acid molecule targeting the NEK6 gene (final concentration 10 nmol/L).
[Fig. 109]
   Fig. 109 is a graph showing hRluc gene relative expression levels when cells transfected with a reporter plasmid for measuring the activity of the sense strand of a nucleic acid molecule targeting NEK6 were transfected with a nucleic acid molecule targeting the NEK6 gene (final concentration 10 nmol/L).
[Fig. 110]

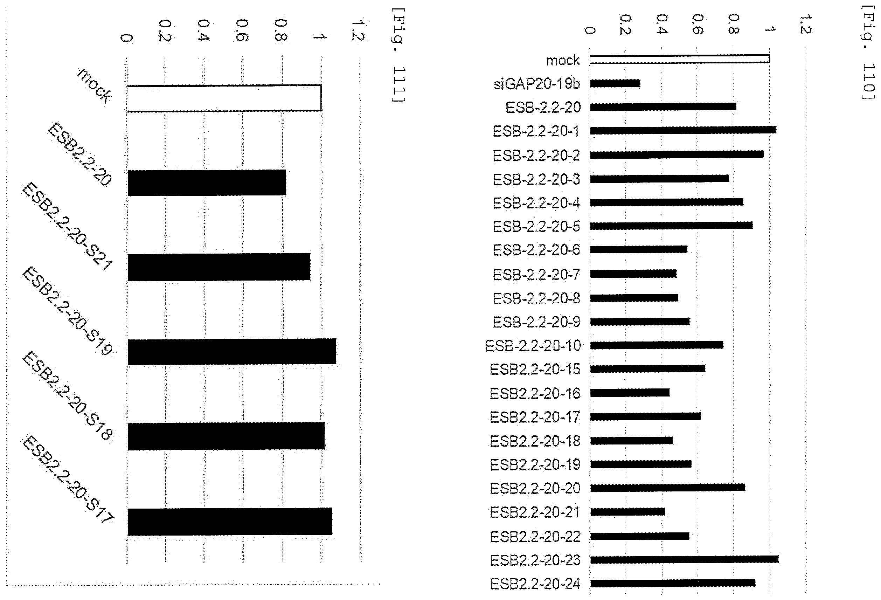

   Fig. 110 is a graph showing hRluc gene relative expression levels when cells transfected with a reporter plasmid for measuring the activity of the sense strand of a nucleic acid molecule targeting GAPDH were transfected with a nucleic acid molecule targeting the GAPDH gene (final concentration 10 nmol/L).
[Fig. 111]
   Fig. 111 is a graph showing hRluc gene relative expression levels when cells transfected with a reporter plasmid for measuring the activity of the sense strand of a nucleic acid molecule targeting GAPDH were transfected with a nucleic acid molecule targeting the GAPDH gene (final concentration 10 nmol/L).
[Fig. 112]

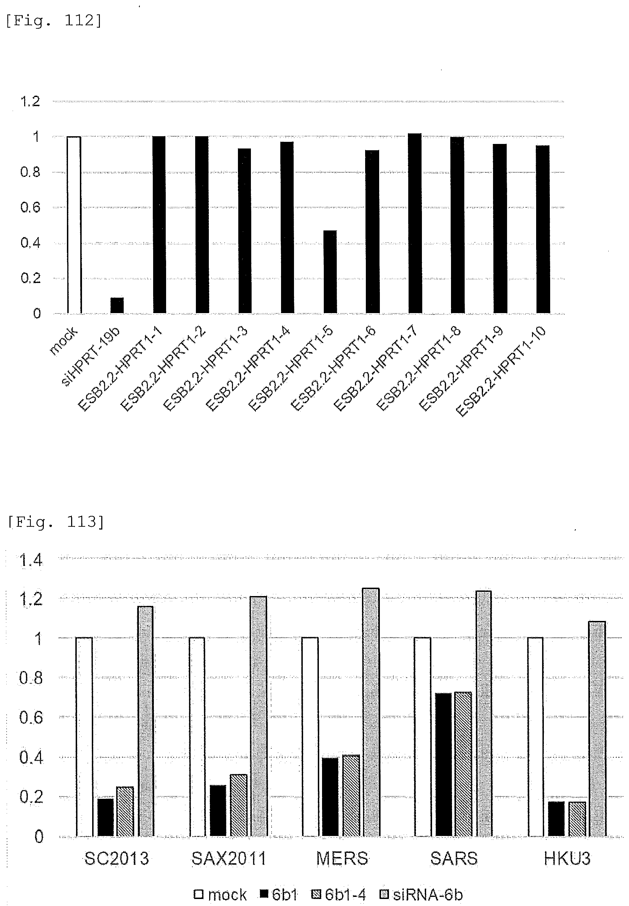

   Fig. 112 is a graph showing hRluc gene relative expression levels when cells transfected with a reporter plasmid for measuring the activity of the sense strand of a nucleic acid molecule targeting HPRT1 were transfected with a nucleic acid molecule targeting the HPRT1 gene (final concentration 10 nmol/L).
[Fig. 113]
   Fig. 113 is a graph showing the results of a gene expression-suppressing effect obtained by preparing a sugar cross-linked nucleic acid molecule targeting coronavirus and examining its suppressing effect in a reporter assay system.

### [Description of Embodiments]

### (I) the nucleic acid molecule of the present invention

The present invention provides a nucleic acid molecule represented by the following formula: 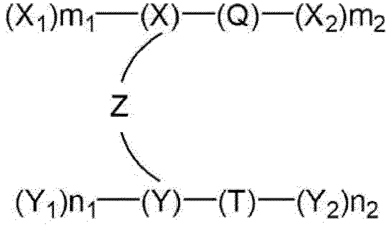
wherein X, Y, X₁, Y₁, X₂, and Y₂ are each independently an optionally modified ribonucleotide residue or an optionally modified deoxyribonucleotide residue,
T and Q are a sequence consisting of 14 to 30 consecutive, optionally-modified ribonucleotide residues and complementary to the target nucleic acid sequence, and a ribonucleotide sequence complementary thereto (one of which is a sequence complementary to the target nucleic acid sequence, and the other is a sequence complementary thereto),
Z is a linker connecting the 2'-position of the sugar moiety of (X) and the 2'-position of the sugar moiety of (Y);
m₁ and m₂ are each independently an integer of 0 to 5; and n₁ and n₂ are each independently an integer of 0 to 5.

In the present specification, the ribonucleotide sequence complementary to the target nucleic acid sequence is in an "antisense" relationship if the target nucleic acid sequence is "sense", and thus to be also simply referred to as an "antisense strand sequence". On the other hand, the ribonucleotide sequence complementary to the antisense strand sequence is in a homologous relationship with the target nucleic acid sequence, and thus to be also simply referred to as a "sense strand sequence".

In the above-mentioned formula, when Q or (X)-(Q) is a sense strand sequence, (X₁)ₘ₁-(X)-(Q)-(X₂)ₘ₂ as a sense strand and (Y₁)ₙ₁-(Y)-(T)-(Y₂)ₙ₂ as an antisense strand are crosslinked by a linker Z. When Q or (X)-(Q) is an antisense strand sequence, (X₁)ₘ₁-(X)-(Q)-(X₂)ₘ₂ as an antisense strand and (Y₁)ₙ₁-(Y)-(T)-(Y₂)ₙ₂ as a sense strand are crosslinked by Z. The present invention provides a nucleic acid molecule in which a sense strand and an antisense strand are crosslinked. Preferably, (Q) or (X)-(Q) is a sense strand sequence and (T) or (Y)-(T) is an antisense strand sequence.

The nucleic acid molecule of the present invention is characterized in that when Q is a sense strand sequence, the 2'-position of the sugar moiety of (X) which is a ribonucleotide residue in proximity to the sense strand sequence (Q) and the 2'-position of the sugar moiety of (Y) which is a ribonucleotide residue in proximity to the antisense strand sequence (T) are connected by linker Z; and when Q is an antisense strand sequence, the 2'-position of the sugar moiety of (X) which is a ribonucleotide residue in proximity to the antisense strand sequence (Q) and the 2'-position of the sugar moiety of (Y) which is a ribonucleotide residue in proximity to the sense strand sequence (T) are connected by linker Z.

In addition, it is characterized in that when (X)-(Q) is a sense strand sequence, the 2'-position of the sugar moiety of (X) of the sense strand sequence (X)-(Q) and the 2'-position of the sugar moiety of (Y) of the antisense strand sequence (Y)-(T) are connected by linker Z; and when (X)-(Q) is an antisense strand sequence, the 2'-position of the sugar moiety of (X) of the antisense strand sequence (X)-(Q) and the 2'-position of the sugar moiety of (Y) of the sense strand sequence (Y)-(T) are connected by linker Z.

The strand bias rule in the present invention is that, in the case of siRNA, since the strand bias is towards the strand with the 5'-end on the loose base pairing (i.e., AU-rich) side, the siRNA sequence is selected such that the 5'-end side of the antisense strand sequence is AU-rich and the 3'-end side thereof is GC-rich in conventional siRNA design. However, in the nucleic acid molecule of the present invention, a wide range of sequences, including not only those that meet the above-mentioned conditions but also those that do not, can be selected as targets by cross-linking the sense strand with the antisense strand. In conventional siRNA design, siRNA sequences in which genes other than the target gene contain sequences complementary to the sense strand sequence were excluded from the candidate sequences since the sense strand sequence is retained in RISC and may exhibit off-target effects. In the case of the nucleic acid molecule of the present invention, off-target effects due to the sense strand are highly suppressed, and thus the range of sequences that can be selected as target sequences is further widened.

The "antisense strand sequence" in the nucleic acid molecule of the present invention may be any ribonucleotide sequence as long as it is a sequence complementary to any target nucleic acid sequence. As used herein, the "complementary sequence" includes not only sequences completely complementary to a target nucleic acid sequence (i.e., hybridizes without a mismatch), but also a sequence containing mismatch of 1 to several nucleotides, preferably 1 or 2 nucleotides, as long as it can hybridize with a nucleic acid containing a target sequence under, for example, physiological conditions of mammalian cells. For example, sequences having 90% or more, preferably 95% or more, 97% or more, 98% or more, 99% or more, identity to the complete complementary strand sequence of the target nucleic acid sequence in the target gene can be mentioned. The "identity of nucleotide sequence" in the present invention can be calculated using homology calculation algorithm NCBI BLAST (National Center for Biotechnology Information Basic Local Alignment Search Tool) under the following conditions (expectancy =10; gap allowed; filtering=ON; match score=1; mismatch score=-3). Complementarity in individual bases is not limited to forming Watson-Crick base pairs with the target bases, but also includes forming Hoogsteen base pairs and Wobble base pairs.

Alternatively, the "complementary sequence" is a nucleotide sequence that hybridizes with the target nucleic acid sequence under stringent conditions. As used herein, the "stringent conditions" refers to, for example, the conditions described in Current Protocols in Molecular Biology, John Wiley & Sons,6.3.1-6.3.6, 1999, for example, hybridization in 6×SSC (sodium chloride/sodium citrate)/45°C followed by washing once or more in 0.2×SSC/0.1% SDS/50 - 65°C, and the like. Those of ordinary skill in the art can appropriately select hybridization conditions that achieve stringency equivalent thereto.

The "sense strand sequence" and "antisense strand sequence" in the nucleic acid molecule of the present invention are preferably complementary sequences. However, they may be sequences containing mismatch of 1 to several nucleotides, preferably 1 or 2 nucleotides. As used herein, the "complementary sequence" is as defined above for the complementarity of the antisense sequence to the target nucleic acid sequence.

The lengths of the antisense strand sequence and sense strand sequence are not particularly limited as long as they can specifically hybridize with the target nucleic acid sequence and suppress expression of the gene containing the target nucleic acid sequence, and the length of each may be the same or different. The length of them is, for example, 14 to 30 nucleotides, preferably 15 to 27 nucleotides, more preferably 16 to 23 nucleotides, further preferably 19 to 23 nucleotides. The length of the antisense strand sequence is preferably 19 to 23 nucleotides, further preferably 21 to 23 nucleotides. The length of the sense strand sequence is preferably 17 to 23 nucleotides, further preferably 19 to 23 nucleotides.

The antisense strand and sense strand may have (X₁)ₘ₁, (X₂)ₘ₂, (Y₁)ₙ₁, or (Y₂)ₙ₂ in the above-mentioned formula as an additional ribonucleotide at the 5'-terminal and/or 3'-terminal. In one embodiment in which the sense strand sequence is (Q) and the antisense strand sequence is (T), the number (m1) of ribonucleotide residue (X₁) and the number (m2) of (X₂) to be added to the terminals of the sense strand sequence (Q) is 0 to 5, and the number (n1) of ribonucleotide residue (Y₁) and the number (n2) of (Y₂) to be added to the terminals of the antisense strand sequence (T) is also 0 to 5. In one preferred embodiment, they are nucleic acid molecules corresponding to the compounds produced in the following Examples, wherein m1=0, m2=0 or 2, n¹=1, and n²=0.

As the constitutional unit of the nucleic acid molecule of the present invention, ribonucleotide residues can be mentioned. The nucleotide residue may be, for example, modified or non-modified. (Since the modification includes substitution of the 2'-position OH group by a hydrogen atom, a deoxyribonucleotide residue may also be contained as a constitutional unit of the nucleic acid molecule of the present invention. In this case, it is indicated as a ribonucleotide residue or a deoxyribonucleotide residue. Therefore, in the following explanation of the modified ribonucleotide residue, it is simply referred to as "nucleotide residue" at times). The nucleic acid molecule of the present invention containing, for example, a modified ribonucleotide residue may show enhanced nuclease resistance and improved stability. Furthermore, the nucleic acid molecule of the present invention may further contain, for example, a non-nucleotide residue in addition to the aforementioned nucleotide residue.

In the nucleic acid molecule of the present invention, the constitutional unit of the regions other than linker (Z) is preferably a nucleotide residue. Each region is composed of, for example, any of the following residues (1 to (3):
(1) an unmodified nucleotide residue(s)
(2) a modified nucleotide residue(s)
(3) an unmodified nucleotide residue(s) and a modified nucleotide residue(s).

In the nucleic acid molecule of the present invention, linker (Z) is preferably a non-nucleotide structure, more preferably a structure having an alkyl chain having an amide bond inside.

In the preferred embodiment, the sturture of 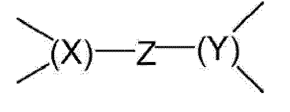 in the nucleic acid molecule of the present invention is 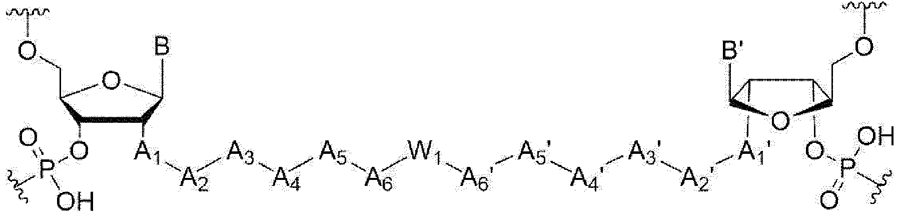 or

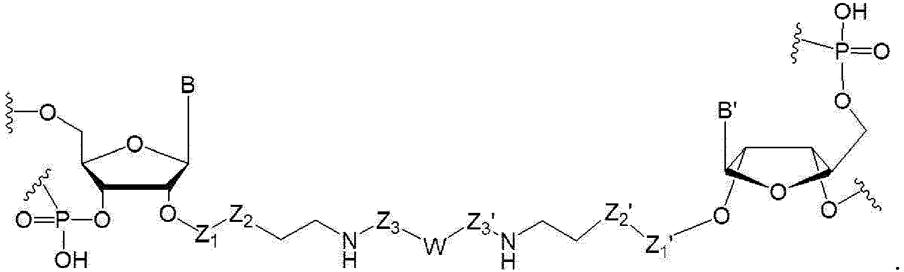

As already described, linker Z connects the 2'-position of the sugar moiety of (X) and the 2'-position of the sugar moiety of (Y).

In the above-mentioned formulas,
B and B' are each independently an atomic group having a nucleic acid base backbone,
A₁ and A₁' are each independently -O-, -NR^{1a}-, -S- or -CR^{1a}R^{1b}-(wherein R^{1a} and R^{1b} are each independently a hydrogen atom or an alkyl group having 1 - 10 carbon atoms),
A₂ and A₂' are each independently -CR^{2a}R^{2b}-, -CO-, an alkynyl group, an alkenyl group, or a single bond (wherein R^{2a} and R^{2b} are each independently a hydrogen atom or an alkyl group having 1 - 10 carbon atoms),
A₃ and A₃' are each independently -O- or -NR^{3a}-, -S-, -CR^{3a}R^{3b}- or a single bond (wherein R^{3a} and R^{3b} are each independently a hydrogen atom or an alkyl group having 1 - 10 carbon atoms),
A₄ and A₄' are each independently -(CR^{4a}R^{4b})n-, -(CR^{4a}R^{4b})n-ring D- (wherein ring D is an aryl group having 6 - 10 carbon atoms, a heteroaryl group having 2 - 10 carbon atoms, a cycloalkyl group having 4 - 10 carbon atoms, or a heterocycloalkyl group having 4 - 10 carbon atoms, R^{4a} and R^{4b} are each independently a hydrogen atom or an alkyl group having 1 - 10 carbon atoms) or a single bond,
As and A₅' are each independently -NR^{5a}- or a single bond
   (wherein R^{5a} is a hydrogen atom or an alkyl group having 1 - 10 carbon atoms),
A₆ and A₆' are each independently -(CR^{6a}R^{6b})n- or a single bond (wherein R^{6a} and R^{6b} are each independently a hydrogen atom or an alkyl group having 1 - 10 carbon atoms), and
W₁ is -(CR¹R²)n-, -CO-, -(CR¹R²)n-COO-(CR¹R²)n-COO-(CR¹R²)n, - (CR¹R²)n-O-(CR¹R²CR¹R^{2O})n-CH₂-, -(CR¹R²)n- ring D-(CR¹R²)n-, or - (CR¹R²)n-SS-(CR¹R²)n- (wherein ring D is an aryl group having 6 - 10 carbon atoms, a heteroaryl group having 2 - 10 carbon atoms, a cycloalkyl group having 4 - 10 carbon atoms, or a heterocycloalkyl group having 4 - 10 carbon atoms; R¹ and R², R^{1a} and R^{2a}, and R^{1b} and R^{2b} are each independently a hydrogen atom or an alkyl group having 1 - 10 carbon atoms).
Z₁ and Z₁' are each independently -CH₂- or -CO-,
Z₂ and Z₂' are each independently -O- or -NH-,
Z₃ and Z₃' are each -CO-, -CH₂-, or a single bond (absent), and W is -CR¹⁰R²⁰- or -CR^{10a}R^{20a}-N(R³⁰)-CR^{10b}R^{20b}-.

R¹⁰ and R²⁰, R^{10a} and R^{20a}, and R^{10b} and R^{20b} are each independently a hydrogen atom or an alkyl group having 1 - 10 carbon atoms, R³⁰ is -CO-R⁴⁰-, and R⁴⁰ is a hydrogen atom, an optionally substituted alkyl group having 1 - 20 carbon atoms, or an aryl group having 6 - 10 carbon atoms.

In the above-mentioned formulas, the "alkyl group having 1 - 10 carbon atoms", "aryl group having 6 - 10 carbon atoms", "heteroaryl group having 2 - 10 carbon atoms", "cycloalkyl group having 4 - 10 carbon atoms", and "heterocycloalkyl group having 4 - 10 carbon atoms" may be substituted at a substitutable position. As the substituent, those described in the below-mentioned substituent group A can be mentioned. Similarly, as the substituent of the "optionally substituted alkyl group having 1 - 20 carbon atoms", those described in the below-mentioned substituent group A can be mentioned.

The terms and symbols used in the present invention are defined in the following.

Examples of the "alkyl group having 1 - 20 carbon atoms" include methyl, ethyl, n-propyl, isopropyl, n-butyl, isobutyl, sec-butyl and tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, and the like. Examples of the "alkyl group having 1 - 10 carbon atoms" include methyl, ethyl, n-propyl, isopropyl, n-butyl, isobutyl, sec-butyl, tert-butyl, pentyl, hexyl, heptyl, octyl, nonyl, decyl, and the like, preferably C₁₋₆ alkyl group (e.g., methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl). Examples of the "aryl group having 6 - 10 carbon atoms" include phenyl group, tolyl group, xylyl group, and naphthyl group.

The "amino-protecting group" specifically includes trichloroacetyl, trifluoroacetyl, N-phthalimide, and the like.

The "hydroxyl-protecting group" means a general hydroxyl-protecting group known to those of ordinary skill in the art, which is introduced to prevent a reaction of the hydroxyl group. For example, the protecting groups described in Protective Groups in Organic Synthesis, published by John Wiley and Sons (1980) and the like, specifically, acyl-protecting groups such as acetyl, benzoyl and the like, alkyl-protecting groups such as trityl, 4-methoxytrityl, 4,4'-dimethoxytrityl, benzyl and the like, silyl-protecting group such as trimethylsilyl, tertbutyldimethylsilyl, tert-butyldiphenylsilyl and the like can be mentioned.

The "electron-withdrawing group" is a group which easily attracts an electron from the bonded atom side as compared to a hydrogen atom. Specifically, cyano, nitro, alkylsulfonyl (e.g., methylsulfonyl, ethylsulfonyl), halogen (fluorine atom, chlorine atom, bromine atom or iodine atom), arylsulfonyl (e.g., phenylsulfonyl, naphthylsulfonyl), trihalomethyl (e.g., trichloromethyl, trifluoromethyl), trialkyl amino (e.g., trimethyl amino) and the like can be mentioned.

Examples of the "halogen" include fluorine atom, chlorine atom, bromine atom, and iodine atom.

The "alkyl group" means a straight chain or branched chain alkyl group having 1 - 30, preferably 1 - 12, more preferably 1 - 6, particularly preferably 1 - 4, carbon atoms. Specific examples thereof include methyl, ethyl, n-propyl, isopropyl, n-butyl, isobutyl, sec-butyl and tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl and the like. Preferred are, for example, methyl, ethyl, n-propyl, isopropyl, n-butyl, isobutyl, sec-butyl, tert-butyl, n-pentyl, isopentyl, neopentyl, n-hexyl, isohexyl and the like.

The "alkenyl group" means a straight chain or branched chain alkenyl group having 2 - 30, preferably 2 - 12, more preferably 2 - 8, carbon atoms, and includes the aforementioned alkyl group containing one or plural double bonds and the like. Specific examples thereof include vinyl, 1-propenyl, 2-propenyl, 1-butenyl, 2-butenyl, 3-butenyl, 1,3-butadienyl, 3-methyl-2-butenyl, and the like.

The "alkynyl group" means a straight chain or branched chain alkynyl group having 2 - 30, preferably 2 - 12, more preferably 2 - 8, carbon atoms, and includes the aforementioned alkyl group containing one or plural triple bonds and the like. Specific examples thereof include ethynyl, propynyl, propargyl, butynyl, pentynyl, hexynyl, and the like. The alkynyl group may further have one or plural double bonds.

The "alkoxy group" means a straight chain or branched chain alkoxy group having 1 - 30, preferably 1 - 12, more preferably 1 - 6, particularly preferably 1 - 4, carbon atoms. Specific examples thereof include methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy, and the like.

The "aryl group" means an aryl group having 6 - 24, preferably 6 - 10, carbon atoms. Examples thereof include monocyclic aromatic hydrocarbon groups such as phenyl and the like, and polycyclic aromatic hydrocarbon groups such as 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, and the like. As the "aryl group having 6 - 10 carbon atoms", the above-mentioned aryl groups having 6 - 10 carbon atoms can be mentioned, and phenyl, naphthyl, and the like can be specifically mentioned.

The "heterocycloalkyl group" means a hecycloalkyl group having 6 - 24, preferably 6 - 10, carbon atoms. As the heterocycloalkyl group, the below-mentioned cycloalkyl groups in which one or more carbon atoms forming the cyclic structure are substituted by a nitrogen atom, an oxygen atom, a sulfur atom, or the like can be mentioned. Specific examples thereof include [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, tetrahydrofuryl, and the like. As the "heterocycloalkyl group having 4 - 10 carbon atoms", the above-mentioned heterocycloalkyl groups having 4 - 10 carbon atoms can be mentioned, Specific examples thereof include azetidinyl, pyrrolidinyl, piperidyl, piperadyl, morpholyl, oxetanyl, tetrahydrofuryl, tetrahydropyranyl, thioxetanyl, tetrahydrothienyl, and tetrahydrothiopyranyl group, and the like.

The "aralkyl group" means an aralkyl group having 7 - 30, preferably 7 - 11, carbon atoms. Specific examples thereof include benzyl, 2-phenethyl, naphthalenyl methyl, and the like.

The "cycloalkyl group" means a cycloalkyl group having 3 - 24, preferably 3 - 15, carbon atoms. Specifically, cyclopropyl, cyclobutyl, cyclopentyl, cyclohexyl, cycloheptyl, cyclooctyl, bridged cyclic hydrocarbon group, spiro hydrocarbon group and the like can be mentioned, and preferably, cyclopropyl, cyclobutyl, cyclopentyl, cyclohexyl, bridged cyclic hydrocarbon group and the like can be mentioned. Examples of the "bridged cyclic hydrocarbon group" include bicyclo[2.1.0]pentyl, bicyclo[2.2.1]heptyl, bicyclo[2.2.2]octyl, bicyclo[3.2.1]octyl, tricyclo[2.2.1.0]heptyl, bicyclo[3.3.1]nonane, 1-adamantyl, 2-adamantyl and the like. Examples of the "spiro hydrocarbon group" include spiro[3.4]octyl and the like. Examples of the "cycloalkyl group having 4 - 10 carbon atoms" include the above-mentioned cycloalkyl groups having 4 - 10 carbon atoms, specifically, cyclobutyl, cyclopentyl, cyclohexyl, cycloheptyl, cyclooctyl and the like.

The "cycloalkenyl group" means a cycloalkenyl group having 3 - 24, preferably 3 - 7, carbon atoms and containing at least one, preferably 1 or 2, double bonds. Specific examples thereof include cyclopropenyl, cyclobutenyl, cyclopentenyl, cyclohexenyl, cycloheptenyl, and the like. The aforementioned cycloalkenyl group also includes a bridged cyclic hydrocarbon group and a spirohydrocarbon group having an unsaturated bond in the ring. Examples of the "bridged cyclic hydrocarbon group having an unsaturated bond in the ring" include bicyclo[2.2.2]octenyl, bicyclo[3.2.1]octenyl, tricyclo[2.2.1.0]heptenyl and the like. Examples of the "spiro hydrocarbon group having an unsaturated bond in the ring" include spiro[3.4]octenyl and the like.

The "cycloalkylalkyl group" means an alkyl group (mentioned above) substituted by the aforementioned cycloalkyl group, and is preferably a cycloalkylalkyl group having 4 - 30, more preferably 4 - 11, carbon atoms. Specific examples thereof include cyclopropylmethyl, 2-cyclobutylethyl, cyclopentylmethyl, 3-cyclopentylpropyl, cyclohexylmethyl, 2-cyclohexylethyl, cycloheptylmethyl, and the like.

The "alkoxyalkyl group" means an alkyl group (mentioned above) substituted by the aforementioned alkoxy group, and is preferably a straight chain or branched chain alkoxyalkyl group having 2 - 30, more preferably 2 - 12, carbon atoms. Specific examples thereof include methoxymethyl, methoxyethyl, ethoxymethyl, ethoxyethyl, t-butoxy methyl, and the like.

The "alkylene group" means a straight chain or branched chain alkylene group having 1 - 30, preferably 1 - 12, more preferably 1 - 6, particularly preferably 1 - 4, carbon atoms. Specific examples thereof include, methylene, ethylene, and propylene, and the like.

The "heteroaryl group" encompasses, for example, monocyclic aromatic heterocyclic groups and condensed aromatic heterocyclic groups. Examples of the aforementioned heteroaryl include furyls (e.g., 2-furyl, 3-furyl), thienyls (e.g., 2-thienyl, 3-thienyl), pyrrolyls (e.g., 1-pyrrolyl, 2-pyrrolyl, 3-pyrrolyl), imidazolyls (e.g., 1-imidazolyl, 2-imidazolyl, 4-imidazolyl), pyrazolyls (e.g., 1-pyrazolyl, 3-pyrazolyl, 4-pyrazolyl), triazolyls (e.g., 1,2,4-triazol-1-yl, 1,2,4-triazol-3-yl, 1,2,4-triazol-4-yl), tetrazolyls (e.g., 1-tetrazolyl, 2-tetrazolyl, 5-tetrazolyl), oxazolyls (e.g., 2-oxazolyl, 4-oxazolyl, 5-oxazolyl), isoxazolyls (e.g., 3-isoxazolyl, 4-isoxazolyl, 5-isoxazolyl), thiazolyls (e.g., 2-thiazolyl, 4-thiazolyl, 5-thiazolyl), thiadiazolyls, isothiazolyls (e.g., 3-isothiazolyl, 4-isothiazolyl, 5-isothiazolyl), pyridyls (e.g., 2-pyridyl, 3-pyridyl, 4-pyridyl), pyridazinyls (e.g., 3-pyridazinyl, 4-pyridazinyl), pyrimidinyls (e.g., 2-pyrimidinyl, 4-pyrimidinyl, 5-pyrimidinyl), furazanyls (e.g., 3-furazanyl), pyrazinyls (e.g., 2-pyrazinyl), oxadiazolyls (e.g., 1,3,4-oxadiazol-2-yl), benzofuryls (e.g., 2-benzo[b]furyl, 3-benzo[b]furyl, 4-benzo[b]furyl, 5-benzo[b]furyl, 6-benzo[b]furyl, 7-benzo[b]furyl), benzothienyls (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzimidazolyls (e.g., 1-benzimidazolyl, 2-benzimidazolyl, 4-benzimidazolyl, 5-benzimidazolyl), dibenzofuryls, benzoxazolyls, benzothiazolyls, quinoxalinyls (e.g., 2-quinoxalinyl, 5-quinoxalinyl, 6-quinoxalinyl), cinnolinyls (e.g., 3-cinnolinyl, 4-cinnolinyl, 5-cinnolinyl, 6-cinnolinyl, 7-cinnolinyl, 8-cinnolinyl), quinazolinyls (e.g., 2-quinazolinyl, 4-quinazolinyl, 5-quinazolinyl, 6-quinazolinyl, 7-quinazolinyl, 8-quinazolinyl), quinolyls (e.g., 2-quinolyl, 3-quinolyl, 4-quinolyl, 5-quinolyl, 6-quinolyl, 7-quinolyl, 8-quinolyl), phthalazinyls (e.g., 1-phthalazinyl, 5-phthalazinyl, 6-phthalazinyl), isoquinolyls (e.g., 1-isoquinolyl, 3-isoquinolyl, 4-isoquinolyl, 5-isoquinolyl, 6-isoquinolyl, 7-isoquinolyl, 8-isoquinolyl), puryls, pteridinyls (e.g., 2-pteridinyl, 4-pteridinyl, 6-pteridinyl, 7-pteridinyl), carbazolyls, phenanthridinyls, acridinyls (e.g., 1-acridinyl, 2-acridinyl, 3-acridinyl, 4-acridinyl, 9-acridinyl), indolyls (e.g., 1-indolyl, 2-indolyl, 3-indolyl, 4-indolyl, 5-indolyl, 6-indolyl, 7-indolyl), isoindolyls, phenazinyls (e.g., 1-phenazinyl, 2-phenazinyl), and phenothiazinyls (e.g., 1-phenothiazinyl, 2-phenothiazinyl, 3-phenothiazinyl, 4-phenothiazinyl) and the like.

As the "heteroaryl group having 2 - 10 carbon atoms", the above-mentioned heteroaryl group having 2 - 10 carbon atoms can be mentioned. Specific examples thereof include furyl group, thienyl group, pyrrolyl group, oxazolyl group, triazolyl group, pyridyl group, quinolinyl group, and the like.

In the present invention, the "substituent" is exemplified by those in the following substituent group A. Substituent group A
(1) halogen (fluorine atom, chlorine atom, bromine atom, or iodine atom);
(2) alkyl group (mentioned above);
(3) alkoxy group (mentioned above);
(4) alkenyl group (mentioned above);
(5) alkynyl group (mentioned above);
(6) haloalkyl group (e.g., chloromethyl, fluoromethyl, dichloromethyl, difluoromethyl, dichlorofluoromethyl, trifluoromethyl, pentafluoroethyl, etc.);
(7) aryl group (mentioned above);
(8) heteroaryl group (mentioned above);
(9) aralkyl group (mentioned above)
(10) cycloalkyl group (mentioned above);
(11) cycloalkenyl group (mentioned above);
(12) cycloalkylalkyl group (mentioned above);
(13) cycloalkenylalkyl group (e.g., cyclopentenylethyl, cyclohexenylethyl, cyclohexenylbutyl, etc.);
(14) hydroxyalkyl group (e.g., hydroxymethyl, hydroxyethyl, hydroxypropyl, hydroxybutyl, etc.);
(15) alkoxyalkyl group (mentioned above);
(16) aminoalkyl group (e.g., aminomethyl, aminoethyl, aminopropyl, etc.);
(17) heterocyclyl group (e.g., 1-pyrrolinyl, 2-pyrrolinyl, 3-pyrrolinyl, 1-pyrrolidinyl, 2-pyrrolidinyl, 3-pyrrolidinyl, pyrrolidinone, 1-imidazolinyl, 2-imidazolinyl, 4-imidazolinyl, 1-imidazolidinyl, 2-imidazolidinyl, 4-imidazolidinyl, imidazolidinone, 1-pyrazolinyl, 3-pyrazolinyl, 4-pyrazolinyl, 1-pyrazolidinyl, 3-pyrazolidinyl, 4-pyrazolidinyl, piperidinone, piperidino, 2-piperidinyl, 3-piperidinyl, 4-piperidinyl, 1-piperazinyl, 2-piperazinyl, piperazinone, 2-morpholinyl, 3-morpholinyl, morpholino, tetrahydropyranyl, tetrahydrofuranyl, etc.);
(18) heterocyclylalkenyl group (e.g., 2-piperidinylethenyl, etc.);
(19) heterocyclylalkyl group (e.g., piperidinylmethyl, piperazinylmethyl, etc.);
(20) heteroarylalkyl group (e.g., pyridylmethyl, quinolin-3-yl methyl, etc.);
(21) silyl group;
(22) silyl oxyalkyl group (e.g., silyl oxymethyl, silyl oxyethyl, etc.)
(23) mono-, di- or tri-alkylsilyl group (e.g., methylsilyl, ethylsilyl, etc.); and
(24) mono-, di- or tri-alkylsilyloxyalkyl group (e.g., trimethylsilyloxymethyl, etc.).

The "atomic group having a nucleic acid base backbone" means a functional group having a nucleic acid base backbone in the whole or a part of the structure. The "nucleic acid base backbone" here may be a natural nucleic acid base backbone or an artificial nucleic acid base backbone, and preferably a natural nucleic acid base backbone.

The natural nucleic acid base is more preferably adenine, cytosine, guanine, uracil, thymine or other nitrogen-containing aromatic ring (e.g., 5-alkylpyrimidine, 5-halogenopyrimidine, deazapurine, deazapyrimidine, azapurine, azapyrimidine). It may be the same as the "base" in the below-mentioned nucleotide residues.

The nucleic acid molecule of the present invention may be labeled with, for example, a labeling substance. The labeling substance is not particularly limited, and may be, for example, a fluorescent substance, a dye, an isotope or the like. Examples of the labeling substance include: fluorophores such as pyrene, TAMRA, fluorescein, a Cy3 dye, a Cy5 dye and the like. Examples of the dye include Alexa dyes such as Alexa 488 and the like. Examples of the isotope include stable isotopes and radioisotopes. For example, stable isotopes have a low risk of radiation exposure and require no special facilities. Thus, stable isotopes are superior in handleability and can reduce costs. Moreover, the stable isotope does not change the physical properties of a compound labeled therewith and thus has an excellent property as a tracer. The stable isotope is exemplified by ²H, ¹³C, ¹⁵N, ¹⁷O, ¹⁸O, ³³S, ³⁴S and ³⁶S.

The nucleotide residue includes, as its components, a sugar, a base, and phosphoric acid. The ribonucleotide residue has a ribose residue as the sugar; and adenine (A), guanine (G), cytosine (C), or uracil (U) (which can also be replaced by thymine(T)) as the base. The deoxyribonucleotide residue has a deoxyribose residue as the sugar; and adenine (dA), guanine (dG), cytosine (dC), or thymine (dT) (which can also be replaced by uracil (dU)) as the base.

In the modified nucleotide residue, any of the components of the nucleotide residues is modified. In the present invention, "modification" means, for example, substitution, addition, and/or deletion of any of the components; and substitution, addition, and/or deletion of an atom(s) and/or a functional group(s) in the component(s). The modified nucleotide residue may be a naturally-occurring nucleotide residue or an artificially-modified nucleotide residue. Regarding the naturally-derived modified nucleotide residues, for example, Limbach et al. (1994, Summary: the modified nucleosides of RNA, Nucleic Acids Res. 22: pp. 2183 to 2196) can be referred to.

Examples of the modification of the nucleotide residue include modification of a ribose-phosphate backbone (hereinafter referred to as a "ribophosphate backbone").

In the ribophosphate backbone, for example, a ribose residue may be modified. In the ribose residue, for example, the 2'-position carbon can be modified. Specifically, a hydroxyl group bound to the 2'-position carbon can be, for example, replaced with an atom or group selected from the group consisting of hydrogen atom, a halogen atom such as fluorine, and the like, an -O-alkyl group (e.g., -O-Me group), an -O-acyl group (e.g., -O-COMe group), and an amino group, preferably an atom or group selected from the group consisting of a hydrogen atom, a methoxy group and a fluorine atom. By replacing the hydroxyl group bound to the aforementioned 2'-position carbon with hydrogen, it is possible to replace the ribose residue with deoxyribose. The ribose residue can be replaced with its stereoisomer, for example, and may be, for example, replaced with an arabinose residue.

The ribophosphate backbone may be replaced with, for example, a non-ribophosphate backbone having a non-ribose residue and/or a non-phosphate. The non-ribophosphate backbone may be, for example, the ribophosphate backbone modified to be uncharged. Examples of an alternative obtained by substituting the ribophosphate backbone with the non-ribophosphate backbone in the nucleotide include morpholino, cyclobutyl, and pyrrolidine. Other examples of the alternative include artificial nucleic acid monomer residues. Specific examples thereof include PNA (Peptide Nucleic Acid), LNA (Locked Nucleic Acid), and ENA (2'-O,4'-C-Ethylenebridged Nucleic Acids). Among them, PNA is preferred.

In the ribophosphate backbone, a phosphate group can also be modified. In the ribophosphate backbone, a phosphate group in the closest proximity to the sugar residue is called an "α-phosphate group". The α-phosphate group is charged negatively, and the electric charges are distributed evenly over two oxygen atoms that are not linked to the sugar residue. Among the four oxygen atoms in the α-phosphate group, the two oxygen atoms not linked to the sugar residue in the phosphodiester linkage between the nucleotide residues hereinafter are referred to as "non-linking oxygens". On the other hand, two oxygen atoms that are linked to the sugar residue in the phosphodiester linkage between the nucleotide residues hereinafter are referred to as "linking oxygens". For example, the α-phosphate group is preferably modified to be uncharged, or to render the charge distribution between the non-linking oxygens asymmetric.

In the phosphate group, for example, the non-linking oxygen(s) may be replaced. The non-linking oxygen(s) can be replaced with, for example, any atom selected from S (sulfur), Se (selenium), B (boron), C (carbon), H (hydrogen), N (nitrogen), and OR (R is an alkyl group or an aryl group) and substitution with S is preferred. It is preferable that both non-linking oxygens are replaced, and it is more preferable that both are replaced with S. Examples of such modified phosphate group include phosphorothioates, phosphorodithioates, phosphoroselenates, borano phosphates, borano phosphate esters, hydrogen phosphonates, phosphoroamidates, alkyl or aryl phosphonates, and phosphotriesters. In particular, phosphorodithioate in which both of the aforementioned two non-linking oxygens are replaced with S is preferred.

In the phosphate group, for example, linking oxygen(s) may be replaced. The linking oxygen(s) can be replaced with, for example, any atom selected from S (sulfur), C (carbon), and N (nitrogen). Examples of such modified phosphate group include: bridged phosphoroamidates resulting from the substitution with N; bridged phosphorothioates resulting from the substitution with S; and bridged methylenephosphonates resulting from the substitution with C. Preferably, substitution of the linking oxygen(s) is performed in, for example, at least one of the 5'-terminus nucleotide residue and the 3'-terminus nucleotide residue of the nucleic acid molecule of the present invention. When the substitution is performed on the 5' side, substitution with C is preferred. When the substitution is performed on the 3' side, substitution with N is preferred.

The phosphate group may be replaced with, for example, the phosphorus-free linker. The linker is exemplified by siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, methyleneoxymethylimino, or the like. Preferably, the linker is exemplified by a methylenecarbonylamino group and a methylenemethylimino group.

In the nucleic acid molecule of the present invention, for example, at least one of a nucleotide residue at the 3'-terminus and a nucleotide residue at the 5'-terminus may be modified. The modification is as described above, and it is preferable to modify a phosphate group(s) at the end(s). For example, the entire phosphate group may be modified, or one or more atoms in the phosphate group may be modified. In the former case, for example, the entire phosphate group may be replaced or deleted.

Modification of the nucleotide residue(s) at the end(s) may be, for example, addition of any other molecule. Examples of the other molecule include functional molecules such as labeling substances and protecting groups. Examples of the protecting groups include S (sulfur), Si (silicon), B (boron), and ester-containing groups. The functional molecules such as the aforementioned labeling substances can be used, for example, in the detection and the like of the nucleic acid molecule of the present invention.

The other molecule may be added to the phosphate group of the nucleotide residue or may be added to the phosphate group or the sugar residue via a spacer. For example, the terminal atom of the spacer can be added to or replaced with either one of the linking oxygens of the phosphate group, or O, N, S, or C of the sugar residue. The binding site in the sugar residue preferably is, for example, C at the 3'-position, C at the 5'-position, or any atom bound thereto. For example, the spacer can also be added to or replaced with a terminal atom of the nucleotide alternative such as PNA.

The spacer is not particularly limited, and examples thereof include -(CH₂)n-, -(CH₂)ₙN-, -(CH₂)ₙO-, -(CH₂)ₙS-, O(CH₂CH₂O)ₙCH₂CH₂OH, abasic sugar, amide, carboxy, amine, oxyamine, oxyimine, thioether, disulfide, thiourea, sulfonamide, and morpholino, and also biotin reagents and fluorescein reagents. In the aforementioned formulae, n is a positive integer which is preferably 1 to 6, further preferably n=3 or 6.

Other examples of the molecule to be added to the end include dyes, intercalating agents (e.g., acridine), crosslinking agents (e.g., psoralen, mitomycin C), porphyrins (TPPC4, texaphyrin, sapphyrin), polycyclic aromatic hydrocarbons (e.g., phenazine, dihydrophenazine), artificial endonucleases (e.g., EDTA), lipophilic carriers (e.g., cholesterol, cholic acid, adamantaneacetic acid, 1-pyrenebutyric acid, dihydrotestosterone, 1,3-Bis-O (hexadecyl)glycerol, a geranyloxyhexyl group, hexadecylglycerol, borneol, menthol, 1,3-propanediol, a heptadecyl group, palmitic acid, myristic acid, O3-(oleoyl)lithocholic acid, O3-(oleoyl)cholic acid, dimethoxytrityl, or phenoxathiine), peptide complexes (e.g., Antennapedia peptide, Tat peptide), alkylating agents, phosphoric acid, amino, mercapto, PEG (e.g., PEG-40K), MPEG, [MPEG]₂, polyamino, alkyl, substituted alkyl, radiolabeled markers, enzymes, haptens (e.g., biotin), transport/absorption facilitators (e.g., aspirin, vitamin E, folic acid), and synthetic ribonucleases (e.g., imidazole, bisimidazole, histamine, imidazole clusters, acridine-imidazole complexes, Eu³⁺ complexes of tetraazamacrocycles).

In the nucleic acid molecule of the present invention, for example, the 5'-terminus may be modified with a phosphate group or a phosphate group analog. Examples of the phosphate group include:
5'-monophosphate ((HO)₂(O)P-O-5');
5'-diphosphate ((HO)₂(O)P-O-P(HO)(O)-O-5');
5'-triphosphate ((HO)₂(O)P-O-(HO)(O)P-O-P(HO)(O)-O-5');
5'-guanosine cap (7-methylated or non-methylated, 7 m-G-O-5'-(HO)(O)P-O-(HO)(O)P-O-P(HO)(O)-O-5');
5'-adenosine cap (Appp);
any modified or unmodified nucleotide cap structure (N-O-5'-(HO)(O)P-O-(HO)(O)P-O-P(HO)(O)-O-5');
5'-monothiophosphate (phosphorothioate: (HO)₂(S)P-O-5');
5'-dithiophosphate (phosphorodithioate: (HO) (HS) (S)P-O-5');
5'-phosphorothiolate ((HO)₂(O)P-S-5');
sulfur substituted monophosphate, diphosphate, and
triphosphates (e.g., 5'-α-thiotriphosphate, 5'-γ-thiotriphosphate, and the like);
5'-phosphoramidates ((HO)₂(O)P-NH-5', (HO)(NH₂)(O)P-O-5');
5'-alkylphosphonates (e.g., RP(OH) (O)-O-5', (OH)₂(O)P-5'-CH₂, where R is alkyl (e.g., methyl, ethyl, isopropyl, propyl, or the like)); and
5'-alkyletherphosphonates (e.g., RP(OH) (O)-O-5', where R is alkylether (e.g., methoxymethyl, ethoxymethyl, or the like)).

In the nucleotide residue, the base is not particularly limited. The base may be a natural base or a non-natural base. For example, a common base, a modified analog thereof, and the like can be used.

Examples of the base include: purine bases such as adenine and guanine; and pyrimidine bases such as cytosine, uracil, and thymine. Other examples of the aforementioned base include thymine, xanthine, hypoxanthine, and examples of the nucleoside include nubularine, isoguanosine, tubercidine, and the like. Examples of the base also include: 2-aminoadenine, alkyl derivatives such as 6-methylated purine; alkyl derivatives such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl)uracil, 5-aminoallyluracil; 8-halogenated, aminated, thiolated, thioalkylated, hydroxylated, and other 8-substituted purines; 5-trifluoromethylated and other 5-substituted pyrimidines; 7-methylguanine; 5-substituted pyrimidines; 6-azapyrimidines; N-2, N-6, and O-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazoles; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; and O-alkylated bases. Examples of the purines and pyrimidines include those disclosed in U.S. Patent No. 3,687,808, "Concise Encyclopedia of Polymer Science and Engineering", pp. 858 to 859, edited by Kroschwitz J. I, John Wiley & Sons, 1990, and Englisch et al, Angewandte Chemie, International Edition, 1991, vol. 30, p. 613.

Other examples of the modified nucleotide residue include those having no base, i.e., those having an abasic ribophosphate backbone. Furthermore, as the modified nucleotide residue, for example, those described in U.S. Provisional Application No. 60/465,665 (filing date: April 25, 2003) and International Application No. PCT/US04/07070 (filing date: March 8, 2004) can be used and these documents are incorporated herein by reference.

In a preferred embodiment, the nucleic acid molecule of the present invention contains at least any one of the above-mentioned modified nucleotides. Preferably, the nucleic acid molecule of the present invention contains at least any one of the above-mentioned modified nucleotides in the sense strand. Preferably, the aforementioned modified nucleotide is selected from the group consisting of 2'-O-methyl-modified nucleotide, nucleotide containing a 5'-phosphorothioate group, deoxy-nucleotide, 3'-terminal deoxy-thymine (dT) nucleotide, 2'-fluoro-modified nucleotide, 2'-deoxy-modified nucleotide, terminal nucleotide bound with a cholesteryl derivative or a dodecanoic acid bisdecylamide group, fixed nucleotide, non-fixed nucleotide, conformationally restricted nucleotide, constrained ethyl nucleotide, abasic nucleotide, 2'-amino-modified nucleotide, 2'-O-allyl-modified nucleotide, 2'-C-alkyl-modified nucleotide, 2'-hydroxyl-modified nucleotide, 2'-methoxyethyl-modified nucleotide, 2'-O-alkyl-modified nucleotide, morpholino nucleotide, phosphoramidate, nucleotide containing non-natural base, tetrahydropyran-modified nucleotide, 1,5-anhydrohexitol-modified nucleotide, cyclohexenyl-modified nucleotide, nucleotide containing a phosphorothioate group, nucleotide containing a methylphosphonate group, nucleotide containing 5'-phosphate, and nucleotide containing a 5'-phosphate mimic.

The method for synthesizing the nucleic acid molecule of the present invention is not particularly limited, and a conventionally known method can be employed. Examples of the aforementioned synthesis method include chemical synthesis methods and synthesis methods according to genetic engineering procedures. The aforementioned chemical synthesis methods are not particularly limited, and examples thereof include a phosphoramidite method, an H-phosphonate method and the like. The aforementioned chemical synthesis methods can be carried out, for example, using a commercially available automated nucleic acid synthesizer. In the aforementioned chemical synthesis methods, an amidite is generally used. The aforementioned amidite is not particularly limited, and examples of commercially available RNA amidite include DMT-2'-O-TBDMS amidite (Proligo), ACE amidite and TOM amidite, CEE amidite, CEM amidite, TEM amidite and the like. Examples of the aforementioned genetic engineering procedures include: synthesis methods utilizing in vitro transcription; methods using a vector; methods carried out using a PCR cassette and the like. The aforementioned vector is not particularly limited, and examples thereof include non-virus vectors such as plasmid, and virus vectors.

Use of an amidite compound having the following structure instead of such commercially available amidites is a preferred embodiment of the present invention: 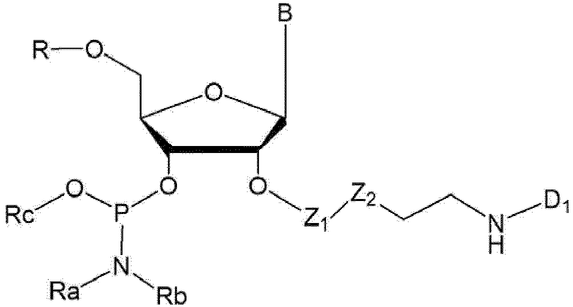
wherein B is an atomic group having a nucleic acid base backbone,
Z₁ is -CH₂- or -CO-,
Z₂ is -O- or -NH-,
R is a hydroxyl-protecting group,
Rₐ and R_{b} are the same or different and each a hydrogen atom or a substituent; R_{c} is a hydrogen atom, an electron-withdrawing group, or a substituent optionally substituted by an electron-withdrawing group, and
D₁ is an amino-protecting group.

An amidite compound wherein Z₁ is -CH₂- and Z₂ is -O- is also to be referred to as trifluoroacetylaminoethoxymethyl(AEM)amidite.

An amidite compound wherein Z₁ is -CO- and Z₂ is -NH- is also to be referred to as trifluoroacetylaminoethoxycarbamoyl(AEC)amidite.

The structure of the preferred AEM amidite is as follows: 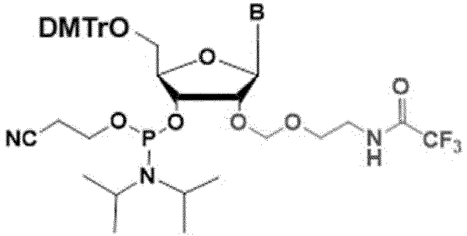 wherein B is an atomic group having a nucleic acid base backbone, and DMTr means 4,4'-dimethoxytrityl.

The structure of the preferred AEC amidite is as follows: 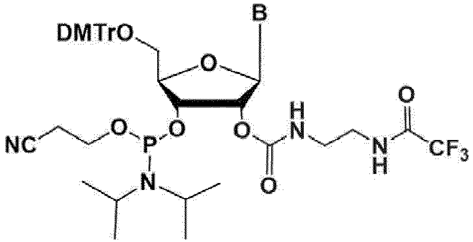 wherein B is an atomic group having a nucleic acid base backbone, and DMTr means 4,4'-dimethoxytrityl.

The amidite compound of the present invention can be produced according to a conventional method.

### (II) Use of the nucleic acid molecule of the present invention

The nucleic acid molecule of the present invention has a target gene expression suppressing activity equal to or higher than that of siRNA, and the off-target effect by the sense strand is remarkably attenuated. Therefore, it can be formulated as it is, or together with pharmacologically acceptable additives, as an expression inhibitor of a gene containing a target nucleic acid sequence. The gene expression inhibitor can be used, for example, as a research reagent to suppress expression of a target gene in an in vitro system (e.g., isolated cells, tissues or organs), or used to suppress the expression thereof by in vivo administration to a subject having the target gene. When the gene expression inhibitor of the present invention is administered in vivo, the subject of administration is exemplified by humans and nonhuman animals such as nonhuman mammals excluding humans.

Administration of the gene expression inhibitor of the present invention to, for example, a patient who has or may have in the future a disease caused by the target gene can inhibit the expression of the target gene, thereby treating the aforementioned disease. In the present specification, the term "treatment" is used to encompass all of the prevention of the aforementioned disease, delay in onset of the disease, improvement of the disease, improvement in prognosis, and the like.

In the present invention, the disease to be treated is not particularly limited, and examples thereof include diseases caused by high expression of certain genes. Depending on the kind of the aforementioned disease, a gene involved in the disease is set to the aforementioned target gene, and further, depending on the aforementioned target gene, an expression inhibitory sequence i.e., antisense strand sequence), and a sense strand sequence complementary thereto may be set as appropriate.

As a specific example, the TGF-β1 gene can be recited as the aforementioned target gene. The nucleic acid molecule of the present invention having an antisense strand sequence against the TGF-β1 gene can be used, for example, in the treatment of fibrotic diseases, specifically idiopathic pulmonary fibrosis and the like. Similarly, when the target gene is NIMA-related kinase 6 (NEK6), which plays an important role in tumor formation, the nucleic acid molecule of the present invention can be used, for example, in the treatment of cancer or fibrosis (WO/2019/022257).

Examples of the pharmacologically acceptable additive include, but are not limited to, excipients such as sucrose, starch and the like, binders such as cellulose, methylcellulose and the like, disintegrants such as starch, carboxymethylcellulose and the like, lubricants such as magnesium stearate, aerogel and the like, flavors such as citric acid, menthol and the like, preservatives such as sodium benzoate, sodium bisulfite and the like, stabilizers such as citric acid, sodium citrate and the like, suspending agents such as methylcellulose, polyvinyl pyrrolidone and the like, dispersing agents such as surfactant and the like, diluents such as water, physiological saline and the like, base wax and the like.

To facilitate the introduction of the nucleic acid molecule of the present invention into the target cell, the gene expression inhibitor of the present invention can further comprise a reagent for nucleic acid introduction. Cationic lipids such as atelocollagen; liposome; nanoparticle; Lipofectin, Lipofectamine, DOGS (Transfectam), DOPE, DOTAP, DDAB, DHDEAB, HDEAB, polybrene, or poly(ethyleneimine) (PEI) and the like, and the like can be used as the reagent for nucleic acid introduction.

In one preferable embodiment, the gene expression inhibitor of the present invention may be a pharmaceutical composition wherein the nucleic acid molecule of the present invention is encapsulated in a liposome. A liposome is a microscopic closed vesicle having an internal phase enclosed by one or more lipid bilayers, and typically can retain a water-soluble substance in the internal phase and a lipophilic substance in the lipid bilayer. Herein, when the term "encapsulated" is used, the nucleic acid molecule of the present invention may be retained in the internal phase or in the lipid bilayer, of the liposome. The liposome to be used in the present invention may be a single-layer film or a multilayer film. The particle size of the liposome can be appropriately selected within the range of, for example, 10 - 1000 nm, preferably 50 - 300 nm. Considering the delivery efficiency to the target tissue, the particle size can be, for example, 200 nm or less, preferably 100 nm or less.

Methods of encapsulating a water-soluble compound such as nucleic acid into a liposome include lipid film method (vortex method), reversed-phase evaporation method, surfactant removal method, freeze-thawing method, remote loading method and the like, but are not limited thereto, and any known method can be appropriately selected.

While the gene expression inhibitor of the present invention can be orally or parenterally administered to a mammal (e.g., human, cat, ferret, mink, rat, mouse, guinea pig, rabbit, sheep, horse, swine, bovine, monkey), it is desirably administered parenterally.

Preparations suitable for parenteral administration (for example, subcutaneous injection, intramuscular injection, topical injection, intraperitoneal administration and the like) include aqueous and non-aqueous isotonic sterile injectable liquids, which may contain an antioxidant, a buffer solution, a bacteriostatic agent, an isotonizing agent and the like. Aqueous and non-aqueous sterile suspensions can also be mentioned, which may contain a suspending agent, a solubilizer, a thickening agent, a stabilizer, an antiseptic and the like. These preparations can be encapsulated in containers such as ampoules and vials for unit dosage or a plurality of dosages. It is also possible to freeze-dry the active ingredient and a pharmacologically acceptable additive, and store the preparation in a state that may be dissolved or suspended in an appropriate sterile vehicle just before use.

As other preparations preferable for parenteral administration, spray and the like can be mentioned.

The content of the nucleic acid molecule of the present invention in the gene expression inhibitor is, for example, about 0.1 - 100 wt% of the whole preparation.

The dose of the gene expression inhibitor of the present invention varies depending on the object of administration, method of administration, kind of the target disease, severity, situation of the subject of administration (sex, age, body weight and the like). For systemic administration to, for example, an adult, generally, a single dose of the nucleic acid of the present invention is not less than 2 nmol/kg and not more than 50 nmol/kg. For topical administration, it is desirably not less than 1 pmol/kg and not more than 10 nmol/kg. Such dose is desirably administered once to 10 times, more preferably 5 to 10 times.

The present invention is explained in further detail in the following by referring to Examples. The present invention is not limited to these Examples.

### [Example]

### Production Example 1: Synthesis of AEM reagent

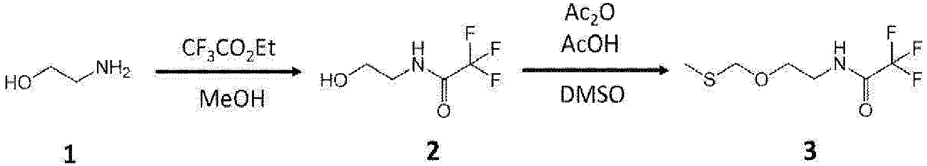

### (1) Synthesis of compound 2

To a solution of compound 1 (6.1 g, 100 mmol) in methanol (40 mL) was added ethyl trifluoroacetate (13.1 mL, 1.1 eq.) under an argon atmosphere, and the mixture was stirred at room temperature overnight. The reaction mixture was concentrated under reduced pressure to give the target compound 2 as a white solid.
ESI-Mass:156.03[M-H]-

### (2) Synthesis of compound 3

To a solution of compound 2 (100 mmol) in dimethylsulfoxide (100 mL) were added acetic anhydride (71.8 mL, 7.6 eq.), acetic acid (51.5 mL, 9.0 eq.) under an argon atmosphere, and the mixture was stirred at room temperature for 1 day. To the reaction mixture was added slowly a suspension of sodium hydrogen carbonate and water, and the mixture was stirred at room temperature for 1 hr. Water was added to the reaction mixture, and the mixture was extracted with ethyl acetate. The organic layer was washed with water, and dried over magnesium sulfate. The solution was concentrated under reduced pressure, and the obtained residue was purified by silica gel column chromatography (n-hexane:ethyl acetate=100:0 - 50:50) to give the target compound 3 (AEM reagent) as a colorless oily substance (8.4 g).
ESI-Mass:240.03[M+Na]⁺

### Production Example 2: Synthesis of uridine AEM amidite (U5)

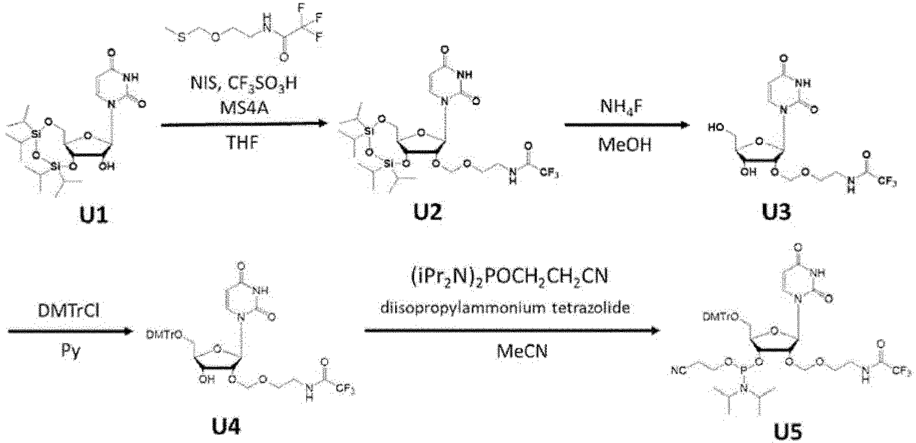

### (1) Synthesis of 3',5'-O-(tetraisopropyldisiloxane-1,3-diyl)-2'-O-[2-(trifluoroacetyl)aminoethoxymethyl]uridine (U2)

Compound U1 (3.3 g, 6.8 mmol) was azeotropically distilled with a mixed solvent of toluene and tetrahydrofuran, and vacuum dried for 1 hr. Under an argon atmosphere, dehydrated tetrahydrofuran (30 mL), molecular sieves 4A (3.3 g), and N-iodosuccinimide (2.3 g, 1.5 eq.) were added and the mixture was stirred and cooled to -45°C. After stirring for 5 min, trifluoromethanesulfonic acid (0.9 mL, 1.5 eq.) was added dropwise. After stirring for 5 min, an AEM reagent (compound 3; 2.2 g, 1.5 eq.) dissolved in tetrahydrofuran (7 mL) was added, and the mixture was stirred at -45°C for 1 hr. To the reaction mixture was added at -45°C a mixed solution of a saturated aqueous sodium thiosulfate solution and a saturated aqueous sodium hydrogen carbonate solution, and the mixture was stirred at room temperature for 30 min until the brown color disappeared. Water was added to the reaction mixture, and the mixture was extracted with ethyl acetate. The organic layer was washed once with saturated aqueous sodium thiosulfate solution, once with saturated aqueous sodium hydrogen carbonate solution, and once with saturated aqueous sodium chloride solution, and dried over magnesium sulfate. The solution was concentrated under reduced pressure, and the obtained residue was purified by silica gel column chromatography (n-hexane:ethyl acetate=90:10 - 50:50) to give the target compound U2 as a high purity fraction, white foamy substance (3.1 g) and as a low purity fraction, a pale-yellow oily substance (1.7 g). ESI-Mass:678.24 [M+Na]⁺

### (2) Synthesis of 2'-O-[2-(trifluoroacetyl)aminoethoxymethyl]uridine (U3)

To a solution of compound U2 (3.1 g, 4.7 mmol) in dehydrated methanol (30 mL) was added ammonium fluoride (0.7 g, 4.0 eq.) under an argon atmosphere, and the mixture was stirred at 50°C for 4 hr. To the reaction mixture were added methanol and silica gel and the solution was concentrated under reduced pressure. The obtained residue was filled in a column and purified by silica gel column chromatography (chloroform:20% methanol/80% chloroform=100:0 - 35:65) to give the target compound U3 as a white foamy substance (1.5 g).
ESI-Mass:412.09[M-H]-

### (3) Synthesis of 5'-O-(4,4'-dimethoxytrityl)-2'-O-[2-(trifluoroacetyl)aminoethoxymethyl]uridine (U4)

To a solution of compound U3 (1.9 g, 4.7 mmol) in dehydrated pyridine (20 mL) was added DMTr-Cl (1.9 g, 1.2 eq.) under an argon atmosphere at 0°C, and the mixture was stirred at room temperature overnight. To the reaction mixture was added DMTr-Cl (0.3 g,0.2eq.), and the mixture was stirred at room temperature for 4 hr. To the reaction mixture was added methanol (10 mL), and the mixture was stirred for 10 min. The solution was concentrated under reduced pressure, saturated aqueous sodium hydrogen carbonate solution was added, and the mixture was extracted with dichloromethane. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=70:30 - 0:100, containing 0.05% pyridine) to give the target compound U4 as a pale-yellow foamy substance (2.8 g).
ESI-Mass:738.22 [M+Na]⁺

### (4) Synthesis of 5'-O-(4,4'-dimethoxytrityl)-2'-O-[2-(trifluoroacetyl)aminoethoxymethyl]uridine 3'-O-(2-cyanoethyl N,N-diisopropyl phosphoramidite) (U5)

To a solution of compound U4 (2.8 g, 3.9 mmol) in dehydrated acetonitrile (40 mL) was added diisopropyl ammonium tetrazolide (0.8 g, 1.2 eq.), and 2-cyanoethyl N,N,N',N'-tetraisopropylphosphorodiamidite (1.5 mL, 1.2 eq.) under an argon atmosphere at room temperature, and the mixture was stirred at 40°C for 2 hr. To the reaction mixture were added water, saturated aqueous sodium chloride solution, and saturated aqueous sodium hydrogen carbonate solution, and the mixture was extracted with ethyl acetate. The organic layer was washed with saturated aqueous sodium chloride solution and dried over magnesium sulfate. The solution was concentrated under reduced pressure, and the obtained residue was purified by silica gel column chromatography (n-hexane:ethyl acetate=70:30 - 0:100, containing 0.1% triethylamine) to give the target compound U5 as a white foamy substance (3.2 g). ³¹P-NMR (202 MHz, CDCl₃): 151.99, 149.74(<integral ratio>1.0:1.2)
¹H-NMR (500 MHz, CDCl₃) δ: 1.00-1.20(m, 12H), 2.43-2.47(m, 1H), 2.60-2.65(m, 1H), 3.44-4.10(m, 10H), 3.79-3.81(-OMe, 6H), 4.15-4.25(m, 1H), 4.35-4.37(m, 1H), 4.54-4.68(m, 1H), 4.79-5.24(m, 3H), 5.86-5.91(m, 1H), 6.81-6.87(m, 4H), 7.23-7.43(m, 9H), 7.81-7.92(m, 1H), 8.12-8.20(m, 1H), 8.83-9.15(m, 1H)
ESI-Mass:938.32 [M+Na]⁺

### Production Example 3: Synthesis of adenosine AEM amidite (A5)

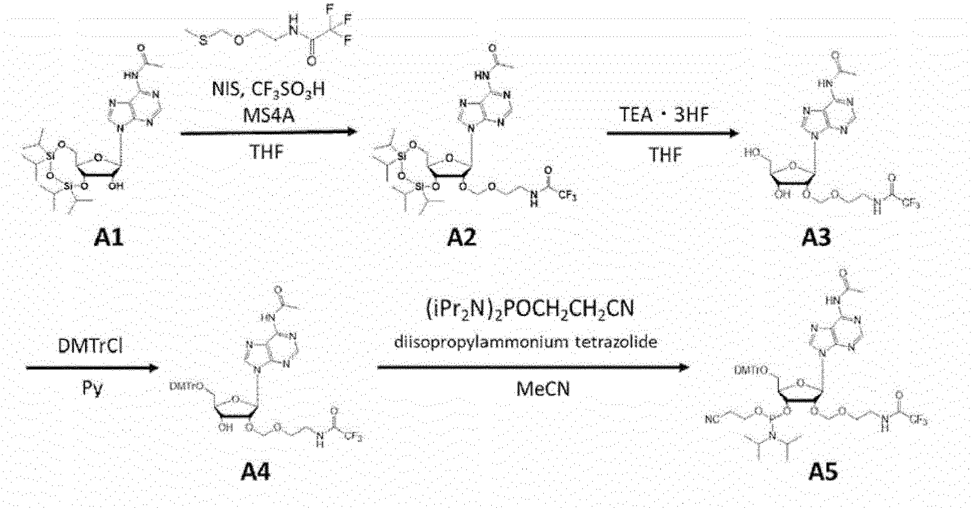

### (1) Synthesis of N⁶-acetyl-3',5'-O-(tetraisopropyldisiloxane-1,3-diyl)-2'-O-[2-(trifluoroacetyl)aminoethoxymethyl]adenosine (A2)

Compound A1 (3.7 g, 6.8 mmol) was azeotropically distilled with a mixed solvent of toluene and tetrahydrofuran and vacuum dried for 1 hr. Under an argon atmosphere, dehydrated tetrahydrofuran (30 mL), molecular sieves 4A (3.7 g), and N-iodosuccinimide (2.0 g, 1.3 eq.) were added and the mixture was stirred. The mixture was cooled to -45°C. After stirring for 5 min, trifluoromethanesulfonic acid (0.8 mL, 1.3 eq.) was added dropwise. After stirring for 5 min, AEM reagent (compound 3; 2.0 g, 1.3 eq.) dissolved in tetrahydrofuran (7 mL) was added, and the mixture was stirred at -45°C for 3 hr and at 0°C for 1 hr. To the reaction mixture was added a mixed solution of a saturated aqueous sodium thiosulfate solution and a saturated aqueous sodium hydrogen carbonate solution at 0°C, and the mixture was stirred at room temperature for 30 min until the brown color disappeared. To the reaction mixture were added water and saturated aqueous sodium chloride solution, and the mixture was extracted with ethyl acetate. The organic layer was washed once with saturated aqueous sodium thiosulfate solution, once with saturated aqueous sodium hydrogen carbonate solution, and once with saturated aqueous sodium chloride solution, and dried over magnesium sulfate. The solution was concentrated under reduced pressure, and the obtained residue was purified by silica gel column chromatography (n-hexane:ethyl acetate=50:50 - 0:100) to give the target compound A2 as a high purity fraction, white foamy substance (3.1 g) and as a low purity fraction, white foamy substance (1.8 g).
ESI-Mass:721.30[M+H]⁺

### (2) Synthesis of N⁶-acetyl-2'-O-[2-(trifluoroacetyl)aminoethoxymethyl]adenosine (A3)

To a solution of compound A2 (3.1 g, 4.3 mmol) in dehydrated tetrahydrofuran (30 mL) was added TEA/3HF (0.8 mL, 1.2 eq.) under an argon atmosphere, and the mixture was stirred at room temperature for 5 hr. To the reaction mixture were added methanol and silica gel and the solution was concentrated under reduced pressure. The obtained residue was filled in a column and purified by silica gel column chromatography (chloroform:20% methanol/80% chloroform=100:0 - 35:65) to give the target compound A3 as a white foamy substance (1.6 g).
ESI-Mass:501.13[M+Na]⁺

### (3) Synthesis of N⁶-acetyl-5'-O-(4,4'-dimethoxytrityl)-2'-O-[2-(trifluoroacetyl)aminoethoxymethyl]adenosine (A4)

To a solution of compound A3 (2.1 g, 4.3 mmol) in dehydrated pyridine (20 mL) was added DMTr-Cl (2.2 g, 1.5 eq.) under an argon atmosphere at 0°C, and the mixture was stirred at room temperature for 4 hr. To the reaction mixture was added DMTr-Cl (1.5 g, 1.0 eq.), and the mixture was stirred at room temperature for 2 hr. To the reaction mixture was added methanol (10 mL) and the mixture was stirred for 10 min. The solution was concentrated under reduced pressure, and the obtained residue was purified by silica gel column chromatography (dichloromethane:10% methanol/90% dichloromethane=100:0 - 0:100, containing 0.05% pyridine) to give the target compound A4 as a pale-yellow foamy substance (2.9 g).
ESI-Mass:803.27 [M+Na]⁺

### (4) Synthesis of N⁶-acetyl-5'-O-(4,4'-dimethoxytrityl)-2'-O-[2-(trifluoroacetyl)aminoethoxymethyl]adenosine 3'-O-(2-cyanoethyl N,N-diisopropyl phosphoramidite) (A5)

To a solution of compound A4 (2.9 g, 3.7 mmol) in dehydrated acetonitrile (40 mL) were added diisopropyl ammonium tetrazolide (0.8 g, 1.2 eq.), 2-cyanoethyl N,N,N',N'-tetraisopropylphosphorodiamidite (1.4 mL, 1.2 eq.) under an argon atmosphere at room temperature, and the mixture was stirred at 40°C for 1 hr. The reaction mixture was concentrated under reduced pressure, water and a saturated aqueous sodium hydrogen carbonate solution, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=50:50 - 0:100, containing 0.1% triethylamine) to give the target compound A5 as a white foamy substance (3.2 g).
³¹P-NMR (202 MHz, CDCl₃): 151.75,150.87, 14.83 (<integral ratio>1.1:1.0:0.4)
¹H-NMR (500 MHz, CDCl₃) δ: 1.04-1.30(m, 12H), 2.37-2.41(m, 1H), 2.58-2.64(m, 4H), 3.35-3.93(m, 11H), 3.76-3.79(-OMe, 6H), 4.32-4.41(m, 1H), 4.63-4.71(m, 1H), 4.73-4.78(m, 1H), 4.80-4.96(m, 2H), 6.21-6.25(m, 1H), 6.77-6.85(m, 4H), 7.20-7.47(m, 10H), 8.25(s, 0.5H), 8.27(s, 0.5H), 8.61(brs, 1H), 8.71(brs, 1H)
ESI-Mass:1003.38 [M+Na]⁺

### Production Example 4: Synthesis of guanosine AEM amidite (G5)

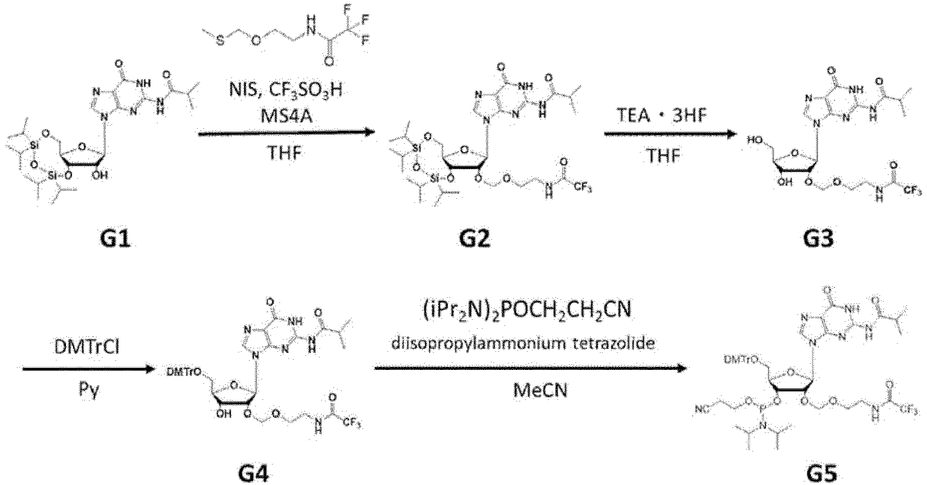

### (1) Synthesis of N²-isobutyryl-3',5'-O-(tetraisopropyldisiloxane-1,3-diyl)-2'-O-[2-(trifluoroacetyl)aminoethoxymethyl]guanosine (G2)

Compound G1 (4.0 g, 6.8 mmol) was azeotropically distilled with a mixed solvent of toluene and tetrahydrofuran and vacuum dried for 1 hr. Under an argon atmosphere, dehydrated tetrahydrofuran (30 mL), molecular sieves 4A (4.0 g), and N-iodosuccinimide (2.3 g, 1.5 eq.) were added and the mixture was stirred. The mixture was cooled to -45°C. After stirring for 5 min, trifluoromethanesulfonic acid (0.9 mL, 1.5 eq.)was added dropwise. After stirring for 5 min, an AEM reagent (compound 3; 2.2 g, 1.5 eq.) dissolved in tetrahydrofuran (5 mL) was added, and the mixture was stirred at -45°C for 1 hr. To the reaction mixture was added a mixed solution of a saturated aqueous sodium thiosulfate solution and a saturated aqueous sodium hydrogen carbonate solution at -45°C, and the mixture was stirred at room temperature for 30 min until the brown color disappeared. Water was added to the reaction mixture, and the mixture was extracted with ethyl acetate. The organic layer was washed once with saturated aqueous sodium thiosulfate solution, once with saturated aqueous sodium hydrogen carbonate solution, and once with saturated aqueous sodium chloride solution, and dried over magnesium sulfate. The solution was concentrated under reduced pressure to give a crude product of the target compound G2 as a white foamy substance (5.2 g).
ESI-Mass:787.32[M+Na]⁺

### (2) Synthesis of N²-isobutyryl-2'-O-[2-(trifluoroacetyl)aminoethoxymethyl]guanosine (G3)

To a solution of compound G2 (5.1 g, 6.8 mmol) in dehydrated tetrahydrofuran (60 mL) was added TEA/3HF (1.3 mL, 1.2 eq.) under an argon atmosphere, and the mixture was stirred at room temperature for 6 hr. To the reaction mixture were added methanol and silica gel and the solution was concentrated under reduced pressure. The obtained residue was filled in a column and purified by silica gel column chromatography (chloroform:20% methanol/80% chloroform=100:0 - 35:65) to give the target compound G3 as a white foamy substance (3.4 g).
ESI-Mass:545.16[M+Na]⁺

### (3) Synthesis of N²-isobutyryl-5'-O-(4,4'-dimethoxytrityl)-2'-O-[2-(trifluoroacetyl)aminoethoxymethyl]guanosine (G4)

To a solution of compound G3 (3.4 g, 6.5 mmol) in dehydrated pyridine (35 mL) was added DMTr-Cl (3.3 g, 1.5 eq.) under an argon atmosphere at 0°C, and the mixture was stirred at room temperature for 3 hr. To the reaction mixture was added DMTr-Cl (3.3 g, 1.5 eq.), and the mixture was stirred at room temperature for 4 hr. To the reaction mixture was added DMTr-Cl (3.3 g, 1.5 eq.), and the mixture was stirred at room temperature for 1 hr. To the reaction mixture was added methanol (20 mL) and the mixture was stirred for 10 min. The solution was concentrated under reduced pressure, and the obtained residue was crudely purified by silica gel column chromatography (dichloromethane:10% methanol/90% dichloromethane=100:0 - 0:100, containing 0.05% pyridine). The obtained crude product was purified by silica gel column chromatography (dichloromethane:10% methanol/90% dichloromethane=50:50, containing 0.05% pyridine) to give the target compound G4 as a pale-yellow foamy substance (3.1 g).
ESI-Mass:825.31 [M+H]⁺

### (4) Synthesis of N²-isobutyryl-5'-O-(4,4'-dimethoxytrityl)-2'-O-[2-(trifluoroacetyl)aminoethoxymethyl]guanosine 3'-O-(2-cyanoethyl N,N-diisopropyl phosphoramidite) (G5)

To a solution of compound G4 (3.0 g, 3.7 mmol) in dehydrated acetonitrile (40 mL) were added diisopropyl ammonium tetrazolide (0.8 g, 1.3 eq.) and 2-cyanoethyl N,N,N',N'-tetraisopropylphosphorodiamidite (1.5 mL, 1.3 eq.) under an argon atmosphere at room temperature, and the mixture was stirred at 40°C for 4 hr. To the reaction mixture was added 2-cyanoethyl N,N,N',N'-tetraisopropylphosphorodiamidite (0.8 mL,0.7eq.), and the mixture was stirred at 40°C for 1 hr. To the reaction mixture were added water and saturated aqueous sodium hydrogen carbonate solution, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=50:50 - 0:100, containing 0.1% triethylamine) to give a crude purified product (2.0 g). After sonication of the crude purified product (1.6 g) with ethyl acetate and n-hexane, the solid was collected by filtration and dried under reduced pressure to give the target compound G5 as a white solid (0.7 g).
³¹P-NMR (202 MHz, CDCl₃): 150.99,150.37(<integral ratio>1.0:0.5) ¹H-NMR (500 MHz, CDCl₃) δ: 0.78-1.20(m, 18H), 1.79-1.85(m, 0.33H), 2.05-2.11(m, 0.67H), 2.31(t, J= 6.0 Hz, 0.67H), 2.68-2.72(m, 1.33H), 3.17-3.22(m, 1H), 3.33-4.00(m, 9H), 3.75-3.80(-OMe, 6H), 4.22-4.34(m, 1H), 4.50-4.60(m, 1H), 4.71-4.84(m, 2H), 4.95-5.09(m, 0.67H), 5.11-5.16(m, 0.33H), 5.90(d, J= 7.0 Hz, 0.33H), 6.00(d, J= 7.0 Hz, 0.67H), 6.78-6.83(m, 4H), 7.07-7.13(m, 1H), 7.20-7.55(m, 9H), 7.81(s, 0.33H), 7.83(s, 0.67H), 8.08(brs, 0.33H), 8.50(brs, 0.67H), 12.05(brs, 1H)
ESI-Mass:1047.38 [M+Na]⁺

### Production Example 5: Synthesis of cytidine AEM amidite (C5)

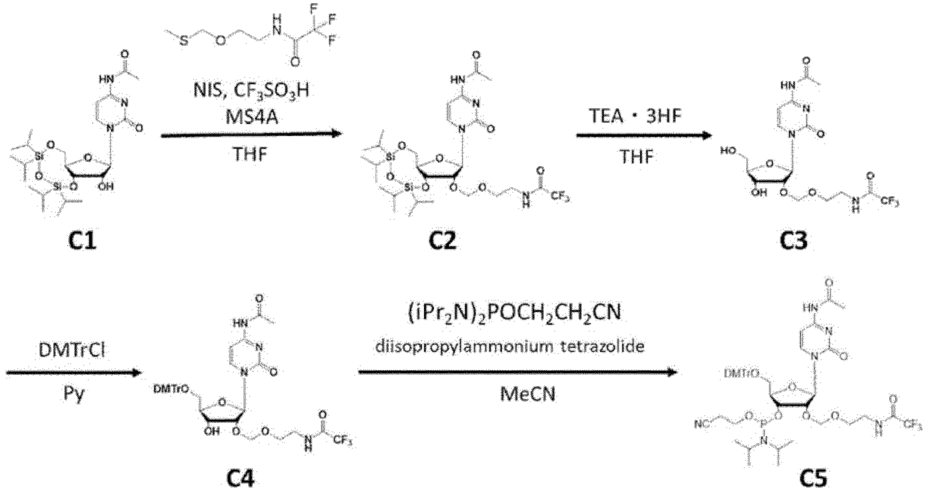

### (1) Synthesis of N⁴-acetyl-3',5'-O-(tetraisopropyldisiloxane-1,3-diyl)-2'-O-[2-(trifluoroacetyl)aminoethoxymethyl]cytidine (C2)

Compound C1 (3.6 g, 6.8 mmol) was vacuum dried for 1 hr. Under an argon atmosphere, dehydrated tetrahydrofuran (30 mL), molecular sieves 4A (3.6 g), and N-iodosuccinimide (2.3 g, 1.5 eq.) were added and the mixture was stirred. The mixture was cooled to -45°C. After stirring for 5 min, trifluoromethanesulfonic acid (0.9 mL, 1.5 eq.) was added dropwise. After stirring for 5 min, an AEM reagent (compound 3; 2.2 g, 1.5 eq.) dissolved in tetrahydrofuran (5 mL) was added, and the mixture was stirred at -45°C for 1 hr. To the reaction mixture was added a mixed solution of a saturated aqueous sodium thiosulfate solution and a saturated aqueous sodium hydrogen carbonate solution at -45°C, and the mixture was stirred at room temperature for 30 min until the brown color disappeared. Water was added to the reaction mixture, and the mixture was extracted with ethyl acetate. The organic layer was washed once with saturated aqueous sodium thiosulfate solution, once with saturated aqueous sodium hydrogen carbonate solution, and once with saturated aqueous sodium chloride solution, and dried over magnesium sulfate. The solution was concentrated under reduced pressure to give a crude product of the target compound C2 as a white foamy substance (5.5 g). ESI-Mass:719.27[M+Na]⁺

### (2) Synthesis of N⁴-acetyl-2'-O-[2-(trifluoroacetyl)aminoethoxymethyl]cytidine (C3)

To a solution of compound C2 (5.5 g, 7.9 mmol) in dehydrated tetrahydrofuran (70 mL) was added TEA/3HF (1.3 mL, 1.0 eq.) under an argon atmosphere, and the mixture was stirred at room temperature for 1 day. After adding ethyl acetate to the reaction mixture and sonicating the mixture, insoluble material was collected by filtration to give solid <1>. The filtrate was concentrated under reduced pressure, and the obtained residue was purified by silica gel column chromatography (chloroform:20% methanol/80% chloroform=100:0 - 35:65) to give a purified product <2>. The solid <1> and the purified product <2> were mixed to give the target compound C3 as a white solid (3.1 g).
ESI-Mass:477.12[M+Na]⁺

### (3) Synthesis of N⁴-acetyl-5'-O-(4,4'-dimethoxytrityl)-2'-O-[2-(trifluoroacetyl)aminoethoxymethyl]cytidine (C4)

To a solution of compound C3 (3.1 g, 6.8 mmol) in dehydrated pyridine (35 mL) was added DMTr-Cl (3.5 g, 1.5 eq.) under an argon atmosphere at 0°C, and the mixture was stirred at room temperature overnight. To the reaction mixture was added DMTr-Cl (1.7 g, 0.8 eq.), and the mixture was stirred at room temperature for 2 hr. To the reaction mixture was added methanol (20 mL) and the mixture was stirred for 1 hr. The solution was concentrated under reduced pressure, and the obtained residue was purified by silica gel column chromatography (dichloromethane:10% methanol/90% dichloromethane=100:0 - 50:50, containing 0.05% pyridine) to give the target compound C4 as a pale-yellow foamy substance (4.8 g).
ESI-Mass:779.25 [M+Na]⁺

### (4) Synthesis of N⁴-acetyl-5'-O-(4,4'-dimethoxytrityl)-2'-O-[2-(trifluoroacetyl)aminoethoxymethyl]cytidine 3'-O-(2-cyanoethyl N,N-diisopropyl phosphoramidite) (C5)

To a solution of compound C4 (4.8 g, 6.3 mmol) in dehydrated acetonitrile (50 mL) were added diisopropyl ammonium tetrazolide (1.6 g, 1.5 eq.) and 2-cyanoethyl N,N,N',N'-tetraisopropylphosphorodiamidite (3.0 mL, 1.5 eq.) under an argon atmosphere at room temperature, and the mixture was stirred at 40°C for 2 hr. To the reaction mixture were added water, saturated aqueous sodium hydrogen carbonate solution, and saturated aqueous sodium chloride solution, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=50:50 - 0:100, containing 0.1% triethylamine) to give the target compound C5 as a white foamy substance (5.1 g).
³¹P-NMR (202 MHz, CDCl₃): 152.32,149.93, 14.75 (<integral ratio>0.5:1.0:0.5)
¹H-NMR (500 MHz, CDCl₃) δ: 0.97-1.30(m, 12H), 2.18-2.22(m, 3H), 2.37-2.60(m, 2H), 3.45-3.85(m, 10H), 3.80-3.83(-OMe, 6H), 4.20-4.30(m, 1H), 4.35-4.38(m, 1H), 4.50-4.59(m, 1H), 4.81(d, J= 7.0Hz, 0.33H), 4.90(d, J= 7.0Hz, 0.67H), 5.04(d, J= 7.0 Hz, 0.67H), 5.19(d, J= 7.0 Hz, 0.33H), 5.90-5.95(m, 1H), 6.82-7.02(m, 5H), 7.25-7.47(m, 9H), 8.39(brs, 0.67H), 8.56(brs, 0.33H), 8.60(d, J= 7.5 Hz, 0.33H),
8.63-8.68(m, 1H), 8.77(brs, 0.67H)
ESI-Mass:979.37 [M+Na]⁺

### Production Example 6: Synthesis of AEC reagent

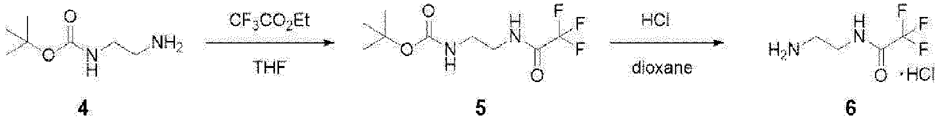

### (1) Synthesis of compound 5

To a solution of compound 4 (25 g, 0.155 mol) in tetrahydrofuran (100 mL) was added ethyl trifluoroacetate (23.1 g, 1.05 eq.) under an argon atmosphere, and the mixture was stirred at room temperature overnight. The reaction mixture was concentrated under reduced pressure, and n-hexane was added to the residue to give the target compound 5 as a white solid (25.4 g, yield 64%) .
¹H-NMR (500 MHz, CDCl₃) δ (ppm) :1.44 (9H,s),
3.36(2H,dd,J=4.5,10.5Hz), 3.46(2H,dd,J=5.5,19.5Hz), 4.96(1H,s), 7.80 (1H,s).

### (2) Synthesis of compound 6

To a solution of compound 5 (25.4 g, 98.8 mmol) in methylene chloride (25 mL) was added dropwise a 4 mol/L hydrochloric acid-dioxane solution water acetic acid (150 mL, 6 eq.) over 30 min under ice-cooling, and the mixture was further stirred at room temperature overnight. To the reaction mixture was slowly added a suspension of sodium hydrogen carbonate and water, and the mixture was stirred at room temperature for 1 hr. The reaction mixture was concentrated under reduced pressure. The residue was crystallized by adding ethyl acetate to give the target compound 6 (AEC reagent) as a white solid (19.0 g, quant.).
¹H-NMR (500 MHz, DMSO-d₆) δ(ppm) :3.43-3.61(4H,m), 8.79(3H,brs), 9.68 (1H,brs).

### Production Example 7: Synthesis of uridine AEC amidite

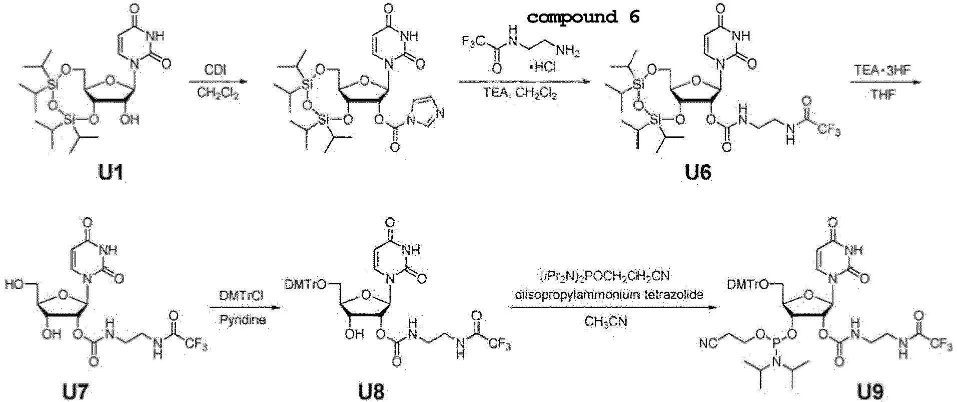

### (1) Synthesis of 3',5'-O-(tetraisopropyldisiloxane-1,3-diyl)-2'-[N-[2-[(trifluoroacetyl)amino]ethyl]carbamate]uridine (U6)

To compound U1 (5.0 g, 10.3 mmol) were added dehydrated dichloromethane (50 mL) and N,N'-carbonyldiimidazole (3.3 g, 20.5 mmol), and the mixture was stirred at room temperature for 1 hr. N,N'-carbonyldiimidazole (0.16 g, 1.0 mmol) was added, and the mixture was further stirred for 3 hr (reaction mixture 1) .

AEC reagent (compound 6; 3.0 g, 15.5 mmol) was dissolved in dehydrated dichloromethane (50 mL), and the mixture was cooled to 0°C. Triethylamine (1.6 g, 15.5 mmol) was added and the mixture was stirred at 0°C for 40 min. Reaction mixture 1 was added, and the mixture was stirred at 0°C for 80 min and at room temperature for 17 hr. Water was added to the reaction mixture, and the mixture was extracted with dichloromethane. The organic layer was washed once with saturated aqueous sodium hydrogen carbonate solution, once with saturated aqueous sodium chloride solution, and dried over magnesium sulfate. The solution was concentrated under reduced pressure to give a white foamy substance (6.4 g) containing the target compound U6. ESI-Mass:691.2386[M+Na]⁺

### (2) Synthesis of 2'-[N-[2-[(trifluoroacetyl)amino]ethyl]carbamate]uridine (U7)

To a solution of compound U6 (3.0 g, 4.5 mmol) in dehydrated tetrahydrofuran (30 mL) was added triethylaminetrihydrofluoride (0.88 mL, 5.4 mmol), and the mixture was stirred at room temperature for 17 hr. The reaction mixture was concentrated under reduced pressure, and the obtained residue was purified by silica gel column chromatography (chloroform:methanol=95:5 - 80:20) to give the target compound U7 as a white solid (0.5 g).
ESI-Mass:449.0869[M+Na]⁺,425.0948[M-H]⁻

### (3) Synthesis of 5'-O-(4,4'-dimethoxytrityl)-2'-[N-[2-[(trifluoroacetyl)amino]ethyl]carbamate]uridine (U8)

To a solution of compound U7 (1.2 g, 2.8 mmol) in dehydrated pyridine (12 mL) was added 4,4'-dimethoxytritylchloride (1.14 g, 3.4 mmol) under an argon atmosphere at 0°C, and the mixture was stirred at room temperature for 2 hr. To the reaction mixture was added methanol (7 mL) and the mixture was stirred for 1 hr. The solution was concentrated under reduced pressure, saturated aqueous sodium hydrogen carbonate solution was added, and the mixture was extracted with dichloromethane. The organic layer was washed with saturated aqueous sodium chloride solution and dried over sodium sulfate. The solution was concentrated under reduced pressure, and the obtained residue was purified by silica gel column chromatography (dichloromethane:methanol=100:0 - 90:10, containing 0.05% pyridine) to give the target compound U8 as a pale-yellow foamy substance (1.76 g).
ESI-Mass:751.2245[M+Na]⁺

### (4) Synthesis of 5'-O-(4,4'-dimethoxytrityl)-2'-[N-[2-[(trifluoroacetyl)amino]ethyl]carbamate]uridine 3'-O-(2-cyanoethyl N,N-diisopropyl phosphoramidite)(U9)

Compound U8 (1.76 g, 2.4 mmol) was azeotropically distilled with dehydrated acetonitrile, and vacuum dried. Diisopropyl ammonium tetrazolide (0.5 g, 3.0 mmol) was added and the mixture was further vacuum dried for 1 hr. Dehydrated acetonitrile (15 mL) was added, and the mixture was stirred under an argon atmosphere at 40°C for 5 min. 2-Cyanoethyl N,N,N',N'-tetraisopropylphosphorodiamidite (0.9 g, 3.0 mmol) was added, and the mixture was stirred at 40°C for 2 hr. The solution was concentrated under reduced pressure, saturated aqueous sodium hydrogen carbonate solution was added, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=40:60 - 10:90, containing 0.1% triethylamine) to give the target compound U9 as a white foamy substance (1.45 g). ESI-Mass:1030.4556[M+TEA+H]⁺
³¹P-NMR (202 MHz, CDCl₃) :151.08,150.80
¹H-NMR (500 MHz, CDCl₃):1.06-1.25(m,12H), 2.44(t,1H), 2.59-2.76(m,1H), 3.35-3.94(m,10H), 3.78-3.80(-OMe,6H), 4.23-4.35(m,1H), 4.67-4.71(m,1H), 5.23-5.42(m,2H), 5.73-5.94(m,1H), 6.20-6.24(m,1H), 6.80-6.86(m,4H), 7.21-7.40(m,9H), 7.72-7.76(m,1H), 7.80-7.89(m,1H), 9.09-9.24(m,1H)

### Production Example 8

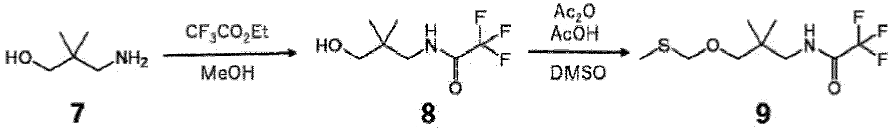

### (1) Synthesis of compound 8

To a solution of compound 7 (5.1 g, 49.7 mmol) in methanol (50 mL) was added ethyl trifluoroacetate (6.5 mL, 1.1 eq.) under an argon atmosphere, and the mixture was stirred at room temperature overnight. The reaction mixture was concentrated under reduced pressure to give the target compound 8 as a colorless oily substance (10.0 g).
ESI-Mass:222.08[M+Na]⁺

### (2) Synthesis of compound 9

To a solution of compound 8 (5.0 g, 25.1 mmol) in dimethyl sulfoxide (17.8 mL) were added acetic acid (12.9 mL, 9.0 eq.) and acetic anhydride (19.0 mL, 8.0 eq.) under an argon atmosphere, and the mixture was stirred at 40°C for 6 hr and at room temperature overnight. Water was added to the reaction mixture, and the mixture was extracted with ethyl acetate. To the organic layer was added slowly a suspension of sodium hydrogen carbonate and water and the mixture was stirred at room temperature for 30 min. Water was added, and the mixture was extracted with ethyl acetate. The organic layer was washed with water, and dried over magnesium sulfate. The solution was concentrated under reduced pressure, and the obtained residue was purified by silica gel column chromatography (n-hexane:ethyl acetate=100:0 - 80:20) to give the target compound 9 as a colorless oily substance (2.4 g).
ESI-Mass:282.08[M+Na]⁺

### Production Example 9

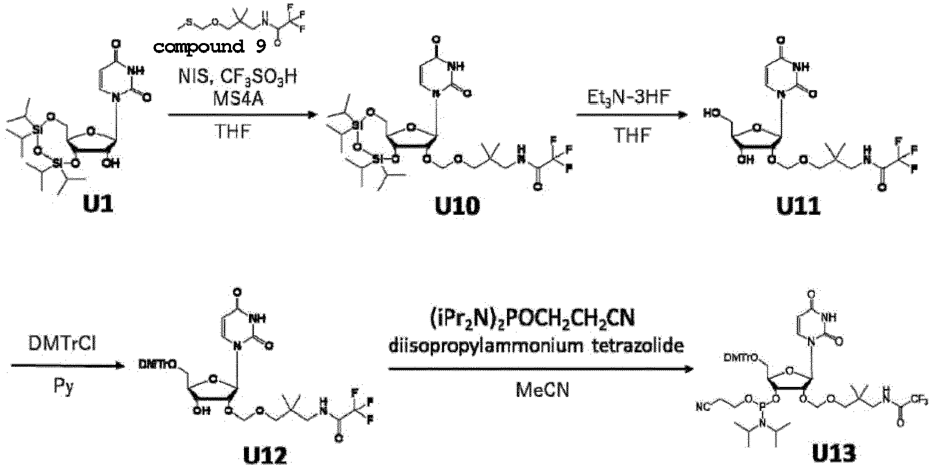

### (1) Synthesis of 3',5'-O-(tetraisopropyldisiloxane-1,3-diyl)-2'-O-[3-(trifluoroacetyl)amino-2,2-dimethylpropoxymethyl]uridine (U10)

To compound U1 (3.3 g, 6.8 mmol) were added dehydrated tetrahydrofuran (30 mL), molecular sieves 4A (3.3 g), and N-iodosuccinimide (1.9 g, 1.2 eq.) under an argon atmosphere, and the mixture was stirred. The mixture was cooled to -45°C. After stirring for 5 min, trifluoromethanesulfonic acid (0.73 mL, 1.2 eq.) was added dropwise. After stirring for 5 min, compound 9 (2.1 g, 1.2 eq.) dissolved in tetrahydrofuran (8 mL) was added, and the mixture was stirred at -45°C for 1 hr. To the reaction mixture was added a mixed solution of a saturated aqueous sodium thiosulfate solution and a saturated aqueous sodium hydrogen carbonate solution at -45°C, and the mixture was stirred at room temperature for 5 min until the brown color disappeared. Water was added to the reaction mixture, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=100:0 - 50:50) to give the target compound U10 as a white foamy substance (4.6 g).
ESI-Mass:720.30[M+Na]⁺

### (2) Synthesis of 2'-O-[3-(trifluoroacetyl)amino-2,2-dimethylpropoxymethyl]uridine (U11)

To a solution of compound U10 (4.5 g, 6.4 mmol) in dehydrated tetrahydrofuran (40 mL) was added TEA/3HF (1.3 mL, 1.2 eq.) under an argon atmosphere, and the mixture was stirred at room temperature for 6 hr. To the reaction mixture were added methanol and silica gel and the solution was concentrated under reduced pressure. The obtained residue was filled in a column and purified by silica gel column chromatography (chloroform:20% methanol/80% chloroform=100:0 - 35:65) to give the target compound U11 as a white foamy substance (2.8 g).
ESI-Mass:478.14[M+Na]⁺

### (3) Synthesis of 5'-O-(4,4'-dimethoxytrityl)-2'-O-[3-(trifluoroacetyl)amino-2,2-dimethylpropoxymethyl]uridine (U12)

To a solution of compound U11 (2.8 g, 6.0 mmol) in dehydrated pyridine (25 mL) was added DMTr-Cl (3.1 g, 1.5 eq.) under an argon atmosphere at 0°C, and the mixture was stirred at room temperature for 5 hr. To the reaction mixture was added DMTr-Cl (1.5 g, 0.75 eq.), and the mixture was stirred at room temperature for 1 hr. To the reaction mixture was added methanol (30 mL), and the mixture was stirred at room temperature for 15 min. The solution was concentrated under reduced pressure and methanol (20 mL) was added. The solution was concentrated under reduced pressure, saturated aqueous sodium hydrogen carbonate solution and water were added, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=80:20 - 30:70, containing 0.1% triethylamine) to give the target compound U12 as a white foamy substance (3.9 g).
ESI-Mass:780.27[M+Na]⁺

### (4) Synthesis of 5'-O-(4,4'-dimethoxytrityl)-2'-O-[3-(trifluoroacetyl)amino-2,2-dimethylpropoxymethyl]uridine 3'-O-(2-cyanoethyl N,N-diisopropyl phosphoramidite)(U13)

To a solution of compound U12 (3.8 g, 5.1 mmol) in dehydrated acetonitrile (40 mL) was added diisopropyl ammonium tetrazolide (1.1 g, 1.3 eq.), 2-cyanoethyl N,N,N',N'-tetraisopropylphosphorodiamidite (2.1 mL, 1.3 eq.) under an argon atmosphere at room temperature, and the mixture was stirred at 40°C for 3 hr. To the reaction mixture were added at room temperature diisopropyl ammonium tetrazolide (0.57 g, 0.65 eq.) and 2-cyanoethyl N,N,N',N'-tetraisopropylphosphorodiamidite (1.0 mL, 0.65 eq.), and the mixture was stirred at 40°C for 1 hr. About half of the solvent was concentrated under reduced pressure, water and saturated aqueous sodium hydrogen carbonate solution were added to the reaction mixture, and the mixture was extracted with ethyl acetate. The organic layer was washed with saturated aqueous sodium chloride solution, and dried over magnesium sulfate. The solution was concentrated under reduced pressure, and the obtained residue was purified by silica gel column chromatography (n-hexane:ethyl acetate=100:30 - 30:70, containing 0.1% triethylamine) to give the target compound U13 as a white foamy substance (3.6 g).
ESI-Mass:980.37[M+Na]⁺

### Production Example 10: Synthesis of each nucleic acid molecule

The nucleic acid molecules shown in the following Examples were synthesized by a nucleic acid synthesizer (trade name: ABI 3900 DNA Synthesizer, Applied Biosystems) based on the phosphoramidite method. Solid phase synthesis was performed using, from the 3'-side, EMM amidite (WO/2013/027843) or TBDMS amidite as RNA amidite, deoxy guanosine (ibu) CED phosphoramidite for g as DNA amidite, as 2'-modified amidite, 2'-fluoroguanosine (ibu) CED phosphoramidite (ChemGenes) for Gf, 2'-O-Methyl Uridine CED phosphoramidite (ChemGenes) for U (italics), 2'-O-Methyl Cytidine (Ac) CED phosphoramidite (ChemGenes) for C (italics), and amidites for crosslinking for the underlined parts in the sequence. As amidites for crosslinking, 2'-Amino(TFA)-uridine-3'-CEP (ChemGenes) for U in ESB2.2-20 of Example 94, AEC amidite (compound U9; Production Example 7) for U in ESB2.2-20 of Example 95, 2'-Amino(TFA)-uridine-3'-CEP (ChemGenes) for U in ESB2.2-20 antisense strand of Example 96, 2'-O-propargyl Uridine CED phosphoramidite (ChemGenes) for U in ESB2.2-20 sense strand of Example 98, 2'-O-Trifluoroacetamido propyl Uridine CED phosphoramidite (ChemGenes) for U in ESB2.2-20 of Example 99, and compound U13 (Production Example 9) for U in ESB2.2-20 of Example 100 were respectively used, and in other Examples, AEM amidites (compound U5; Production Example 2, compound A5; Production Example 3, compound G5; Production Example 4, compound C5; Production Example 5) were used as amidites for crosslinking. Deprotection of the aforementioned amidites was performed according to a conventional method. The synthesized nucleic acid molecules were purified by HPLC.

The sequence information of the nucleic acid molecules used is described in Table 1-1 to -2, Table 2-1 to -3, and Table 3-1 to -19.
SEQ ID NO: 1: si-PH-0153 sense strand (upper panel), AEM4, AEM1, AEM6, AEM28
SEQ ID NO: 2: si-PH-0153 antisense strand (lower panel), AEM2, AEM5, AEM3, AEM8, AEM31, AEM29
SEQ ID NO: 3: AEM7
SEQ ID NO: 4: AEM9
SEQ ID NO: 5: AEM10
SEQ ID NO: 6: AEM13
SEQ ID NO: 7: AEM14
SEQ ID NO: 8: AEM15
SEQ ID NO: 9: AEM17
SEQ ID NO: 10: AEM18, AEM26, AEM27
SEQ ID NO: 11: AEM19
SEQ ID NO: 12: AEM20
SEQ ID NO: 13: AEM21
SEQ ID NO: 14: AEM22
SEQ ID NO: 15: AEM23
SEQ ID NO: 16: AEM32, AEM33, AEM34

**[Table 1-1]**

| | BNC name | structural formula |
|---|---|---|
| Reference Example | si-PH-0153 | 5′-GAUAAGAUGAAUCUCUUCUCC-3′ ; ; ; ; ; ; ; ; ; ; ; ; ; ; ; ; ; ; ; 3′-CUCUAUUCUACUUAGAGAAGA-5′ |
| | AEM4+2 | GAUAAGAUGAAUCUCUUCUCC CUCUAUUCUACUUAGAGAAGA |
| | AEM1+2 | GAUAAGAUGAAUCUCUUCUCC CUCUAUUCUACUUAGAGAAGA |
| | AEM1+5 | GAUAAGAUGAAUCUCUUCUCC CUCUAUUCUACUUAGAGAAGA |
| | AEM6+2 | GAUAAGAUGAAUCUCUUCUCC CUCUAUUCUACUUAGAGAAGA |
| | AEM1+3 | GAUAAGAUGAAUCUCUUCUCC CUCUAUUCUACUUAGAGAAGA |
| | AEM7+8 | AGAUAAGAUGAAUCUCUUCUCC CUCUAUUCUACUUAGAGAAGA |
| | AEM9+10 | UGGAGAUAAGAUGAAUCUCUUCUCC ACCUCUAUUCUACUUAGAGAAGA |
| | AEM13+8 | AGUAAAGAUGAAUCUCUUCUCC CUCUAUUCUACUUAGAGAAGA |
| | AEM14+15 | AUAAGAUGAAUCUCUUCUCC UAUUCUACUUAGAGAAGA |
| | AEM17+18 | UGAUAAGAUGAAUCUCUUCUCC UCUAUUCUACUUAGAGAAGA |
| | AEM7+18 | AGAUAAGAUGAAUCUCUUCUCC UCUAUUCUACUUAGAGAAGA |
| | AEM14+18 | AUAAGAUGAAUCUCUUCUCC UCUAUUCUACUUAGAGAAGA |

**[Table 1-2]**

| | | |
|---|---|---|
| | AEM19+20 | UAGAUAAGAUGAAUCUCUUCUCC UUCUAUUCUACUUAGAGAAGA |
| | AEM21+22 | UAUAAGAUGAAUCUCUUCUCC UUAUAUUCUACUUAGAGAAGA |
| | AEM21+23 | UAUAAGAUGAAUCUCUUCUCC UAUAUUCUACUUAGAGAAGA |
| | AEM14+26 | AUAAGAUGAAUCUCUUCUCC UCUAUUCUACUUAGAGAAGA |
| | AEM14+27 | AUAAGAUGAAUCUCUUCUCC UCUAUUCUACUUAGAGAAGA |
| | AEM28+29 | GAUAAGAUGAAUCUCUUCUCC CUCUAUUCUACUUAGAGAAGA |
| | AEM28+8 | GAUAAGAUGAAUCUCUUCUCC CUCUAUUCUACUUAGAGAAGA |
| | AEM28+31 | GAUAAGAUGAAUCUCUUCUCC CUCU AUUCUACUUAGAGAAGA |
| | AEM7+32 | AGAUAAGAUGAAUCUCUUCUCC CCUCUAUUCUACUUAGAGAAGA |
| | AEM7+33 | AGAUAAGAUGAAUCUCUUCUCC CCUCUAUUCUACUUAGAGAAGA |
| | AEM7+34 | AGAUAAGAUGAAUCUCUUCUCC CCUCUAUUCUACUUAGAGAAGA |

### (2) Example 1 (synthesis of BBN-1)

Using AEM4 and AEM2 described in Table 1-1, an AEM4+2 aqueous solution was produced. Specifically, to a mixture of 1 mmol/L AEM4 aqueous solution (100 µL) and 1 mmol/L AEM2 aqueous solution (100 µL) were added distilled water for injection (70 µL) and 10xAnnealing Buffer (100 mM Tris-HCl (pH 7.5), 200 mM NaCl; 30 µL) at room temperature, and the mixture was shaken at 95°C for 15 min. The mixture was allowed to gradually cool to room temperature to give an AEM4+2 aqueous solution (300 µL). To a mixture of 333 µmol/L AEM4+2 aqueous solution (10 µL), distilled water for injection (26.9 µL), and 1 mol/L phosphate buffer (10 µL) at pH 8.5 was added a dimethylformamide solution (30 eq, 3.1 µL) of 32 mmol/L disuccinimidyl succinate at room temperature, and the mixture was stirred at 30°C for 2 hr. A dimethylformamide solution (60 eq, 6.2 µL) of 32 mmol/L disuccinimidyl succinate was added at room temperature, and the mixture was stirred at 30°C overnight. A dimethylformamide solution (30 eq, 3.1 µL) of 32 mmol/L disuccinimidyl succinate was added at room temperature, and the mixture was stirred at 30°C for 2 hr. Ethanol precipitation of the reaction mixture was performed, and the resulting precipitate was dissolved in distilled water for injection to give BBN-1 with a purity of 68.6%. mass spectrometry: 13488.8 (Calculated: 13488.2). RP-HPLC chart is shown in Fig. 1.

In this Example, disuccinimidyl succinate was used as a compound having a linker structure (linker compound).

### (3) Example 2 (synthesis of BBN-2)

By a method similar to that in (2), synthesis was performed using AEM1 and AEM2 to give BBN-2 with a purity of 66.9%. mass spectrometry: 13488.9 (Calculated: 13488.2). RP-HPLC chart is shown in Fig. 2.

### (4) Example 3 (synthesis of BBN-3)

To a mixture of 333 µmol/L AEM4+2 aqueous solution (10 µL), distilled water for injection (26.7 µL), and 1 mol/L phosphate buffer (10 µL) at pH 8.5 was added a 31 mmol/L dimethylformamide solution (30 eq, 3.3 µL) of disuccinimidyl glutarate at room temperature, and the mixture was stirred at 30°C overnight. Ethanol precipitation of the reaction mixture was performed, and the resulting precipitate was dissolved in distilled water for injection to give BBN-3 with a purity of 77.9%. mass spectrometry: 13502.9 (Calculated: 13502.2). RP-HPLC chart is shown in Fig. 3.

In this Example, disuccinimidyl glutarate was used as a linker compound.

### (5) Example 4 (synthesis of BBN-4)

To a mixture of 333 µmol/L AEM4+2 aqueous solution (10 µL), distilled water for injection (24.3 µL), and 1 mol/L phosphate buffer (10 µL) at pH 8.5 was added a 17 mmol/L BS3 aqueous solution (30 eq, 5.7 µL) at room temperature, and the mixture was stirred at 30°C overnight. Ethanol precipitation of the reaction mixture was performed, and the resulting precipitate was dissolved in distilled water for injection to give BBN-4 with a purity of 81.6%. mass spectrometry: 13545.0 (Calculated: 13544.3). RP-HPLC chart is shown in Fig. 4.
BS3: Bis(sulfosuccinimidyl)suberate, disodium salt (DOJINDO LABORATORIES)

In this Example, BS3 was used as a linker compound.

### (6) Example 5 (synthesis of BBN-5)

To a mixture of 333 µmol/L AEM4+2 aqueous solution (50 µL), distilled water for injection (145.7 µL), and 1 mol/L phosphate buffer (50 µL) at pH 8.5 was added a dimethylformamide solution (10 eq, 4.3 µL) of 39 mmol/L succinimidyl carbonate at room temperature, and the mixture was stirred at 15°C for 2 hr. A dimethylformamide solution (30 eq, 12.9 µL) of 39 mmol/L succinimidyl carbonate was added at room temperature, and the mixture was stirred at 15°C overnight. A dimethylformamide solution (20 eq, 4.3 µL) of 78 mmol/L succinimidyl carbonate was added at room temperature, and the mixture was stirred at 15°C for 2 hr. 1 mol/L phosphate buffer (50 µL) at pH8.5 and a dimethylformamide solution (20 eq, 4.3 µL) of 78 mmol/L succinimidyl carbonate were added at room temperature, and the mixture was stirred at 15°C for 3 hr. 1 mol/L phosphate buffer (50 µL) at pH8.5 and a dimethylformamide solution (30 eq, 6.5 µL) of 78 mmol/L succinimidyl carbonate were added at room temperature, and the mixture was stirred at 15°C for 2 hr. 1 mol/L phosphate buffer (50 µL) at pH8.5 and a dimethylformamide solution (30 eq, 6.5 µL) of 78 mmol/L succinimidyl carbonate were added at room temperature, and the mixture was stirred at 15°C for 1 hr. Ethanol precipitation of the reaction mixture was performed, and the resulting precipitate was dissolved in distilled water for injection, and purified by HPLC (column: XBridge Oligonucleotide, BEH C18, 2.5 µm, 10 mmx50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 50% CH₃CN). Ethanol precipitation of the purified product was performed, and the resulting precipitate was dissolved in distilled water for injection to give BBN-5 with a purity of 99.1%. mass spectrometry: 13432.8 (Calculated: 13432.1). RP-HPLC chart is shown in Fig. 5.

In this Example, succinimidyl carbonate was used as a linker compound.

### (7) Example 6 (synthesis of BBN-6)

To a mixture of 1100 µmol/L AEM4 aqueous solution (18.2 µL) and 0.2 mol/L phosphate buffer (200 µL) at pH 8.5 was added a dimethylformamide solution (50 eq, 23.7 µL) of 42 mmol/L N-succinimidylbromacetate at room temperature, and the mixture was stirred at 25°C for 1 hr. A dimethylformamide solution (25 eq, 11.8 µL) of 42 mmol/L N-succinimidylbromoacetate was added at room temperature, and the mixture was stirred at 25°C for 1 hr. Ethanol precipitation of the reaction mixture was performed to give an intermediate. To a mixture of the intermediate and 0.2 mol/L phosphate buffer (150 µL) at pH 8.5 was added a 879 µmol/L AEM2 aqueous solution (1.0 eq, 22.8 µL) at room temperature, and the mixture was stirred at 25°C overnight. 1 mol/L phosphate buffer (50 µL) at pH8.5 was added at room temperature, and the mixture was stirred at 50°C for 8 hr. The reaction mixture was purified by HPLC (column: XBridge Oligonucleotide, BEH C18, 2.5 µm, 10 mm x 50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 50% CH₃CN). Ethanol precipitation of the purified product was performed, and the resulting precipitate was dissolved in distilled water for injection to give BBN-6 with a purity of 100%. mass spectrometry: 13446.9 (Calculated: 13446.1). RP-HPLC chart is shown in Fig. 6.

In this Example, N-succinimidylbromoacetate was used as a linker compound.

### (8) Example 7 (synthesis of BBN-7)

Synthesis by a method similar to that in (7) and using AEM4 and AEM2 gave BBN-7 with a purity of 100%. mass spectrometry: 13446.9 (Calculated: 13446.1). RP-HPLC chart is shown in Fig. 7. In this Example, BBN-7 was synthesized by exchanging the AEM4 aqueous solution and the AEM2 aqueous solution in BBN-6 synthesis.

### (9) Example 8 (synthesis of BBN-8)

To a mixture of 333 µmol/L AEM1+5 aqueous solution (50 µL) and 0.2 mol/L phosphate buffer (200 µL) at pH 8.5 was added a dimethylformamide solution (30 eq, 15.6 µL) of 32 mmol/L disuccinimidyl succinate at room temperature, and the mixture was stirred at 25°C for 1 hr. Ethanol precipitation of the reaction mixture was performed, and the resulting precipitate was dissolved in distilled water for injection and purified by HPLC (column: XBridge Oligonucleotide, BEH C18, 2.5 µm, 10 mmx50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 50% CH₃CN). Ethanol precipitation of the purified product was performed, and the resulting precipitate was dissolved in distilled water for injection to give BBN-8 with a purity of 99.7%. mass spectrometry: 13489.1 (Calculated: 13488.2). RP-HPLC chart is shown in Fig. 8.

In this Example, disuccinimidyl succinate was used as a linker compound.

### (10) Examples 9, 10 (synthesis of BBN-9, 10)

BBN-9, 10 were synthesized by a method similar to that in (9) and using AEM6+2 aqueous solution and AEM1+3 aqueous solution, respectively.

BBN-9 with a purity of 99.9% was obtained. mass spectrometry: 13489.0 (Calculated: 13488.2). RP-HPLC chart is shown in Fig. 9.

BBN-10 with a purity of 100% was obtained. mass spectrometry: 13488.9 (Calculated: 13488.2). RP-HPLC chart is shown in Fig. 10.

### (11) Example 11 (synthesis of BBN-11)

To a mixture of 250 µmol/L AEM7+8 aqueous solution (80 µL), distilled water for injection (116 µL), and 1 mol/L phosphate buffer (52 µL) at pH 8.5 was added a dimethylformamide solution (30 eq, 12 µL) of 50 mmol/L disuccinimidyl succinate at room temperature, and the mixture was stirred at 25°C for 3 hr. Ethanol precipitation of the reaction mixture was performed, and the resulting precipitate was dissolved in distilled water for injection and purified by HPLC (column: XBridge Oligonucleotide, BEH C18, 2.5 µm, 10 mmx50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 50% CH₃CN). Ethanol precipitation of the purified product was performed, and the resulting precipitate was dissolved in distilled water for injection to give BBN-11 with a purity of 96.3%. mass spectrometry: 13818.0 (Calculated: 13817.4). RP-HPLC chart is shown in Fig. 11.

In this Example, disuccinimidyl succinate was used as a linker compound.

### (12) Examples 13, 17, 19 (synthesis of BBN-13, 17, 19)

BBN-13, 17, 19 were synthesized by a method similar to that in (11) and using AEM9+10 aqueous solution, AEM13+18 aqueous solution, AEM14+15 aqueous solution, respectively.

BBN-13 with a purity of 99.6% was obtained. mass spectrometry: 15449.0 (Calculated: 15448.4). RP-HPLC chart is shown in Fig. 13.

BBN-17 with a purity of 100% was obtained. mass spectrometry: 13818.1 (Calculated: 13817.4). RP-HPLC chart is shown in Fig. 15.

BBN-19 with a purity of 99.8% was obtained. mass spectrometry: 12227.1 (Calculated: 12226.5). RP-HPLC chart is shown in Fig. 17.

### (13) Example 12 (synthesis of BBN-12)

To a mixture of 250 µmol/L AEM7+8 aqueous solution (80 µL), distilled water for injection (116 µL), and 1 mol/L phosphate buffer (52 µL) at pH 8.5 was added an aqueous solution (30 eq, 12 µL) of 50 mmol/L BS3 at room temperature, and the mixture was stirred at 25°C for 3 hr. The reaction mixture was purified by HPLC (column: XBridge Oligonucleotide, BEH C18, 2.5 µm, 10 mm x 50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 50% CH₃CN). Ethanol precipitation of the purified product was performed, and the resulting precipitate was dissolved in distilled water for injection to give BBN-12 with a purity of 100%. mass spectrometry: 13874.0 (Calculated: 13873.5). RP-HPLC chart is shown in Fig. 12.

In this Example, BS3 was used as a linker compound.

### (14) Examples 14, 18, 20 to 34 (synthesis of BBN-14, 18, 20 to 34)

BBN-14, 18, 20 to 34 were synthesized by a method similar to that in (13) and using AEM9+10 aqueous solution, AEM13+8 aqueous solution, AEM14+15 aqueous solution, AEM17+18 aqueous solution, AEM7+18 aqueous solution, AEM14+18 aqueous solution, AEM19+20 aqueous solution, AEM21+22 aqueous solution, AEM21+23 aqueous solution, AEM14+26 aqueous solution, AEM14+27 aqueous solution, AEM28+29 aqueous solution, AEM28+8 aqueous solution, AEM28+31 aqueous solution, AEM7+32 aqueous solution, AEM7+33 aqueous solution, AEM7+34 aqueous solution, respectively.

BBN-14 with a purity of 99.0% was obtained. mass spectrometry: 15505.0 (Calculated: 15504.5). RP-HPLC chart is shown in Fig. 14.

BBN-18 with a purity of 99.6% was obtained. mass spectrometry: 13874.1 (Calculated: 13873.5). RP-HPLC chart is shown in Fig. 16.

BBN-20 with a purity of 99.6% was obtained. mass spectrometry: 12283.1 (Calculated: 12282.6). RP-HPLC chart is shown in Fig. 18.

BBN-21 with a purity of 99.3% was obtained. mass spectrometry: 13546.0 (Calculated: 13545.3). RP-HPLC chart is shown in Fig. 19.

BBN-22 with a purity of 100% was obtained. mass spectrometry: 13568.9 (Calculated: 13568.3). RP-HPLC chart is shown in Fig. 20.

BBN-23 with a purity of 99.9% was obtained. mass spectrometry: 12894.5 (Calculated: 12893.9). RP-HPLC chart is shown in Fig. 21-1.

BBN-24 with a purity of 99.9% was obtained. mass spectrometry: 14181.3 (Calculated: 14180.7). RP-HPLC chart is shown in Fig. 22.

BBN-25 with a purity of 100% was obtained. mass spectrometry: 13530.9 (Calculated: 13530.3). RP-HPLC chart is shown in Fig. 23.

BBN-26 with a purity of 99.9% was obtained. mass spectrometry: 13224.8 (Calculated: 13224.1). RP-HPLC chart is shown in Fig. 24.

BBN-27 with a purity of 98.7% was obtained. mass spectrometry: 12894.8 (Calculated: 12893.9). RP-HPLC chart is shown in Fig. 25-1.

BBN-28 with a purity of 99.9% was obtained. mass spectrometry: 12894.6 (Calculated: 12893.9). RP-HPLC chart is shown in Fig. 26-1.

BBN-29 with a purity of 99.4% was obtained. mass spectrometry: 13545.1 (Calculated: 13544.3). RP-HPLC chart is shown in Fig. 27-1.

BBN-30 with a purity of 100% was obtained. mass spectrometry: 13545.0 (Calculated: 13544.3). RP-HPLC chart is shown in Fig. 28-1.

BBN-31 with a purity of 99.8% was obtained. mass spectrometry: 13544.9 (Calculated: 13544.3). RP-HPLC chart is shown in Fig. 29.

BBN-32 with a purity of 99.6% was obtained. mass spectrometry: 14179.5 (Calculated: 14178.7). RP-HPLC chart is shown in Fig. 30-1.

BBN-33 with a purity of 99.9% was obtained. mass spectrometry: 14179.4 (Calculated: 14178.7). RP-HPLC chart is shown in Fig. 31-1.

BBN-34 with a purity of 99.8% was obtained. mass spectrometry: 14179.3 (Calculated: 14178.7). RP-HPLC chart is shown in Fig. 32.

### <nucleic acid molecules list-1>

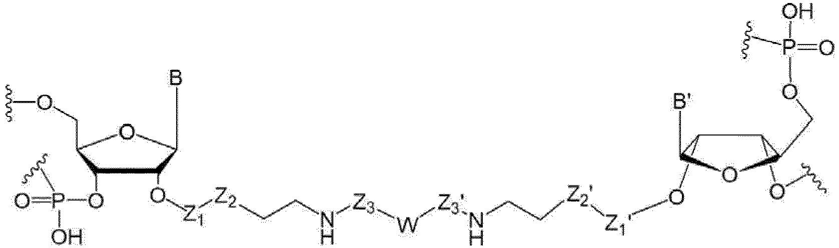

**[Table 2-1]**

| Ex. No. | BNC name | structural formula | linker |
|---|---|---|---|
| Reference Example | si-PH-0153 | 5′-GAUAAGAUGAAUCUCUUCUCC-3 ; ; ; ; ; ; ; ; ; ; ; ; ; ; ; ; ; ; ; 3′-CUCUAUUCUACUUAGAGAAGA-5′ | - |
| 1 | BBN-1 | GAUAAGAUGAAUCUCUUCUCC CUCUAUUCUACUUAGAGAAGA | 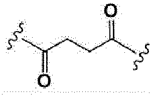 |
| 2 | BBN-2 | GAUAAGAUGAAUCUCUUCUCC CUCUAUUCUACUUAGAGAAGA | 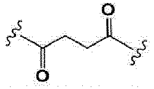 |
| 3 | BBN-3 | GAUAAGAUGAAUCUCUUCUCC CUCUAUUCUACUUAGAGAAGA | 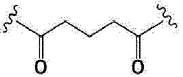 |
| 4 | BBN-4 | GAUAAGAUGAAUCUCUUCUCC CUCUAUUCUACUUAGAGAAGA | 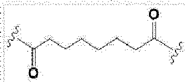 |
| 5 | BBN-5 | GAUAAGAUGAAUCUCUUCUCC CUCUAUUCUACUUAGAGAAGA | 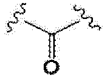 |
| 6 | BBN-6 | GAUAAGAUGAAUCUCUUCUCC CUCUAUUCUACUUAGAGAAGA | 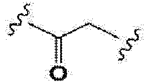 |
| 7 | BBN-7 | GAUAAGAUGAAUCUCUUCUCC CUCUAUU CUACUUAGAGAAGA | 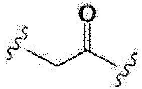 |
| 8 | BBN-8 | GAUAAGAUGAAUCUCUUCUCC CUCUAUUCUACUUAGAGAAGA | 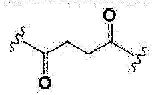 |
| 9 | BBN-9 | GAUAAGAUGAAUCUCUUCUCC CUCUAUUCUACUUAGAGAAGA | 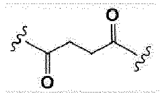 |
| 10 | BBN-10 | GAUAAGAUGAAUCUCUUCUCC CUCUAUUCUACUUAGAGAAGA | 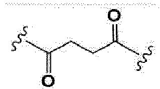 |
| 11 | BBN-11 | AGAUAAGAUGAAUCUCUUCUCC CUCUAUUCUACUUAGAGAAGA | 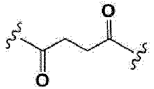 |
| 12 | BBN-12 | AGAUAAGAUGAAUCUCUUCUCC CUCUAUUCUACUUAGAGAAGA | 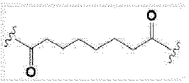 |

Reference example: SEQ ID NO: 1, SEQ ID NO: 2
BBN1:SEQ ID NO: 1, SEQ ID NO: 2
BBN2:SEQ ID NO: 1, SEQ ID NO: 2
BBN3:SEQ ID NO: 1, SEQ ID NO: 2
BBN4:SEQ ID NO: 1, SEQ ID NO: 2
BBN5:SEQ ID NO: 1, SEQ ID NO: 2
BBN6:SEQ ID NO: 1, SEQ ID NO: 2
BBN7:SEQ ID NO: 1, SEQ ID NO: 2
BBN8:SEQ ID NO: 1, SEQ ID NO: 2
BBN9:SEQ ID NO: 1, SEQ ID NO: 2
BBN10:SEQ ID NO: 1, SEQ ID NO: 2
BBN11:SEQ ID NO: 3, SEQ ID NO: 2
BBN12:SEQ ID NO: 3, SEQ ID NO: 2

**[Table 2-2]**

| | | | |
|---|---|---|---|
| 13 | BBN-13 | UGGAGAUAAGAUGAAUCUCUUCUCC ACCUCUAUUCUACUUAGAGAAGA | 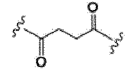 |
| 14 | BBN-14 | UGGAGAUAAGAUGAAUCUCUUCUCC ACCUCUAUUCUACUUAGAGAAGA | 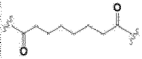 |
| 17 | BBN-17 | AGUAAAGAUGAAUCUCUUCUCC CUCUAUUCUACUUAGAGAAGA | 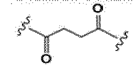 |
| 18 | BBN-18 | AGUAAAGAUGAAUCUCUUCUCC CUCUAUUCUACUUAGAGAAGA | 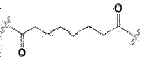 |
| 19 | BBN-19 | AUAAGAUGAAUCUCUUCUCC UAUUCUACUUAGAGAAGA | 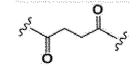 |
| 20 | BBN-20 | AUAAGAUGAAUCUCUUCUCC UAUUCUACUUAGAGAAGA | 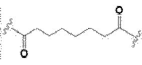 |
| 21 | BBN-21 | UGAUAAGAUGAAUCUCUUCUCC UCUAUUCUACUUAGAGAAGA | 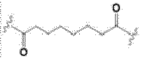 |
| 22 | BBN-22 | AGAUAAGAUGAAUCUCUUCUCC UCUAUUCUACUUAGAGAAGA | 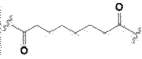 |
| 23 | BBN-23 | AUAAGAUGAAUCUCUUCUCC UCUAUUCUACUUAGAGAAGA | 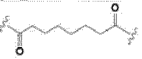 |
| 24 | BBN-24 | UAGAUAAGAUGAAUCUCUUCUCC UUCUAUUCUACUUAGAGAAGA | 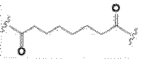 |
| 25 | BBN-25 | UAUAAGAUGAAUCUCUUCUCC UUAUAUUCUACUUAGAGAAGA | 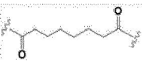 |
| 26 | BBN-26 | UAUAAGAUGAAUCUCUUCUCC UAUAUUCUACUUAGAGAAGA | 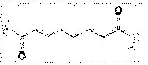 |

BBN13:SEQ ID NO: 4, SEQ ID NO: 5
BBN14:SEQ ID NO: 4, SEQ ID NO: 5
BBN17:SEQ ID NO: 6, SEQ ID NO: 2
BBN18:SEQ ID NO: 6, SEQ ID NO: 2
BBN19:SEQ ID NO: 7, SEQ ID NO: 8
BBN20:SEQ ID NO: 7, SEQ ID NO: 8
BBN21:SEQ ID NO: 9, SEQ ID NO: 10
BBN22:SEQ ID NO: 3, SEQ ID NO: 10
BBN23:SEQ ID NO: 7, SEQ ID NO: 10
BBN24:SEQ ID NO: 11, SEQ ID NO: 12
BBN25:SEQ ID NO: 13, SEQ ID NO: 14
BBN26:SEQ ID NO: 13, SEQ ID NO: 15

**[Table 2-3]**

| | | | |
|---|---|---|---|
| 27 | BBN-27 |   AUAAGAUGAAUCUCUUCUCC UCUAUUCUACUUAGAGAAGA | 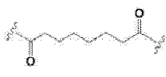 |
| 28 | BBN-28 | AUAAGAUGAAUCUCUUCUCC UCUAUUCUACUUAGAGAAGA | 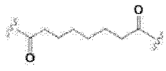 |
| 29 | BBN-29 | GAUAAGAUGAAUCUCUUCUCC CUCUAUUCUACUUAGAGAAGA | 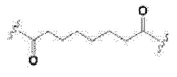 |
| 30 | BBN-30 | GAUAAGAUGAAUCUCUUCUCC CUCUAUUCUACUUAGAGAAGA | 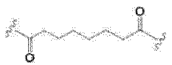 |
| 31 | BBN-31 | GAUAAGAUGAAUCUCUUCUCC CUCUAUUCUACUUAGAGAAGA | 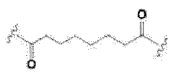 |
| 32 | BBN-32 | AGAUAAGAUGAAUCUCUUCUCC CCUCUAUUCUACUUAGAGAAGA | 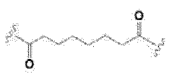 |
| 33 | BBN-33 | AGAUAAGAUGAAUCUCUUCUCC CCUCUAUUCUACUUAGAGAAGA | 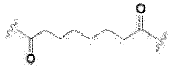 |
| 34 | BBN-34 |   AGAUAAGAUGAAUCUCUUCUCC CCUCUAUUCUACUUAGAGAAGA | 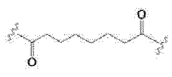 |

BBN27:SEQ ID NO: 7, SEQ ID NO: 10
BBN28:SEQ ID NO: 7, SEQ ID NO: 10
BBN29:SEQ ID NO: 1, SEQ ID NO: 10
BBN30:SEQ ID NO: 1, SEQ ID NO: 10
BBN31:SEQ ID NO: 1, SEQ ID NO: 10
BBN32:SEQ ID NO: 3, SEQ ID NO: 16
BBN33:SEQ ID NO: 3, SEQ ID NO: 16
BBN34:SEQ ID NO: 3, SEQ ID NO: 16

### Example 23-2

In a 200 mM phosphate buffer (pH 8.5), 10 nmol each of the sense strand and antisense strand of ESB2.2_HPRT1-3 were mixed to a final concentration of 100 µM, a 300 nmol BS3 aqueous solution was added, and the mixture was stirred at 25°C for 3 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 23-2 with a purity of 99.5% was obtained.
mass spectrometry: 12918.8 (Calculated: 12918.9)
RP-HPLC chart after purification is shown in Fig. 21-2.

In this Example, BS3 was used as a linker compound. BS3: Bis(sulfosuccinimidyl)suberate, disodium salt (DOJINDO LABORATORIES)

### Example 27-2

Synthesis by a method similar to that in Example 23-2 and using the sense strand and antisense strand of ESB2.2-20-2 gave Example 27-2 with a purity of 99.8%.
mass spectrometry: 13111.0 (Calculated: 13111.1)
RP-HPLC chart after purification is shown in Fig. 25-2.

### Example 28-2

Synthesis by a method similar to that in Example 23-2 and using the sense strand and antisense strand of ESB2.2-20-3 gave Example 28-2 with a purity of 99.9%.
mass spectrometry: 13110.6 (Calculated: 13111.1)
RP-HPLC chart after purification is shown in Fig. 26-2.

### Example 29-2

Synthesis by a method similar to that in Example 23-2 and using the sense strand and antisense strand of ESB2.2-20-4 gave Example 29-2 with a purity of 99.6%.
mass spectrometry: 13723.3 (Calculated: 13723.4)
RP-HPLC chart after purification is shown in Fig. 27-2.

### Example 30-2

Synthesis by a method similar to that in Example 23-2 and using the sense strand and antisense strand of ESB2.2-20-5 gave Example 30-2 with a purity of 99.2%.
mass spectrometry: 13723.3 (Calculated: 13723.4)
RP-HPLC chart after purification is shown in Fig. 28-2.

### Example 30-3

Synthesis by a method similar to that in Example 23-2 and using the sense strand and antisense strand of ESB_HPRT1 gave Example 30-3 with a purity of 96.5%.
mass spectrometry: 13670.3 (Calculated: 13669.6)
RP-HPLC chart after purification is shown in Fig. 28-3.

### Example 30-4

Synthesis by a method similar to that in Example 23-2 and using the sense strand and antisense strand of ESB2.2_HPRT1-4 gave Example 30-4 with a purity of 100.0%.
mass spectrometry: 13529.3 (Calculated: 13529.3)
RP-HPLC chart after purification is shown in Fig. 28-4.

### Example 30-5

Synthesis by a method similar to that in Example 23-2 and using the sense strand and antisense strand of 6b1 gave Example 30-5 with a purity of 94.5%.
mass spectrometry: 13709.9 (Calculated: 13709.5)
RP-HPLC chart after purification is shown in Fig. 28-5.

### Example 32-2

Synthesis by a method similar to that in Example 23-2 and using the sense strand and antisense strand of ESB2.2-20-6 gave Example 32-2 with a purity of 100.0%.
mass spectrometry: 14334.9 (Calculated: 14334.8)
RP-HPLC chart after purification is shown in Fig. 30-2.

### Example 33-2

Synthesis by a method similar to that in Example 23-2 and using the sense strand and antisense strand of ESB2.2-17 gave Example 33-2 with a purity of 100.0%.
mass spectrometry: 14295.4 (Calculated: 14294.8)
RP-HPLC chart after purification is shown in Fig. 31-2.

### Example 33-3

Synthesis by a method similar to that in Example 23-2 and using the sense strand and antisense strand of ESB2.2-18 gave Example 33-3 with a purity of 99.9%.
mass spectrometry: 14374.3 (Calculated: 14373.8)
RP-HPLC chart after purification is shown in Fig. 31-3.

### Example 33-4

Synthesis by a method similar to that in Example 23-2 and using the sense strand and antisense strand of ESB2.2-19 gave Example 33-4 with a purity of 100.0%.
mass spectrometry: 14390.3 (Calculated: 14389.8)
RP-HPLC chart after purification is shown in Fig. 31-4.

### Example 33-5

Synthesis by a method similar to that in Example 23-2 and using the sense strand and antisense strand of ESB2.2-20 gave Example 33-5 with a purity of 99.9%.
mass spectrometry: 14335.4 (Calculated: 14334.8)
RP-HPLC chart after purification is shown in Fig. 31-5.

### Example 33-6

Synthesis by a method similar to that in Example 23-2 and using the sense strand and antisense strand of ESB2.2-21 gave Example 33-6 with a purity of 100.0%.
mass spectrometry: 14413.2 (Calculated: 14412.9)
RP-HPLC chart after purification is shown in Fig. 31-6.

### Example 33-7

Synthesis by a method similar to that in Example 23-2 and using the sense strand and antisense strand of ESB2.2-22 gave Example 31-7 with a purity of 100.0%.
mass spectrometry: 14363.5 (Calculated: 14362.8)
RP-HPLC chart after purification is shown in Fig. 31-7.

### Example 33-8

Synthesis by a method similar to that in Example 23-2 and using the sense strand and antisense strand of ESB2.2-23 gave Example 33-8 with a purity of 99.8%.
mass spectrometry: 14342.3 (Calculated: 14341.8)
RP-HPLC chart after purification is shown in Fig. 31-8.

### Example 33-9

Synthesis by a method similar to that in Example 23-2 and using the sense strand and antisense strand of ESB2.2-24 gave Example 33-9 with a purity of 100.0%.
mass spectrometry: 14364.3 (Calculated: 14363.8)
RP-HPLC chart after purification is shown in Fig. 31-9.

### Example 33-10

Synthesis by a method similar to that in Example 23-2 and using the sense strand and antisense strand of ESB2.2-25 gave Example 33-10 with a purity of 99.9%.
mass spectrometry: 14351.1 (Calculated: 14350.8)
RP-HPLC chart after purification is shown in Fig. 31-10.

### Example 33-11

Synthesis by a method similar to that in Example 23-2 and using the sense strand and antisense strand of ESB2.2-26 gave Example 33-11 with a purity of 100.0%.
mass spectrometry: 14390.4 (Calculated: 14389.8)
RP-HPLC chart after purification is shown in Fig. 31-11.

### Example 33-12

Synthesis by a method similar to that in Example 23-2 and using the sense strand and antisense strand of ESB2.2-27 gave Example 33-12 with a purity of 99.8%.
mass spectrometry: 14428.4 (Calculated: 14427.9)
RP-HPLC chart after purification is shown in Fig. 31-12.

### Example 33-13

Synthesis by a method similar to that in Example 23-2 and using the sense strand and antisense strand of ESB2.2-28 gave Example 33-13 with a purity of 100.0%.
mass spectrometry: 14414.5 (Calculated: 14413.8)
RP-HPLC chart after purification is shown in Fig. 31-13.

### Example 33-14

Synthesis by a method similar to that in Example 23-2 and using the sense strand and antisense strand of ESB2.2-29 gave Example 31-14 with a purity of 99.9%.
mass spectrometry: 14389.5 (Calculated: 14388.8)
RP-HPLC chart after purification is shown in Fig. 31-14.

### Example 33-15

Synthesis by a method similar to that in Example 23-2 and using the sense strand and antisense strand of ESB2.2-30 gave Example 33-15 with a purity of 99.9%.
mass spectrometry: 14360.3 (Calculated: 14359.8)
RP-HPLC chart after purification is shown in Fig. 31-15.

### Example 33-16

Synthesis by a method similar to that in Example 23-2 and using the sense strand and antisense strand of ESB2.2-31 gave Example 33-16 with a purity of 100.0%.
mass spectrometry: 14318.3 (Calculated: 14317.8)
RP-HPLC chart after purification is shown in Fig. 31-16.

### Example 33-17

Synthesis by a method similar to that in Example 23-2 and using the sense strand and antisense strand of ESB2.2-32 gave Example 33-17 with a purity of 94.3%.
mass spectrometry: 14317.2 (Calculated: 14316.8)
RP-HPLC chart after purification is shown in Fig. 31-17.

### Example 33-18

Synthesis by a method similar to that in Example 23-2 and using the sense strand and antisense strand of ESB2.2-33 gave Example 33-18 with a purity of 99.9%.
mass spectrometry: 14357.4 (Calculated: 14356.8)
RP-HPLC chart after purification is shown in Fig. 31-18.

### Example 33-19

Synthesis by a method similar to that in Example 23-2 and using the sense strand and antisense strand of ESB2.2-34 gave Example 33-19 with a purity of 99.8%.
mass spectrometry: 14335.3 (Calculated: 14334.8)
RP-HPLC chart after purification is shown in Fig. 31-19.

### Example 33-20

Synthesis by a method similar to that in Example 23-2 and using the sense strand and antisense strand of ESB2.2-35 gave Example 33-20 with a purity of 85.3%.
mass spectrometry: 14304.4 (Calculated: 14303.8)
RP-HPLC chart after purification is shown in Fig. 31-20.

### Example 33-21

Synthesis by a method similar to that in Example 23-2 and using the sense strand and antisense strand of ESB2.2-36 gave Example 33-21 with a purity of 99.8%.
mass spectrometry: 14241.4 (Calculated: 14240.7)
RP-HPLC chart after purification is shown in Fig. 31-21.

### Example 33-22

Synthesis by a method similar to that in Example 23-2 and using the sense strand and antisense strand of ESB2.2-37 gave Example 33-22 with a purity of 98.9%.
mass spectrometry: 14344.2 (Calculated: 14343.8)
RP-HPLC chart after purification is shown in Fig. 31-22.

### Example 33-23

Synthesis by a method similar to that in Example 23-2 and using the sense strand and antisense strand of ESB2.2-38 gave Example 33-23 with a purity of 96.0%.
mass spectrometry: 14328.3 (Calculated: 14327.8)
RP-HPLC chart after purification is shown in Fig. 31-23.

### Example 33-24

Synthesis by a method similar to that in Example 23-2 and using the sense strand and antisense strand of ESB2.2-39 gave Example 33-24 with a purity of 97.4%.
mass spectrometry: 14337.6 (Calculated: 14336.8)
RP-HPLC chart after purification is shown in Fig. 31-24.

### Example 33-25

The 879 µmol/L sense strand (11.4 µL) of ESB2.2-20-fd, the 956 µmol/L antisense strand (10.5 µL) of ESB2.2-20-fd, a 30 mmol/L BS3 aqueous solution (10 µL), distilled water for injection (8.2 µL), and 1 mol/L phosphate buffer (pH 8.5, 10 µL) were mixed and stirred at 30°C for 2.5 hr. Ethanol precipitation of the reaction mixture was performed, and the resulting precipitate was dissolved in distilled water for injection to give Example 33-25 with a purity of 80.6%. mass spectrometry: 14321.10 (Calculated: 14320.77). RP-HPLC chart after purification is shown in Fig. 31-25.

### Example 33-26

Synthesis by a method similar to that in Example 23-2 and using the sense strand and antisense strand of ESB2.2_HPRT1-1 gave Example 33-26 with a purity of 97.8%.
mass spectrometry: 14164.8 (Calculated: 14164.6)
RP-HPLC chart after purification is shown in Fig. 31-26.

### Example 33-27

Synthesis by a method similar to that in Example 23-2 and using the sense strand and antisense strand of 6b1-4 gave Example 33-27 with a purity of 97.1%.
mass spectrometry: 14321.4 (Calculated: 14320.7)
RP-HPLC chart after purification is shown in Fig. 31-27.

### Example 35

To a mixture of the sense strand (10 nmol, 7.8 µL) and antisense strand (10 nmol, 13.6 µL) of BBN39 were added distilled water for injection (68.6 µL) and 10×Annealing Buffer (100 mM Tris-HCl (pH 7.5), 200 mM NaCl; 10 µL) at room temperature, and the mixture was shaken at 95°C for 15 min and allowed to gradually cool to room temperature. To an aqueous solution of this double stranded RNA were added distilled water for injection (14 µL), 1 mol/L phosphate buffer (30 µL) at pH 8.5, and a 50 mmol/L BS3 aqueous solution (6 µL) at room temperature, and the mixture was stirred at 25°C for 4 hr. The reaction mixture was purified by HPLC (column: XBridge Oligonucleotide, BEH C18, 2.5 µm, 10 mm×50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 90% CH₃CN). Ethanol precipitation of the purified product was performed, and the resulting precipitate was dissolved in distilled water for injection to give Example 35 with a purity of 93.8%. mass spectrometry: 11647.9 (Calculated: 11647.2). RP-HPLC chart is shown in Fig. 33.

In this Example, BS3 was used as a linker compound.

### Example 36

Synthesis by a method similar to that in Example 35 and using the sense strand and antisense strand of BBN40 gave Example 36 with a purity of 94.2%. mass spectrometry: 11647.8 (Calculated: 11647.2). RP-HPLC chart is shown in Fig. 34.

### Example 37

Synthesis by a method similar to that in Example 35 and using the sense strand and antisense strand of BBN41 gave Example 37 with a purity of 95.3%. mass spectrometry: 11647.8 (Calculated: 11647.2). RP-HPLC chart is shown in Fig. 35.

### Example 38

Synthesis by a method similar to that in Example 35 and using the sense strand and antisense strand of BBN42 gave Example 38 with a purity of 99.3%. mass spectrometry: 12259.2 (Calculated: 12258.5). RP-HPLC chart is shown in Fig. 36-1.

### Example 38-2

Synthesis by a method similar to that in Example 23-2 and using the sense strand and antisense strand of ESB2.2-20-1 gave Example 38-2 with a purity of 99.9%.
mass spectrometry: 12475.7 (Calculated: 12475.7)
RP-HPLC chart after purification is shown in Fig. 36-2.

### Example 39

Synthesis by a method similar to that in Example 35 and using the sense strand and antisense strand of BBN43 gave Example 39 with a purity of 98.5%. mass spectrometry: 12259.2 (Calculated: 12258.5). RP-HPLC chart is shown in Fig. 37.

### Example 40

Synthesis by a method similar to that in Example 35 and using the sense strand and antisense strand of BBN44 gave Example 40 with a purity of 99.1%. mass spectrometry: 12259.1 (Calculated: 12258.5). RP-HPLC chart is shown in Fig. 38-1

### Example 40-2

Synthesis by a method similar to that in Example 23-2 and using the sense strand and antisense strand of ESB2.2_HPRT1-2 gave Example 40-2 with a purity of 100.0%.
mass spectrometry: 12306.7 (Calculated: 12306.6)
RP-HPLC chart after purification is shown in Fig. 38-2.

### Example 41

Synthesis by a method similar to that in Example 35 and using the sense strand and antisense strand of BBN45 gave Example 41 with a purity of 97.5%. mass spectrometry: 14853.9 (Calculated: 14853.1). RP-HPLC chart is shown in Fig. 39-1.

### Example 41-2

Synthesis by a method similar to that in Example 23-2 and using the sense strand and antisense strand of ESB2.2-20-8 gave Example 41-2 with a purity of 96.4%.
mass spectrometry: 14969.2 (Calculated: 14969.2)
RP-HPLC chart after purification is shown in Fig. 39-2.

### Example 42

Synthesis by a method similar to that in Example 35 and using the sense strand and antisense strand of BBN46 gave Example 42 with a purity of 99.3%. mass spectrometry: 14853.9 (Calculated: 14853.1). RP-HPLC chart is shown in Fig. 40-1.

### Example 42-2

Synthesis by a method similar to that in Example 23-2 and using the sense strand and antisense strand of ESB2.2-20-9 gave Example 42-2 with a purity of 97.9%.
mass spectrometry: 14969.2 (Calculated: 14969.2)
RP-HPLC chart after purification is shown in Fig. 40-2.

### Example 43

Synthesis by a method similar to that in Example 35 and using the sense strand and antisense strand of BBN47 gave Example 43 with a purity of 99.7%. mass spectrometry: 14853.8 (Calculated: 14853.1). RP-HPLC chart is shown in Fig. 41-1.

### Example 43-2

Synthesis by a method similar to that in Example 23-2 and using the sense strand and antisense strand of ESB2.2-20-10 gave Example 43-2 with a purity of 98.9%.
mass spectrometry: 14969.2 (Calculated: 14969.2)
RP-HPLC chart after purification is shown in Fig. 41-2.

### Example 44

Synthesis by a method similar to that in Example 35 and using the sense strand and antisense strand of BBN48 gave Example 44 with a purity of 98.2%. mass spectrometry: 15505.0 (Calculated: 15504.5). RP-HPLC chart is shown in Fig. 42.

### Example 45

Synthesis by a method similar to that in Example 35 and using the sense strand and antisense strand of BBN49 gave Example 45 with a purity of 94.1%. mass spectrometry: 15505.2 (Calculated: 15504.5). RP-HPLC chart is shown in Fig. 43.

### Example 46

Synthesis by a method similar to that in Example 35 and using the sense strand and antisense strand of BBN50 gave Example 46 with a purity of 95.7%. mass spectrometry: 15505.1 (Calculated: 15504.5). RP-HPLC chart is shown in Fig. 44-1.

### Example 46-2

Synthesis by a method similar to that in Example 23-2 and using the sense strand and antisense strand of ESB2.2_HPRT1-5 gave Example 46-2 with a purity of 99.5%.
mass spectrometry: 15529.4 (Calculated: 15529.5)
RP-HPLC chart after purification is shown in Fig. 44-2.

### Example 47

Synthesis by a method similar to that in Example 35 and using the sense strand and antisense strand of BBN51 gave Example 47 with a purity of 98.9%. mass spectrometry: 13545.0 (Calculated: 13544.3). RP-HPLC chart is shown in Fig. 45.

### Example 48

Synthesis by a method similar to that in Example 35 and using the sense strand and antisense strand of BBN52 gave Example 48 with a purity of 97.1%. mass spectrometry: 14179.3 (Calculated: 14178.7). RP-HPLC chart is shown in Fig. 46-1.

### Example 48-2

Synthesis by a method similar to that in Example 23-2 and using the sense strand and antisense strand of ESB2.2-20-7 gave Example 48-2 with a purity of 100.0%.
mass spectrometry: 14334.9 (Calculated: 14334.8)
RP-HPLC chart after purification is shown in Fig. 46-2.

### Example 49

Synthesis by a method similar to that in Example 35 and using the sense strand and antisense strand of BBN53 gave Example 49 with a purity of 97.3%. mass spectrometry: 14179.4 (Calculated: 14178.7). RP-HPLC chart is shown in Fig. 47.

### Example 50

In a 200 mM phosphate buffer (pH 8.5), 10 nmol each of the sense strand and antisense strand of ESB2.2-1 were mixed to a final concentration of 100 µM, a 300 nmol BS3 aqueous solution was added, and the mixture was stirred at 25°C for 3 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 50 with a purity of 97.1% was obtained.
mass spectrometry: 13545.0 (Calculated: 13544.3)
RP-HPLC chart after purification is shown in Fig. 48-1.

In this Example, BS3 was used as a linker compound.

### Example 50-2

Synthesis by a method similar to that in Example 50 and using the sense strand and antisense strand of ESB2.2_HPRT1-6 gave Example 50-2 with a purity of 99.7%.
mass spectrometry: 14164.8 (Calculated: 14164.6)
RP-HPLC chart after purification is shown in Fig. 48-2.

### Example 51

Synthesis by a method similar to that in Example 50 and using the sense strand and antisense strand of ESB2.2-2 gave Example 51 with a purity of 99.3%.
mass spectrometry: 13544.9 (Calculated: 13544.3)
RP-HPLC chart after purification is shown in Fig. 49-1.

### Example 51-2

Synthesis by a method similar to that in Example 50 and using the sense strand and antisense strand of ESB2.2-20-15 gave Example 51-2 with a purity of 99.0%.
mass spectrometry: 14334.9 (Calculated: 14334.8)
RP-HPLC chart after purification is shown in Fig. 49-2.

### Example 52

Synthesis by a method similar to that in Example 50 and using the sense strand and antisense strand of ESB2.2-3 gave Example 52 with a purity of 97.0%.
mass spectrometry: 13545.0 (Calculated: 13544.3)
RP-HPLC chart after purification is shown in Fig. 50-1.

### Example 52-2

Synthesis by a method similar to that in Example 50 and using the sense strand and antisense strand of ESB2.2-20-16 gave Example 52-2 with a purity of 97.1%.
mass spectrometry: 14335.5 (Calculated: 14334.8)
RP-HPLC chart after purification is shown in Fig. 50-2.

### Example 53

Synthesis by a method similar to that in Example 50 and using the sense strand and antisense strand of ESB2.2-4 gave Example 53 with a purity of 96.6%.
mass spectrometry: 13544.9 (Calculated: 13544.3)
RP-HPLC chart after purification is shown in Fig. 51-1.

### Example 53-2

Synthesis by a method similar to that in Example 50 and using the sense strand and antisense strand of ESB2.2-20-17 gave Example 53-2 with a purity of 100.0%.
mass spectrometry: 14334.9 (Calculated: 14334.8)
RP-HPLC chart after purification is shown in Fig. 51-2.

### Example 54

Synthesis by a method similar to that in Example 50 and using the sense strand and antisense strand of ESB2.2-5 gave Example 54 with a purity of 97.7%.
mass spectrometry: 13569.0 (Calculated: 13568.3)
RP-HPLC chart after purification is shown in Fig. 52-1.

### Example 54-2

Synthesis by a method similar to that in Example 50 and using the sense strand and antisense strand of ESB2.2-20-22 gave Example 54-2 with a purity of 98.9%.
mass spectrometry: 13724.0 (Calculated: 13723.4)
RP-HPLC chart after purification is shown in Fig. 52-2.

### Example 55

Synthesis by a method similar to that in Example 50 and using the sense strand and antisense strand of ESB2.2-6 gave Example 55 with a purity of 95.9%.
mass spectrometry: 13874.0 (Calculated: 13873.5)
RP-HPLC chart after purification is shown in Fig. 53-1.

### Example 55-2

Synthesis by a method similar to that in Example 50 and using the sense strand and antisense strand of ESB2.2-20-21 gave Example 55-2 with a purity of 99.8%.
mass spectrometry: 14029.6 (Calculated: 14029.6)
RP-HPLC chart after purification is shown in Fig. 53-2.

### Example 55-3

Synthesis by a method similar to that in Example 50 and using the sense strand and antisense strand of ESB2.2_HPRT1-9 gave Example 55-3 with a purity of 98.8%.
mass spectrometry: 13858.4 (Calculated: 13858.5)
RP-HPLC chart after purification is shown in Fig. 53-3.

### Example 56

Synthesis by a method similar to that in Example 50 and using the sense strand and antisense strand of ESB2.2-7 gave Example 56 with a purity of 97.5%.
mass spectrometry: 14508.5 (Calculated: 14507.9)
RP-HPLC chart after purification is shown in Fig. 54-1.

### Example 56-2

Synthesis by a method similar to that in Example 50 and using the sense strand and antisense strand of ESB2.2-20-19 gave Example 56-2 with a purity of 100.0%.
mass spectrometry: 14640.0 (Calculated: 14640.0)
RP-HPLC chart after purification is shown in Fig. 54-2.

### Example 57

Synthesis by a method similar to that in Example 50 and using the sense strand and antisense strand of ESB2.2-8 gave Example 57 with a purity of 98.3%.
mass spectrometry: 14508.4 (Calculated: 14507.9)
RP-HPLC chart after purification is shown in Fig. 55-1.

### Example 57-2

Synthesis by a method similar to that in Example 50 and using the sense strand and antisense strand of ESB2.2-20-20 gave Example 57-2 with a purity of 99.3%.
mass spectrometry: 14640.0 (Calculated: 14640.0)
RP-HPLC chart after purification is shown in Fig. 55-2.

### Example 58

Synthesis by a method similar to that in Example 50 and using the sense strand and antisense strand of ESB2.2-9 gave Example 58 with a purity of 97.3%.
mass spectrometry: 14508.6 (Calculated: 14507.9)
RP-HPLC chart after purification is shown in Fig. 56.

### Example 58-2

Synthesis by a method similar to that in Example 50 and using the sense strand and antisense strand of ESB2.2_HPRT1-8 gave Example 58-2 with a purity of 98.0%.
mass spectrometry: 14509.9 (Calculated: 14509.8)
RP-HPLC chart after purification is shown in Fig. 56-2.

### Example 59

Synthesis by a method similar to that in Example 50 and using the sense strand and antisense strand of ESB2.2-10 gave Example 59 with a purity of 97.8%.
mass spectrometry: 14814.7 (Calculated: 14814.1)
RP-HPLC chart after purification is shown in Fig. 57-1.

### Example 59-2

Synthesis by a method similar to that in Example 50 and using the sense strand and antisense strand of ESB2.2-20-18 gave Example 59-2 with a purity of 96.9%.
mass spectrometry: 14985.1 (Calculated: 14985.2)
RP-HPLC chart after purification is shown in Fig. 57-2.

### Example 60

Synthesis by a method similar to that in Example 50 and using the sense strand and antisense strand of ESB2.2-11 gave Example 60 with a purity of 92.6%.
mass spectrometry: 14814.7 (Calculated: 14814.1)
RP-HPLC chart after purification is shown in Fig. 58-1.

### Example 60-2

Synthesis by a method similar to that in Example 50 and using the sense strand and antisense strand of ESB2.2_HPRT1-7 gave Example 60-2 with a purity of 99.9%.
mass spectrometry: 14855.1 (Calculated: 14855.1)
RP-HPLC chart after purification is shown in Fig. 58-2.

### Example 61

Synthesis by a method similar to that in Example 50 and using the sense strand and antisense strand of ESB2.2-12 gave Example 61 with a purity of 98.4%.
mass spectrometry: 14814.6 (Calculated: 14814.1)
RP-HPLC chart after purification is shown in Fig. 59.

### Example 62

Synthesis by a method similar to that in Example 50 and using the sense strand and antisense strand of ESB2.2-20-23 gave Example 62 with a purity of 99.0%.
mass spectrometry: 13644.2 (Calculated: 13644.4)
RP-HPLC chart after purification is shown in Fig. 60-1.

### Example 62-2

Synthesis by a method similar to that in Example 50 and using the sense strand and antisense strand of ESB2.2_HPRT1-10 gave Example 62-2 with a purity of 98.0%.
mass spectrometry: 13554.2 (Calculated: 13554.3)
RP-HPLC chart after purification is shown in Fig. 60-2.

### Example 63

Synthesis by a method similar to that in Example 50 and using the sense strand and antisense strand of ESB2.2-20-24 gave Example 63 with a purity of 98.5%.
mass spectrometry: 14946.2 (Calculated: 14946.1)
RP-HPLC chart after purification is shown in Fig. 61.

### Example 64

Synthesis by a method similar to that in Example 50 and using the sense strand and antisense strand of ESB2.2-NEK-S21 gave Example 64 with a purity of 100.0%.
mass spectrometry: 13849.5 (Calculated: 13849.5)
RP-HPLC chart after purification is shown in Fig. 62-1.

### Example 64-2

Synthesis by a method similar to that in Example 50 and using the sense strand and antisense strand of ESB2.2-20-S21 gave Example 64-2 with a purity of 98.7%.
mass spectrometry: 14028.7 (Calculated: 14028.6)
RP-HPLC chart after purification is shown in Fig. 62-2.

### Example 65

Synthesis by a method similar to that in Example 50 and using the sense strand and antisense strand of ESB2.2-NEK-S19 gave Example 65 with a purity of 99.8%.
mass spectrometry: 13174.9 (Calculated: 13175.1)
RP-HPLC chart after purification is shown in Fig. 63-1.

### Example 65-2

Synthesis by a method similar to that in Example 50 and using the sense strand and antisense strand of ESB2.2-20-S19 gave Example 65-2 with a purity of 99.8%.
mass spectrometry: 13416.1 (Calculated: 13416.3)
RP-HPLC chart after purification is shown in Fig. 63-2.

### Example 66

Synthesis by a method similar to that in Example 50 and using the sense strand and antisense strand of ESB2.2-NEK-S18 gave Example 66 with a purity of 98.8%.
mass spectrometry: 12868.9 (Calculated: 12868.9)
RP-HPLC chart after purification is shown in Fig. 64-1.

### Example 66-2

Synthesis by a method similar to that in Example 50 and using the sense strand and antisense strand of ESB2.2-20-S18 gave Example 66-2 with a purity of 99.9%.
mass spectrometry: 13071.0 (Calculated: 13071.1)
RP-HPLC chart after purification is shown in Fig. 64-2.

### Example 67

Synthesis by a method similar to that in Example 50 and using the sense strand and antisense strand of ESB2.2-NEK-S17 gave Example 67 with a purity of 99.9%.
mass spectrometry: 12539.7 (Calculated: 12539.7)
RP-HPLC chart after purification is shown in Fig. 65-1.

### Example 67-2

Synthesis by a method similar to that in Example 50 and using the sense strand and antisense strand of ESB2.2-20-S17 gave Example 67-2 with a purity of 98.8%.
mass spectrometry: 12725.9 (Calculated: 12725.9)
RP-HPLC chart after purification is shown in Fig. 65-2.

### Example 68

Synthesis by a method similar to that in Example 6 and using the sense strand and antisense strand of ESB2.2-15 gave Example 68.
mass spectrometry: 13446.7 (Calculated: 13446.1)

### Example 69

In a 200 mM phosphate buffer (pH 8.5), 3 nmol each of the sense strand and antisense strand of ESB2.2-15 were mixed to a final concentration of 100 µM, a 50 mM disuccinimidyl succinate/DMF solution (90 nmol) was added, and the mixture was stirred at 25°C for 3 hr. The reaction mixture was purified (column: XBridge Oligonucleotide BEH C18, 2.5 µm, 4.6×50 mm; flow rate: 1 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 69 with a purity of 97.4% was obtained.
mass spectrometry: 13488.8 (Calculated: 13488.2)
RP-HPLC chart after purification is shown in Fig. 66.

### Example 70

Synthesis by a method similar to that in Example 6 and using the sense strand and antisense strand of ESB2.2-16 gave Example 70 with a purity of 98.2%.
mass spectrometry: 13446.6 (Calculated: 13446.1)
RP-HPLC chart after purification is shown in Fig. 67.

### Example 71

Synthesis by a method similar to that in Example 69 and using the sense strand and antisense strand of ESB2.2-16 gave Example 71 with a purity of 94.0%.
mass spectrometry: 13488.9 (Calculated: 13488.2)
RP-HPLC chart after purification is shown in Fig. 68.

### Example 72

Synthesis by a method similar to that in Example 6 and using the sense strand and antisense strand of ESB2.2-13 gave Example 72 with a purity of 97.0%.
mass spectrometry: 14081.2 (Calculated: 14080.5)
RP-HPLC chart after purification is shown in Fig. 69.

### Example 73

Synthesis by a method similar to that in Example 69 and using the sense strand and antisense strand of ESB2.2-13 gave Example 73 with a purity of 97.7%.
mass spectrometry: 14123.3 (Calculated: 14122.6)
RP-HPLC chart after purification is shown in Fig. 70.

### Example 74

Synthesis by a method similar to that in Example 6 and using the sense strand and antisense strand of ESB2.2-14 gave Example 74 with a purity of 92.9%.
mass spectrometry: 14081.1 (Calculated: 14080.5)
RP-HPLC chart after purification is shown in Fig. 71.

### Example 75

Synthesis by a method similar to that in Example 69 and using the sense strand and antisense strand of ESB2.2-14 gave Example 75 with a purity of 97.7%.
mass spectrometry: 14123.4 (Calculated: 14122.6)
RP-HPLC chart after purification is shown in Fig. 72.

### Production Example 11

### Synthesis of linker compound 10

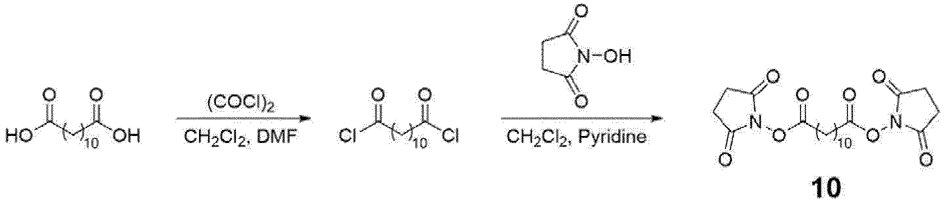

To a solution of dodecanedioic acid (115 mg, 0.5 mmol) in dichloromethane (2 mL) was added N,N-dimethylformamide (20 µL), and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 1 hr. The reaction mixture was concentrated under reduced pressure. To a solution of the obtained residue in dichloromethane (2 mL) were added N-hydroxysuccinimide (140 mg, 1.2 mmol) and pyridine (0.5 mL), and the mixture was stirred at room temperature for 2 hr. Water was added to the reaction mixture, and the mixture was extracted with dichloromethane. The organic layer was washed with saturated aqueous sodium chloride solution and dried over sodium sulfate. The solution was concentrated under reduced pressure to give a crude product (232 mg) containing compound 10.

### Example 76

100 nmol each of the sense strand and antisense strand of ESB2.2-16 were mixed, and distilled water for injection was added to adjust to 480 µL. Isopropanol (300 µL) was added, and a 20 mM compound 10/DMF solution (200 µL) and triethylamine (20 µL) were successively added, and the mixture was stirred at 25°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 76 with a purity of 90.0% was obtained. mass spectrometry: 13601.4 (Calculated: 13600.4). RP-HPLC chart after purification is shown in Fig. 73-1.

### Example 76-2

100 nmol each of the sense strand and antisense strand of ESB2.2-20 were mixed, and distilled water for injection was added to adjust to 480 µL. Isopropanol (300 µL) was added, and a 20 mM compound 10/DMF solution (200 µL) and triethylamine (20 µL) were successively added, and the mixture was stirred at 25°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 76-2 with a purity of 84.4% was obtained. mass spectrometry: 14391.4 (Calculated: 14390.9). RP-HPLC chart after purification is shown in Fig. 73-2.

### Production Example 12

### Synthesis of linker compound 11

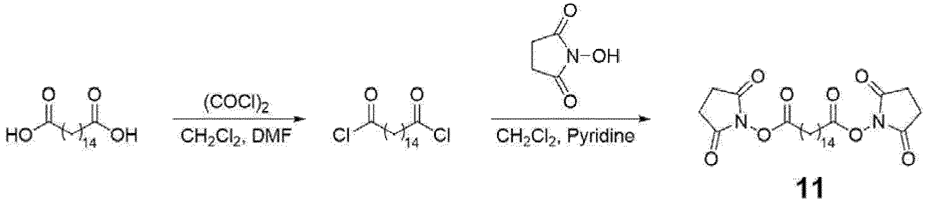

To a solution of hexadecanedioic acid (143 mg, 0.5 mmol) in dichloromethane (2 mL) was added N,N-dimethylformamide (20 µL), and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 1 hr. The reaction mixture was concentrated under reduced pressure. To a solution of the obtained residue in dichloromethane (2 mL) were added N-hydroxysuccinimide (140 mg, 1.2 mmol) and pyridine (0.5 mL), and the mixture was stirred at room temperature for 2 hr. Water was added to the reaction mixture, and the mixture was extracted with dichloromethane. The organic layer was washed with saturated aqueous sodium chloride solution and dried over sodium sulfate. The solution was concentrated under reduced pressure to give a crude product (288 mg) containing compound 11.

### Example 77

100 nmol each of the sense strand and antisense strand of ESB2.2-16 were mixed, and distilled water for injection was added to 480 µL. Isopropanol (300 µL) was added, and a 20 mM compound 11/DMF solution (200 µL) and triethylamine (20 µL) were successively added, and the mixture was stirred at 25°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 77 with a purity of 99.3% was obtained. mass spectrometry: 13657.2 (Calculated: 13656.5). RP-HPLC chart after purification is shown in Fig. 74-1.

### Example 77-2

100 nmol each of the sense strand and antisense strand of ESB2.2-20 were mixed, and distilled water for injection was added to 480 µL. Isopropanol (300 µL) was added, and a 20 mM compound 11/DMF solution (200 µL) and triethylamine (20 µL) were successively added, and the mixture was stirred at 25°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 77-2 with a purity of 79.3% was obtained. mass spectrometry: 14447.6 (Calculated: 14447.0). RP-HPLC chart after purification is shown in Fig. 74-2.

### Production Example 13

### Synthesis of linker compound 12

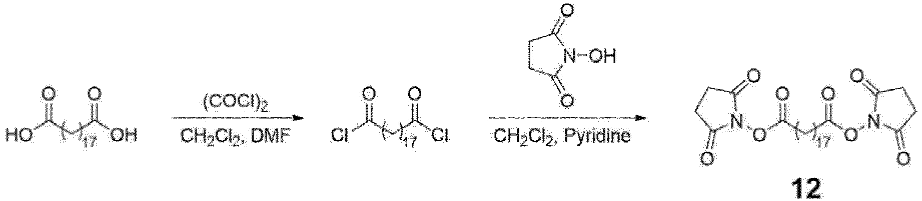

To a solution of nonadecanedioic acid (164 mg, 0.5 mmol) in dichloromethane (2 mL) was added N,N-dimethylformamide (20 µL), and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 1 hr. The reaction mixture was concentrated under reduced pressure. To a solution of the obtained residue in dichloromethane (2 mL) were added N-hydroxysuccinimide (140 mg, 1.2 mmol) and pyridine (0.5 mL), and the mixture was stirred at room temperature for 2 hr. Water was added to the reaction mixture, and the mixture was extracted with dichloromethane. The organic layer was washed with saturated aqueous sodium chloride solution and dried over sodium sulfate. The solution was concentrated under reduced pressure to give a crude product (301 mg) containing compound 12.

### Example 78

100 nmol each of the sense strand and antisense strand of ESB2.2-16 were mixed, and distilled water for injection was added to 480 µL. Isopropanol (300 µL) was added, and a 20 mM compound 12/DMF solution (200 µL) and triethylamine (20 µL) were successively added, and the mixture was stirred at 25°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 78 with a purity of 96.1% was obtained. mass spectrometry: 13699.4 (Calculated: 13698.6). RP-HPLC chart after purification is shown in Fig. 75-1.

### Example 78-2

100 nmol each of the sense strand and antisense strand of ESB2.2-20 were mixed, and distilled water for injection was added to 480 µL. Isopropanol (300 µL) was added, and a 20 mM compound 12/DMF solution (200 µL) and triethylamine (20 µL) were successively added, and the mixture was stirred at 25°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 78-2 with a purity of 94.8% was obtained. mass spectrometry: 14489.7 (Calculated: 14489.1). RP-HPLC chart after purification is shown in Fig. 75-2.

### Example 79

1.4 mmol/L AEM28 (13.7 µL), 1.2 mmol/L AEM8 (15.8 µL), a 50 mmol/L aqueous solution (12 µL) of Sulfo-EGS Crosslinker (Funakoshi Co., Ltd.), distilled water for injection (38.5 µL), and 1 mol/L phosphate buffer (pH 8.5, 20 µL) were mixed and stirred at 30°C for 3 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 79 with a purity of 99.3% was obtained. mass spectrometry: 13633.31 (Calculated: 13632.38). RP-HPLC chart after purification is shown in Fig. 76-1.
Sulfo-EGS: Ethylene glycolbis(sulfosuccinimidylsuccinate) (Funakoshi Co., Ltd.)

### Example 79-2

A 890 µmol/L sense strand (22.5 µL) of ESB2.2-20, a 956 µmol/L antisense strand (20.9 µL) of ESB2.2-20, a 50 mmol/L aqueous solution (12 µL) of Sulfo-EGS Crosslinker (Funakoshi Co., Ltd.), distilled water for injection (24.6 µL), and 1 mol/L phosphate buffer (pH 8.5, 20 µL) were mixed and stirred at 25°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 79-2 with a purity of 95.3% was obtained. mass spectrometry: 14423.20 (Calculated: 14422.78). RP-HPLC chart after purification is shown in Fig. 76-2.

### Production Example 14

### Synthesis of linker compound 13

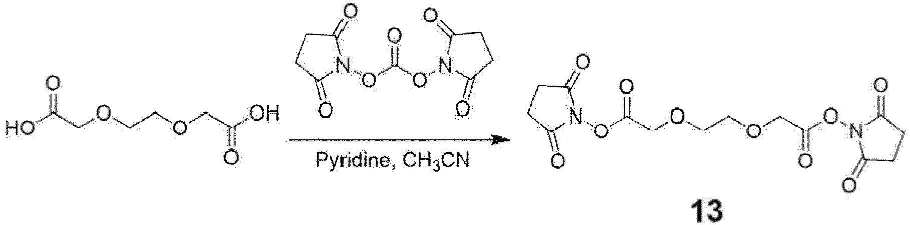

To a solution of 3,6-dioxaoctanedioic acid (178 mg, 1.0 mmol) in acetonitrile (3 mL) were added pyridine (162 µL) and N,N'-disuccinimidylcarbonate (512 mg, 2.0 mmol), and the mixture was stirred at room temperature for 3 hr. The solution was concentrated under reduced pressure, 1 mol/L hydrochloric acid was added, and the mixture was extracted with dichloromethane. The organic layer was dried over sodium sulfate and the solution was concentrated under reduced pressure to give a crude product (179 mg) containing compound 13.
ESI-Mass:395.0699[M+Na]⁺

### Example 80

1.4 mmol/L AEM28 (27.4 µL), 1.2 mmol/L AEM8 (31.7 µL), a 50 mmol/L compound 13/DMSO solution (16 µL), distilled water for injection (44.9 µL), and 1 mol/L phosphate buffer (pH 8.5, 30 µL) were mixed and stood still at 21°C for 15 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 80 with a purity of 98.8% was obtained. mass spectrometry: 13549.4 (Calculated: 13548.2). RP-HPLC chart after purification is shown in Fig. 77.

### Production Example 15

### Synthesis of linker compound 14

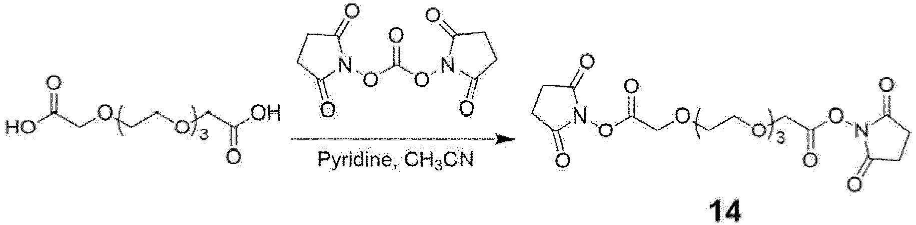

To a solution of COOH-PEG5-COOH (134 mg, 0.5 mmol) in acetonitrile (2 mL) were added pyridine (82 µL) and N,N'-disuccinimidylcarbonate (258 mg, 1.0 mmol), and the mixture was stirred at room temperature for 2.5 hr. The solution was concentrated under reduced pressure, 1 mol/L hydrochloric acid was added, and the mixture was extracted with dichloromethane. The organic layer was dried over magnesium sulfate and the solution was concentrated under reduced pressure to give a crude product (129 mg) containing compound 14.
ESI-Mass:483.1192 [M+Na] ⁺

### Example 81

1.4 mmol/L AEM28 (27.4 µL), 1.2 mmol/L AEM8 (31.7 µL), 50 mmol/L compound 14/DMSO solution (16 µL), distilled water for injection (44.9 µL), and 1 mol/L phosphate buffer (pH 8.5, 30 µL) were mixed and stirred at 30°C for 45 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 81 with a purity of 99.8% was obtained. mass spectrometry: 13637.20 (Calculated: 13636.42). RP-HPLC chart after purification is shown in Fig. 78-1.

### Example 81-2

A 890 µmol/L sense strand (33.7 µL) of ESB2.2-20, a 956 µmol/L antisense strand (31.4 µL) of ESB2.2-20, a 50 mmol/L compound 14/DMF solution (18 µL), distilled water for injection (36.9 µL), and 1 mol/L phosphate buffer (pH 8.5, 30 µL) were mixed and stirred at 25°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 81-2 with a purity of 91.9% was obtained. mass spectrometry: 14427.30 (Calculated: 14426.82). RP-HPLC chart after purification is shown in Fig. 78-2.

### Production Example 16

### Synthesis of linker compound 15

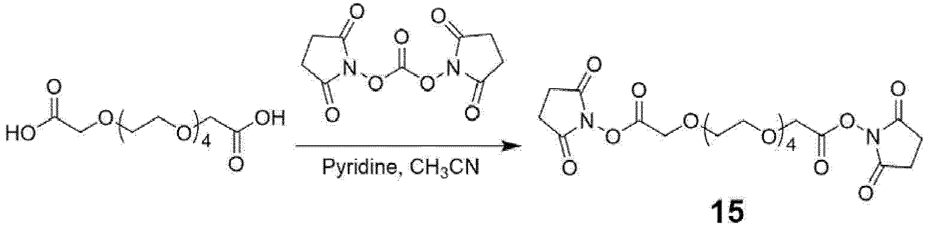

To a solution of COOH-PEG6-COOH (152 mg, 0.5 mmol) in acetonitrile (2 mL) were added pyridine (81 µL) and N,N'-disuccinimidylcarbonate (251 mg, 1.0 mmol), and the mixture was stirred at room temperature for 2.5 hr. The solution was concentrated under reduced pressure, 1 mol/L hydrochloric acid was added, and the mixture was extracted with dichloromethane. The organic layer was dried over magnesium sulfate and the solution was concentrated under reduced pressure to give a crude product (205 mg) containing compound 15.
ESI-Mass:527.1419[M+Na]⁺

### Example 82

1.4 mmol/L AEM28 (27.4 µL), 1.2 mmol/L AEM8 (31.7 µL), 50 mmol/L compound 15/DMSO solution (16 µL), distilled water for injection (44.9 µL), and 1 mol/L phosphate buffer (pH 8.5, 30 µL) were mixed and stirred at 30°C for 45 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 82 with a purity of 99.4% was obtained. mass spectrometry: 13681.30 (Calculated: 13680.47). RP-HPLC chart after purification is shown in Fig. 79-1.

### Example 82-2

A 890 µmol/L sense strand (33.7 µL) of ESB2.2-20, a 956 µmol/L antisense strand (31.4 µL) of ESB2.2-20, a 50 mmol/L compound 15/DMF solution (18 µL), distilled water for injection (36.9 µL), and 1 mol/L phosphate buffer (pH 8.5, 30 µL) were mixed and stirred at 25°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 82-2 with a purity of 93.6% was obtained. mass spectrometry: 14471.10 (Calculated: 14470.87). RP-HPLC chart after purification is shown in Fig. 79-2.

### Example 83

1.4 mmol/L AEM28 (13.7 µL), 1.2 mmol/L AEM8 (15.8 µL), a 50 mmol/L DMF solution (12 µL) of DST Crosslinker (Funakoshi Co., Ltd.), distilled water for injection (38.5 µL), and 1 mol/L phosphate buffer (pH 8.5, 20 µL) were mixed and stirred at 30°C for 3 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 83 with a purity of 99.6% was obtained. mass spectrometry: 13521.10 (Calculated: 13520.26). RP-HPLC chart after purification is shown in Fig. 80-1.
DST: Disuccinimidyl tartrate (Funakoshi Co., Ltd.)

### Example 83-2

A 890 µmol/L sense strand (22.5 µL) of ESB2.2-20, a 956 µmol/L antisense strand (20.9 µL) of ESB2.2-20, a 50 mmol/L DMF solution (12 µL) of DST Crosslinker (Funakoshi Co., Ltd.), distilled water for injection (24.6 µL), and 1 mol/L phosphate buffer (pH 8.5, 20 µL) were mixed and stirred at 25°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 83-2 with a purity of 95.1% was obtained. mass spectrometry: 14311.20 (Calculated: 14310.66). RP-HPLC chart after purification is shown in Fig. 80-2.

### Production Example 17

### Synthesis of linker compound 16

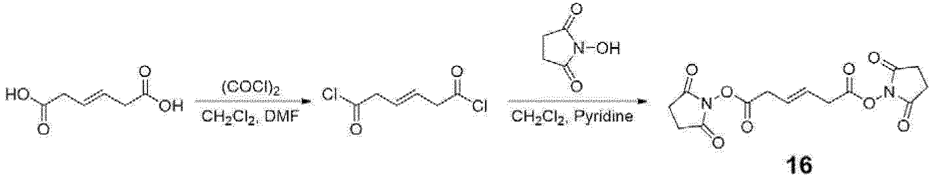

To a solution of trans-3-hexenedioic acid (72 mg, 0.5 mmol) in dichloromethane (2 mL) was added N,N-dimethylformamide (20 µL) , and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 50 min. The reaction mixture was concentrated under reduced pressure. To a solution of the obtained residue in dichloromethane (2 mL) were added N-hydroxysuccinimide (140 mg, 1.2 mmol) and pyridine (0.5 mL), and the mixture was stirred at room temperature for 2 hr. Water was added to the reaction mixture, and the mixture was extracted with dichloromethane. The organic layer was washed with saturated aqueous sodium chloride solution and dried over magnesium sulfate. The solution was concentrated under reduced pressure to give a crude product (186 mg) containing compound 16.

### Example 84

1.4 mmol/L AEM28 (20.5 µL), 1.2 mmol/L AEM8 (23.8 µL), a 50 mmol/L compound 16/DMSO solution (18 µL), distilled water for injection (17.7 µL), and 1 mol/L phosphate buffer (pH 8.5, 20 µL) were mixed and stirred at 30°C for 30 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 84 with a purity of 100% was obtained. mass spectrometry: 13515.00 (Calculated: 13514.30). RP-HPLC chart after purification is shown in Fig. 81-1.

### Example 84-2

A 890 µmol/L sense strand (33.7 µL) of ESB2.2-20, a 956 µmol/L antisense strand (31.4 µL) of ESB2.2-20, a 50 mmol/L compound 16/DMF solution (18 µL), distilled water for injection (36.9 µL), and 1 mol/L phosphate buffer (pH 8.5, 30 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 84-2 with a purity of 95.2% was obtained. mass spectrometry: 14305.20 (Calculated: 14304.70). RP-HPLC chart after purification is shown in Fig. 81-2.

### Production Example 18

### Synthesis of linker compound 17

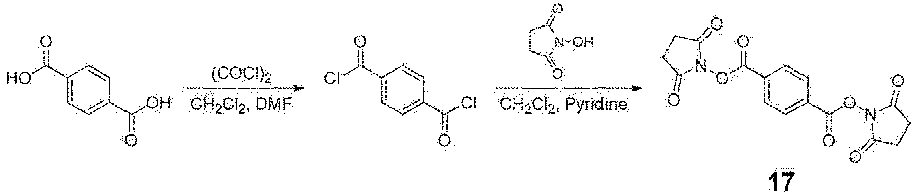

To a solution of terephthalic acid (84 mg, 0.5 mmol) in dichloromethane (2 mL) was added N,N-dimethylformamide (20 µL), and the mixture was cooled to 0°C. Oxalylchloride (103 µL, 1.2 mmol) was added, and the mixture was stirred at room temperature for 45 min. Oxalylchloride (103 µL, 1.2 mmol) was further added, and the mixture was stirred at room temperature for 80 min. The reaction mixture was concentrated under reduced pressure. To a solution of the obtained residue in dichloromethane (2 mL) were added N-hydroxysuccinimide (140 mg, 1.2 mmol) and pyridine (0.5 mL), and the mixture was stirred at room temperature for 1 hr. Water was added to the reaction mixture, and the mixture was extracted with dichloromethane. The organic layer was washed with saturated aqueous sodium chloride solution and dried over magnesium sulfate. The solution was concentrated under reduced pressure to give a white crude product containing compound 17.

### Example 85

1.4 mmol/L AEM28 (20.5 µL), 1.2 mmol/L AEM8 (23.8 µL), a 50 mmol/L compound 17/DMSO solution (18 µL), distilled water for injection (17.7 µL), and 1 mol/L phosphate buffer (pH 8.5, 20 µL) were mixed and stirred at 30°C for 40 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 85 with a purity of 99.9% was obtained. mass spectrometry: 13537.30 (Calculated: 13536.30). RP-HPLC chart after purification is shown in Fig. 82-1.

### Example 85-2

A 890 µmol/L sense strand (33.7 µL) of ESB2.2-20, a 956 µmol/L antisense strand (31.4 µL) of ESB2.2-20, a 50 mmol/L compound 17/DMF solution (18 µL) , distilled water for injection (36.9 µL), and 1 mol/L phosphate buffer (pH 8.5, 30 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 85-2 with a purity of 95.7% was obtained. mass spectrometry: 14327.20 (Calculated: 14326.70). RP-HPLC chart after purification is shown in Fig. 82-2.

### Production Example 19

### Synthesis of linker compound 18

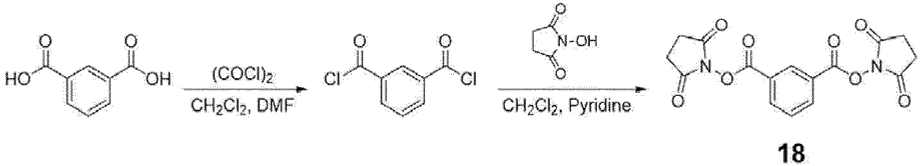

To a solution of isophthalic acid (84 mg, 0.5 mmol) in dichloromethane (2 mL) was added N,N-dimethylformamide (20 µL), and the mixture was cooled to 0°C. Oxalylchloride (103 µL, 1.2 mmol) was added and the mixture was stirred at room temperature for 45 min. Oxalylchloride (103 µL, 1.2 mmol) was further added, and the mixture was stirred at room temperature for 80 min.. The reaction mixture was concentrated under reduced pressure. To a solution of the obtained residue in dichloromethane (2 mL) were added N-hydroxysuccinimide (140 mg, 1.2 mmol) and pyridine (0.5 mL), and the mixture was stirred at room temperature for 45 min. Water was added to the reaction mixture, and the mixture was extracted with dichloromethane. The organic layer was washed with saturated aqueous sodium chloride solution, and dried over magnesium sulfate, and the solution was concentrated under reduced pressure to give a crude product containing compound 18.

### Example 86

1.4 mmol/L AEM28 (20.5 µL), 1.2 mmol/L AEM8 (23.8 µL), a 50 mmol/L compound 18/DMSO solution (18 µL), distilled water for injection (17.7 µL), and 1 mol/L phosphate buffer (pH 8.5, 20 µL) were mixed and stirred at 30°C for 40 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 86 with a purity of 98.3% was obtained. mass spectrometry: 13537.20 (Calculated: 13536.30). RP-HPLC chart after purification is shown in Fig. 83-1.

### Example 86-2

A 890 µmol/L sense strand (33.7 µL) of ESB2.2-20, a 956 µmol/L antisense strand (31.4 µL) of ESB2.2-20, a 50 mmol/L compound 18/DMF solution (18 µL), distilled water for injection (36.9 µL), and 1 mol/L phosphate buffer (pH 8.5, 30 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 86-2 with a purity of 94.5% was obtained. mass spectrometry: 14327.10 (Calculated: 14326.70). RP-HPLC chart after purification is shown in Fig. 83-2.

### Production Example 20

### Synthesis of linker compound 19

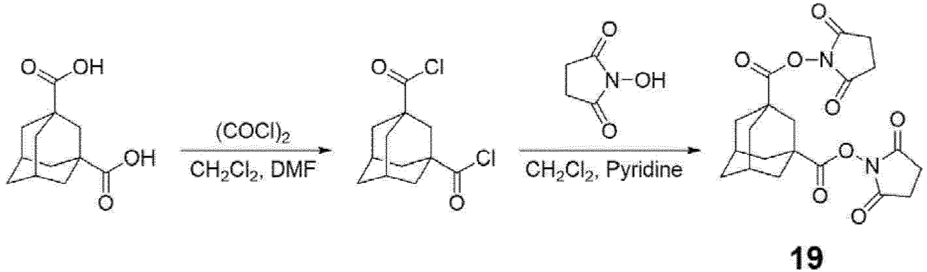

To a solution of 1,3-adamantanedicarboxylic acid (117 mg, 0.5 mmol) in dichloromethane (2 mL) was added N,N-dimethylformamide (20 µL), and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 20 min. The reaction mixture was concentrated under reduced pressure. To a solution of the obtained residue in dichloromethane (2 mL) were added N-hydroxysuccinimide (146 mg, 1.2 mmol) and pyridine (0.5 mL), and the mixture was stirred at room temperature for 45 min. Water was added to the reaction mixture, and the mixture was extracted with dichloromethane. The organic layer was washed with saturated aqueous sodium chloride solution, and dried over magnesium sulfate, and the solution was concentrated under reduced pressure to give a crude product containing compound 19.

### Example 87

1.4 mmol/L AEM28 (27.4 µL), 1.2 mmol/L AEM8 (31.7 µL), a 50 mmol/L compound 19/DMSO solution (40 µL), distilled water for injection (60.9 µL), and 1 mol/L phosphate buffer (pH 8.5, 40 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 87 with a purity of 77.2% was obtained. mass spectrometry: 13595.20 (Calculated: 13594.43). RP-HPLC chart after purification is shown in Fig. 84-1.

### Example 87-2

A 890 µmol/L sense strand (33.7 µL) of ESB2.2-20, a 956 µmol/L antisense strand (31.4 µL) of ESB2.2-20, a 50 mmol/L compound 19/DMF solution (18 µL), distilled water for injection (36.9 µL), and 1 mol/L phosphate buffer (pH 8.5, 30 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 87-2 with a purity of 80.3% was obtained. mass spectrometry: 14385.40 (Calculated: 14384.83). RP-HPLC chart after purification is shown in Fig. 84-2.

### Production Example 21

### Synthesis of linker compound 20

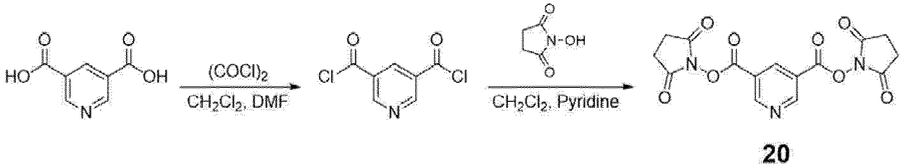

To a solution of 3,5-pyridinedicarboxylic acid (83 mg, 0.5 mmol) in dichloromethane (2 mL) was added N,N-dimethylformamide (20 µL), and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 45 min. The reaction mixture was concentrated under reduced pressure. To a solution of the obtained residue in dichloromethane (2 mL) were added N-hydroxysuccinimide (141 mg, 1.2 mmol) and pyridine (0.5 mL), and the mixture was stirred at room temperature for 75 min. Water was added to the reaction mixture, and the mixture was extracted with dichloromethane. The organic layer was washed with saturated aqueous sodium chloride solution, and dried over magnesium sulfate, and the solution was concentrated under reduced pressure to give a crude product (266 mg) containing compound 20.

### Example 88

1.4 mmol/L AEM28 (20.5 µL), 1.2 mmol/L AEM8 (23.8 µL), a 50 mmol/L compound 20/DMSO solution (18 µL), distilled water for injection (17.7 µL), and 1 mol/L phosphate buffer (pH 8.5, 20 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 88 with a purity of 99.6% was obtained. mass spectrometry: 13537.90 (Calculated: 13537.29). RP-HPLC chart after purification is shown in Fig. 85-1.

### Example 88-2

A 890 µmol/L sense strand (33.7 µL) of ESB2.2-20, a 956 µmol/L antisense strand (31.4 µL) of ESB2.2-20, a 50 mmol/L compound 20/DMF solution (18 µL), distilled water for injection (36.9 µL), and 1 mol/L phosphate buffer (pH 8.5, 30 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 88-2 with a purity of 97.0% was obtained. mass spectrometry: 14328.10 (Calculated: 14327.69). RP-HPLC chart after purification is shown in Fig. 85-2.

### Production Example 22

### Synthesis of linker compound 21

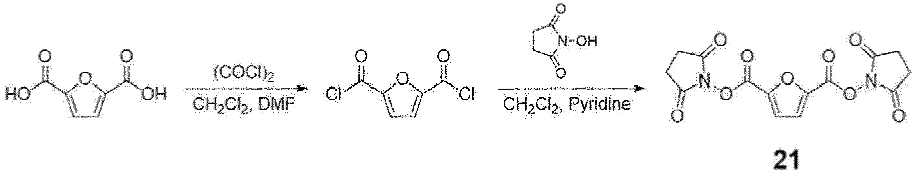

To a solution of 2,5-furandicarboxylic acid (79 mg, 0.5 mmol) in dichloromethane (2 mL) was added N,N-dimethylformamide (20 µL) , and the mixture was cooled to 0°C. Oxalylchloride (103 µL, 1.2 mmol) was added, and the mixture was stirred at room temperature for 30 min. Oxalylchloride (103 µL, 1.2 mmol) was further added, and the mixture was stirred at room temperature for 30 min. The reaction mixture was concentrated under reduced pressure. To a solution of the obtained residue in dichloromethane (2 mL) were added N-hydroxysuccinimide (145 mg, 1.2 mmol) and pyridine (0.5 mL), and the mixture was stirred at room temperature for 1 hr. Water was added to the reaction mixture, and the mixture was extracted with dichloromethane. The organic layer was washed with saturated aqueous sodium chloride solution, and dried over magnesium sulfate, and the solution was concentrated under reduced pressure to give a crude product (210 mg) containing compound 21.

### Example 89

1.4 mmol/L AEM28 (20.5 µL), 1.2 mmol/L AEM8 (23.8 µL), a 50 mmol/L compound 21/DMSO solution (18 µL), distilled water for injection (17.7 µL), and 1 mol/L phosphate buffer (pH 8.5, 20 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 89 with a purity of 99.7% was obtained. mass spectrometry: 13526.90 (Calculated: 13526.26). RP-HPLC chart after purification is shown in Fig. 86-1.

### Example 89-2

A 890 µmol/L sense strand (33.7 µL) of ESB2.2-20, a 956 µmol/L antisense strand (31.4 µL) of ESB2.2-20, a 50 mmol/L compound 21/DMF solution (18 µL), distilled water for injection (36.9 µL), and 1 mol/L phosphate buffer (pH 8.5, 30 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 89-2 with a purity of 86.0% was obtained. mass spectrometry: 14317.10 (Calculated: 14316.66). RP-HPLC chart after purification is shown in Fig. 86-2.

### Production Example 23

### Synthesis of linker compound 25

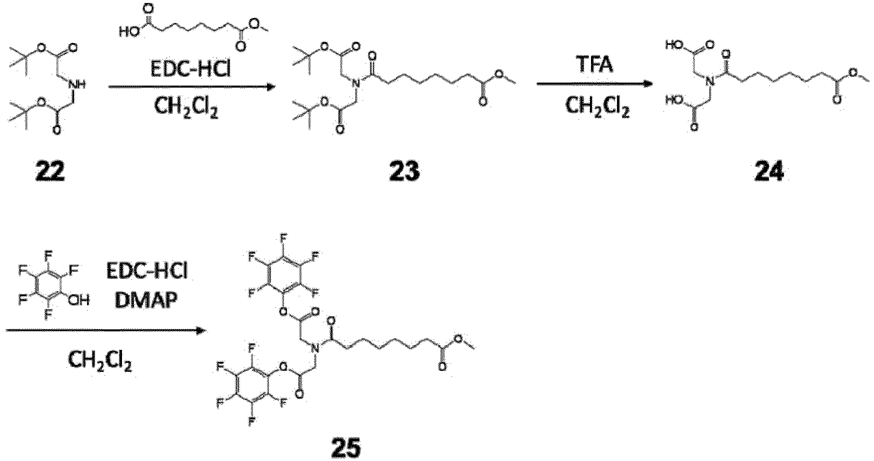

### (1) Synthesis of compound 23

To a solution of compound 22 (5.3 g, 21.6 mmol) and monomethyl suberate (4.9 g, 1.2 eq.) in dichloromethane (50 mL) was added 1-ethyl-3-(3-dimethylaminopropyl)carbodiimide hydrochloride (5.0 g, 1.2 eq.) under an argon atmosphere, and the mixture was stirred at room temperature overnight. To the reaction mixture were added water and saturated aqueous sodium hydrogen carbonate solution, the mixture was extracted with dichloromethane, and the organic layer was dried over magnesium sulfate. The solution was concentrated under reduced pressure, and the obtained residue was purified by silica gel column chromatography (n-hexane:ethyl acetate=30:70 - 0:100) to give the target compound 23 as a colorless oily substance (9.4 g).
ESI-Mass:438.24 [M+Na] ⁺

### (2) Synthesis of compound 24

To a solution of compound 23 (9.4 g, 22.6 mmol) in dichloromethane (80 mL) was added trifluoroacetic acid (17.3 mL, 10 eq.) under an argon atmosphere, and the mixture was stirred at room temperature for 3 hr. The reaction mixture was concentrated under reduced pressure to give the target compound 24 as a pale-yellow oily substance (8.3 g).
ESI-Mass:304.09[M+H]⁺

### (3) Synthesis of compound 25

To a solution of compound 24 (3.0 g, 9.9 mmol) and pentafluorophenol (4.6 g, 2.5 eq.) in dichloromethane (80 mL) were added 1-ethyl-3-(3-dimethylaminopropyl)carbodiimide hydrochloride (4.7 g, 2.5 eq.) and 4-dimethylaminopyridine (121 mg, 0.1 eq.) under an argon atmosphere, and the mixture was stirred at room temperature overnight. The solution was concentrated under reduced pressure, and the obtained residue was purified by silica gel column chromatography (dichloromethane:ethyl acetate=100:0 - 85:15) to give a crude product of the target compound 25 as a pale-yellow oily substance (5.6 g).
ESI-Mass:658.08[M+Na]⁺

### Example 90

1.4 mmol/L AEM28 (27.4 µL), 1.2 mmol/L AEM8 (31.7 µL), a 30 mmol/L compound 25/DMF solution (60 µL), distilled water for injection (41 µL), and 1 mol/L phosphate buffer (pH 8.5, 40 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 90 with a purity of 99.4% was obtained. mass spectrometry: 13674.10 (Calculated: 13673.48). RP-HPLC chart after purification is shown in Fig. 87.

### Example 91

1.4 mmol/L AEM28 (14 µL), 1.2 mmol/L AEM8 (16 µL), 30 mmol/L DSP/DMF solution (20 µL), 1% triethylamine aqueous solution (20 µL), and 2-propanol (30 µL) were mixed and stirred at 30°C for 45 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 91 with a purity of 99.2% was obtained. mass spectrometry: 13581.30 (Calculated: 13580.43). RP-HPLC chart after purification is shown in Fig. 88-1.
DSP: Dithiobis(succinimidyl propionate) (DOJINDO LABORATORIES)

### Example 91-2

A 890 µmol/L sense strand (22.5 µL) of ESB2.2-20, a 956 µmol/L antisense strand (20.9 µL) of ESB2.2-20, 30 mmol/L DSP/DMF solution (20 µL), 1% triethylamine aqueous solution (26.6 µL), and 2-propanol (40 µL) were mixed and stirred at 30°C for 100 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 91-2 with a purity of 95.0% was obtained. mass spectrometry: 14371.30 (Calculated: 14370.83). RP-HPLC chart after purification is shown in Fig. 88-2.

### Example 92

1.4 mmol/L AEM28 (14 µL), 1.2 mmol/L AEM8 (16 µL), 30 mmol/L DSH/DMF solution (20 µL), 1% triethylamine aqueous solution (20 µL), and 2-propanol (30 µL) were mixed and stirred at 30°C for 30 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 92 with a purity of 99.2% was obtained. mass spectrometry: 13665.50 (Calculated: 13664.59). RP-HPLC chart after purification is shown in Fig. 89-1.
DSH: Dithiobis(succinimidyl hexanoate) (DOJINDO LABORATORIES)

### Example 92-2

A 890 µmol/L sense strand (22.5 µL) of ESB2.2-20, a 956 µmol/L antisense strand (20.9 µL) of ESB2.2-20, 30 mmol/L DSH/DMF solution (20 µL), 1% triethylamine aqueous solution (26.6 µL), and 2-propanol (40 µL) were mixed and stirred at 30°C for 100 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 92-2 with a purity of 91.6% was obtained. mass spectrometry: 14455.50 (Calculated: 14454.99). RP-HPLC chart after purification is shown in Fig. 89-2.

### Example 93

1.4 mmol/L AEM28 (14 µL), 1.2 mmol/L AEM8 (16 µL), 30 mmol/L DSO/DMF solution (20 µL), 1% triethylamine aqueous solution (20 µL), and 2-propanol (30 µL) were mixed and stirred at 30°C for 1 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 93 with a purity of 99.5% was obtained. mass spectrometry: 13721.50 (Calculated: 13720.70). RP-HPLC chart after purification is shown in Fig. 90-1.
DSO: Dithiobis(succinimidyl octanoate) (DOJINDO LABORATORIES)

### Example 93-2

A 890 µmol/L sense strand (22.5 µL) of ESB2.2-20, a 956 µmol/L antisense strand (20.9 µL) of ESB2.2-20, 30 mmol/L DSO/DMF solution (20 µL), 1% triethylamine aqueous solution (26.6 µL), and 2-propanol (40 µL) were mixed and stirred at 30°C for 100 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 93-2 with a purity of 92.4% was obtained. mass spectrometry: 14511.50
(Calculated: 14511.10). RP-HPLC chart after purification is shown in Fig. 90-2.

### Example 94

A 1.1 mmol/L (purity 35%) sense strand (51.5 µL) of ESB2.2-20 (U: 2'-Amino(TFA)-uridine-3'-CEP was introduced), a 1.3 mmol/L antisense strand (15.9 µL) of ESB2.2-20 (U: 2'-Amino(TFA)-uridine-3'-CEP was introduced), 50 mmol/L BS3 aqueous solution (20 µL), distilled water for injection (72.6 µL), and 1 mol/L phosphate buffer (pH 8.5, 40 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 94 with a purity of 77.4% was obtained. mass spectrometry: 14186.90 (Calculated: 14186.67). RP-HPLC chart after purification is shown in Fig. 91.

### Example 95

A 625 µmol/L sense strand (24 µL) of ESB2.2-20 (U: compound U9 (Production Example 7) was introduced), a 625 µmol/L antisense strand (24 µL) of ESB2.2-20 (U: compound U9 (Production Example 7) was introduced), 50 mmol/L BS3 aqueous solution (9 µL), distilled water for injection (63 µL), and 1 mol/L phosphate buffer (pH 8.5, 30 µL) were mixed and stirred at 30°C for 30 min. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 95 with a purity of 85.2% was obtained. mass spectrometry: 14361.10 (Calculated: 14360.77). RP-HPLC chart after purification is shown in Fig. 92.

### Example 96

A 890 µmol/L sense strand (11.2 µL) of ESB2.2-20, a 1.3 mmol/L antisense strand (8.0 µL) of ESB2.2-20 (U: 2'-Amino(TFA)-uridine-3'-CEP) was introduced), 50 mmol/L BS3 aqueous solution (10 µL), distilled water for injection (10.8 µL), and 1 mol/L phosphate buffer (pH 8.5, 10 µL) were mixed and stirred at 30°C for 2 hr. The reaction mixture was purified (column: XBridge Oligonucleotide BEH C18, 2.5 µm, 10×50 mm; flow rate: 4.7 mL/min; column temperature: 60°C; detection: UV 260 nm; Buffer A: 50 mmol/L TEAA (pH 7.4), 5% CH₃CN; Buffer B: 50 mmol/L TEAA (pH 7.4), 90% CH₃CN), and the peak of the target was fractionated. The separated fraction was subjected to ethanol precipitation, and the resulting precipitate was dissolved in distilled water for injection. Example 96 with a purity of 96.9% was obtained. mass spectrometry: 14261.20 (Calculated: 14260.67). RP-HPLC chart after purification is shown in Fig. 93.

### Example 97

(1) To a solution of azidoacetic acid (14.3 mg, 141 µmοl) in dehydrated dimethylformamide (0.5 mL) were added a dehydrated dimethylformamide solution of N,N,N',N'-tetramethyl-O-(N-succinimidyl)uronium tetrafluoroborate (594 mM, 0.24 mL, 1.0 eq.) and a dehydrated dimethylformamide solution of diisopropyl ethylamine (594 mM, 0.24 mL, 1.0 eq.) at 0°C, and the mixture was stirred at 0°C for 30 min to give an intermediate.
   1.6 mmol/L AEM28 (31.4 µL), distilled water for injection (38.2 µL), and 1 mol/L phosphate buffer (20 µL) at pH 8.5 were mixed with the aforementioned intermediate (10.4 µL), and the mixture was stirred at 30°C overnight. Ethanol precipitation of the reaction mixture was performed, and the resulting precipitate was dissolved in distilled water for injection to give an AEM28-azide compound with a purity of 92.1%. mass spectrometry: 6774.60 (Calculated: 6774.15). RP-HPLC chart is shown in Fig. 94-1.
(2) To a solution of 4-pentynoic acid (9.4 mg, 96 µmοl) in dehydrated dimethylformamide (0.5 mL) were added a dehydrated dimethylformamide solution of N,N,N',N'-tetramethyl-O-(N-succinimidyl)uronium tetrafluoroborate (239 mM, 0.2 mL, 0.5 eq.), and a dehydrated dimethylformamide solution of diisopropyl ethylamine (239 mM, 0.20 mL, 0.5 eq.) at 0°C, and the mixture was stirred at 0°C for 30 min to give an intermediate.
   1.4 mmol/L AEM8 (35.8 µL), distilled water for injection (56 µL), and 1 mol/L phosphate buffer (30 µL) at pH 8.5 were mixed with the aforementioned intermediate (28.2 µL), and the mixture was stirred at 30°C for 30 min. Ethanol precipitation of the reaction mixture was performed, and the resulting precipitate was dissolved in distilled water for injection to give an AEM28-alkyne compound with a purity of 93.9%. mass spectrometry: 6795.80 (Calculated: 6795.2). RP-HPLC chart is shown in Fig. 94-2.
(3) To a mixture of 318 µmol/L AEM28-azide compound aqueous solution (20 µL), 378 µmol/L AEM8-alkyne compound aqueous solution (16.8 µL), 1 mol/L phosphate buffer (11.4 µL) at pH 8.5, and 100 mmol/L sodium ascorbate aqueous solution (6.4 µL) was added a mixed solution (dimethylsulfoxide:t-butanol=3:1, 2.6 µL) of 25 mmol/L copper (II)sulfate pentahydrate and 50 mmol/L tris[(1-benzyl-1H-1,2,3-triazol-4-yl)methyl]amine at room temperature, and the mixture was stirred at 30°C for 4 hr. At room temperature, 500 mmol/L EDTA aqueous solution (50 µL) was added and the mixture was filtered. The filtrate was purified by HPLC (column: XBridge Oligonucleotide, BEH C18, 2.5 µm, 10 mm×50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 90% CH₃CN). Ethanol precipitation of the purified product was performed, and the resulting precipitate was dissolved in distilled water for injection to give Example 97 with a purity of 98.7%. mass spectrometry: 13569.5 (Calculated: 13569.3). RP-HPLC chart is shown in Fig. 94-3.

### Example 98

(1) Synthesis by a method similar to that in Example 97(1) and using the antisense strand of ESB2.2-20 gave an ESB2.2-20 antisense strand azide compound with a purity of 89.8%. mass spectrometry: 7163.0 (Calculated: 7162.5). RP-HPLC chart is shown in Fig. 95-1.
(2) To a mixture of an aqueous solution (11.5 µL) of 872 µmol/L sense strand of ESB2.2-20 (U:2'-O-propargyl Uridine CED phosphoramidite was introduced), an aqueous solution (21.5 µL) of 460 µmol/L antisense strand azide compound of ESB2.2-20, and 1 mol/L phosphate buffer (11.3 µL) at pH 8.5 was added a mixed solution (dimethylsulfoxide:t-butanol=3:1, 12 µL) of 25 mmol/L copper(I) bromide and 50 mmol/L tris[(1-benzyl-1H-1,2,3-triazol-4-yl)methyl]amine () at room temperature, and the mixture was stirred at 25°C for 45 min. At room temperature, 500 mmol/L EDTA aqueous solution (50 µL) was added and the mixture was filtered. The filtrate was purified by HPLC (column: XBridge Oligonucleotide, BEH C18, 2.5 µm, 10 mm×50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 90% CH₃CN). Ethanol precipitation of the purified product was performed, and the resulting precipitate was dissolved in distilled water for injection to give Example 98 with a purity of 97.0%. mass spectrometry: 14245.2
   (Calculated: 14244.7). RP-HPLC chart is shown in Fig. 95-2.

### Example 99

To a mixture of an aqueous solution (21.5 µL) of 0.46 mmol/L sense strand of ESB2.2-20 (U: 2'-O-Trifluoroacetamido propyl Uridine CED phosphoramidite was introduced), an aqueous solution (9.3 µL) of 1.1 mmol/L antisense strand of ESB2.2-20 (U: 2'-O-Trifluoroacetamido propyl Uridine CED phosphoramidite was introduced), distilled water for injection (105.2 µL), and 1 mol/L phosphate buffer (40 µL) at pH 8.5 was added 50 mmol/L BS3 aqueous solution (24 µL) at room temperature, and the mixture was stirred at 25°C for 4 hr. The reaction mixture was purified by HPLC (column: XBridge Oligonucleotide, BEH C18, 2.5 µm, 10 mm×50 mm; flow rate: 4.7 mL/min; detection: UV 260 nm; column oven: 60°C; Buffer A: 50 mmol/L TEAA (pH 7.3), 5% CH₃CN; Buffer B: 50 mmol/L TEAA (pH 7.3), 90% CH₃CN). Ethanol precipitation of the purified product was performed, and the resulting precipitate was dissolved in distilled water for injection to give Example 99 with a purity of 99.2%. mass spectrometry: 14303.3 (Calculated: 14302.8). RP-HPLC chart is shown in Fig. 96.

### Example 100

Synthesis by a method similar to that in Example 99 and using the sense strand and antisense strand of ESB2.2-20 (U: compound U13(Production Example 9) was introduced) gave Example 100 with a purity of 90.2%. mass spectrometry: 14419.4 (Calculated: 14419.0). RP-HPLC chart is shown in Fig. 97.

### Examples 101 to 136-3

These compounds can be synthesized by the above-mentioned Example methods or a combination of the above-mentioned methods and known methods.

### <Nucleic acid molecule list-2>

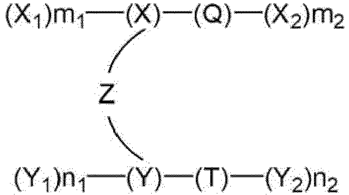

**[Table 3-1]**

| Upper panel: sense strand 5'-3', lower panel: antisense strand 3'-5', N: RNA, **N**: crosslinked nucleotide, *N*: 2'-OMe, Nf: 2'-F, n: DNA | | | | |
|---|---|---|---|---|
| Ex.No. | nucleic acid name | Seq.No | structural formula | Z |
| 23-2 | ESB2.2_HPRT1-3 | 17 | UUUGUUGGAUUUGAAAUUCC ; ; ; ; ; ; ; ; ; ; ; ; ; ; ; ; ; ; UGAAACAACCUAAACUUUAA | 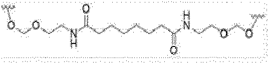 |
| | | 18 | | |
| 27-2 | ESB2.2-20-2 | 19 | U̲GGUCGUAUUGGGCGCCUGG ; ; ; ; ; ; ; ; ; ; ; ; ; ; ; ; ; ; ; PA̲ACCAGCAUAACCCGCGGA | 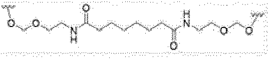 |
| | | 20 | | |
| 28-2 | ESB2.2-20-3 | 21 | UGGUCGUAUUGGGCGCCUGG ; ; ; ; ; ; ; ; ; ; ; ; ; ; ; ; ; ; ; AAACCAGCAUAACCCGCGGA | 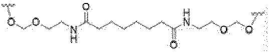 |
| | | 22 | | |
| 29-2 | ESB2.2-20-4 | 23 |   UUGGUCGUAUUGGGCGCCUGG ;;;;;;;;;;;;;;;;;;; UAAACCAGCAUAACCCGCGGA | 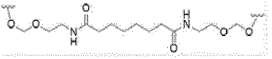 |
| | | 24 | | |
| 30-2 | ESB2.2-20-5 | 25 | UUGGUCGUAUUGGGCGCCUGG ;;;;;;;;;;;;;;;;;;;; UAAACCAGCAUAACCCGCGGA | 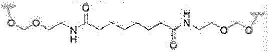 |
| | | 26 | | |
| 30-3 | ESB_HPRT1 | 27 | C̲UUUGUUGGAUUUGAAAUUCC ; ; ; ; ; ; ; ; ; ; ; ; ; ; ; ; ; ; ; CU̲GAAACAACCUAAACUUUAA | 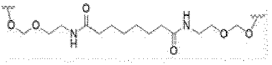 |
| | | 28 | | |
| 30-4 | ESB2.2_HPRT1-4 | 29 |  CUUUGUUGGAUUUGAAAUUCC ; ; ; ; ; ; ; ; ; ; ; ; ; ; ; ; ; ; ; ; CUGAAACAACCUAAACUUUAA | 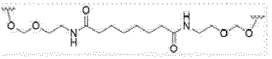 |
| | | 30 | | |
| 30-5 | 6b1 | 31 | UUAUGGGUUGGGACUACCUUA ;;;;;;;;;;;;;;;;;;: GGAAUACCCAACCCUGAUGGG | 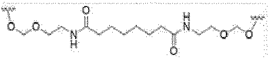 |
| | | 32 | | |
| 32-2 | ESB2.2-20-6 | 33 | UUUGGUCGUAUUGCGGCCCUGG ;;;;;;;;;;;;;;;;;;;;;; CUAAACCAGCAUAACCCCGCGGA | 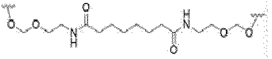 |
| | | 34 | | |
| 33-2 | ESB2.2-17 | 35 | GAUUUGGUCGUAUUGGGCGCCU ; ; ; ; ; ; ; ; ; ; ; ; ; ; ; ; ; ; ; ; ; GCCUAAACCAGCAUAACCCGCGG | 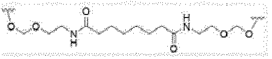 |
| | | 36 | | |
| 33-3 | ESB2.2-18 | 37 |   CGUCAAGGCUGAGAACGGCAAG ; ; ; ; ; ; ; ; ; ; ; ; ; ; ; ; ; ; ; ; UGGCAGUUCCGACUCUUGCCCU | 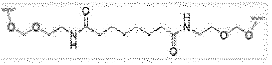 |
| | | 38 | | |
| 33-4 | ESB2.2-19 | 39 |  CACUCCUCCACCUUUGACGCUG ;;;;;;;;;;;;;;;;;;;;; GGGUGAGGAGGUGGAAACUGCG | 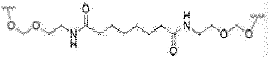 |
| | | 40 | | |
| 33-5 | ESB2.2-20 | 41 | UUUUGGUCGUAUUGGGCGCCU ;;;;;;;;;;;;;;;;;;;;; CUAAACCAGCAUAACCCGCGGA | 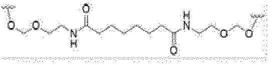 |
| | | 42 | | |

**[Table 3-2]**

| | | | | |
|---|---|---|---|---|
| 33-6 | ESB2.2-21 | 43 |   AUGGGGAAGGUGAAGGUCGGAG ;;;;;;;;;;;;;;;;;;;; GGUACCCCUUCCACUUCCAGCC | 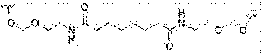 |
| | | 44 | | |
| 33-7 | ESB2.2-22 | 45 | GGUGAAGCAGGCGUCGGAGGGC ; ; ; ; ; ; ; ; ; ; ; ; ; ; ; ; ; ; ; ; CACCACUUCGUCCGCAGCCUCC | 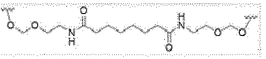 |
| | | 46 | | |
| 33-8 | ESB2.2-23 | 47 | CUGACUUCAACAGCGACACCCA ;;;;;;;;;;;;;;;;;;;; GAGACUGAAGUUGUCGCUGUGG | 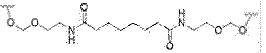 |
| | | 48 | | |
| 33-9 | ESB2.2-24 | 49 | UGGUGAAGCAGGCGUCGGAGGG ; ; ; ; ; ; ; ; ; ; ; ; ; ; ; ; ; ; ; ; CCACCACUUCGUCCGCAGCCUC | 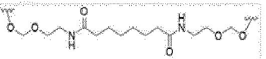 |
| | | 50 | | |
| 33-10 | ESB2.2-25 | 51 | UGGGGAAGGUGAAGGUCGGAGU ; ; ; ; ; ; ; ; ; ; ; ; ; ; ; ; ; ; ; ; GUACCCCUUCCACUUCCAGCCU | 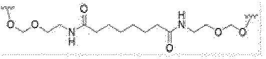 |
| | | 52 | | |
| 33-11 | ESB2.2-26 | 53 | CUCCUCCACCUUUGACGCUGGG ;;;;;;;;;;;;;;;;;;;; GUGAGGAGGUGGAAACUGCGAC | 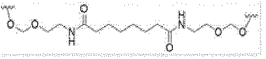 |
| | | 54 | | |
| 33-12 | ES82.2-27 | 55 | CCUCCGGGAAACUGUGGCGUGA ;;;;;;;;;;;;;;;;;;;;;; GGGGAGGCCCUUUGACACCGCA | 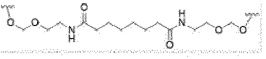 |
| | | 56 | | |
| 33-13 | ESB2.2-28 | 57 | ACUCCUCCACCUUUGACGCUGG ; ; ; ; ; ; ; ; ; ; ; ; ; ; ; ; ; ; ; ; ; GGUGAGGAGGUGGAAACUGCGA | 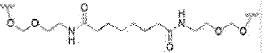 |
| | | 58 | | |
| 33-14 | ESB2.2-29 | 59 | GGCGCUGAGUACGUCGUGGAGU ;;;;;;;;;;;;;;;;;;;; GACCGCGACUCAUGCAGCACCU | 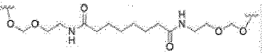 |
| | | 60 | | |
| 33-15 | ESB2.2-30 | 61 | AAAUCAAGUGGGGCGAUGCUGG ; ; ; ; ; ; ; ; ; ; ; ; ; ; ; ; ; ; ; ; ; GUUUUAGUUCACCCCGCUACGA | 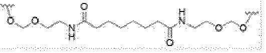 |
| | | 62 | | |
| 33-16 | ESB2.2-31 | 63 | CUGAUGCCCCAUGUUCGUCAU ; ; ; ; ; ; ; ; ; ; ; ; ; ; ; ; ; ; ; ACGACUACGGGGUACAAGCAG | 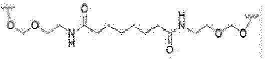 |
| | | 64 | | |
| 33-17 | ESB2.2-32 | 65 | GGAAGGUGAAGGUCGGAGUCAA ; ; ; ; ; ; ; ; ; ; ; ; ; ; ; ; ; ; ; ; CCCCUUCCACUUCCAGCCUCAG | 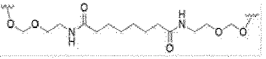 |
| | | 66 | | |
| 33-18 | ESB2.2-33 | 67 | GCUGAUGCCCCCAUGUUCGUCA ;;;;;;;;;;;;;;;;;;;; GACGACUACGGGGGUACAAGCA | 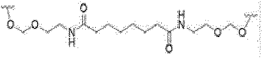 |
| | | 68 | | |

**[Table 3-3]**

| | | | | |
|---|---|---|---|---|
| 33-19 | ESB22-34 | 69 | UGAUGCCCCCAUGUUCGUCAUG ;;;;;;;;;;;;;;;; ;;;; CGACUACGGGGGUACAAGCAGU | 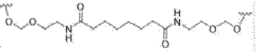 |
| | | 70 | | |
| 33-20 | ESB2.2-35 | 71 | C̲AAAAUCAAGUGGGGCGAUGCU ;;;;;;;;;;;;;;;;;;;;;; AG̲GUUUUAGUUCACCCCGCUAC | 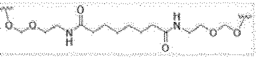 |
| | | 72 | | |
| 33-21 | ESB2.2-36 | 73 | CCAAGGUCAUCCAUGACAACUU ;;;;;;;;;;;;;;;;;;;; CCGGUUCCAGUAGGUACUGUUG | 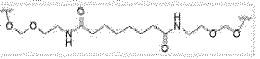 |
| | | 74 | | |
| 33-22 | ESB22-37 | 75 | **A**AAUUCCAUGGCACCGUCAAGG ; ; ; ; ; ; ; ; ; ; ; ; ; ; ; ; ; ; ; ; C**G**UUUAAGGUACCGUGGCAGUU | 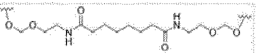 |
| | | 76 | | |
| 33-23 | ESB2.2-38 | 77 | CAACGGAUUUGGUCGUAUUGGG ; ; ; ; ; ; ; ; ; ; ; ; ; ; ; ; ; ; ; ; CAGUUGCCUAAACCAGCAUAAC | 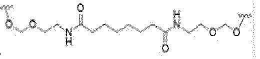 |
| | | 78 | | |
| 33-24 | ESB2.2-39 | 79 | C̲CUGGUAUGACAACGAAUUUGG ;;;;;;;;;;;;;;;;;;;; AA̲GGACCAUACUGUUGCUUAAA | 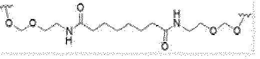 |
| | | 80 | | |
| 33-25 | ESB2.2-20-fd | 81 | uUUGGUCGUAUUGGGCGCCUGfg ;;;;;;;;;;;;;;;;;;;; CUAAACCAGCAUAACCCGCGGA | 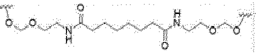 |
| | | 82 | | |
| 33-26 | ESB2.2_HPRT1-1 | 83 |   ACUUUGUUGGAUUUGAAAUUCC ;;;;;;;;;;;;;;;;;;;; UCUGAAACAACCUAAACUUUAA | 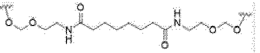 |
| | | 84 | | |
| 33-27 | 6b1-4 | 85 | CUUAUGGGUUGGGACUACCUUA ;;;;;;;;;;;;;;;;;;;;: UGGAAUACCCAACCCUGAUGGG | 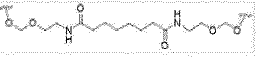 |
| | | B6 | | |
| 35 | BBN39 | 87 | AAGAUGAAUCUCUUCUCC ;;;;;;;;;;;;;;;;; UAUUCUACUUAGAGAAGA | 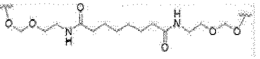 |
| | | 88 | | |
| 36 | BBN40 | 89 | AAGAUGAAUCUCUUCUCC ; ; ; ; ; ; ; ; ; ; ; ; ; ; ; ; ; UAUUCUACUUAGAGAAGA | 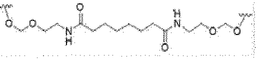 |
| | | 90 | | |
| 37 | BBN41 | 91 | AAGAUGAAUCUCUUCUCC ;;;;;;;;;;;;;;;; UAUUCUACUUAGAGAAGA | 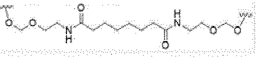 |
| | | 92 | | |
| 38 | BBN42 | 93 | U̲AAGAUGAAUCUCUUCUCC ; ; ; ; ; ; ; ; ; ; ; ; ; ; ; ; ; C̲UAUUCUACUUAGAGAAGA | 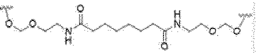 |
| | | 94 | | |
| 38-2 | ESB2.2-20-1 | 95 | GGUCGUAUUGGGCGCCUGG ; ; ; ; ; ; ; ; ; ; ; ; ; ; ; ; ; AACCAGCAUAACCCGCGGA | 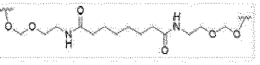 |
| | | 96 | | |
| 39 | BBN43 | 97 | UAAGAUGAAUCUCUUCUCC ; ; ; ; ; ; ; ; ; ; ; ; ; ; ; ; ; CUAUUCUACUUAGAGAAGA | 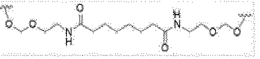 |
| | | 98 | | |

**[Table 3-4]**

| | | | | |
|---|---|---|---|---|
| 40 | BBN44 | 99 | UAAGAUGAAUCUCUUCUCC ; ; ; ; ; ; ; ; ; ; ; ; ; ; ; ; ; CUAUUCUACUUAGAGAAGA | 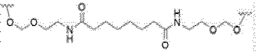 |
| | | 100 | | |
| 40-2 | ESB2.2_HPRT1-2 | 101 | UUGUUGGAUUUGAAAUUCC ; ; ; ; ; ; ; ; ; ; ; ; ; ; ; ; ; GAAACAACCUAAACUUUAA | 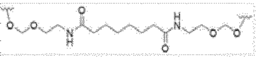 |
| | | 102 | | |
| 41 | BBN45 | 103 | G̲AGAUAAGAUGAAUCUCUUCUCC ;;;;;;;;;;;;;;;;;;;;; A̲CCUCUAUUCUACUUAGAGAAGA | 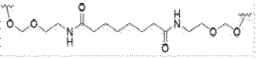 |
| | | 104 | | |
| 41-2 | ESS2.2-20-8 | 105 | A UUUGGUCGUAUUGGGCGCCUGG ; ; ; ; ; ; ; ; ; ; ; ; ; ; ; ; ; ; ; ; ; ; C CUAAACCAGCAUAACCCGCGGA | 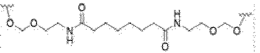 |
| | | 106 | | |
| 42 | BBN46 | 107 | G̲AGAUAAGAUGAAUCUCUUCUCC ;;;;;;;;;;;;;;;;;;;;; AC̲CUCUAUUCUACUUAGAGAAGA | 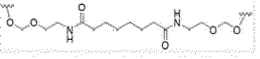 |
| | | 108 | | |
| 42-2 | ESB2.2-20-9 | 109 | AUUUGGUCGUAUUGGGCGCCUGG ; ; ; ; ; ; ; ; ; ; ; ; ; ; ; ; ; ; ; ; ; CCUAAACCAGCAUAACCCGCGGA | 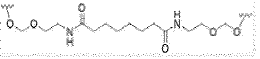 |
| | | 110 | | |
| 43 | BBN47 | 111 | GAGAUAAGAUGAAUCUCUUCUCC ; ; ; ; ; ; ; ; ; ; ; ; ; ; ; ; ; ; ; ; ; ACCUCUAUUCUACUUAGAGAAGA | 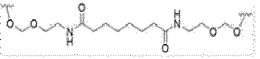 |
| | | 112 | | |
| 43-2 | ESB2.2-20-10 | 113 | AUUUGGUCGUAUUGGGCGCCUGG ; ; ; ; ; ; ; ; ; ; ; ; ; ; ; ; ; ; ; ; ; CCUAAACCAGCAUAACCCGCGGA | 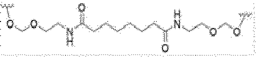 |
| | | 114 | | |
| 44 | BBN48 | 115 | G̲GAGAUAAGAUGAAUCUCUUCUCC ; ; ; ; ; ; ; ; ; ; ; ; ; ; ; ; ; ; ; ; ; ; U̲ACCUCUAUUCUACUUAGAGAAGA | 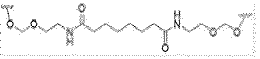 |
| | | 116 | | |
| 45 | BBN49 | 117 | GGAGAUAAGAUGAAUCUCUUCUCC ; ; ; ; ; ; ; ; ; ; ; ; ; ; ; ; ; ; ; ; ; UACCUCUAUUCUACUUAGAGAAGA | 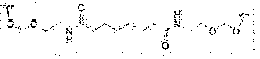 |
| | | 118 | | |
| 46 | SBN50 | 119 | GGAGAUAAGAUGAAUCUCUUCUCC ;;;;;;;;;;;;;;;;;;;;;; UACCUCUAUUCUACUUAGAGAAGA | 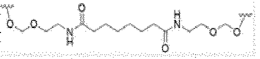 |
| | | 120 | | |
| 46-2 | ES82.2_HPRTI-5 | 121 | AGACUUUGUUGGAUUUGAAAUUCC ;;;;;;;;;;;;;;;;;;;;;; GGUCUGAAACAACCUAAACUUUAA | 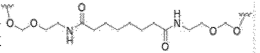 |
| | | 122 | | |
| 47 | BBN51 | 123 | GAUAAGAUGAAUCUCUUCUCC ;;;;;;;;;;;;;;;;;;; CUCUAUUCUACUUAGAGAAGA | 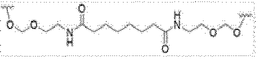 |
| | | 124 | | |
| 48 | B8N52 | 125 | AGAUAAGAUGAAUCUCUUCUCC ;;;;;;;;;;;;;;;;;;;;; CCUCUAUUCUACUUAGAGAAGA | 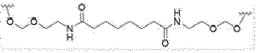 |
| | | 126 | | |

**[Table 3-5]**

| | | | | |
|---|---|---|---|---|
| 48-2 | ESB2.2-20-7 | 127 | UUUGGUCGUAUUGGGCGCCUGG ;;;;;;;;;;;;;;;;;;;; CUAAACCAGCAUAACCCGCGGA | 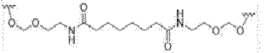 |
| | | 128 | | |
| 49 | BBN53 | 129 | 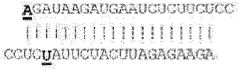 AGAUAAGAUGAAUCUCUUCGCC GCUCUAUUCUACUUAGAGAAGA | 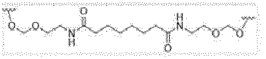 |
| | | 130 | | |
| 50 | ESB2.2-1 | 131 | GAUAAGAUGAAUCUCUUCUCC ; ; ; ; ; ; ; ; ; ; ; ; ; ; ; ; ; ; ; CUCUAUUCUACUUAGAGAAGA | 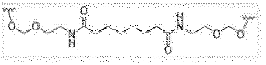 |
| | | 132 | | |
| 50-2 | ESB2.2_HPRT1-6 | 133 | AC UUUGUUGGAUUUGAAAUUCC ;;;;;;;;;;;;;;;;;;;; UCUGAAACAACCUAAACUUUAA | 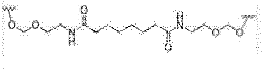 |
| | | 134 | | |
| 51 | ESB22-2 | 135 | GAUAAGAUGAAUCUCUUCUCC ;;;;;;;;;;;;;;;;;;; CUCUAUUCUACUUAGAGAAGA | 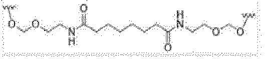 |
| | | 136 | | |
| 51-2 | ESB2.2-20-15 | 137 | UuUGGUCGUAUUGGGCGCCUGG ;;;;;;;;;;;;;;;;;;; CUAAACCAGCAUAACCCGCGGA | 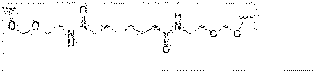 |
| | | 138 | | |
| 52 | ESB2.2-3 | 139 |   GAUAAGAUGAAUCUCUUCUCC ;;;;;;;;;;;;;;;;;;; CUCUAUUCUACUUAGAGAAGA | 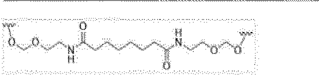 |
| | | 140 | | |
| 52-2 | ES82 2-20-16 | 141 | 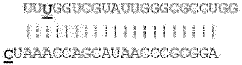 UUUGGUCGUAUUGGGCGCCUGG CUAAACCAGCAUAACCCGCGGA | 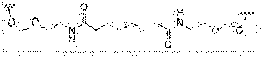 |
| | | 142 | | |
| 53 | ES82.2-4 | 143 |   GAUAAGAUGAAUCUCUUCUCC ;;;;;;;;;;;;;;;;;;; CUCUAUUCUACUUAGAGAAGA | 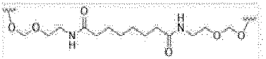 |
| | | 144 | | |
| 53-2 | ESB2.2-20-17 | 145 | UUUGCUCGUAUUGGGCGCCUGG ;;;;;;;;;;;;;;;;;;;; CUAAACGAGCAUAACCCGCGGA | 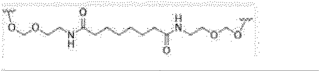 |
| | | 146 | | |
| 54 | ESB2.2-5 | 147 | AGAUAAGAUGAAUCUCUUCUCC ;;;;;;;;;;;;;;;;;;;; UCUAUUCUACUUAGAGAAGA | 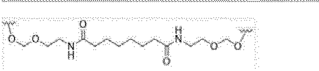 |
| | | 148 | | |
| 54-2 | ESB2.2-20-22 | 149 | UUUGGUCGUAUUGGGGCGCCUGG ; ; ; ; ; ; ; ; ; ; ; ; ; ; ; ; ; ; ; ; AAACCAGCAUAACCCCGCGGA | 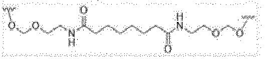 |
| | | 150 | | |
| 55 | ESB2.2-6 | 151 | AGAUAAGAUGAAUCUCUUCUCC ;;;;;;;;;;;;;;;;;;; CGCUAUUCUACUUAGAGAAGA | 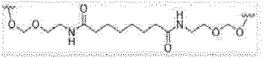 |
| | | 152 | | |
| 55-2 | ESB2.2-20-21 | 153 | 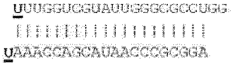 U̲UUGGUCGUAUUGGGCGCCUGG U̲AAACCAGCAUAACCCGCGGA | 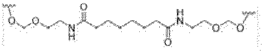 |
| | | 154 | | |
| 55-3 | ESB2.2_HPRT1-9 | 155 | ACUUUGUUGGAUUUGAAAUUCC ;;;;;;;;;;;;;;;;;;;; CUGAAACAACCUAAACUUUAA | 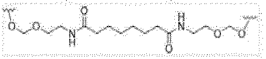 |
| | | 156 | | |
| 56 | ESB2.2-7 | 157 | AGAUAAGAUGAAUCUCUUCUCG ;;;;;;;;;;;;;;;;;;;; ACCUCUAUUCUACUUAGAGAAGA | 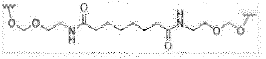 |
| | | 158 | | |

**[Table 3-6]**

| | | | | |
|---|---|---|---|---|
| 56-2 | ESB2.2-20-19 | 159 | U̲UUGGUCGUAUUGGCGCCUGG ; ; ; ; ; ; ; ; ; ; ; ; ; ; ; ; ; ; ; C̲CUAAACCAGCAUAACCGCGGA | 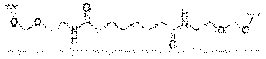 |
| | | 160 | | |
| 57 | ESB2.2-8 | 161 | AGAUAAGAUGAAUCUCUUCUCC ; ; ; ; ; ; ; ; ; ; ; ; ; ; ; ; ; ; ; ; ; ACCUCUAUUCUACUUAGAGAAGA | 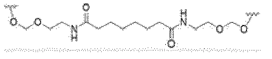 |
| | | 162 | | |
| 57-2 | ESB2.2-20-20 | 163 | U̲UUGGUCGUAUUGGGGGCCUGG ; ; ; ; ; ; ; ; ; ; ; ; ; ; ; ; ; ; ; ; ; CC̲UAAACCAGCAUAACCCGGCGGA | 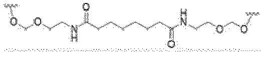 |
| | | 164 | | |
| 58 | ESB2.2-9 | 165 | AGAUAAGAUGAAUCUCUUCUCC ;;;;;;;;;;;;;;;;;;;;;; ACCUCUAUUCUACUUAGAGAAGA | 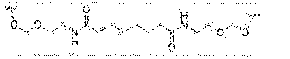 |
| | | 166 | | |
| 58-2 | ESB2.2_HPRT1-8 | 167 | ACUUUGUUGGAUUUGAAAUUCC ;;;;;;;;;;;;;;;;;;;;; GUCUGAAACAACCUAAACUUUAA | 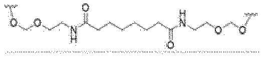 |
| | | 168 | | |
| 59 | ESB2.2-10 | 169 | AGAUAAGAUGAAUCUCUUCUCC ; ; ; ; ; ; ; ; ; ; ; ; ; ; ; ; ; ; ; ; UACCUCUAUUCUACUUAGAGAAGA | 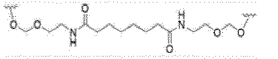 |
| | | 170 | | |
| 59-2 | ESB2.2-20-18 | 171 | U UUUGGUCGUAUUGGCGCCUGG ; ; ; ; ; ; ; ; ; ; ; ; ; ; ; ; ; ; ; ; GCCUAAACCAGCAUAACCGCGGA | 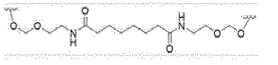 |
| | | 112 | | |
| 60 | ESB2.2-11 | 173 | AGAUAAGAUGAAUCUCUUCUCC ;;;;;;;;;;;;;;;;;;;;;; UACCUCUAUUCUACUUAGAGAAGA | 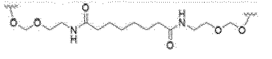 |
| | | 174 | | |
| 50-2 | ESB2.2_HPRT1-7 | 175 |   ACUUUGUUGGAUUUGAAAUUCC ;;;;;;;;;;;;;;;;;;;;;; GGUCUGAAACAACCUAAACUUUAA | 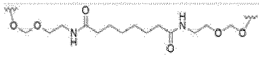 |
| | | 176 | | |
| 61 | ESB2.2-12 | 177 | AGAUAAGAUGAAUCUCUUCUCC ; ; ; ; ; ; ; ; ; ; ; ; ; ; ; ; ; ; ; ; ; UACCUCUAUUCUACUUAGAGAAGA | 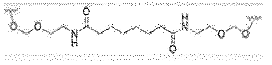 |
| | | 178 | | |
| 62 | ESB2.2-20-23 | 119 | UUUGGUCGUAUUGGGCGCCU ;;;;;;;;;;;;;;;;;;;; CUAAACCAGCAUAACCCGCGGA | 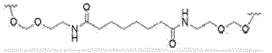 |
| | | 180 | | |
| 62-2 | ESB2.2_HPRT1-10 | 161 | A̲CUUUGUUGGAUUUGAAAUU ; ; ; ; ; ; ; ; ; ; ; ; ; ; ; ; ; ; ; UC̲UGAAACAACCUAAACUUUAA | 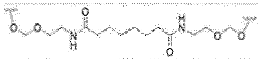 |
| | | 182 | | |

**[Table 3-7]**

| | | | | |
|---|---|---|---|---|
| 63 | ESB2.2-20-24 | 183 | U̲UUGGUCGUAUUGGGCGCCUGGUC ; ; ; ; ; ; ; ; ; ; ; ; ; ; ; ; ; ; ; ; ; CU̲AAACCAGCAUAACCCGCGGA | 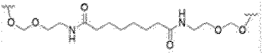 |
| | | 184 | | |
| 64 | ESB2.2-NEK-S21 | 185 | GAUAAGAUGAAUCUCUUCUCC ; ; ; ; ; ; ; ; ; ; ; ; ; ; ; ; ; ; ; CCUCUAUUCUACUUAGAGAAGA | 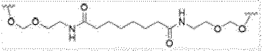 |
| | | 186 | | |
| 64-2 | ES82.2-2Q-521 | 187 | uUGGUCGUAUUGGGCGCCUGG ; ; ; ; ; ; ; ; ; ; ; ; ; ; ; ; ; ; ; CuAAACCAGCAUAACCCGCGGA | 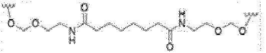 |
| | | 188 | | |
| 65 | ESB2.2-NEK-S19 | 189 | UAAGAUGAAUCUCUUCUCC ;;;;;;;;;;;;;;;;; CCUCUAUUCUACUUAGAGAAGA | 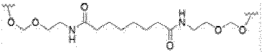 |
| | | 100 | | |
| 65-2 | ESB2.2-20-S19 | 191 | U̲GGUCGUAUUGGGCGCCUGG ; ; ; ; ; ; ; ; ; ; ; ; ; ; ; ; ; ; CU̲AAACCAGCAUAACCCGCGGA | 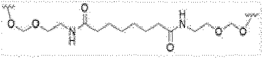 |
| | | 192 | | |
| 66 | ESB2.2-NEK-S18 | 193 | AAGAUGAAUCUCUUCUCC ;;;;;;;;;;;;;;;;; CCUCUAUUCUACUUAGAGAAGA | 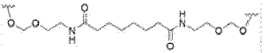 |
| | | 194 | | |
| 66-2 | ESB2.2-20-S18 | 195 | *G*GUCGUAUUGGGCGCCUGG ; ; ; ; ; ; ; ; ; ; ; ; ; ; ; ; ; ; ; CUAAACCAGCAUAACCCGCGGA | 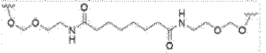 |
| | | 196 | | |
| 67 | ESB2.2-NEK-S17 | 197 |        AGAUGAAUCUCUUCUCC ;;;;;;;;;;;;;;; CCUCUAUUCUACUUAGAGAAGA | 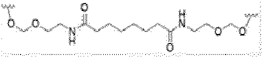 |
| | | 198 | | |
| 67-2 | ESB2.2-20-S17 | 199 200 | GUCGUAUUGGGCGGCCUGG ;;;;;;;;;;;;;;;;; CUAAACCAGCAUAACCCGCGGA | 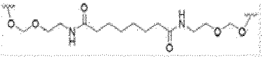 |
| 68 | ESB2.2-15-C2 | 201 202 | GAUAAGAUGAAUCUCUUCUCC ;;;;;;;;;;;;;;;;;;; CUCUAUUCUACUUAGAGAAGA | 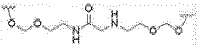 |
| 69 | ESB2.2-15-C4 | 203 | GAUAAGAUGAAUCUCUUCUCC ; ; ; ; ; ; ; ; ; ; ; ; ; ; ; ; ; ; ; CUCUAUUCUACUUAGAGAAGA | 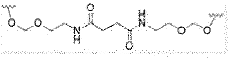 |
| | | 204 | | |
| 70 | ESB2.2-16-C2 | 205 | GAUAAGAUGAAUCUCUUCUCC ; ; ; ; ; ; ; ; ; ; ; ; ; ; ; ; ; ; ; CUCUAUUCUACUUAGAGAAGA | 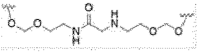 |
| | | 206 | | |
| 71 | ESB2.2-16-C4 | 207 | GAUAAGAUGAAUCUCUUCUCC ; ; ; ; ; ; ; ; ; ; ; ; ; ; ; ; ; ; ; CUCUAUUCUACUUAGAGAAGA | 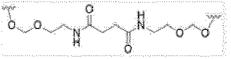 |
| | | 208 | | |
| 72 | ESB2.2-13-C2 | 209 | AGAUAAGAUGAAUCUCUUCUCC ; ; ; ; ; ; ; ; ; ; ; ; ; ; ; ; ; ; ; ; CCUCUAUUCUACUUAGAGAAGA | 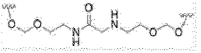 |
| | | 210 | | |

**[Table 3-8]**

| | | | | |
|---|---|---|---|---|
| 73 | ESB2.2-13-C4 | 211 | AGAUAAGAUGAAUCUCUUCUCC ; ; ; ; ; ; ; ; ; ; ; ; ; ; ; ; ; ; ; ; CCUCUAUUCUACUUAGAGAAGA | 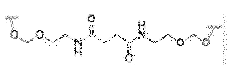 |
| | | 212 | | |
| 74 | ESB2.2-14-C2 | 213 | A̲GAUAAGAUGAAUCUCUUCUCC ;;;;;;;;;;;;;;;;;;;; CC̲UCUAUUCUACUUAGAGAAGA | 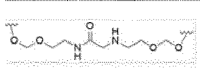 |
| | | 214 | | |
| 75 | ESB2.2-14-C4 | 215 | AGAUAAGAUGAAUCUCUUCUCC ;;;;;;;;;;;;;;;;;;;;;; CCUCUAUUCUACUUAGAGAAGA | 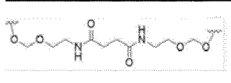 |
| | | 216 | | |
| 76 | ESB2.2-16-C12 | 217 | GAUAAGAUGAAUCUCUUCUCC ;;;;;;;;;;;;;;;;;;; CUCUAUUCUACUUAGAGAAGA | 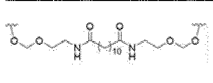 |
| | | 218 | | |
| 76-2 | ESB2.2-20-C12 | 219 | UUUGGUCGUAUUGGGCGCCUGG ;;;;;;;;;;;;;;;;;;;; CUAAACCAGCAUAACCCGCGGA | 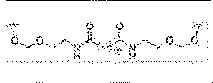 |
| | | 220 | | |
| 77 | ESB2.2-16-C16 | 221 |   GAUAAGAUGAAUCUCUUCUCC ;;;;;;;;;;;;;;;;;;; CUCUAUUCUACUUAGAGAAGA | 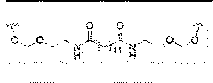 |
| | | 222 | | |
| 77-2 | ESB2.2-20-C16 | 223 | UUUGGUCGUAUUGGCGCCUGG ; ; ; ; ; ; ; ; ; ; ; ; ; ; ; ; ; ; ; CUAAACCAGCAUAACCGCGGA | 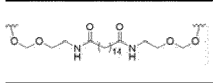 |
| | | 224 | | |
| 78 | ESB2.2-16-C19 | 225 | GAUAAGAUGAAUCUCUUCUCC ; ; ; ; ; ; ; ; ; ; ; ; ; ; ; ; ; ; ; CUCUAUUCUACUUAGAGAAGA | 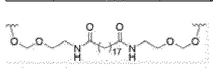 |
| | | 226 | | |
| 78-2 | ESB2.2-20-C19 | 227 | U̲UUGGUCGUAUUGGGCGCCUGG ;;;;;;;;;;;;;;;;;;;; CU̲AAACCAGCAUAACCCGCGGA | 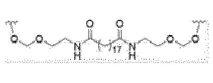 |
| | | 228 | | |
| 79 | ESB2.2-16-EGS | 229 | GAUAAGAUGAAUCUGUUCUCC ; ; ; ; ; ; ; ; ; ; ; ; ; ; ; ; ; ; ; CUCUAUUCUACUUAGAGAAGA | 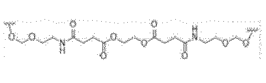 |
| | | 230 | | |
| 79-2 | ESB2.2-20-EGS | 231 | UUUGGUCGUAUUGGGCGCCUGG ;;;;;;;;;;;;;;;;;;;; CUAAACCAGCAUAACCCGCGGA | 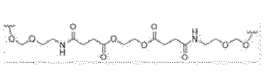 |
| | | 232 | | |
| 80 | ESB2.2-16-C602 | 233 |   GAUAAGAUGAAUCUCUUCUCC ;;;;;;;;;;;;;;;;;;; CUCUAUUCUACUUAGAGAAGA | 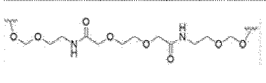 |
| | | 234 | | |
| 81 | ESB2.2-16-PEG5 | 235 |   GAUAAGAUGAAUCUCUUCUCC ;;;;;;;;;;;;;;;;;;; CUCUAUUCUACUUAGAGAAGA | 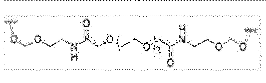 |
| | | 236 | | |
| 81-2 | ESB2.2-20-PEG5 | 237 | UUUGGUCGUAUUGGGCGCCUGG ; ; ; ; ; ; ; ; ; ; ; ; ; ; ; ; ; ; ; ; CUAAACCAGCAUAACCCGCGGA | 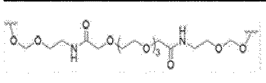 |
| | | 238 | | |

**[Table 3-9]**

| | | | | |
|---|---|---|---|---|
| 82 | ESB2.2-16-PEG6 | 239 |   GAUAAGAUGAAUCUCUUCUCC ;;;;;;;;;;;;;;;;;;; CUCUAUUCUACUUAGAGAAGA | 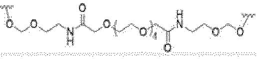 |
| | | 240 | | |
| 82-2 | ESB2.2-20-PEG6 | 241 | UUUCGGUCGUAUUGGGCGCCUGG ;;;;;;;;;;;;;;;;;;;;;; CUAAACCAGCAUAACCCGCGGA | 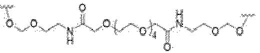 |
| | | 242 | | |
| 83 | ESB2.2-16-DST | 243 | GAUAAGAUGAAUCUCUUCUCC ; ; ; ; ; ; ; ; ; ; ; ; ; ; ; ; ; ; ; CUCUAUUCUACUUAGAGAAGA | 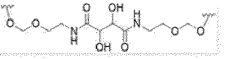 |
| | | 244 | | |
| 83-2 | ESB2.2-20-DST | 245 | UUUGGUCGUAUUGGCCGCCCUGG ;;;;;;;;;;;;;;;;;;;; CUAAACCAGCAUAACCGGCGGA | 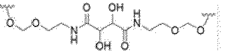 |
| | | 246 | | |
| 84 | ESB2.2-16 -trans-3-hexene | 247 | GAUAAGAUGAAUCUCUUCUCC ;;;;;;;;;;;;;;;;;;; CUCUAUUCUACUUAGAGAAGA | 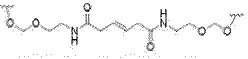 |
| | | 248 | | |
| 84-2 | ESB2.2-20 -trans-3-hexene | 249 | UUUGGUCGUAUUGGGCGCCUGG ;;;;;;;;;;;;;;;;;;;; CUAAACCAGCAUAACCCGCGGA | 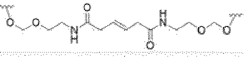 |
| | | 250 | | |
| 85 | ESB2.2-16-PP | 251 | GAUAAGAUGAAUCUCUUCUCC ; ; ; ; ; ; ; ; ; ; ; ; ; ; ; ; ; ; ; CUCUAUUCUACUUAGAGAAGA | 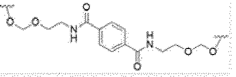 |
| | | 252 | | |
| 85-2 | ESB2.2-20-PP | 253 | U̲UUGGUCGUAUUGGGCGCCUGG ; ; ; ; ; ; ; ; ; ; ; ; ; ; ; ; ; ; ; ; ; CU̲AAACCAGCAUAACCCGCGGA | 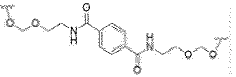 |
| | | 254 | | |
| 86 | ESB2.2-16-MP | 255 | GAUAAGAUGAAUCUCUUCUCC ; ; ; ; ; ; ; ; ; ; ; ; ; ; ; ; ; ; ; CUCUAUUCUACUUAGAGAAGA | 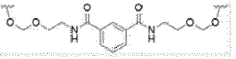 |
| | | 256 | | |
| 86-2 | ESB2.2-20-MP | 257 | UUUGGUCGUAUUGGGCGCCUGG ;;;;;;;;;;;;;;;;;;;; CUAAACCAGCAUAACCCGCGGA | 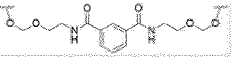 |
| | | 258 | | |
| 87 | ESB2.2-16 -adamantane | 259 | GAUAAGAUGAAUCUCUUCUCC ; ; ; ; ; ; ; ; ; ; ; ; ; ; ; ; ; ; ; CUCUAUUCUACUUAGAGAAGA | 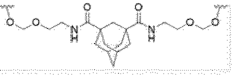 |
| | | 260 | | |
| 87-2 | ESB2.2-20 -adamantane | 261 | UUUUGGUCGUAUUGGCCGCCU ;;;;;;;;;;;;;;;;;;;; CUAAACCAGCAUAACCGGCGGA | 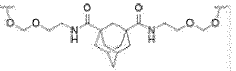 |
| | | 262 | | |
| 88 | ESB2.2-16-pyridine | 263 |   GAUAAGAUGAAUCUCUUCUCC ;;;;;;;;;;;;;;;;;;; CUCUAUUCUACUUAGAGAAGA | 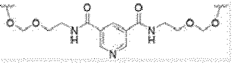 |
| | | 264 | | |

**[Table 3-10]**

| | | | | |
|---|---|---|---|---|
| 88-2 | ESB2.2-20-pyridine | 265 | UUUGGUCGUAUUGGGCGCCUGG ;;;;;;;;;;;;;;;;;;;; CUAAACCAGCAUAACCCGCGGA | 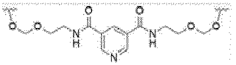 |
| | | 266 | | |
| 89 | ESB2.2-16-furan | 267 | GAUAAGAUGAAUCUCUUCUCC ;;;;;;;;;;;;;;;;;;; CUCUAUUCUACUUAGAGAAGA | 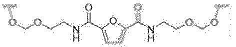 |
| | | 268 | | |
| 89-2 | ESB2.2-20-furan | 269 | U̲UUGGUCGUAUUGGGCGCCUGG ; ; ; ; ; ; ; ; ; ; ; ; ; ; ; ; ; ; ; ; ; CU̲AAACCAGCAUAACCCGCGGA | 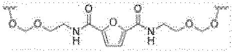 |
| | | 270 | | |
| 90 | ESB2 2-16-134-72 | 271 | GAUAAGAUGAAUCUCUUCUCC ;;;;;;;;;;;;;;;;;;; CUCUAUUCUACUUAGAGAAGA | 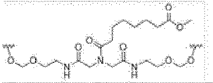 |
| | | 272 | | |
| 91 | ESB2.2-16-DSP | 273 | GAUAAGAUGAAUCUUCUCC ;;;;;;;;;;;;;;;;;;; CUCUAUUCUACUUAGAGAAGA | 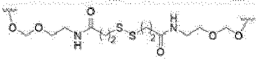 |
| | | 274 | | |
| 91-2 | ESB2.2-20-DSP | 275 | UUUGGUCGUAUUGGGCGCCUGG ;;;;;;;;;;;;;;;;;;;; CUAAACCAGCAUAACCCGCGGA | 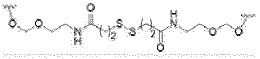 |
| | | 276 | | |
| 92 | ESB2.2-16-DSH | 277 | GAUAAGAUGAAUCUCUUCUCC ;;;;;;;;;;;;;;;;;;; CUCUAUUCUACUUAGAGAAGA | 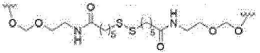 |
| | | 278 | | |
| 92-2 | ESB2.2-20-DSH | 279 | UUUCCUCGUAUUGGGCCCCUGG ;;;;;;;;;;;;;;;;;;;; CUAAAGGAGCAUAACCCGGGGA | 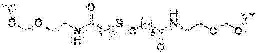 |
| | | 280 | | |
| 93 | ESB2.2-16-DSO | 281 | GAUAAGAUGAAUCUCUUCUCC ; ; ; ; ; ; ; ; ; ; ; ; ; ; ; ; ; ; ; CUCUAUUCUACUUAGAGAAGA | 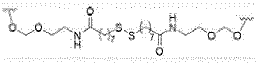 |
| | | 282 | | |
| 93-2 | ESB22-20-DSO | 283 | UUUGGUCGUAUUGGGCGCCUGG ;;;;;;;;;;;;;;;;;;;; CUAAACCAGCAUAACCCGCGGA | 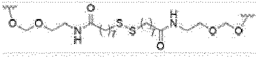 |
| | | 284 | | |
| 94 | ESB2.2-20-NH2 | 285 | UUUUGGUCGUAUUGGGCGCC ;;;;;;;;;;;;;;;;;;;; CUAAACCAGCAUAACCCGCGGA | 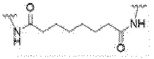 |
| | | 286 | | |
| 95 | ESB2.2-20-AEC | 287 | UUUGGUCGUAUUGGGCGCCUGG ;;;;;;;;;;;;;;;;;;;; CUAAACCAGCAUAACCCGCGGA | 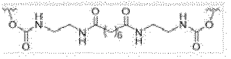 |
| | | 288 | | |
| 96 | ESB2.2-20 -asNH2-ssAEM | 289 | UUUGGUCGUAUUGGGCGCCUGG ;;;;;;;;;;;;;;;;;;;; CUAAACCAGCAUAACCCGCGGA | 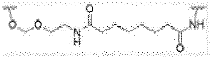 |
| | | 290 | | |
| 97 | ESB2.2-16 -141-152 | 281 | GAUAAGAUGAAUCUCUUCUCC ;;;;;;;;;;;;;;;;;;; CUCUAUUCUACUUAGAGAAGA | 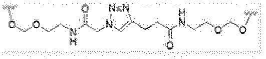 |
| | | 292 | | |
| 98 | ESB2.2-20 -141-178 | 293 | U̲UUGGUCGUAUUGGGCGCCUGG ;;;;;;;;;;;;;;;;;;;; CU̲AAACCAGCAUAACCCGCGGA | 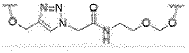 |
| | | 294 | | |

**[Table 3-11]**

| | | | | |
|---|---|---|---|---|
| 99 | ESB2.2-20 -141-171-1 | 295 | UUUGGUCGUAUUGGGCGCCUGG ; ; ; ; ; ; ; ; ; ; ; ; ; ; ; ; ; ; ; ; ; CUAAACCAGCAUAACCCGCGGA | 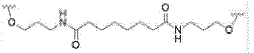 |
| | | 296 | | |
| 100 | ESB2.2-20 -141-171-2 | 297 | UUUGGUCGUAUUGGGCGCCUGG ;;;;;;;;;;;;;;;;;;;; CUAAACCAGCAUAACCCGCGGA | 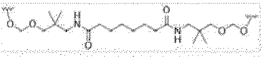 |
| | | 298 | | |
| 101 | ESB2.2-16-B01 | 289 | GAUAAGAUGAAUCUCUUCUCC ; ; ; ; ; ; ; ; ; ; ; ; ; ; ; ; ; ; ; CUCUAUUCUACUUAGAGAAGA | 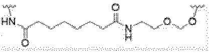 |
| | | 300 | | |
| 101-2 | ESB2.2-20-B01 | 301 | UUUGGUCGUAUUGGGCGCCUGG ; ; ; ; ; ; ; ; ; ; ; ; ; ; ; ; ; ; ; ; CUAAACCAGCAUAACCCGCGGA | 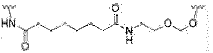 |
| | | 302 | | |
| 101-3 | ESBS2.2_HPRT1-1 -B01 | 303 | ACUUUGUUGGAUUUGAAAUUCC ; ; ; ; ; ; ; ; ; ; ; ; ; ; ; ; ; ; ; ; UCUGAAACAACCUAAACUUUAA | 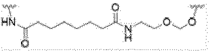 |
| | | 304 | | |
| 102 | ESB2.2-16-B02 | 305 |   GAUAAGAUGAAUCUCUUCUCC ; ; ; ; ; ; ; ; ; ; ; ; ; ; ; ; ; ; ; CUCUAUUCUACUUAGAGAAGA | 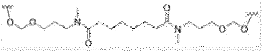 |
| | | 306 | | |
| 102-2 | ES82.2-20-B02 | 307 | U̲UUGGUCGUAUUGGGCGCCUGG ; ; ; ; ; ; ; ; ; ; ; ; ; ; ; ; ; ; ; ; ; CU̲AAACCAGCAUAACCCGCGGA | 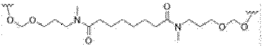 |
| | | 308 | | |
| 102-3 | ESB2.2_HPRT1-1 -B02 | 309 | ACUUUGUUGGAUUUGAAAUUCC ;;;;;;;;;;;;;;;;;;;; UCUGAAACAACCUAAACUUUAA | 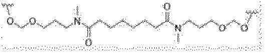 |
| | | 310 | | |
| 103 | ESB2.2-16-B03 | 311 | GAUAAGAUGAAUCUCUUCUCC ;;;;;;;;;;;;;;;;;;; CUCUAUUCUACUUAGAGAAGA | 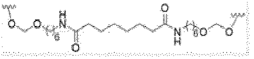 |
| | | 312 | | |
| 103-2 | ESB2.2-20-B03 | 313 | UUUGGUCGUAUUGGGCGCCUGG ; ; ; ; ; ; ; ; ; ; ; ; ; ; ; ; ; ; ; ; CUAAACCAGCAUAACCCGCGGA | 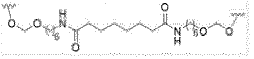 |
| | | 314 | | |
| 103-3 | ESB2.2_HPRT1-1 -B03 | 315 | ACUUUGUUGGAUUUGAAAUUCC ;;;;;;;;;;;;;;;;;;;; UCUGAAACAACCUAAACUUUAA | 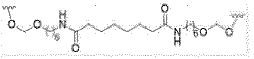 |
| | | 316 | | |
| 104 | ESB2.2-16-B04 | 317 | GAUAAGAUGAAUCUCUUCUCC ; ; ; ; ; ; ; ; ; ; ; ; ; ; ; ; ; ; ; CUCUAUUCUACUUAGAGAAGA | 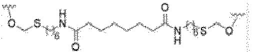 |
| | | 318 | | |
| 104-2 | ESB2.2-20-B04 | 319 | UUUGGUCGUAUUGGGCCGCCUGG ; ; ; ; ; ; ; ; ; ; ; ; ; ; ; ; ; ; ; ; ; CUAAACCAGCAUAACCCGGCGGA | 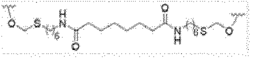 |
| | | 320 | | |
| 104-3 | ESB2.2_HPRT1-1 -B04 | 321 | ACUUUGUUGGAUUUGAAAUUCC ; ; ; ; ; ; ; ; ; ; ; ; ; ; ; ; ; ; ; ; UCUGAAACAACCUAAACUUUAA | 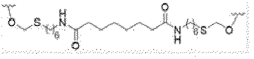 |
| | | 322 | | |

**[Table 3-12]**

| | | | | |
|---|---|---|---|---|
| 105 | ESB2.2-16-B05 | 323 | GAUAAGAUGAAUCUCUUCUCC ; ; ; ; ; ; ; ; ; ; ; ; ; ; ; ; ; ; ; CUCUAUUCUACUUAGAGAAGA | 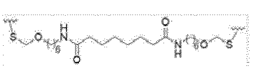 |
| | | 324 | | |
| 105-2 | ESB2.2-20-B05 | 325 | UUUGGUCGUAUUGGGCGCCUGG ; ; ; ; ; ; ; ; ; ; ; ; ; ; ; ; ; ; ; ; ; CUAAACCAGCAUAACCCGCGGA | 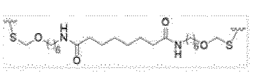 |
| | | 326 | | |
| 105-3 | ESB2.2_HPRT1-1 -B05 | 327 | ACUUUGUUGGAUUUGAAAUUCC ; ; ; ; ; ; ; ; ; ; ; ; ; ; ; ; ; ; ; UCUGAAACAAGCUAAACUUUAA | 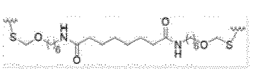 |
| | | 328 | | |
| 106 | ESB2.2-16-B06 | 329 |   GAUAAGAUGAAUCUCUUCUCC ;;;;;;;;;;;;;;;;;;; CUCUAUUCUACUUAGAGAAGA | 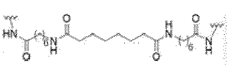 |
| | | 330 | | |
| 106-2 | ESB2.2-20-B06 | 331 | UUUGGUCGUAUUGGGCGCCUGG ;;;;;;;;;;;;;;;;;;;;; CUAAACCAGCAUAACCCGCGGA | 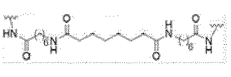 |
| | | 332 | | |
| 106-3 | ESB2.2_HPRT1-1 -B06 | 333 | A̲CUUUGUUGGAUUUGAAAUUCC ; ; ; ; ; ; ; ; ; ; ; ; ; ; ; ; ; ; ; ; UC̲UGAAACAACCUAAACUUUAA | 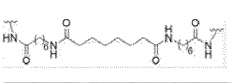 |
| | | 334 | | |
| 107 | ESB2.2-16-B07 | 335 |   GAUAAGAUGAAUCUCUUCUCC ;;;;;;;;;;;;;;;;;;; CUCUAUUCUACUUAGAGAAGA | 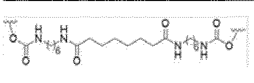 |
| | | 336 | | |
| 107-2 | ESB2.2-20-B07 | 337 | UUUGGUCGUAUUGGGCGCCUGG ; ; ; ; ; ; ; ; ; ; ; ; ; ; ; ; ; ; ; ; CUAAACCAGCAUAACCCGCGGA | 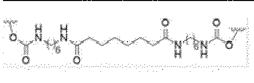 |
| | | 338 | | |
| 107-3 | ESB2.2_HPRT1-1 -B07 | 339 | ACUUUGUUGGAUUUGAAAUUCC ;;;;;;;;;;;;;;;;;;;; UCUGAAACAACCUAAACUUUAA | 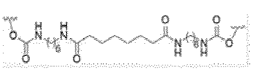 |
| | | 340 | | |
| 108 | ESB2.2-16-B08 | 341 | GAUAACAUGAAUCUCUUCUCC ; ; ; ; ; ; ; ; ; ; ; ; ; ; ; ; ; ; ; CUCUAUUGUACUUAGAGAAGA | 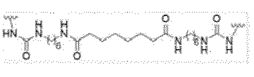 |
| | | 342 | | |
| 108-2 | ESB2.2-20-B08 | 343 | UUUGGUCGUAUUGGGCGCCUGG ;;;;;;;;;;;;;;;;;;;; CUAAACCAGCAUAACCCGCGGA | 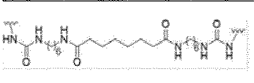 |
| | | 344 | | |
| 108-3 | ESB2.2_HPRT1-1 -B08 | 345 | ACUUUGUUGGAUUUGAAUUCC ;;;;;;;;;;;;;;;;;;;; UCUGAAACAACCUAAACUUUAA | 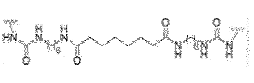 |
| | | 346 | | |
| 109 | ESB2.2-16-B09 | 347 | GAUAAGAUGAAUCUCUUCUCC ; ; ; ; ; ; ; ; ; ; ; ; ; ; ; ; ; ; ; CUCUAUUCUACUUAGAGAAGA | 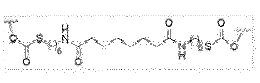 |
| | | 348 | | |
| 109-2 | ESB2.2-20-B09 | 349 | UUUGGUCGUAUUGGGCGCCUGG ;;;;;;;;;;;;;;;;;;;;; CUAAACCAGCAUAACCCGCGGA | 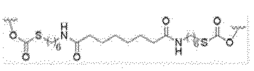 |
| | | 350 | | |

**[Table 3-13]**

| | | | | |
|---|---|---|---|---|
| 109-3 | ESB2.2_HPRT1-1 -B09 | 351 | A̲CUUUGUUGGAUUUGAAAUUCC ;;;;;;;;;;;;;;;;;;;; UC̲UGAAACAACCUAAACUUUAA | 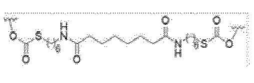 |
| | | 352 | | |
| 110 | ESB2.2-16-B10 | 353 | GAUAAGAUGAAUCUCUUCUCC ; ; ; ; ; ; ; ; ; ; ; ; ; ; ; ; ; ; ; CUCUAUUCUACUUAGAGAAGA | 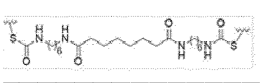 |
| | | 354 | | |
| 110-2 | ESB2.2-20-B10 | 355 | UUUGGUCGUAUUGGGCGGCCUGG ;;;;;;;;;;;;;;;;;;;;; CUAAACCAGCAUAACCCGCCGGA | 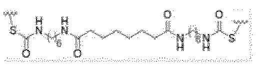 |
| | | 356 | | |
| 110-3 | ESB2.2_HPRT1-1 -B10 | 357 | ACUUUGUUGGAUUUGAAAUUCC ; ; ; ; ; ; ; ; ; ; ; ; ; ; ; ; ; ; ; ; UCUGAAACAACCUAAACUUUAA | 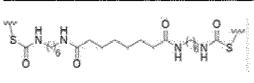 |
| | | 358 | | |
| 111 | ESB2.2-16-B11 | 359 | GAUAAGAUGAAUCUCUUCUCC ;;;;;;;;;;;;;;;;;;; CUCUAUUCUACUUAGAGAAGA | 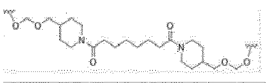 |
| | | 360 | | |
| 111-2 | ESB2.2-20-B11 | 361 | UUUGGUCGUAUUGGGCGCCUGG ;;;;;;;;;;;;;;;;;;;; CUAAACCAGCAUAACCCGCGGA | 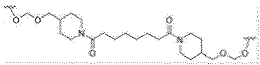 |
| | | 362 | | |
| 111-3 | ESB2.2_HPRT1-1 -B11 | 363 | ACUUUGUUGGAUUUGAAAUUCC ; ; ; ; ; ; ; ; ; ; ; ; ; ; ; ; ; ; ; ; UCUGAAACAACCUAAACUUUAA | 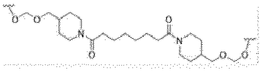 |
| | | 364 | | |
| 112 | ESB2.2-16-B12 | 365 | GAUAAGAUGAAUCUCUUCUCC ; ; ; ; ; ; ; ; ; ; ; ; ; ; ; ; ; ; ; CUCUAUUCUACUUAGAGAAGA | 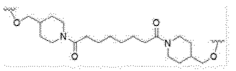 |
| | | 366 | | |
| 112-2 | ESB2.2-20-B12 | 367 | U̲UUGGUCGUAUUGGGCGCCUGG ; ; ; ; ; ; ; ; ; ; ; ; ; ; ; ; ; ; ; ; ; CU̲AAACCAGCAUAACCCGCGGA | 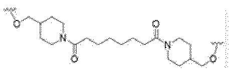 |
| | | 368 | | |
| 112-3 | ESB2.2_HPRT1-1 -612 | 369 | A ACUUUGUUGGAUUUGAAAUUCC ; ; ; ; ; ; ; ; ; ; ; ; ; ; ; ; ; ; ; ; UCUGAAACAACCUAAACUUUAA C | 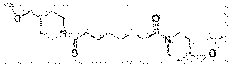 |
| | | 370 | | |
| 113 | ESB2.2-16-B13 | 371 | GAUAAGAUGAAUCUCUUCUCC ; ; ; ; ; ; ; ; ; ; ; ; ; ; ; ; ; ; ; CUCUAUUCUACUUAGAGAAGA | 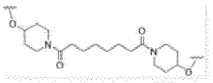 |
| | | 372 | | |
| 113-2 | ESB2.2-20-B13 | 373 | U̲UUGGUCGUAUUGGGCGCCUGG ; ; ; ; ; ; ; ; ; ; ; ; ; ; ; ; ; ; ; ; CU̲AAACCAGCAUAACCCGCGGA | 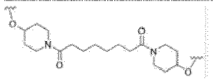 |
| | | 374 | | |
| 113-3 | ESB2.2_HPRT1-1 -B13 | 375 | A̲CUUUGUUGGAUUUGAAAUUCC ; ; ; ; ; ; ; ; ; ; ; ; ; ; ; ; ; ; ; ; ; UC̲UGAAACAACCUAAACUUUAA | 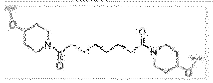 |
| | | 376 | | |
| 114 | ESB2.2-16-B14 | 377 |   GAUAAGAUGAAUCUCUUCUCC ;;;;;;;;;;;;;;;;;;; CUCUAUUCUACUUAGAGAAGA | 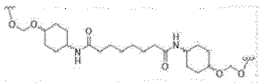 |
| | | 378 | | |

**[Table 3-14]**

| | | | | |
|---|---|---|---|---|
| 114-2 | ESB2.2-20-B14 | 379 |   UUUGGUCGUAUUGGGCGCCUGG ;;;;;;;;;;;;;;;;;;;; CUAAACCAGCAUAACCCGCGGA | 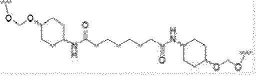 |
| | | 380 | | |
| 114-3 | ESB2.2_HPRT1-1 -814 | 381 | ACUUUGUUGGAUUUGAAAUUCC ;;;;;;;;;;;;;;;;;;;; UCUGAAACAACCUAAACUUUAA | 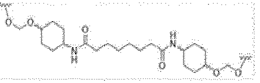 |
| | | 382 | | |
| 115 | ESB2.2-16-B15 | 383 | GAUAAGAUGAAUCUCUUCUCC ;;;;;;;;;;;;;;;;;;; CUCUAUUCUACUUAGAGAAGA | 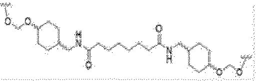 |
| | | 384 | | |
| 115-2 | ESB2.2-20-B15 | 385 | UUUGGUCGUAUUGGGCGCCUGG ;;;;;;;;;;;;;;;;;;;; CUAAACCAGCAUAACCCGCGGA | 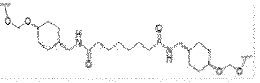 |
| | | 386 | | |
| 115-3 | ESB2.2_HPRT1-1 -B15 | 387 | A̲CUUUGUUGGAUUUGAAAUUCC ;;;;;;;;;;;;;;;;;;;; UC̲UGAAACAACCUAAACUUUAA | 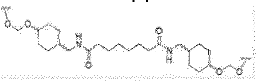 |
| | | 388 | | |
| 116 | ESB2.2-16-B16 | 389 |   GAUAAGAUGAAUCUCUUCUCC ;;;;;;;;;;;;;;;;;;; CUCUAUUCUACUUAGAGAAGA | 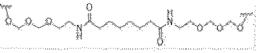 |
| | | 390 | | |
| 116-2 | ES82.2-20-B16 | 391 | U̲UUGGUCGUAUUGGGCGCCUGG ;;;;;;;;;;;;;;;;;;;; CU̲AAACCAGCAUAACCCGCGGA | 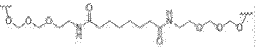 |
| | | 392 | | |
| 116-3 | ESB2.2_HPRT1-1 -B16 | 393 | ACUUUGUUGGAUUUGAAAUUCC ; ; ; ; ; ; ; ; ; ; ; ; ; ; ; ; ; ; ; ; UCUGAAACAACCUAAACUUUAA | 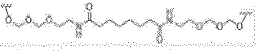 |
| | | 394 | | |
| 117 | ESB2.2-16-B17 | 395 | GAUAAGAUCAAUCUCUUCUCC ;;;;;;;;;;;;;;;;;;; CUCUAUUCUAGUUAGAGAAGA | 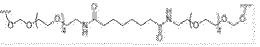 |
| | | 396 | | |
| 117-2 | ESB2.2-20-B17 | 397 | UUUGGUCGUAUUGGGCGCCUGG ;;;;;;;;;;;;;;;;;;;; CUAAACCAGCAUAACCCGCGGA | 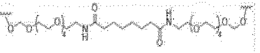 |
| | | 398 | | |
| 117-3 | ESB2.2_HPRT1-1 -B17 | 399 | **A**CUUUGUUGGAUUUGAAAUUCC ; ; ; ; ; ; ; ; ; ; ; ; ; ; ; ; ; ; ; ; ; U**C**UGAAACAACCUAAACUUUAA | 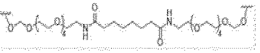 |
| | | 400 | | |
| 118 | ESB2.2-16-B18 | 401 | GAUAAGAUGAAUCUCUUCUCC ;;;;;;;;;;;;;;;;;;; CUCUAUUCUACUUAGAGAAGA | 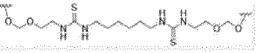 |
| | | 402 | | |
| 118-2 | ESB2.2-20-B18 | 403 | U̲UUGGUCGUAUUGGGCGCCUGG ; ; ; ; ; ; ; ; ; ; ; ; ; ; ; ; ; ; ; ; CU̲AAACCAGCAUAACCCGCGGA | 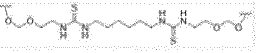 |
| | | 404 | | |
| 118-3 | ESB2.2_HPRT1-1 -B18 | 405 | A̲CUUUGUUGGAUUUGAAAUUCC ;;;;;;;;;;;;;;;;;;;; UC̲UGAAACAACCUAAACUUUAA | 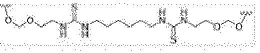 |
| | | 406 | | |

**[Table 3-15]**

| | | | | |
|---|---|---|---|---|
| 119 | ESB2.2-16-B19 | 407 | GAUAAGAUGAAUCUCUUCUCC ; ; ; ; ; ; ; ; ; ; ; ; ; ; ; ; ; ; ; CUCUAUUCUACUUAGAGAAGA | 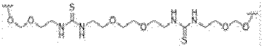 |
| | | 408 | | |
| 119-2 | ESB2.2-20-B19 | 409 |   UUUGGUCGUAUUGGCGCCUGG ;;;;;;;;;;;;;;;;;;; CUAAACCAGCAUAACCGCGGA | 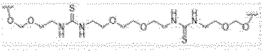 |
| | | 410 | | |
| 119-3 | ESB2.2 _HPRT1-1 -B19 | 411 | ACUUUGUUGGAUUUGAAAUUCG ;;;;;;;;;;;;;;;;;;;; UCUGAAACAACCUAAACUUUAA | 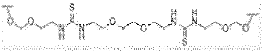 |
| | | 412 | | |
| 120 | ESB2.2-16-B20 | 413 | GAUAAGAUGAAUCUCUUCUCC ;;;;;;;;;;;;;;;;;;; CUCUAUUCUACUUAGAGAAGA | 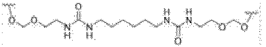 |
| | | 414 | | |
| 120-2 | ESB2.2-20-B20 | 415 | UUUGGUCGUAUUGGGCGCCUGG ;;;;;;;;;;;;;;;;;;;; CUAAACCAGCAUAACCCGCGGA | 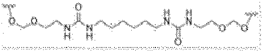 |
| | | 416 | | |
| 120-3 | ESB2.2_HPRT1-1 -B20 | 417 | ACUUUGUUGGAUUUGAAAUUCC ;;;;;;;;;;;;;;;;;;;; UCUGAAACAACCUAAACUUUAA | 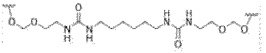 |
| | | 418 | | |
| 121 | ESB2.2-16-B21 | 419 |   GAUAAGAUGAAUCUCUUCUCC ;;;;;;;;;;;;;;;;;;; CUCUAUUCUACUUAGAGAAGA | 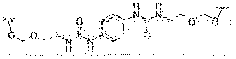 |
| | | 420 | | |
| 121-2 | ESB2.2-20-B21 | 421 | U̲UUGGUCGUAUUGGGCGCCUGG ; ; ; ; ; ; ; ; ; ; ; ; ; ; ; ; ; ; ; ; ; CU̲AAACCAGCAUAACCCGCGGA | 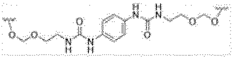 |
| | | 422 | | |
| 121-3 | ESB2.2_HPRT1-1 -B21 | 423 |   ACUUUGUUGGAUUUGAAAUUCC ;;;;;;;;;;;;;;;;;;;; UCUGAAACAACCUAAACUUUAA | 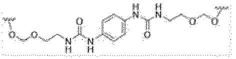 |
| | | 424 | | |
| 122 | ESB2.2-16-B22 | 425 | GAUAAGAUGAAUCUCUUCUCC ;;;;;;;;;;;;;;;;;;; CUCUAUUCUACUUAGAGAAGA | 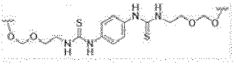 |
| | | 426 | | |
| 122-2 | ESB2.2-20-822 | 427 | U̲UUGGUCGUAUUGGGCGCCUGG CU̲AAACCAGCAUAACCCGCGGCA | 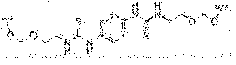 |
| | | 428 | | |
| 122-3 | ESB2.2_HPRT1-1 -B22 | 429 | A̲CUUUGUUGGAUUUGAAAUUCC ; ; ; ; ; ; ; ; ; ; ; ; ; ; ; ; ; ; ; ; UC̲UGAAACAACCUAAACUUUAA | 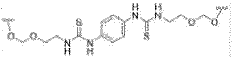 |
| | | 430 | | |
| 123 | ESB2.2-16-B23 | 431 | GAUAAGAUGAAUCUCUUCUCC ;;;;;;;;;;;;;;;;;;;; CUCUAUUCUACUUAGAGAAGA | 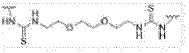 |
| | | 432 | | |
| 123-2 | ESB2.2-20-B23 | 433 | UUUGGUCGUAUUGGGCGCCUGG ;;;;;;;;;;;;;;;;;;;; CUAAACCAGCAUAACCCGCGGA | 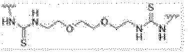 |
| | | 434 | | |

**[Table 3-16]**

| | | | | |
|---|---|---|---|---|
| 123-3 | ESB2.2_HPRT1-1 -B23 | 435 | ACUUUGUUGGAUUUGAAAUUCC ; ; ; ; ; ; ; ; ; ; ; ; ; ; ; ; ; ; ; ; UCUGAAACAACCUAAACUUUAA | 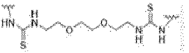 |
| | | 436 | | |
| 124 | ESB2.2-16-B24 | 437 |   GAUAAGAUGAAUCUCUUCUCC ;;;;;;;;;;;;;;;;;;; CUCUAUUCUACUUAGAGAAGA | 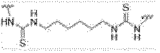 |
| | | 438 | | |
| 124-2 | ESB2.2-20-B24 | 439 | U̲UUGGUCGUAUUGGGCGGCCUGG ;;;;;;;;;;;;;;;;;;;;; CU̲AAACCAGCAUAACCCGCCGGA | 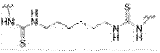 |
| | | 440 | | |
| 124-3 | ESB2.2_HPRT1-1 -B24 | 441 | ACUUUGUUGGAUUUGAAAUUCC ;;;;;;;;;;;;;;;;;;;; UCUGAAACAACCUAAACUUUAA | 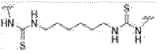 |
| | | 442 | | |
| 125 | ESB2.2-16-B25 | 443 |   GAUAAGAUGAAUCUCUUCUCC ;;;;;;;;;;;;;;;;;;; CUCUAUUCUACUUAGAGAAGA | 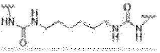 |
| | | 444 | | |
| 125-2 | ESB2.2-20-B25 | 445 | U̲UUGGUCGUAUUGGGCGCCUGG ;;;;;;;;;;;;;;;;;;;;;; CU̲AAACCAGCAUAACCCGCGGA | 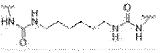 |
| | | 446 | | |
| 125-3 | ESB2.2_HPRT1-1 -B25 | 447 | ACUUUGUUGGAUUUGAAAUUCC ;;;;;;;;;;;;;;;;;;;;; UCUGAAACAACCUAAACUUUAA | 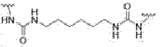 |
| | | 448 | | |
| 126 | ESB2.2-16-B26 | 449 | GAUAAGAUGAAUCUCUUCUCC ;;;;;;;;;;;;;;;;;;; CUCUAUUCUACUUAGAGAAGA | 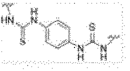 |
| | | 450 | | |
| 126-2 | ESB2.2-20-B26 | 451 | U̲UUGGUCGUAUUGGCGCCUGG ;;;;;;;;;;;;;;;;;;; CU̲AAACCAGCAUAACCGCGGA | 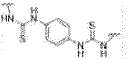 |
| | | 452 | | |
| 126-3 | ESB2.2_HPRT1-1 -B26 | 453 | ACUUUCUUGCAUUUGAAAUUCC ;;;;;;;;;;;;;;;;;;;; UCUGAAAGAACGUAAACUUUAA | 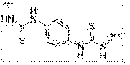 |
| | | 454 | | |
| 127 | ESB2.2-16-B27 | 455 | GAUAAGAUGAAUCUCUUCUCC ;;;;;;;;;;;;;;;;;;; CUCUAUUCUACUUAGAGAAGA | 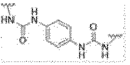 |
| | | 456 | | |
| 127-2 | ESB2.2-20-B27 | 457 | U̲UUGGUCGUAUUGGGCGCCUGG ; ; ; ; ; ; ; ; ; ; ; ; ; ; ; ; ; ; ; CU̲AAACCAGCAUAACCCGCGGA | 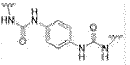 |
| | | 458 | | |
| 127-3 | ESB2.2_HPRT1-1 -B27 | 459 |   ACUUUGUUGGAUUUGAAAUUCC ;;;;;;;;;;;;;;;;;;;; UCUGAAACAACCUAAACUUUAA | 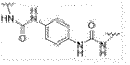 |
| | | 460 | | |
| 128 | ESB2.2-16-B28 | 461 |   GAUAAGAUGAAUCUCUUCUCC ; ; ; ; ; ; ; ; ; ; ; ; ; ; ; ; ; ; ; CUCUAUUCUACUUAGAGAAGA | 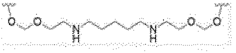 |
| | | 462 | | |

**[Table 3-17]**

| | | | | |
|---|---|---|---|---|
| 128-2 | ESB2.2-20-B28 | 463 | UUUGGUCGUAUUGGGCGCCUGG ; ; ; ; ; ; ; ; ; ; ; ; ; ; ; ; ; ; ; : ; ; CUAAACCAGCAUAACCCGCGGA | 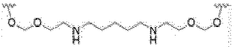 |
| | | 464 | | |
| 128-3 | ESB2.2_HPRT1-1 -B28 | 465 | A̲CUUUGUUGGAUUUGAAAUUCC ;;;;;;;;;;;;;;;;;;;; UC̲UGAAACAACCUAAACUUUAA | 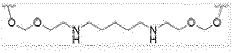 |
| | | 466 | | |
| 129 | ESB2.2-16-B29 | 467 | GAUAAGAUGAAUCUCUUCUCC ; ; ; ; ; ; ; ; ; ; ; ; ; ; ; ; ; ; ; CUCUAUUCUACUUAGAGAAGA | 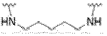 |
| | | 468 | | |
| 129-2 | ESB2.2-20-B29 | 469 | UUUGGUCGUAUUGGGCGCCUGG ; ; ; ; ; ; ; ; ; ; ; ; ; ; ; ; ; ; ; ; ; CUUAAACCAGCAUAACCCGCGGA | 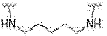 |
| | | 470 | | |
| 129-3 | ESB2.2_HPRT1-1 -B29 | 471 | ACUUUGUUGGAUUUGAAAUUCC ; ; ; ; ; ; ; ; ; ; ; ; ; ; ; ; ; ; ; ; ; UCUGAAACAACCUAAACUUUAA | 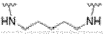 |
| | | 472 | | |
| 130 | ESB2.2-16-B30 | 473 |   GAUAAGAUGAAUCUCUUCUCC ; ; ; ; ; ; ; ; ; ; ; ; ; ; ; ; ; ; ; CUCUAUUCUACUUAGAGAAGA | 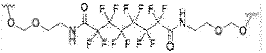 |
| | | 474 | | |
| 130-2 | ESB2.2-20-B30 | 475 | UUUGGUCGUAUUGGGCGCCUGG ;;;;;;;;;;;;;;;;;;;;;; CUAAACCAGCAUAACCCGCGGA | 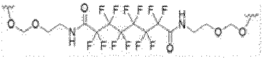 |
| | | 476 | | |
| 130-3 | ESB2.2_HPRT1-1 -B30 | 477 | A̲CUUUGUUGGAUUUGAAAUUCC ;;;;;;;;;;;;;;;;;;;; UC̲UGAAACAACCUAAACUUUAA | 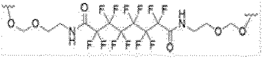 |
| | | 478 | | |
| 131 | ESB2.2-16-B31 | 478 |   GAUAAGAUGAAUCUCUUCUCC ;;;;;;;;;;;;;;;;;;; CUCUAUUCUACUUAGAGAAGA | 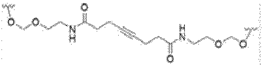 |
| | | 480 | | |
| 131-2 | ESB2.2-20-B31 | 481 | U̲UUGGUCGUAUUGGGCCGCCUGG ;;;;;;;;;;;;;;;;;;;;;; CU̲AAACCAGCAUAACCCGGCGGA | 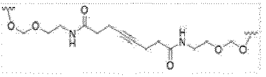 |
| | | 482 | | |
| 131-3 | ESB2.2_HPRT1-1 -B31 | 483 | ACUUUGUUGGAUUUGAAAUUCC ; ; ; ; ; ; ; ; ; ; ; ; ; ; ; ; ; ; ; ; UCUGAAACAACCUAAACUUUAA | 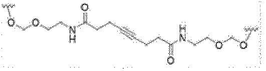 |
| | | 484 | | |
| 132 | ESB2.2-16-B32 | 485 | GAUAAGAUGAAUCUCUUCUCC ; ; ; ; ; ; ; ; ; ; ; ; ; ; ; ; ; ; ; CUCUAUUCUACUUAGAGAAGA | 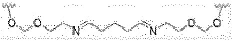 |
| | | 486 | | |
| 132-2 | ESB2.2-20-632 | 487 | UUUGGUCGUAUUGGGCGCCUGG ; ; ; ; ; ; ; ; ; ; ; ; ; ; ; ; ; ; ; ; ; CUAAACCAGCAUAACCCGCGGA | 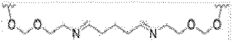 |
| | | 488 | | |
| 132-3 | ESB2.2_HPRT1-1 -832 | 489 | A̲CUUUGUUGGAUUUGAAAUUCC ; ; ; ; ; ; ; ; ; ; ; ; ; ; ; ; ; ; ; ; UC̲UGAAACAACCUAAACUUUAA | 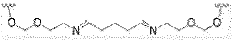 |
| | | 490 | | |

**[Table 3-18]**

| | | | | |
|---|---|---|---|---|
| 133 | ESB2.2-16-B33 | 491 |   GAUAAGAUGAAUCUCUUCUCC ; ; ; ; ; ; ; ; ; ; ; ; ; ; ; ; ; ; ; CUCUAUUCUACUUAGAGAAGA | 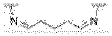 |
| | | 492 | | |
| 133-2 | ESB2.2-20-B33 | 493 | UUUGGUCGUAUUGGGCGCCUGG ; ; ; ; ; ; ; ; ; ; ; ; ; ; ; ; ; ; ; : ; CUAAACCAGCAUAACCCGCGGA | 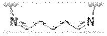 |
| | | 494 | | |
| 133-3 | ESB2.2_HPRT1-1 -B33 | 495 | ACUUUGUUGGAUUUGAAAUUCC ; ; ; ; ; ; ; ; ; ; ; ; ; ; ; ; ; ; ; ; UCUGAAACAACCUAAACUUUAA | 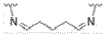 |
| | | 496 | | |
| 134 | ESB2.2-16-B34 | 497 |   GAUAAGAUGAAUCUCUUCUCC ;;;;;;;;;;;;;;;;;;; CUCUAUUCUACUUAGAGAAGA | 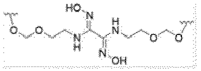 |
| | | 498 | | |
| 134-2 | ESB2.2-20-B34 | 499 | UUUGGUCGUAUUGGGCGCCUGG ;;;;;;;;;;;;;;;;;;;; CUAAACCAGCAUAACCCGCGGA | 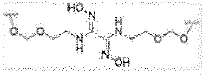 |
| | | 500 | | |
| 134-3 | ESB2.2_HPRT1-1 -B34 | 509 | ACUUUGUUGGAUUUGAAAUUCC ;;;;;;;;;;;;;;;;;;;; UCUGAAACAACCUAAACUUUAA | 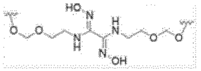 |
| | | 502 | | |
| 135 | ESB2.2-16-B35 | 503 | GAUAAGAUGAAUCUCUUCUCC ;;;;;;;;;;;;;;;;;;; CUCUAUUCUACUUAGAGAAGA | 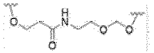 |
| | | 504 | | |
| 135-2 | ESB2.2-20-B35 | 505 | UUUGGUCGUAUUGGGCGCCUGG ; ; ; ; ; ; ; ; ; ; ; ; ; ; ; ; ; ; ; ; ; CUAAACCAGCAUAACCCGCGGA | 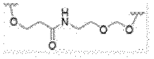 |
| | | 506 | | |
| 135-3 | ESB2.2_HPRT1-1 -B35 | 507 | ACUUUGUUGGAUUUGAAAUUCC ; ; ; ; ; ; ; ; ; ; ; ; ; ; ; ; ; ; UCUGAAACAACCUAAACUUUAA | 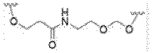 |
| | | 508 | | |
| 136 | ESB2.2-16-B36 | 509 | GAUAAGAUGAAUCUCUUCUCC ;;;;;;;;;;;;;;;;;;; CUCUAUUCUACUUAGAGAAGA | 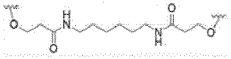 |
| | | 510 | | |
| 136-2 | ESB2.2-20-B36 | 511 | U̲UUGGUCGUAUUGGGCGCCUGG ;;;;;;;;;;;;;;;;;;;; CU̲AAACCAGCAUAACCCGCGGA | 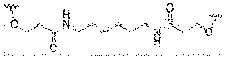 |
| | | 512 | | |
| 136-3 | ESB2.2_HPRT1-1 -B36 | 513 |   ACUUUGUUGGAUUUGAAAUUCC ;;;;;;;;;;;;;;;;;;;; UCUGAAACAACCUAAACUUUAA | 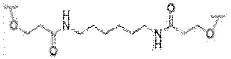 |
| | | 514 | | |
| Ref. Ex. 1 | si-PH-0153 | 1 |   GAUAAGAUGAAUCUCUUCUCC ;;;;;;;;;;;;;;;;;;; CUCUAUUCUACUUAGAGAAGA | |
| | | 2 | | |
| Ref. Ex. 2 | siGAP20-19b | 515 | UUGGUCGUAUUGGGCGCCUGG ; ; ; ; ; ; ; ; ; ; ; ; ; ; ; ; ; ; ; UAAACCAGCAUAACCCGCGGA | |
| | | 516 | | |

**[Table 3-19]**

| | | | | |
|---|---|---|---|---|
| Ref. Ex. 3 | siHPRT1-19b | 517 | CUUUGUUGGAUUUGAAAUUCC ; ; ; ; ; ; ; ; ; ; ; ; ; ; ; ; ; ; ; CUGAAACAACCUAAACUUUAA | |
| | | 518 | | |
| Ref. Ex. 4 | siRNA-6b | 519 | UUAUGGGUUGGGACUACCCUA ;;;;;;;;;;;;;;;;;;; GGAAUACCCAACCCUGAUGGG | |
| | | 520 | | |

### Experimental Example 1: Evaluation of gene expression suppressing effect

### 1-1. NEK6 gene expression level measurement method

H1299 cells were used for NEK6 gene expression measurement. A 10% FBS-containing RPMI (Invitrogen) was used as the medium. The culture conditions were set to 37°C and 5% CO₂.

First, the cells were cultured in the medium, and the cell suspension was dispensed to a 24-well plate so that each well contained 400 µL at 5 × 10⁴ cells/well. The cells in the wells were transfected with the nucleic acid molecules described in Tables 2-1 to -3 (for convenience, also to be referred to as the sugar cross-linked nucleic acid molecule of the present invention) by using a transfection reagent RNAiMAX (Invitrogen) according to the attached protocol. Specifically, the transfection was carried out by setting the composition per well as follows. In the following composition, (B) is Opti-MEM (Invitrogen) and (C) is a nucleic acid molecule solution, and they were added in a total of 100 µL. The final concentration of the nucleic acid molecule in each well was set to 0.01, 0.1, 1 nmol/L (nM), or 1 or 10 nmol/L (nM).

### (Composition per well: µL)

| | | |
|---|---|---|
| | culture medium | 400 |
| | transfection reagent | 1.0 |
| | (B)+(C) | 99.0 |
| Total | | 500 |

After the transfection, the cells were cultured for 24 hours, and RNA was collected using RNeasy Mini Kit (QIAGEN) and according to the attached protocol. Then, the expression level of the NEK6 gene and the expression level of the internal standard GAPDH gene were measured using One Step TB Green PrimeScript PLUS RT-PCR Kit (Takara) and according to the attached protocol. The expression level of NEK6 gene was normalized with reference to that of the GAPDH gene.

PCR was performed using One Step TB Green PrimeScript PLUS RT-PCR Kit (Takara) as the reagent, and Light Cycler480 Instrument II (Roche) as the instrument. NEK6 gene and GAPDH were respectively amplified using the following primer sets. primer set for NEK6 gene
5'-ggagttccaacaacctctgc-3' (SEQ ID NO: 521)
5'-gagccactgtcttcctgtcc-3' (SEQ ID NO: 522) primer set for GAPDH gene
5'-atggggaaggtgaaggtcg-3' (SEQ ID NO: 523)
5'-gggtcattgatggcaacaatatc-3' (SEQ ID NO: 524)

The expression level of the gene was also measured (mock) as a control for the cells subjected to the same transfection procedures except that the RNA solution was not added and that 1.0 µL of the transfection reagent and (B) were added to the total amount of 100 µL.

As for the normalized expression level of NEK6 gene, the relative NEK6 expression value in the cell introduced with each nucleic acid molecule was determined based on the expression level in the cells of the control (mock) as 1.

### 1-2. GAPDH gene expression level measurement method

For the GAPDH gene expression level measurement, similar to the 1-1. NEK6 gene expression level measurement, H1299 cells were transfected with the nucleic acid molecules described in Tables 3-1 to -19 (final concentration 10 nmol/L), RNA was collected, and then the expression level of the GAPDH gene and the expression level of the internal standard HPRT1 gene were measured using One Step TB Green PrimeScript PLUS RT-PCR Kit (Takara) and according to the attached protocol. The expression level of GAPDH gene was normalized with reference to that of the HPRT1 gene.

PCR was performed using One Step TB Green PrimeScript PLUS RT-PCR Kit (Takara) as the reagent, and Light Cycler480 Instrument II (Roche) as the instrument. HPRT1 gene and GAPDH were respectively amplified using the following primer sets. primer set for HPRT1 gene
5'- gaaaaggaccccacgaagtgt-3' (SEQ ID NO: 525)
5'- agtcaagggcatatcctacaaca-3' (SEQ ID NO: 526) primer set for GAPDH gene
5'-atggggaaggtgaaggtcg-3' (SEQ ID NO: 523)
5'-gggtcattgatggcaacaatatc-3' (SEQ ID NO: 524)

The expression level of the gene was also measured (mock) as a control for the cells subjected to the same transfection procedures except that the RNA solution was not added and that 1.0 µL of the transfection reagent and (B) were added to the total amount of 100 µL.

As for the normalized expression level of GAPDH gene, the relative GAPDH expression value in the cell introduced with each nucleic acid molecule was determined based on the expression level in the cells of the control (mock) as 1.

### 1-3. HPRT1 gene expression level measurement method

For the HPRT1 gene expression level measurement, similar to the 1-1. NEK6 gene expression level measurement, H1299 cells were transfected with the various nucleic acid molecules shown in Figs. 98 to 106 (final concentration 10 nmol/L), RNA was collected, and then the expression level of the HPRT1 gene and the expression level of the internal standard GAPDH gene were measured using One Step TB Green PrimeScript PLUS RT-PCR Kit (Takara) and according to the attached protocol. The expression level of HPRT1 gene was normalized with reference to that of the GAPDH gene.

PCR was performed using One Step TB Green PrimeScript PLUS RT-PCR Kit (Takara) as the reagent, and Light Cycler480 Instrument II (Roche) as the instrument. HPRT1 gene and GAPDH were respectively amplified using the following primer sets. primer set for HPRT1 gene
5'- gaaaaggaccccacgaagtgt-3' (SEQ ID NO: 525)
5'- agtcaagggcatatcctacaaca-3' (SEQ ID NO: 526) primer set for GAPDH gene
5'-atggggaaggtgaaggtcg-3' (SEQ ID NO: 523)
5'-gggtcattgatggcaacaatatc-3' (SEQ ID NO: 524)

The expression level of the gene was also measured (mock) as a control for the cells subjected to the same transfection procedures except that the RNA solution was not added and that 1.0 µL of the transfection reagent and (B) were added to the total amount of 100 µL.

As for the normalized expression level of HPRT1 gene, the relative HPRT1 expression value in the cell introduced with each nucleic acid molecule was determined based on the expression level in the cells of the control (mock) as 1.

### 1-4. Results

The gene expression level measurement results are shown in Fig. 98 to Fig. 106.

Figs. 98 to 101 are graphs showing the NEK6 gene relative expression level when transfected with a nucleic acid molecule targeting the NEK6 gene.

Fig. 98 shows the transfection results at a final concentration of 0.01, 0.1 or 1 nmol/L nucleic acid molecule, Fig. 99 shows the transfection results at a final concentration of 1 nmol/L nucleic acid molecule, and Figs. 100 and 101 show the transfection results at a final concentration of 10 nmol/L nucleic acid molecule.

Figs. 102 to 105 are graphs showing the GAPDH gene relative expression level when transfected with a nucleic acid molecule (final concentration 10 nmol/L) targeting the GAPDH gene.

Fig. 106 is a graph showing the HPRT1 gene relative expression level when transfected with a nucleic acid molecule (final concentration of 10 nmol/L) targeting the HPRT1 gene.

It was shown that many of the sugar cross-linked nucleic acid molecules of the present invention have an expression suppressing effect regardless of the target gene, antisense strand sequence, position of crosslinked nucleotide, chain length, terminal structure, and crosslinked structure.

### Experimental Example 2: Evaluation of off-target effect using reporter assay system

A reporter plasmid was produced and the off-target effect caused by the sense strand was confirmed as described below.

### (1) Production of reporter plasmid

The reporter plasmid to be used in the following reporter assay was produced. The production was outsourced to GENEWIZ.

Based on the sequence information of the complementary sequence (sense strand) of the loaded expression suppressing sequence (antisense strand), the following two artificial DNAs (hereinafter referred to as synthetic fragments) were chemically synthesized. A restriction enzyme recognition sequence (XhoI; CTCGAG) was added to the 5'-terminal of the synthetic fragment, and a restriction enzyme recognition sequence (Not I; GCGGCCGC) was added to the 3'-terminal. Reporter plasmids, Pass10 and GAPDH20-HPRT1, were produced by incorporating the above-mentioned synthetic fragment into the restriction enzyme recognition sites XhoI and NotI of the psiCHECK-2 vector (Promega Corporation, GenBank accession number AY535007), which is an expression vector of Renilla Luciferase (hereinafter hRluc) and firefly luciferase (hereinafter hluc+), according to a conventional method. Pass10 is a reporter plasmid that measures the activity of the sense strand of nucleic acid molecules targeting NEK6, and GAPDH20-HPRT1 is a reporter plasmid that measures the activity of the sense strand of nucleic acid molecules targeting GAPDH and HPRT1. In cultured cells, the fusion mRNA (target mRNA) of the hRluc gene and the synthetic fragment, and hluc+ (mRNA for normalization) are expressed from the above-mentioned reporter plasmids.
Pass10(SEQ ID NO: 527) 
5’-

GGAGAAGAGATTCATCTTATCTCTATATGCTGTGTGTACTCTGCTTTCTAGCACCATTTGAAA

TCAGTGTTAGTGGCAGTGTCTTAGCTGGTTGTCGCGTACCAAAAGTAATAATGCGCAAATCCG

AGTCCACGTGTGGAAATAAACCGCCAGCATTCAAGGAGAGAACGGCTAAGAAACTTGCCCAAG

AAAACGACTCAAGTAAGGCAAGCTACCGTGGCTCGCTGAACCAC-3’
GAPDH20-HPRT1(SEQ ID NO: 528)
   5'-GACCAGGCGCCCAATACGACCAAATCCTGGAATTTCAAATCCAACAAAGTCT-3'

The underlined portion in the above-mentioned sequences indicates the target sequence of the sense strand of the below-mentioned nucleic acid molecule.

### (2) Measurement of hRluc gene expression suppressing effect in reporter assay system

Reporter plasmid and nucleic acid molecules were transfected with cultured cells, and the hRluc gene expression suppressing effect was confirmed.

### (a) Material and method

Each nucleic acid molecule solution was prepared using distilled water for injection (Otsuka Pharmaceutical Co., Ltd.). Reporter plasmid solution was prepared using TE buffer (10 mM Tris-HCl, 1 mM EDTA, pH 8.0).

HCT116 cells (DS Pharma Biomedical Co., Ltd.) were used. A 10% FBS-containing DMEM (GIBCO) was used as the medium, and the cells were cultured under the conditions of 37°C, 5% CO₂.

First, cells were seeded in a 10-cm dish at 2×10⁶ cells. After further culturing the cells for 24 hr, 3 µg of the reporter plasmid was transfected using a transfection reagent Lipofectamine 2000 (Invitrogen) according to the protocol attached to the transfection reagent. Specifically, the transfection was carried out by setting the composition per well as follows. In the following composition, (B) is Opti-MEM (Invitrogen) and (C) is a 100 ng/µL reporter plasmid solution, and they were added in a total of 1.955 mL.

### (Composition per well: mL)

| | | |
|---|---|---|
| | culture medium | 8 |
| | Lipofectamine 2000 | 0.045 |
| | (B)+(C) | 1.955 |
| Total | | 10 |

After 24 hr, the cells were collected, the number of cells was counted, and a cell suspension was prepared at 2×10⁵ cells/mL using a medium. The cells were transfected with nucleic acid molecules in a 96-well plate (B&W IsoPlate-96 TC, PerkinElmer) using the transfection reagent Lipofectamine RNAiMAX (Invitrogen) according to the protocol attached to the transfection reagent. Specifically, the transfection was carried out by setting the composition per well as follows. In the following composition, (B) is Opti-MEM (Invitrogen) and (C) is a nucleic acid molecule solution, and they were added in a total of 14.8 µL. The final concentration of the nucleic acid molecule was set to 1 nmol/L or 10 nmol/L. As a control (reference) in the transfection, a well not containing (C) and containing 14.8 µL of (B) alone was set (mock).

### (Composition per well: µL)

| | | |
|---|---|---|
| | cell suspension | 60 |
| | (A) Lipofectamine RNAiMAX | 0.2 |
| | (B)+(C) | 14.8 |
| Total | | 75 |

After culturing for 24 hr more, the luminescence of hRluc and hluc+ was measured using the Dual-Glo Luciferase Assay System (Promega Corporation) according to the attached protocol. The hRluc/hluc+ ratio of each well was obtained from each measured value. Furthermore, the hRluc relative expression level of each well was calculated with the hRluc/hluc+ ratio of the control (reference) as 1.

### (b) Results

The gene expression level measurement results are shown in Fig. 107 to Fig. 112.

Figs. 107 to 109 are graphs showing the hRluc gene relative expression level when cells transfected with a reporter plasmid that measures the activity of the sense strand of a nucleic acid molecule targeting NEK6 are transfected with a nucleic acid molecule targeting the NEK6 gene.

Fig. 107 shows the transfection results at a final concentration of 1 nmol/L nucleic acid molecule, and Figs. 108 and 109 show the transfection results at a final concentration of 10 nmol/L nucleic acid molecule.

Figs. 110 to 111 are graphs showing the hRluc gene relative expression level when cells transfected with a reporter plasmid that measures the activity of the sense strand of a nucleic acid molecule targeting GAPDH are transfected with a nucleic acid molecule (final concentration 10 nmol/L) targeting the GAPDH gene.

Fig. 112 is a graph showing the hRluc gene relative expression level when cells transfected with a reporter plasmid that measures the activity of the sense strand of a nucleic acid molecule targeting HPRT1 are transfected with a nucleic acid molecule (final concentration 10 nmol/L) targeting the HPRT1 gene.

It was shown that the sugar cross-linked nucleic acid molecules of the present invention strongly suppress the off-target effect caused by the sense strand as compared with the siRNA of Reference Example, regardless of the target gene, antisense strand sequence, position of crosslinked nucleotide, chain length, terminal structure, and crosslinked structure.

### Experimental Example 3: Application to coronavirus

As an application example of the sugar cross-linked nucleic acid molecule, sugar cross-linked nucleic acid molecules targeting coronavirus were produced and the gene expression suppressing effects thereof in the reporter assay system were confirmed.

The sequence highly conserved among α coronavirus genus SC2013 (GenBank accession number NC_028833), α coronavirus genus SAX2011 (GenBank accession number NC_028811), β coronavirus genus MERS (GenBank accession number NC_019843), β coronavirus genus SARS (GenBank accession number NC_004718), and β coronavirus genus HKU3 (GenBank accession number DQ022305) was selected as the target sequence, and an siRNA as Reference Example and a sugar cross-linked nucleic acid molecule of the present invention were produced (Tables 3-1 to -19, 6b1, 6bl-4).

### (1) Production of reporter plasmid

The reporter plasmid to be used in the following reporter assay was produced. The production was outsourced to GENEWIZ.

Similar to Experimental Example 2, five artificial DNAs (hereinafter referred to as synthetic fragments) containing target sequences derived from each coronavirus were chemically synthesized based on the genomic sequence information of coronavirus. A restriction enzyme recognition sequence (XhoI; CTCGAG) was added to the 5'-terminal of the synthetic fragment shown below, and a restriction enzyme recognition sequence (Not I; GCGGCCGC) was added to the 3'-terminal thereof. Reporter plasmids, SC2013, SAX2011, MERS, SARS, and HKU3, were produced by incorporating the above-mentioned synthetic fragment into the restriction enzyme recognition sites XhoI and NotI of the psiCHECK-2 vector (Promega Corporation, GenBank accession number AY535007), which is an expression vector of Renilla Luciferase (hereinafter hRluc) and firefly luciferase (hereinafter hluc+), according to a conventional method.
SC2013(SEQ ID NO: 529) 
5’-
gttgacaacccttgtcttatgggttgggactaccctaagtgtgatagagcaatggaagggttg
tgcctattctggaactgtaatgttgatatgtatcctg -3
SAX2011(SEQ ID NO: 530) 
5’-
gttgaaaatccacaccttatgggttgggactatcctaagtgtgaccgtgcaatgaatggtttg
tgcctattctggaactgtaacgttgatatgtaccctg -3’
MERS(SEQ ID NO: 531) 
5’-
gttgataatccgcatcttatgggttgggattaccctaagtgtgatagagctgctgatgggctc
 tgcttattttggaactgtaatgtaccaaaatatccta -3'
SARS(SEQ ID NO: 532) 
5'-
gtagaaactccacaccttatgggttgggattatccaaaatgtgacagagccactgatggtgtt
tgtttgttttggaattgtaacgttgatcgttacccag -3'
HKU3(SEQ ID NO: 533)

5'-
gtagaaagtcctcacctcatgggttggactacccaaaatgtgacagagctacagatggcgtg
tgtttgttttggaactgtaacgtggatcgttacccctt -3'

The underlined portion in the above-mentioned sequences indicates the target sequence of the antisense strand of the nucleic acid molecules.

### (2) Gene expression level measurement using reporter assay system

The measurement of the gene expression level was performed in the same manner as in Experimental Example 2 except that the above-mentioned five reporter plasmids were used, the final concentration of the siRNA as Reference Example was 100 nmol/L, and the final concentration of the nucleic acid molecule of the present invention was 20 nmol/L.

### (3) Results

The measurement results are shown in Fig. 113. While the siRNA as Reference Example did not exhibit gene expression suppressing effects on all coronaviruses, the sugar cross-linked nucleic acid molecule of the present invention was found to exhibit gene expression-suppressing effects on all coronaviruses. That is, it was shown that even a sequence that does not have an expression suppressing effect as an siRNA exhibits an expression suppressing effect by being incorporated into the sugar cross-linked nucleic acid molecule of the present invention.

From the above results, since the nucleic acid molecule of the present invention (1) has a gene expression suppressing activity equivalent to or higher than that of siRNA, and (2) shows a sufficiently attenuated off-target effect of the sense strand, it can be used more safely than conventional siRNA. In addition, since at least the off-target effect of the sense strand can be ignored, (3) it is possible to design a wider range of antisense strand sequences than conventional siRNA.

### [Industrial Applicability]

Since the nucleic acid molecule of the present invention has the superior properties of the above-mentioned (1) to (3), it is extremely useful as a novel gene expression inhibitor that replaces conventional siRNA.

This application is based on a patent application No. 2020-142170 filed in Japan (filing date: August 25, 2020), the contents of which are incorporated in full herein by reference.

## Claims

1. A nucleic acid molecule represented by the following formula: 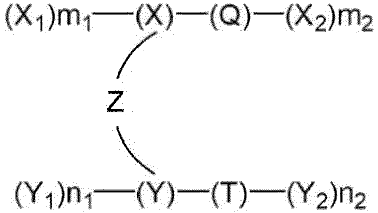
wherein X, Y, X₁, Y₁, X₂, and Y₂ are each independently an optionally modified ribonucleotide residue or an optionally modified deoxyribonucleotide residue,
T and Q are a sequence consisting of 14 to 30 consecutive, optionally-modified ribonucleotide residues and complementary to the target nucleic acid sequence, and a ribonucleotide sequence complementary thereto (one of which is a sequence complementary to the target nucleic acid sequence, and the other is a sequence complementary thereto),
Z is a linker connecting the 2'-position of the sugar moiety of (X) and the 2'-position of the sugar moiety of (Y);
m₁ and m₂ are each independently an integer of 0 to 5; and
n₁ and n₂ are each independently an integer of 0 to 5.

2. The nucleic acid molecule according to claim 1, wherein the linker Z is a non-nucleotide structure having an alkyl chain with an amide bond therein.

3. The nucleic acid molecule according to claim 1 or 2, wherein the following structure 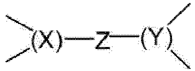 containing the linker Z is 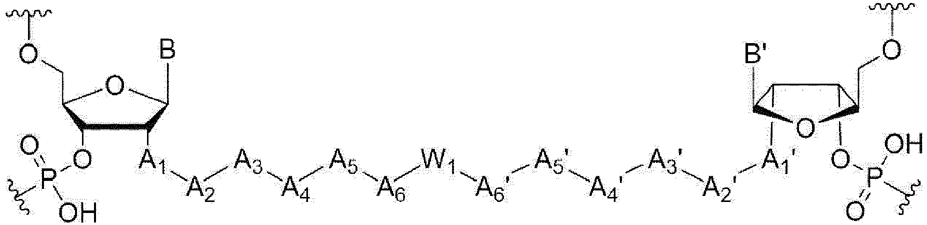
wherein B and B' are each independently an atomic group having a nucleic acid base backbone,
A₁ and A₁' are each independently -O-, -NR^{1a}-, -S- or -CR^{1a}R^{1b}-(wherein R^{1a} and R^{1b} are each independently a hydrogen atom or an alkyl group having 1 - 10 carbon atoms),
A₂ and A₂' are each independently -CR^{2a}R^{2b}-, -CO-, an alkynyl group, an alkenyl group, or a single bond (wherein R^{2a} and R^{2b} are each independently a hydrogen atom or an alkyl group having 1 - 10 carbon atoms),
A₃ and A₃' are each independently -O- or -NR^{3a}-, -S-, -CR^{3a}R^{3b}- or a single bond (wherein R^{3a} and R^{3b} are each independently a hydrogen atom or an alkyl group having 1 - 10 carbon atoms),
A₄ and A₄' are each independently - (CR^{4a}R^{4b})n-, -(CR^{4a}R^{4b})n-ring D- (wherein ring D is an aryl group having 6 - 10 carbon atoms, a heteroaryl group having 2 - 10 carbon atoms, a cycloalkyl group having 4 - 10 carbon atoms, or a heterocycloalkyl group having 4 - 10 carbon atoms, R^{4a} and R^{4b} are each independently a hydrogen atom or an alkyl group having 1 - 10 carbon atoms) or a single bond,
A₅ and A₅' are each independently -NR^{5a}- or a single bond (wherein R^{5a} is a hydrogen atom or an alkyl group having 1 - 10 carbon atoms),
A₆ and A₆' are each independently -(CR^{6a}R^{6b})n- or a single bond (wherein R^{6a} and R^{6b} are each independently a hydrogen atom or an alkyl group having 1 - 10 carbon atoms), and
W₁ is -(CR¹R²)n-, -CO-, -(CR¹R²)n-COO-(CR¹R²)n-COO-(CR¹R²)n, - (CR¹R²)n-O- (CR¹R²CR¹R²O) n-CH₂-, - (CR¹R²)n- ring D- (CR¹R²)n-, or - (CR¹R²)n-SS-(CR¹R²)n- (wherein ring D is an aryl group having 6 - 10 carbon atoms, a heteroaryl group having 2 - 10 carbon atoms, a cycloalkyl group having 4 - 10 carbon atoms, or a heterocycloalkyl group having 4 - 10 carbon atoms; R¹ and R², R^{1a} and R^{2a}, and R^{1b} and R^{2b} are each independently a hydrogen atom or an alkyl group having 1 - 10 carbon atoms).

4. The nucleic acid molecule according to claim 1 or 2, wherein the following structure 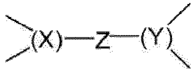 containing the linker Z is 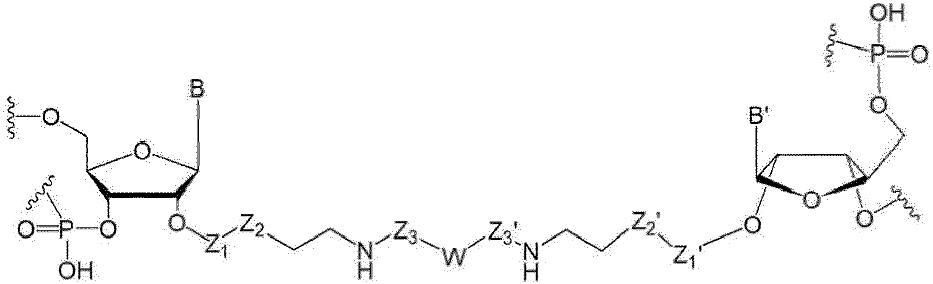
wherein B and B' are each independently an atomic group having a nucleic acid base backbone,
Z₁ and Z₁' are each independently -CH₂- or -CO-,
Z₂ and Z₂' are each independently -O- or -NH-,
Z₃ and Z₃' are each -CO-, -CH₂-, or a single bond (absent), and
W is -CR¹⁰R²⁰- or -CR^{10a}R^{20a}-N(R³⁰)-CR^{10b}R^{20b}- (wherein R¹⁰ and R²⁰,
R^{10a} and R^{20a}, and R^{10b} and R^{20b} are each independently a hydrogen atom or an alkyl group having 1 - 10 carbon atoms, R³⁰ is -COR⁴⁰-, R⁴⁰ is a hydrogen atom, an optionally substituted alkyl group having 1 - 20 carbon atoms, or an aryl group having 6 - 10 carbon atoms).

5. The nucleic acid molecule according to any one of claims 1 to 4, comprising at least one modified nucleotide.

6. The nucleic acid molecule according to any one of claims 1 to 5, wherein the aforementioned modified nucleotide is selected from the group consisting of 2'-O-methyl-modified nucleotide, nucleotide containing a 5'-phosphorothioate group, deoxy-nucleotide, 3'-terminal deoxy-thymine (dT) nucleotide, 2'-O-methyl-modified nucleotide, 2'-fluoro-modified nucleotide, 2'-deoxy-modified nucleotide, terminal nucleotide bound with a cholesteryl derivative or a dodecanoic acid bisdecylamide group, 2'-deoxy-2'-fluoro-modified nucleotide, fixed nucleotide, non-fixed nucleotide, conformationally restricted nucleotide, constrained ethyl nucleotide, abasic nucleotide, 2'-amino-modified nucleotide, 2'-O-allyl-modified nucleotide, 2'-C-alkyl-modified nucleotide, 2'-hydroxyl-modified nucleotide, 2'-methoxyethyl-modified nucleotide, 2'-O-alkyl-modified nucleotide, morpholino nucleotide, phosphoramidate, nucleotide containing non-natural base, tetrahydropyran-modified nucleotide, 1,5-anhydrohexitol-modified nucleotide, cyclohexenyl-modified nucleotide, nucleotide containing a phosphorothioate group, nucleotide containing a methylphosphonate group, nucleotide containing 5'-phosphate, and nucleotide containing a 5'-phosphate mimic.

7. A medicament comprising the nucleic acid molecule according to any one of claims 1 to 6.

8. A target gene expression inhibitor comprising the nucleic acid molecule according to any one of claims 1 to 6, wherein the target gene comprises the aforementioned target nucleic acid sequence.

9. A therapeutic agent for cancer or fibrosis comprising the nucleic acid molecule according to claim 8.

10. A method for suppressing expression of a target gene, comprising contacting an effective amount of the nucleic acid molecule according to any one of claims 1 to 6 with the target gene.

11. A method for treating cancer or fibrosis, comprising contacting an effective amount of the nucleic acid molecule according to any one of claims 1 to 6 with a target gene.

12. The method according to claim 10 or 11, comprising a step of administering the aforementioned nucleic acid molecule to a cell, a tissue, or an organ.

13. The method according to any one of claims 10 to 12, wherein the aforementioned nucleic acid molecule is administered in vivo or in vitro.

14. The method according to any one of claims 10 to 13, wherein the aforementioned nucleic acid molecule is administered to a non-human animal.

15. An amidite compound having the following structure 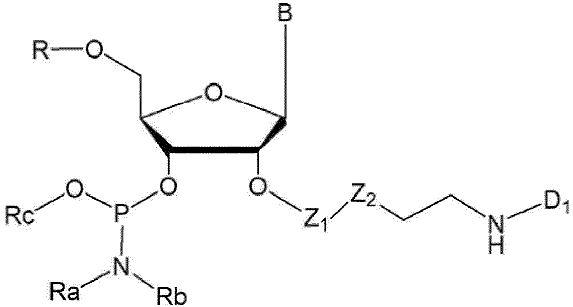
wherein B is an atomic group having a nucleic acid base backbone,
Z₁ is -CH₂- or -CO-,
Z₂ is -O- or -NH-,
R is a hydroxyl-protecting group,
Rₐ and R_{b} are the same or different and each a hydrogen atom or
a substituent; R_{c} is a hydrogen atom, an electron-withdrawing group, or a substituent optionally substituted by an electron-withdrawing group, and
D₁ is an amino-protecting group.

16. The amidite compound according to claim 15, wherein Z₁ is -CH₂- and Z₂ is -O-.

17. The amidite compound according to claim 15, wherein Z₁ is -CO- and Z₂ is -NH-.
